# EUROPEAN PATENT APPLICATION

(11) **EP 4 684 799 A2**
(43) Date of publication of application: **28.01.2026**
(21) Application number: 25217775.3
(22) Date of filing: 03.12.2020
(51) Int. Cl.: A61K 39/395

(54) **ANTIBODIES THAT DISRUPT THE INTERACTION OF GAL3 AND INSULIN RECEPTOR OR INTEGRINS AND METHODS OF USE THEREOF**

(30) Priority: 06.12.2019 US 201962944833 P
(62) Divisional of application: 20896254.8
(71) Applicant: TrueBinding, Inc., Foster City, CA 94404 (US)
(72) Inventor: SUN, Dongxu, FOSTER CITY 94404 (US); ZHANG, Jing, FOSTER CITY 94404 (US); BONACORSI, Maja, FOSTER CITY 94404 (US); WU, Yinan, FOSTER CITY 94404 (US); YU, Yadong, FOSTER CITY 94404 (US); GORDON, Catherine A., FOSTER CITY 94404 (US); TSAI, Tsung-Huang, FOSTER CITY 94404 (US); SHCHORS, Ksenya, FOSTER CITY 94404 (US); WILLIAMS, Samuel A.F., FOSTER CITY 94404 (US)
(74) Representative: Ipsilon

(57) **Abstract**

Disclosed herein are methods and compositions for disrupting an interaction between Galectin-3 and insulin receptor or integrins. Further disclosed herein are methods and compositions for the treatment of a disease or a disorder in a subject, such as the treatment of diabetes mellitus, inflammatory bowel syndrome, non-alcoholic fatty liver disease, and non-alcoholic steatohepatitis.

## Description

### CROSS-REFERENCE TO RELATED APPLICATIONS

This application claims the benefit of priority of U.S. Provisional Patent Application No. 62/944,833, filed December 6, 2019, which is hereby expressly incorporated by reference in its entirety.

### REFERENCE TO SEQUENCE LISTING

The present application is being filed along with a Sequence Listing in electronic format. The Sequence Listing is provided as a file entitled SeqListingIMMUT006WO.TXT, which was created and last modified on December 2, 2020, which is 405,344 bytes in size. The information in the electronic Sequence Listing is hereby incorporated by reference in its entirety.

### FIELD

Aspects of the present disclosure relate generally to antibodies or binding fragments thereof that block or disrupt the interaction between Galectin-3 (Gal3) and insulin receptor (INSR) or integrins (ITG). Further disclosed herein are methods and compositions for the treatment of diseases or disorders, such as (but not limited to) diabetes mellitus, inflammatory bowel syndrome, non-alcoholic fatty liver disease, and non-alcoholic steatohepatitis, which can be associated with INSR and/or ITG dysfunction.

### BACKGROUND

Galectin-3 (Gal3, GAL3) is a lectin, or a carbohydrate-binding protein, with specificity towards beta-galactosides. In human cells, Gal3 is expressed and can be found in the nucleus, cytoplasm, cell surface, and in the extracellular space. Gal3 recognizes and interacts with beta-galactose conjugates on various proteins.

### SUMMARY

Disclosed herein are methods of disrupting an interaction between galectin-3 (Gal3) and a) insulin receptor (INSR) or b) an integrin, or both a) and b). In some embodiments, the methods may comprise contacting an interaction between Gal3 and the insulin receptor or the integrin, or both, with an anti-Gal3 antibody or binding fragment thereof that selectively binds to Gal3 and disrupts the interaction between Gal3 and the insulin receptor or the integrin, or both.

Also disclosed herein are methods of treating diabetes mellitus in a subject in need thereof. In some embodiments, the methods comprise administering to the subject an anti-Gal3 antibody or binding fragment thereof that selectively binds to Gal3 and disrupts an interaction between Gal3 and insulin receptor or an integrin, or both, thereby treating diabetes mellitus in the subject.

Also disclosed herein are methods of treating inflammatory bowel syndrome in a subject in need thereof. In some embodiments, the methods comprise administering to the subject an anti-Gal3 antibody or binding fragment thereof that selectively binds to Gal3 and disrupts an interaction between Gal3 and an integrin, thereby treating inflammatory bowel syndrome in the subject.

Also disclosed herein are methods of treating non-alcoholic fatty liver disease (NAFLD) in a subject in need there. In some embodiments, the methods comprise administering to the subject an anti-Gal3 antibody or binding fragment thereof that selectively binds to Gal3 and disrupts an interaction between Gal3 and insulin receptor, thereby treating NAFLD in the subject.

Also disclosed herein are methods of treating non-alcoholic steatohepatitis (NASH) in a subject in need thereof. In some embodiments, the methods comprise administering to the subject an anti-Gal3 antibody or binding fragment thereof that selectively binds to Gal3 and disrupts an interaction between Gal3 and insulin receptor, thereby treating NASH in the subject.

Also disclosed herein are anti-Gal3 antibodies comprising 1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein the V_{L}-CDR1 comprises an amino acid sequence having at least 60%, at least 70%, at least 80%, at least 90%, or 100% sequence identity to any amino acid sequence according to **SEQ ID NOs: 100-128,** the V_{L}-CDR2 comprises an amino acid sequence having at least 60%, at least 70%, at least 80%, at least 90%, or 100% sequence identity to any amino acid sequence according to **SEQ ID NOs: 129-144,the** V_{L}-CDR3 comprises an amino acid sequence having at least 60%, at least 70%, at least 80%, at least 90%, or 100% sequence identity to any amino acid sequence according to **SEQ ID NOs: 145-168,** the V_{H}-CDR1 comprises an amino acid sequence having at least 60%, at least 70%, at least 80%, at least 90%, or 100% sequence identity to any amino acid sequence according to **SEQ ID NOs: 27-47,**the V_{H}-CDR2 comprises an amino acid sequence having at least 60%, at least 70%, at least 80%, at least 90%, or 100% sequence identity to any amino acid sequence according to **SEQ ID NOs: 48-69,** and the V_{H}-CDR3 comprises an amino acid sequence having at least 60%, at least 70%, at least 80%, at least 90%, or 100% sequence identity to any amino acid sequence according to **SEQ ID NOs: 70-99.**

Also disclosed herein are pharmaceutical compositions comprising any one or more of the anti-Gal3 antibodies or binding fragments thereof disclosed herein. These pharmaceutical compositions may be used for the treatment of diabetes mellitus, inflammatory bowel disease, NAFLD, or NASH, or any combination thereof.

Also disclosed herein are antibodies that bind to human Gal3 and competes with any one of the anti-Gal3 antibodies or binding fragments disclosed herein for binding to human Gal3. In some embodiments, the antibodies that bind to human Gal3 may compete with an anti-Gal3 antibody or binding fragment thereof that binds to a certain peptide of Gal3. In some embodiments, the antibodies that bind to human Gal3 may compete with an anti-Gal3 antibody or binding fragment thereof that belongs to a certain bin. In some embodiments, the antibodies that bind to human Gal3 may compete with 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, 7D8.2D8, and 15F10.2D6, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, F847.14H4 (847.14H4), mIMT001, IMT001(TB001), or IMT006 (TB006).

Also disclosed herein are methods for identifying an antibody capable of disrupting an interaction between Gal3 and insulin receptor or an integrin. In some embodiments, the methods comprise (a) contacting Gal3 protein with an antibody or binding fragment thereof that selectively binds to Gal3, thereby forming a Gal3-antibody complex; (b) contacting the Gal3-antibody complex with the insulin receptor or the integrin, or both; (c) removing unbound insulin receptor or integrin, or both; and(d) detecting the insulin receptor or integrin, or both, bound to the Gal3-antibody complex. In some embodiments, the antibody or binding fragment thereof is capable of disrupting an interaction of Gal3 and insulin receptor or the integrin, or both, when the insulin receptor or the integrin, or both, is not detected in (d).

Also disclosed herein are methods of producing an anti-Gal3 antibody or binding fragment thereof. In some embodiments, the methods comprise expressing a nucleic acid that encodes for the anti-Gal3 antibody or binding fragment thereof in a cell and isolating the expressed anti-Gal3 antibody or binding fragment thereof from the cell. In some embodiments, the methods may further comprise concentrating the anti-Gal3 antibody or binding fragment thereof to a desired concentration. In some embodiments, the cell is a mammalian cell, insect cell, or bacterial cell. In some embodiments, the anti-Gal3 antibody or binding fragment is any one of the anti-Gal3 antibodies or binding fragments thereof disclosed herein.

### BRIEF DESCRIPTION OF THE DRAWINGS

Various aspects of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
**FIG. 1** demonstrates the ability of galectin-3 targeted antibodies to block the binding of Gal3 and Insulin Receptor (INSR) as measured by enzyme-linked immunosorbent assay (ELISA) at 10, 3, and 1 µg/mL. Bars represent mean +/- standard deviation.
**FIG. 2** depicts titrations of a limited series of galectin-3 targeted antibodies to block Gal3 and Insulin Receptor (INSR) binding as measured by enzyme-linked immunosorbent assay (ELISA). Bars represent mean +/- standard deviation.
**FIG. 3A** depicts a summary of properties for exemplary anti-Gal3 antibodies.
**FIG. 3B** depicts the identification of Gal3-binding antibody bins by antibody competition. Values represent inhibition as assessed by biolayer interferometry.
**FIG. 4** demonstrates the reduction of weight gain in mice fed with a normal diet or with 60% high fat diet (HFD) for 8 weeks and dosed with Isotype control antibody HuIgG4, or with Gal3-targeted IMT001-4 (TB001). Left panel depicts mean absolute values, and right panel depicts mean percent change per animal +/- standard error.
**FIG. 5** depicts glucose tolerance in mice treated as in **FIG. 4****.** Left panel depicts serum mean glucose levels after glucose bolus; right panel depicts mean area under curve (AUC) from data as represented in the left panel +/1 standard error.
**FIG. 6** depicts insulin resistance in mice as treated in **FIG. 4****.** Left panel depicts serum mean glucose levels after insulin bolus; right panel depicts mean area over curve (AOC) from data as represented in the left panel +/1 standard error.
**FIG. 7** depicts hematoxylin and eosin staining of formalin fixed paraffin embedded liver sections from mice as treated in **FIG. 4****.** Note the evidence of steatosis in HFD-fed mice treated with control IgG4 and absence thereof in those treated with IMT001-4 (TB001).
**FIG. 8** depicts serum liver enzyme ALT levels in mice treated as in **FIG. 4****.** Bars represent mean +/- standard error.
**FIG. 9** depicts an assessment of relative binding affinity of integrin beta-1 (ITGb1) to Gal3 as measured by ELISA.
**FIG. 10** depicts an assessment of relative binding affinity of ITGb1 and Gal3 following blockade by anti-Gal3 antibodies as measured by ELISA.
**FIG. 11** depicts an assessment of relative binding affinity of integrin alpha-3 (ITGa3) to Gal3 as measured by ELISA.
**FIG. 12** depicts an assessment of relative binding affinity of ITGa3 and Gal3 following blockade by anti-Gal3 antibodies as measured by ELISA.
**FIG. 13** depicts an assessment of relative binding affinity of integrin beta-3 (ITGb3) to Gal3 as measured by ELISA.
**FIG. 14** depicts an assessment of relative binding affinity of ITGb3 and Gal3 following blockade by anti-Gal3 antibodies as measured by ELISA.
**FIG. 15** depicts an assessment of relative binding affinity of integrin alpha-V (ITGaV) to Gal3 as measured by ELISA.
**FIG. 16** depicts an assessment of relative binding affinity of ITGaV and Gal3 following blockade by anti-Gal3 antibodies as measured by ELISA.
**FIG. 17** depicts an assessment of relative inhibition of Jurkat T cell adhesion following the treatment with anti-Gal3 antibodies or isotype control.
**FIG. 18** depicts the amounts of circulating Gal3 in Bks-Db or C57BL6/J control mice.
**FIG. 19** depicts a Kaplan-Meier curve of healthy C57BL6/J mice and Db/Db mice treated with the anti-Gal3 antibody mTB001, a PBS negative control, or a semaglutide positive control.
**FIG. 20** depicts the change in levels of fasting blood glucose of healthy C57BL6/J mice or Db/Db mice treated with either mTB001 or PBS.
**FIG. 21** depicts the mean survival of NOD/ShiLtJ mice treated with mTB001 compared to untreated control.
**FIG. 22A** depicts the change in levels of fasting blood glucose of NOD/ShiLtJ mice treated with mTB001 compared to untreated control.
**FIG. 22B** depicts the circulating levels of C-peptide in NOD/ShiLtJ mice treated with mTB001 compared to untreated control, and in normal control mice.
**FIG. 23** depicts primers used for RT-qPCR for quantifying inflammatory cytokines in an inflammatory bowel disease (IBD) mouse model.
**FIG. 24** depicts the measured colon length in DSS-induced IBD mice treated with mTB001 or PBS compared to normal mice.
**FIG. 25** depicts the quantification of circulating IFN-γ in DSS-induced IBD mice treated with mTB001 (10mg/kg and 1mg/kg) or PBS compared to normal mice.
**FIG. 26** depicts protein sequences of Gal3, insulin receptor (INSR), integrin beta-1 (ITGb1), integrin alpha-3 (ITGa3), integrin beta-3 (ITGb3), and integrin alpha-V (ITGaV).
**FIG. 27** depicts peptide sequences of Gal3 used to generate and analyze antibodies.
**FIG. 28A** depicts exemplary variable heavy chain complementarity-determining region (CDR) 1 for anti-Gal3 antibodies disclosed herein. In some embodiments, any of the compositions or methods provided herein can include one or more of the HCDR1 provided herein.
**FIG. 28B** depicts exemplary variable heavy chain CDR2 for anti-Gal3 antibodies disclosed herein. In some embodiments, any of the compositions or methods provided herein can include one or more of the HCDR2 provided herein.
**FIG. 28C** depicts exemplary variable heavy chain CDR3 for anti-Gal3 antibodies disclosed herein. In some embodiments, any of the compositions or methods provided herein can include one or more of the HCDR3 provided herein.
**FIG. 29A** depicts exemplary variable light chain CDR1 for anti-Gal3 antibodies disclosed herein. In some embodiments, any of the compositions or methods provided herein can include one or more of the LCDR1 provided herein.
**FIG. 29B** depicts exemplary variable light chain CDR2 for anti-Gal3 antibodies disclosed herein. In some embodiments, any of the compositions or methods provided herein can include one or more of the LCDR2 provided herein.
**FIG. 29C** depicts exemplary variable light chain CDR3 for anti-Gal3 antibodies disclosed herein. In some embodiments, any of the compositions or methods provided herein can include one or more of the LCDR3 provided herein.
**FIG. 30** depicts exemplary heavy chain variable region (VH) sequences for anti-Gal3 antibodies disclosed herein. In some embodiments, any of the compositions or methods provided herein can include one or more of the VH sequences provided herein.
**FIG. 31** depicts exemplary light chain variable region (VL) sequences for anti-Gal3 antibodies disclosed herein. In some embodiments, any of the compositions or methods provided herein can include one or more of the VL sequences provided herein.
**FIG. 32** depicts exemplary heavy chain (HC) sequences for anti-Gal3 antibodies disclosed herein. In some embodiments, any of the compositions or methods provided herein can include one or more of the HC sequences provided herein.
**FIG. 33** depicts exemplary light chain (LC) sequences for anti-Gal3 antibodies disclosed herein. In some embodiments, any of the compositions or methods provided herein can include one or more of the LC sequences provided herein.
**FIG. 34** depicts exemplary combinations of variable heavy chain CDR1, CDR2, and CDR3 of anti-Gal3 antibodies disclosed herein. In some embodiments, any of the compositions or methods provided herein can include one or more of the heavy chain CDR combinations provided herein.
**FIG. 35** depicts exemplary combinations of variable light chain CDR1, CDR2, and CDR3 of anti-Gal3 antibodies disclosed herein. In some embodiments, any of the compositions or methods provided herein can include one or more of the light chain CDR combinations provided herein.
**FIG. 36** depicts exemplary combinations of heavy and light chain CDRs of anti-Gal3 antibodies disclosed herein. In some embodiments, any of the compositions or methods provided herein can include one or more of the heavy and light chain CDR combinations provided herein.
**FIG. 37** depicts exemplary combinations of heavy and light chain variable regions of anti-Gal3 antibodies disclosed herein. In some embodiments, any of the compositions or methods provided herein can include one or more of the heavy and light chain variable region combinations provided herein.
**FIG. 38** depicts exemplary combinations of heavy and light chains of anti-Gal3 antibodies disclosed herein. In some embodiments, any of the compositions or methods provided herein can include one or more of the heavy and light chain combinations provided herein.
**FIG. 39** depicts alignments of some embodiments of the VH CDR or VL CDR regions of various embodiments of anti-Gal3 antibodies. In some embodiments, any of the methods or compositions provided herein can use any 1, 2, 3, 4, 5, 6, or 7 of the consensus CDRs provided herein.
**FIG. 40** depicts peptides that were found to bind to exemplary anti-Gal3 antibodies disclosed herein (according to the peptide nomenclature depicted in **FIG. 27** as discussed herein) and binning of these exemplary antibodies.
**FIG. 41** depicts KD (M) values of Gal3 binding for exemplary anti-Gal3 antibodies disclosed herein.

### DETAILED DESCRIPTION OF THE DISCLOSURE

Diabetes mellitus is a group of metabolic diseases characterized by a high blood sugar level over a prolonged period of time. There are three main types of diabetes mellitus: Type I diabetes, Type II diabetes, and gestational diabetes. Type I diabetes is caused by the failure of the pancreas to produce enough insulin due to the loss of insulin-producing beta cells. Type II diabetes is characterized by insulin resistance and may be due to a variety of lifestyle, dietary, and genetic factors, including obesity, poor diet, and stress. Gestational diabetes occurs when a pregnant woman without a prior history of diabetes develops high blood sugar levels.

Inflammatory bowel disease (IBD) is a general medical term encompassing inflammatory disorders of the small intestine and colon. The two most prevalent IBD are Crohn's disease and ulcerative colitis, which have distinct manifestations and potential treatments. While the causes of IBD are not precisely known, it is suspected that they include activity of the microbiota and diet. The symptoms are associated with abnormal immunological activity and inflammation of the gastrointestinal organs. Some treatments include immunosuppressants (e.g. mesalazine, corticosteroids) or antibiotics to suppress certain bacteria in the microbiota, but lasting relief may only be possible with surgery. There is a lasting need for effective treatments of IBD.

Hepatic steatosis, also sometimes referred to as fatty liver disease, is a condition generally characterized by an abnormal retention of lipids in cells of the liver. Hepatic steatosis affects millions of people worldwide. For example, the prevalence of fatty liver disease has been estimated to range from 10-24% in various countries around the globe. Fatty liver disease can have various causes. For example, non-alcoholic fatty liver disease (NAFLD) generally refers to a spectrum of hepatic lipid disorders characterized by hepatic steatosis with no known secondary cause. NAFLD can be subcategorized into (a) non-alcoholic fatty liver (NAFL), defined as the presence of steatosis in the absence of histological evidence of hepatocellular injury, and (b) non-alcoholic steatohepatitis (NASH), hepatic steatosis accompanied by hepatocyte injury and inflammation; NASH may occur with or without fibrosis, but may progress to fibrosis and cirrhosis. NAFLD is generally associated with energy metabolism pathologies, including obesity, dyslipidemia, diabetes and metabolic syndrome. The prevalence of NAFLD is high. Prevalence in the general population is estimated at 20%, with prevalence of NASH estimated to be 3-5%. There is an estimated ~70% prevalence of NAFLD among patients with obesity or diabetes, and an estimated prevalence of ~50% prevalence of NAFLD among patients with dyslipidemias. However, there are presently no approved pharmaceuticals for the treatment of NAFLD/NASH.

Galectin-3 (Gal3) plays an important role in cell proliferation, adhesion, differentiation, angiogenesis, and apoptosis. This activity is, at least in part, due to immunomodulatory properties and binding affinity towards other immune regulatory proteins, signaling proteins, and other cell surface markers. Gal3 functions by distinct N-terminal and C-terminal domains. The N-terminal domain (isoform 1: amino acids 1-111) comprise a tandem repeat domain (TRD, isoform 1: amino acids 36-109) and is largely responsible for oligomerization of Gal3. The C-terminal domain (isoform 1: amino acids 112-250) comprise a carbohydrate-recognition-binding domain (CRD), which binds to β-galactosides.

Galectin-3 (Gal3) is herein implicated to have immunomodulatory activity. An example of this is the interaction between Gal3 and T-cell immunoglobulin and mucin-domain containing-3 (TIM-3), which causes suppression of immune responses such as T cell activation and may enable cancer cells to evade immune clearance. Anti-Gal3 antibodies and methods of use are explored in WO 2019/023247 and WO 2020/160156, each which is hereby expressly incorporated by reference in its entirety.

Further, Gal3 has been shown to be elevated in obese humans and is believed to cause insulin resistance and glucose intolerance in these subjects. Gal3 has been shown to bind directly to insulin receptor and to inhibit downstream signaling. Thus, Gal3 may contribute to obesity-induced insulin resistance and chronic tissue inflammation.

Disclosed herein, in some embodiments, are methods of treating a subject having, suspected of having, or at risk of developing diabetes mellitus, inflammatory bowel disease, non-alcoholic fatty liver disease, and/or non-alcoholic steatohepatitis with an anti-Gal3 antibody or binding fragment thereof. In some embodiments, the anti-Gal3 antibody or binding fragment thereof disrupts an interaction between Gal3 and insulin receptor. In some embodiments, the anti-Gal3 antibody or binding fragment thereof disrupts an interaction between Gal3 and an integrin. Additionally provided are compositions comprising anti-Gal3 antibodies or binding fragments thereof that disrupt an interaction between Gal3 and insulin receptor and/or Gal3 and an integrin.

In some embodiments, provided herein are various embodiments of anti-Gal3 antibodies or binding fragments thereof. In some embodiments, the various anti-Gal3 antibodies or binding fragments thereof can block the interactions between Gal3 and other molecules with which Gal3 binds. In some embodiments, any of the anti-Gal3 antibodies or binding fragments thereof or any arrangement of any of the anti-Gal3 antibodies or binding fragments provided herein may be substituted with an antigen binding molecule that binds to Gal3. Thus, the present disclosure also envisions antigen binding molecules each time it mentions antibodies or binding fragments thereof, but for brevity, the present disclosure sometimes simply refers to antibodies or binding fragments thereof.

In some embodiments, the anti-Gal3 antibodies or binding fragments disclosed herein disrupt the interaction between Gal3 and insulin receptor (INSR). Some exemplary anti-Gal3 antibodies that strongly disrupt (e.g. at least 90%) the interaction between Gal3 and INSR include but are not limited to 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, and 13A12.2E5. Some exemplary antibodies that moderately disrupt (e.g. at least 75%) the interaction between Gal3 and INSR include but are not limited to 7D8.2D8 and 15F10.2D6. Some exemplary antibodies that minimally disrupt (e.g. 30% or less) the interaction between Gal3 and INSR include but are not limited to 9H2.2H1, 12G5.D7, 13G4.2F8, and 24D12.2H9. In some embodiments, any of the anti-Gal3 antibodies or binding fragments thereof or any arrangement of any of the anti-Gal3 antibodies or binding fragments provided herein may be substituted with an antigen binding molecule that binds to Gal3. It is noted that the term "antigen binding molecule" encompasses antibodies and binding fragments thereof and denotes a broader genus of options.

In some embodiments, the anti-Gal3 antibodies or binding fragments disclosed herein disrupt the interaction between Gal3 and integrin beta-1 (ITGb1). Some exemplary anti-Gal3 antibodies that strongly disrupt (e.g. at least 90%) the interaction between Gal3 and ITGb1 include but are not limited to TB001, TB006 and 846.1F5). Some exemplary anti-Gal3 antibodies that moderately disrupt (e.g. between 40% to 60%) the interaction between Gal3 and ITGb1 include but are not limited to 3B11, 2D10, 7D8, 13A12, 14H10, 15F10, 20D11, 846.2H3, 846T.14A2, 847.10B9, 847.12F12, and 847.26F5. Some exemplary anti-Gal3 antibodies that do not disrupt the interaction between Gal3 and ITGb1 include but are not limited to 6B3.2D3, and 847.14H4. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibodies or binding fragments disclosed herein disrupt the interaction between Gal3 and integrin alpha-3 (ITGa3). Some exemplary anti-Gal3 antibodies that strongly disrupt (e.g. at least 90%) the interaction between Gal3 and ITGa3 include but are not limited to TB001, TB006, 2D10, 3B11, 7D8, 13A12, 14H10, 15F10, 19B5, 20D11.2C6, 20H5, 23H9, 846.1B2, 846.1F5, 846.1H5, 846.1H12, 846.2H3, 846T.14A2, 846T.14E4, 846T.16B5, 847.4B10, 847.10B9, 84712F12, and 847.26F5. Some exemplary anti-Gal3 antibodies that weakly disrupt (less than 35%) the interaction between Gal3 and ITGa3 include but are not limited to 6B3.2D3, 9H2.2H10, 12G5.D7, 13G4.2F8, 847.14H4, and 847.11B1. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibodies or binding fragments disclosed herein disrupt the interaction between Gal3 and integrin beta-3 (ITGb3). Some exemplary anti-Gal3 antibodies that strongly disrupt (e.g. at least 90%) the interaction between Gal3 and ITGb3 include but are not limited to TB001, TB006, 2D10, 3B11.2G2, 13A12.2E5, 14H10.2C9, 19B5, 20D11.2C6, 20H5.A3, 23H9.2E4, 846.1B2, 846.1F5, 846.1H12, 846.2H3, 846T.14A2, 846T.14E4, 846T.16B5, 847.10B9, 847.12F12, and 847.26F5. Some exemplary anti-Gal3 antibodies that weakly disrupt (less than 10%) the interaction between Gal3 and ITGb3 include but are not limited to 6B3.2D3, 9H2.2H10, 12G5.D7, 13G4.2F8, 847.11B1, and 847.14H4. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibodies or binding fragments disclosed herein disrupt the interaction between Gal3 and integrin alpha-V (ITGaV). Some exemplary anti-Gal3 antibodies that strongly disrupt (e.g. at least 90%) the interaction between Gal3 and ITGaV include but are not limited to TB001, TB006, 2D10, 3B11.2G2, 14H10.2C9, 19B5, 20D11.2C6, 20H5.A3, 23H9.2E4, 846.1B2, 846.1F5, 846.2H3, 846T.14A2, 846T.16B5, 847.10B9, 847.12F12, 847.26F5, and 849.8D10. Some exemplary anti-Gal3 antibodies that weakly disrupt (less than 30%) the interaction between Gal3 and ITGaV include but are not limited to 6B3.2D3, 9H2.2H10, 12G5.D7, 13G4.2F8, 847.11B1, and 847.14H4. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, administering any one of the anti-Gal3 antibodies or binding fragments thereof disclosed herein can reduce weight gain, insulin resistance, liver steatosis, or liver dysfunction, or any combination thereof, in a subject.

In some embodiments, disrupting the interaction between Gal3 and an integrin reduces lymphocyte adhesion. In some embodiments, administering any one of the anti-Gal3 antibodies or binding fragments disclosed herein reduces inflammation in a subject.

In some embodiments, administering any one of the anti-Gal3 antibodies or binding fragments thereof disclosed herein reduces changes in levels of fasting blood glucose or restores C-peptide levels, or both and can be used as a treatment for type I diabetes mellitus and/or type II diabetes mellitus.

In some embodiments, administering any one of the anti-Gal3 antibodies or binding fragments thereof disclosed herein can do at least one of, if not both of, restore inflammation-induced colon length reduction or reduce circulating inflammatory cytokines.

### Definitions

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein.

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed.

The section headings used herein are for organizational purposes only and are not to be construed as limiting the subject matter described.

In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

Throughout this specification, unless the context requires otherwise, the words "comprise," "comprises," and "comprising" will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

As used herein, the terms "individual(s)", "subject(s)" and "patient(s)" mean any mammal. In some embodiments, the mammal is a human. In some embodiments, the mammal is a non-human. None of the terms require or are limited to situations characterized by the supervision (e.g. constant or intermittent) of a health care worker (e.g. a doctor, a registered nurse, a nurse practitioner, a physician's assistant, an orderly or a hospice worker).

The terms "polypeptide", "peptide", and "protein" are used interchangeably herein to refer to polymers of amino acids of any length. The polymer may be linear, cyclic, or branched, it may comprise modified amino acids, and it may be interrupted by non-amino acids. The terms also encompass amino acid polymers that have been modified, for example, via sulfation, glycosylation, lipidation, acetylation, phosphorylation, iodination, methylation, oxidation, proteolytic processing, phosphorylation, prenylation, racemization, selenoylation, transfer-RNA mediated addition of amino acids to proteins such as arginylation, ubiquitination, or any other manipulation, such as conjugation with a labeling component.

As used herein the term "amino acid" refers to either natural and/or unnatural or synthetic amino acids, including glycine and both the D or L optical isomers, and amino acid analogs and peptidomimetics.

A polypeptide or amino acid sequence "derived from" a designated protein refers to the origin of the polypeptide. Preferably, the polypeptide has an amino acid sequence that is essentially identical to that of a polypeptide encoded in the sequence, or a portion thereof wherein the portion consists of at least 10-20 amino acids, or at least 20-30 amino acids, or at least 30-50 amino acids, or which is immunologically identifiable with a polypeptide encoded in the sequence. This terminology also includes a polypeptide expressed from a designated nucleic acid sequence.

As used herein, the term "antibody" denotes the meaning ascribed to it by one of skill in the art, and further it is intended to include any polypeptide chain-containing molecular structure with a specific shape that fits to and recognizes an epitope, where one or more noncovalent binding interactions stabilize the complex between the molecular structure and the epitope. Antibodies may be polyclonal antibodies, although monoclonal antibodies are preferred because they may be reproduced by cell culture or recombinantly and can be modified to reduce their antigenicity.

In addition to entire immunoglobulins (or their recombinant counterparts), immunoglobulin fragments or "binding fragments" comprising the epitope binding site (e.g., Fab', F(ab')₂, single-chain variable fragment (scFv), diabody, minibody, nanobody, single-domain antibody (sdAb), or other fragments) are useful as antibody moieties in the present invention. Such antibody fragments may be generated from whole immunoglobulins by ricin, pepsin, papain, or other protease cleavage. Minimal immunoglobulins may be designed utilizing recombinant immunoglobulin techniques. For instance "Fv" immunoglobulins for use in the present invention may be produced by linking a variable light chain region to a variable heavy chain region via a peptide linker (e.g., poly-glycine or another sequence which does not form an alpha helix or beta sheet motif). Nanobodies or single-domain antibodies can also be derived from alternative organisms, such as dromedaries, camels, llamas, alpacas, or sharks. In some embodiments, antibodies can be conjugates, e.g. pegylated antibodies, drug, radioisotope, or toxin conjugates. Monoclonal antibodies directed against a specific epitope, or combination of epitopes, will allow for the targeting and/or depletion of cellular populations expressing the marker. Various techniques can be utilized using monoclonal antibodies to screen for cellular populations expressing the marker(s), and include magnetic separation using antibody-coated magnetic beads, "panning" with antibody attached to a solid matrix (i.e., plate), and flow cytometry (e.g. U.S. Pat. No. 5,985,660, hereby expressly incorporated by reference in its entirety).

As known in the art, the term "Fc region" is used to define a C-terminal region of an immunoglobulin heavy chain. The "Fc region" may be a native sequence Fc region or a variant Fc region. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The numbering of the residues in the Fc region is that of the EU index as in Kabat. Kabat et al., Sequences of Proteins of Immunological Interest, 5th Ed. Public Health Service, National Institutes of Health, Bethesda, Md., 1991. The Fc region of an immunoglobulin generally comprises two constant domains, CH2 and CH3. As is known in the art, an Fc region can be present in dimer or monomeric form.

As known in the art, a "constant region" of an antibody refers to the constant region of the antibody light chain or the constant region of the antibody heavy chain, either alone or in combination.

A "variable region" of an antibody refers to the variable region of the antibody light chain or the variable region of the antibody heavy chain, either alone or in combination. As known in the art, the variable regions of the heavy and light chains each consist of four framework regions (FRs) connected by three complementarity determining regions (CDRs) also known as hypervariable regions, and contribute to the formation of the antigen binding site of antibodies. If variants of a subject variable region are desired, particularly with substitution in amino acid residues outside of a CDR region (i.e., in the framework region), appropriate amino acid substitution, preferably, conservative amino acid substitution, can be identified by comparing the subject variable region to the variable regions of other antibodies which contain CDR1 and CDR2 sequences in the same canonical class as the subject variable region (Chothia and Lesk, J Mol Biol 196(4): 901-917, 1987).

In certain embodiments, definitive delineation of a CDR and identification of residues comprising the binding site of an antibody is accomplished by solving the structure of the antibody and/or solving the structure of the antibody-ligand complex. In certain embodiments, that can be accomplished by any of a variety of techniques known to those skilled in the art, such as X-ray crystallography. In certain embodiments, various methods of analysis can be employed to identify or approximate the CDR regions. In certain embodiments, various methods of analysis can be employed to identify or approximate the CDR regions. Examples of such methods include, but are not limited to, the Kabat definition, the Chothia definition, the IMGT approach (Lefranc et al., 2003) Dev Comp Immunol. 27:55-77), computational programs such as Paratome (Kunik et al., 2012, Nucl Acids Res. W521-4), the AbM definition, and the conformational definition.

The Kabat definition is a standard for numbering the residues in an antibody and is typically used to identify CDR regions. See, e.g., Johnson & Wu, 2000, Nucleic Acids Res., 28: 214-8. The Chothia definition is similar to the Kabat definition, but the Chothia definition takes into account positions of certain structural loop regions. See, e.g., Chothia et al., 1986, J. Mol. Biol., 196: 901-17; Chothia et al., 1989, Nature, 342: 877-83. The AbM definition uses an integrated suite of computer programs produced by Oxford Molecular Group that model antibody structure. See, e.g., Martin et al., 1989, Proc Natl Acad Sci (USA), 86:9268-9272; "AbM.TM., A Computer Program for Modeling Variable Regions of Antibodies," Oxford, UK; Oxford Molecular, Ltd. The AbM definition models the tertiary structure of an antibody from primary sequence using a combination of knowledge databases and ab initio methods, such as those described by Samudrala et al., 1999, "Ab Initio Protein Structure Prediction Using a Combined Hierarchical Approach," in PROTEINS, Structure, Function and Genetics Suppl., 3:194-198. The contact definition is based on an analysis of the available complex crystal structures. See, e.g., MacCallum et al., 1996, J. Mol. Biol., 5:732-45. In another approach, referred to herein as the "conformational definition" of CDRs, the positions of the CDRs may be identified as the residues that make enthalpic contributions to antigen binding. See, e.g., Makabe et al., 2008, Journal of Biological Chemistry, 283:1156-1166. Still other CDR boundary definitions may not strictly follow one of the above approaches, but will nonetheless overlap with at least a portion of the Kabat CDRs, although they may be shortened or lengthened in light of prediction or experimental findings that particular residues or groups of residues do not significantly impact antigen binding. As used herein, a CDR may refer to CDRs defined by any approach known in the art, including combinations of approaches. The methods used herein may utilize CDRs defined according to any of these approaches. For any given embodiment containing more than one CDR, the CDRs may be defined in accordance with any of Kabat, Chothia, extended, IMGT, Paratome, AbM, and/or conformational definitions, or a combination of any of the foregoing.

The term "compete," as used herein with regard to an antibody, means that a first antibody, or an antigen-binding portion thereof, binds to an epitope in a manner sufficiently similar to the binding of a second antibody, or an antigen-binding portion thereof, such that the result of binding of the first antibody with its cognate epitope is detectably decreased in the presence of the second antibody compared to the binding of the first antibody in the absence of the second antibody. The alternative, where the binding of the second antibody to its epitope is also detectably decreased in the presence of the first antibody, can, but need not be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope or ligand, whether to the same, greater, or lesser extent, the antibodies are said to "cross-compete" with each other for binding of their respective epitope(s). Both competing and cross-competing antibodies are encompassed by the present invention. Regardless of the mechanism by which such competition or cross-competition occurs (e.g., steric hindrance, conformational change, or binding to a common epitope, or portion thereof), the skilled artisan would appreciate, based upon the teachings provided herein, that such competing and/or cross-competing antibodies are encompassed and can be useful for the methods disclosed herein.

An antibody that "preferentially binds" or "specifically binds" (used interchangeably herein) to an epitope is a term well understood in the art, and methods to determine such specific or preferential binding are also well known in the art. A molecule is said to exhibit "specific binding" or "preferential binding" if it reacts or associates more frequently, and/or more rapidly, and/or with greater duration and/or with greater affinity with a particular cell or substance than it does with alternative cells or substances. An antibody "specifically binds" or "preferentially binds" to a target if it binds with greater affinity, and/or avidity, and/or more readily, and/or with greater duration than it binds to other substances. For example, an antibody that specifically or preferentially binds to a CFD epitope is an antibody that binds this epitope with greater affinity, and/or avidity, and/or more readily, and/or with greater duration than it binds to other CFD epitopes or non-CFD epitopes. It is also understood by reading this definition that, for example, an antibody (or moiety or epitope) that specifically or preferentially binds to a first target may or may not specifically or preferentially bind to a second target. As such, "specific binding" or "preferential binding" does not necessarily require (although it can include) exclusive binding. Generally, but not necessarily, reference to binding means preferential binding.

As used herein, the term "antigen binding molecule" refers to a molecule that comprises an antigen binding portion that binds to an antigen and, optionally, a scaffold or framework portion that allows the antigen binding portion to adopt a conformation that promotes binding of the antigen binding portion or provides some additional properties to the antigen binding molecule. In some embodiments, the antigen is Gal3. In some embodiments, the antigen binding portion comprises at least one CDR from an antibody that binds to the antigen. In some embodiments, the antigen binding portion comprises all three CDRs from a heavy chain of an antibody that binds to the antigen or from a light chain of an antibody that binds to the antigen. In some embodiments, the antigen binding portion comprises all six CDRs from an antibody that binds to the antigen (three from the heavy chain and three from the light chain). In some embodiments, the antigen binding portion is an antibody fragment.

Nonlimiting examples of antigen binding molecules include antibodies, antibody fragments (e.g., an antigen binding fragment of an antibody), antibody derivatives, and antibody analogs. Further specific examples include, but are not limited to, a single-chain variable fragment (scFv), a nanobody (e.g. VH domain of camelid heavy chain antibodies; VHH fragment, see Cortez-Retamozo et al., Cancer Research, Vol. 64:2853-57, 2004), a Fab fragment, a Fab' fragment, a F(ab')2 fragment, a Fv fragment, a Fd fragment, and a complementarity determining region (CDR) fragment. These molecules can be derived from any mammalian source, such as human, mouse, rat, rabbit, pig, dog, cat, horse, donkey, guinea pig, goat, or camelid. Antibody fragments may compete for binding of a target antigen with an intact antibody and the fragments may be produced by the modification of intact antibodies (e.g. enzymatic or chemical cleavage) or synthesized de novo using recombinant DNA technologies or peptide synthesis. The antigen binding molecule can comprise, for example, an alternative protein scaffold or artificial scaffold with grafted CDRs or CDR derivatives. Such scaffolds include, but are not limited to, antibody-derived scaffolds comprising mutations introduced to, for example, stabilize the three-dimensional structure of the antigen binding molecule as well as wholly synthetic scaffolds comprising, for example, a biocompatible polymer. See, for example, Korndorfer et al., 2003, Proteins: Structure, Function, and Bioinformatics, Volume 53, Issue 1:121-129 (2003); Roque et al., Biotechnol. Prog. 20:639-654 (2004). In addition, peptide antibody mimetics ("PAMs") can be used, as well as scaffolds based on antibody mimetics utilizing fibronectin components as a scaffold.

An antigen binding molecule can also include a protein comprising one or more antibody fragments incorporated into a single polypeptide chain or into multiple polypeptide chains. For instance, antigen binding molecule can include, but are not limited to, a diabody (see, e.g., EP 404,097; WO 93/11161; and Hollinger et al., Proc. Natl. Acad. Sci. USA, Vol. 90:6444-6448, 1993); an intrabody; a domain antibody (single VL or VH domain or two or more VH domains joined by a peptide linker; see Ward et al., Nature, Vol. 341:544-546, 1989); a maxibody (2 scFvs fused to Fc region, see Fredericks et al., Protein Engineering, Design & Selection, Vol. 17:95-106, 2004 and Powers et al., Journal of Immunological Methods, Vol. 251:123-135, 2001); a triabody; a tetrabody; a minibody (scFv fused to CH3 domain; see Olafsen et al., Protein Eng Des Sel. , Vol.17:315-23, 2004); a peptibody (one or more peptides attached to an Fc region, see WO 00/24782); a linear antibody (a pair of tandem Fd segments (VH-CH1-VH-CH1 ) which, together with complementary light chain polypeptides, form a pair of antigen binding regions, see Zapata et al., Protein Eng., Vol. 8:1057-1062, 1995); a small modular immunopharmaceutical (see U.S. Patent Publication No. 20030133939); and immunoglobulin fusion proteins (e.g. IgG-scFv, IgG-Fab, 2scFv-IgG, 4scFv-IgG, VH-IgG, IgG-VH, and Fab-scFv-Fc).

In certain embodiments, an antigen binding molecule can have, for example, the structure of an immunoglobulin. An "immunoglobulin" is a tetrameric molecule, with each tetramer comprising two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function.

The term "humanized" as applies to a non-human (e.g. rodent or primate) antibodies are hybrid immunoglobulins, immunoglobulin chains or fragments thereof which contain minimal sequence derived from non-human immunoglobulin.

As used herein, the terms "treating" or "treatment" (and as well understood in the art) means an approach for obtaining beneficial or desired results in a subject's condition, including clinical results. Beneficial or desired clinical results can include, but are not limited to, alleviation or amelioration of one or more symptoms or conditions, diminishment of the extent of a disease, stabilizing (i.e., not worsening) the state of disease, prevention of a disease's transmission or spread, delaying or slowing of disease progression, amelioration or palliation of the disease state, diminishment of the reoccurrence of disease, and remission, whether partial or total and whether detectable or undetectable. "Treating" and "treatment" as used herein also include prophylactic treatment. Treatment methods comprise administering to a subject a therapeutically effective amount of an active agent. The administering step may consist of a single administration or may comprise a series of administrations. The compositions are administered to the subject in an amount and for a duration sufficient to treat the subject. The length of the treatment period depends on a variety of factors, such as the severity of the condition, the age and genetic profile of the subject, the concentration of active agent, the activity of the compositions used in the treatment, or a combination thereof. It will also be appreciated that the effective dosage of an agent used for the treatment or prophylaxis may increase or decrease over the course of a particular treatment or prophylaxis regime. Changes in dosage may result and become apparent by standard diagnostic assays known in the art. In some instances, chronic administration may be required.

The terms "effective amount" or "effective dose" as used herein have their plain and ordinary meaning as understood in light of the specification, and refer to that amount of a recited composition or compound that results in an observable designated effect. Actual dosage levels of active ingredients in an active composition of the presently disclosed subject matter can be varied so as to administer an amount of the active composition or compound that is effective to achieve the designated response for a particular subject and/or application. The selected dosage level can vary based upon a variety of factors including, but not limited to, the activity of the composition, formulation, route of administration, combination with other drugs or treatments, severity of the condition being treated, and the physical condition and prior medical history of the subject being treated. In some embodiments, a minimal dose is administered, and dose is escalated in the absence of dose-limiting toxicity to a minimally effective amount. Determination and adjustment of an effective dose, as well as evaluation of when and how to make such adjustments, are contemplated herein.

The term "administering" includes oral administration, topical contact, administration as a suppository, intravenous, intraperitoneal, intramuscular, intralesional, intrathecal, intranasal, or subcutaneous administration, or the implantation of a slow-release device, e.g., a mini-osmotic pump, to a subject. Administration is by any route, including parenteral and transmucosal (e.g., buccal, sublingual, palatal, gingival, nasal, vaginal, rectal, or transdermal). Parenteral administration includes, e.g., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, etc. By "co-administer" it is meant that a first compound described herein is administered at the same time, just prior to, or just after the administration of a second compound described herein.

As used herein, the term "therapeutic target" refers to a gene or gene product that, upon modulation of its activity (e.g., by modulation of expression, biological activity, and the like), can provide for modulation of the disease phenotype. As used throughout, "modulation" is meant to refer to an increase or a decrease in the indicated phenomenon (e.g., modulation of a biological activity refers to an increase in a biological activity or a decrease in a biological activity).

As used herein, the term "standard of care", "best practice" and "standard therapy" refers to the treatment that is accepted by medical practitioners to be an appropriate, proper, effective, and/or widely used treatment for a certain disease. The standard of care of a certain disease depends on many different factors, including the biological effect of treatment, region or location within the body, patient status (e.g. age, weight, gender, hereditary risks, other disabilities, secondary conditions), toxicity, metabolism, bioaccumulation, therapeutic index, dosage, and other factors known in the art. Determining a standard of care for a disease is also dependent on establishing safety and efficacy in clinical trials as standardized by regulatory bodies such as the US Food and Drug Administration, International Council for Harmonisation, Health Canada, European Medicines Agency, Therapeutics Goods Administration, Central Drugs Standard Control Organization, National Medical Products Administration, Pharmaceuticals and Medical Devices Agency, Ministry of Food and Drug Safety, and the World Health Organization. The standard of care for a disease may include but is not limited to surgery, radiation, chemotherapy, targeted therapy, or immunotherapy.

The term "% w/w" or "% wt/wt" means a percentage expressed in terms of the weight of the ingredient or agent over the total weight of the composition multiplied by 100.

Unless otherwise specified, the complementarity defining regions disclosed herein follow the IMGT definition. In some embodiments, any of the CDRs disclosed herein can instead be interpreted by Kabat, Chothia, or other definitions accepted by those of skill in the art.

It is understood that an antibody with an antibody name described herein can be referred using a shortened version of the antibody name, as long as there are no conflicts with another antibody described herein. For example, 2D10.2B2 may be referred to as 2D10. This can also refer to fragments of the antibody (e.g., with the same 1, 3, or 6 CDRs).

### Gal3 Binders

Disclosed herein and as applicable to any of the methods or uses disclosed herein are anti-Gal3 antibodies or binding fragments thereof. In some embodiments, the anti-Gal3 antibody or binding fragment thereof binds to the N-terminal domain of Gal3, N-terminus of Gal3, or the tandem repeat domain (TRD) of Gal3. In some embodiments, the anti-Gal3 antibody or binding fragment thereof does not bind to the N-terminus of Gal3, the N-terminal domain of Gal3, or the TRD of Gal3. In some embodiments, the anti-Gal3 antibody or binding fragment thereof binds to the C-terminus of Gal3, the C-terminal domain of Gal3, or the CRD of Gal3. In some embodiments, the anti-Gal3 antibody or binding fragment thereof does not bind to the C-terminus of Gal3, the C-terminal domain of Gal3, or the CRD of Gal3. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibody or binding fragment thereof binds to one or more peptides of **SEQ ID NOs: 3-26.** In some embodiments, the anti-Gal3 antibody or binding fragment thereof binds to an epitope present within a region of Gal3 defined by Peptide 1 (ADNFSLHDALSGSGNPNPQG; **SEQ ID NO: 3),** Peptide 4 (GAGGYPGASYPGAYPGQAPP; **SEQ ID NO: 6),** Peptide 6 (GAYPGQAPPGAYPGAPGAYP; **SEQ ID NO: 8),** Peptide 7 (AYPGAPGAYPGAPAPGVYPG; **SEQ ID NO: 9),** or a combination thereof. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibody or binding fragment thereof binds to an epitope of Gal3 that includes a motif of GxYPG, where x is the amino acids alanine (A), glycine (G), or valine (V). In some embodiments, an anti-Gal3 antibody as described herein binds to an epitope of Gal3 that includes two GxYPG motifs separated by three amino acids, where x is A, G, or V. In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibody or binding fragment thereof comprises (1) a light chain variable region comprising a V_{L}-CDR1, a V_{L}-CDR2, and a V_{L}-CDR3 and (2) a heavy chain variable region comprising a V_{H}-CDR1, a V_{H}-CDR2, and a V_{H}-CDR3. In some embodiments, the V_{L}-CDR1 comprises an amino acid sequence having at least 60%, at least 70%, at least 80%, at least 90%, or 100% sequence identity to any amino acid sequence according to **SEQ ID NOs: 100-128.** In some embodiments, the V_{L}-CDR2 comprises an amino acid sequence having at least 60%, at least 70%, at least 80%, at least 90%, or 100% sequence identity to any amino acid sequence according to **SEQ ID NOs: 129-144.** In some embodiments, the V_{L}-CDR3 comprises an amino acid sequence having at least 60%, at least 70%, at least 80%, at least 90%, or 100% sequence identity to any amino acid sequence according to **SEQ ID NOs: 145-168.** In some embodiments, the V_{H}-CDR1 comprises an amino acid sequence having at least 60%, at least 70%, at least 80%, at least 90%, or 100% sequence identity to any amino acid sequence according to **SEQ ID NOs: 27-47.** In some embodiments, the V_{H}-CDR2 comprises an amino acid sequence having at least 60%, at least 70%, at least 80%, at least 90%, or 100% sequence identity to any amino acid sequence according to **SEQ ID NOs: 48-69.** In some embodiments, the V_{H}-CDR3 comprises an amino acid sequence having at least 60%, at least 70%, at least 80%, at least 90%, or 100% sequence identity to any amino acid sequence according to **SEQ ID NOs: 70-99.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-GAL3 antibody or fragment thereof, has at least one of the HCDRs, e.g., HCDR1, HCDR2, or HCDR3, provided herein.

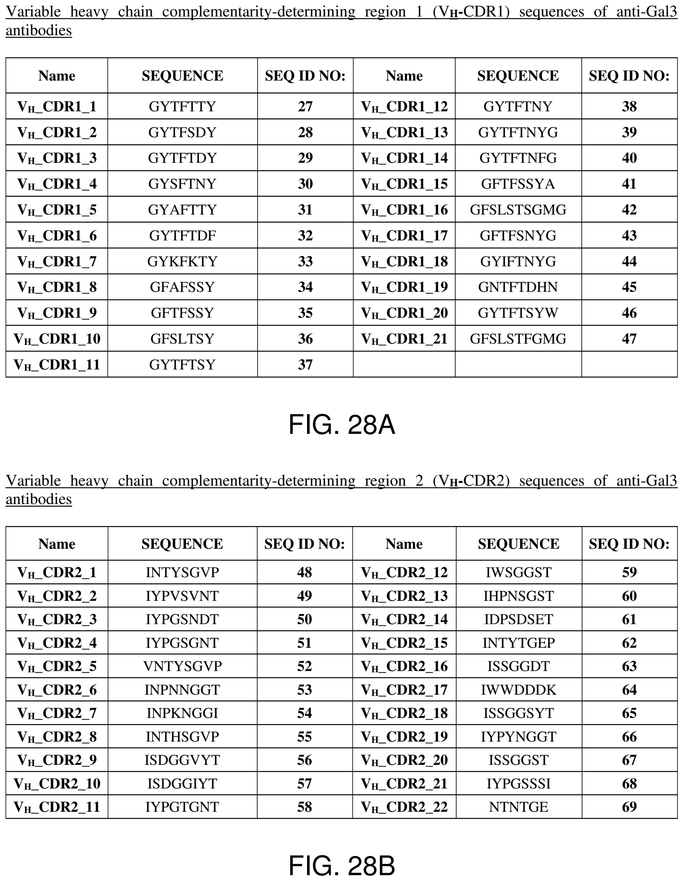

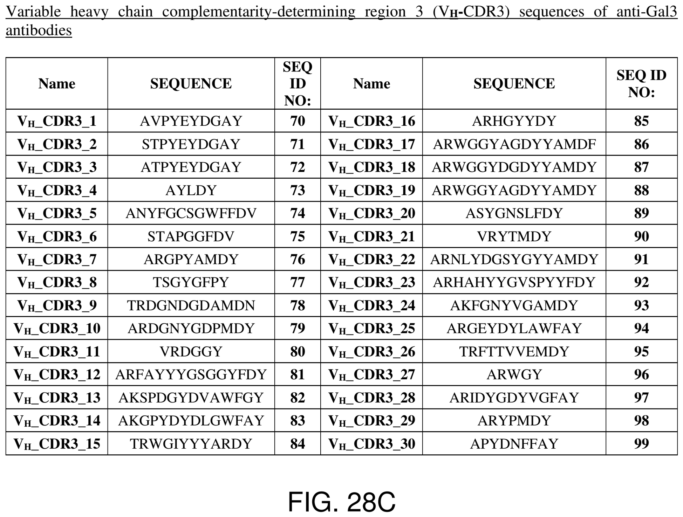

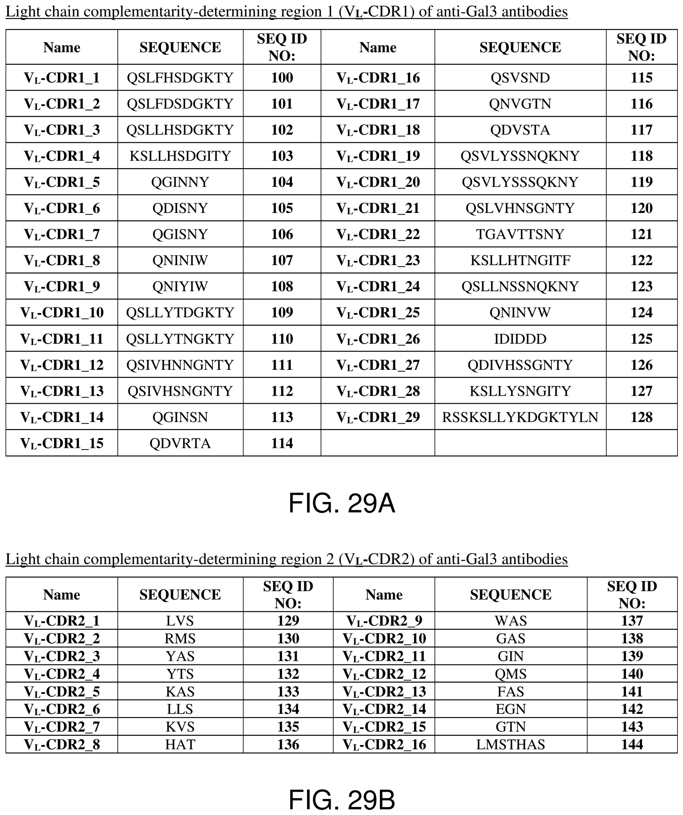

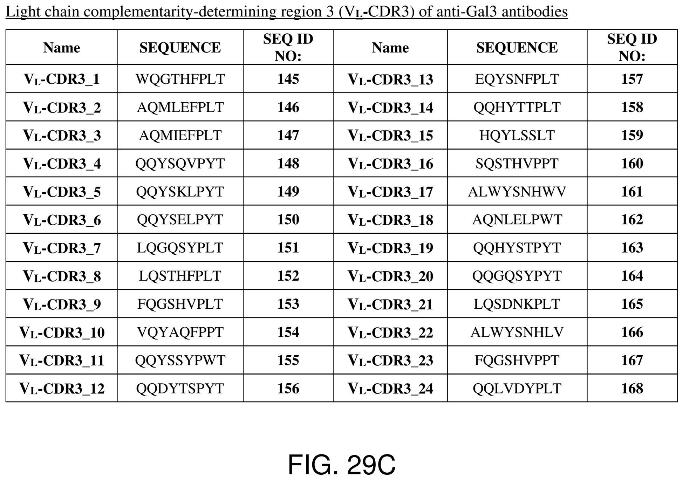

In some embodiments, exemplary V_{H}-CDR1 sequences are depicted in **FIG. 28A****.** In some embodiments, exemplary V_{H}-CDR2 sequences are depicted in **FIG. 28B****.** In some embodiments, exemplary V_{H}-CDR3 sequences are depicted in **FIG. 28C****.** In some embodiments, exemplary V_{L}-CDR1 sequences are depicted in **FIG. 29A****.** In some embodiments, exemplary V_{L}-CDR2 sequences are depicted in **FIG. 29B****.** In some embodiments, exemplary V_{L}-CDR3 sequences are depicted in **FIG. 29C****.**

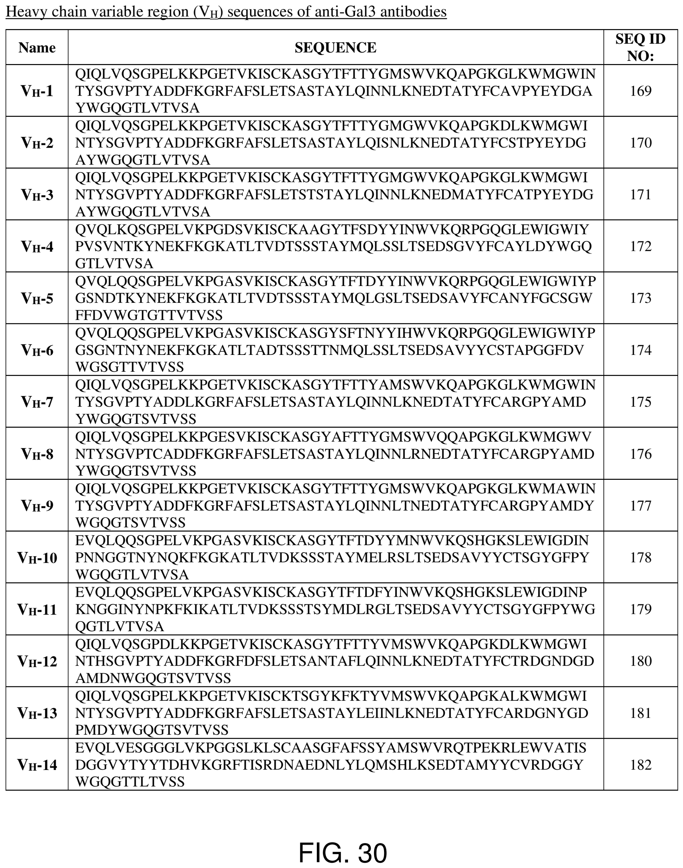

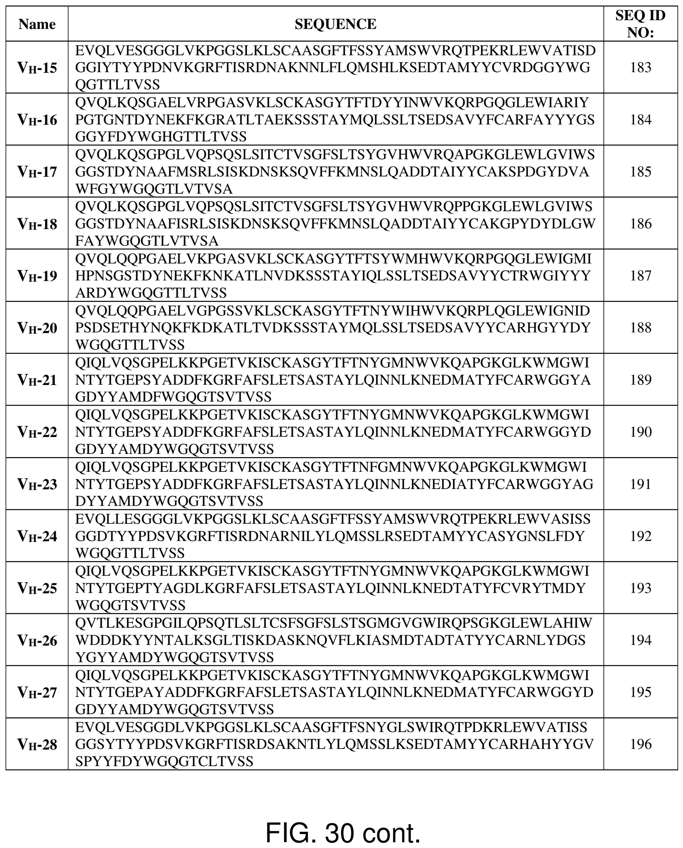

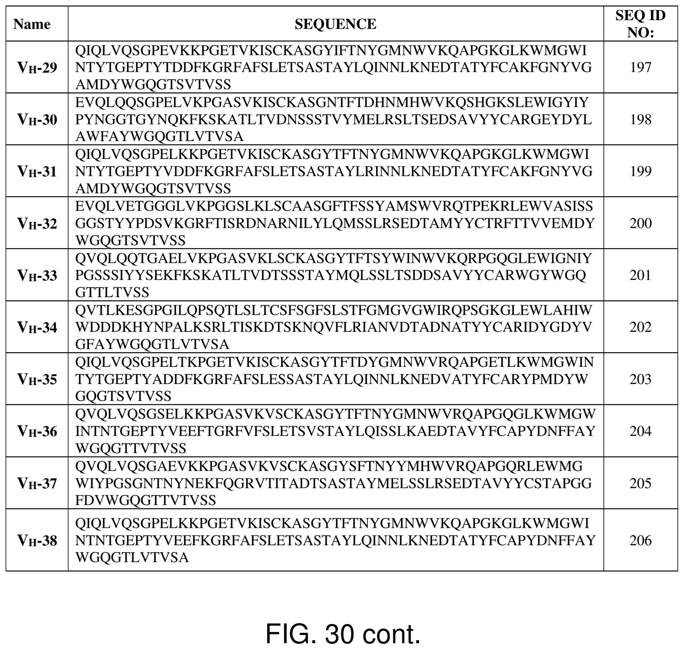

In some embodiments, the heavy chain variable region of any of the anti-Gal3 antibodies or binding fragments thereof disclosed herein comprises an amino acid sequence having at least 75%, 80%, 85%, 90%, 95%, or 100% sequence identity to any sequence according to **SEQ ID NOs: 169-206.** In some embodiments, the heavy chain variable region of any of the anti-Gal3 antibodies or binding fragments thereof disclosed herein is selected from the group consisting of **SEQ ID NOs: 169-206.** In some embodiments, exemplary V_{H} are depicted in **FIG. 30****.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

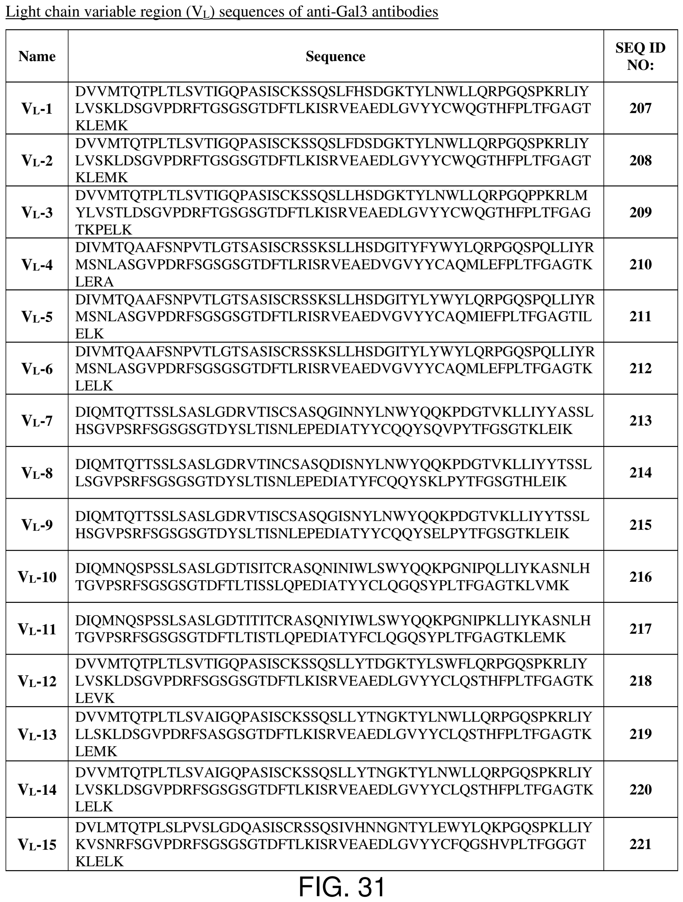

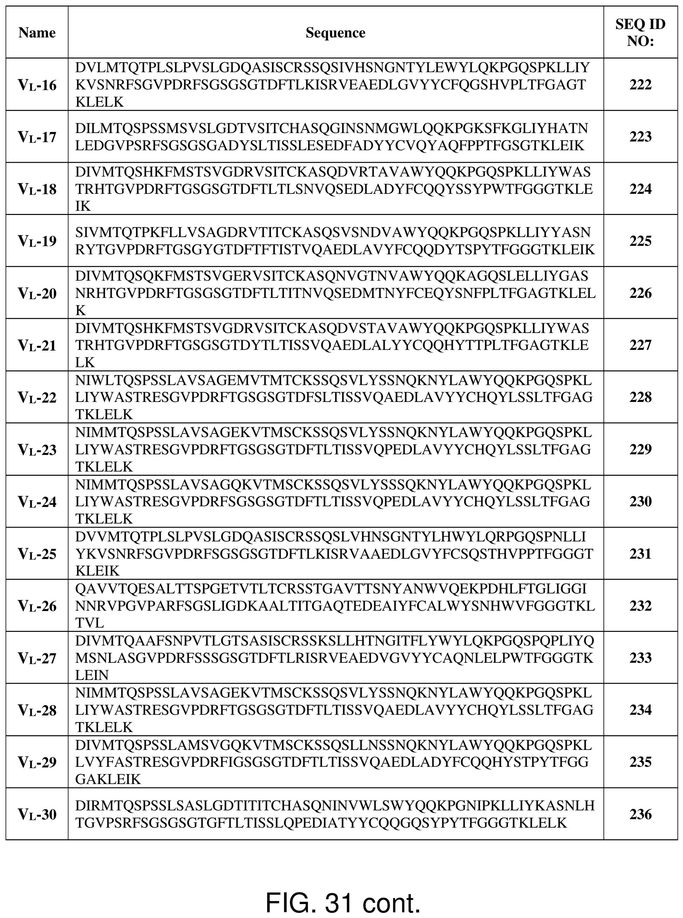

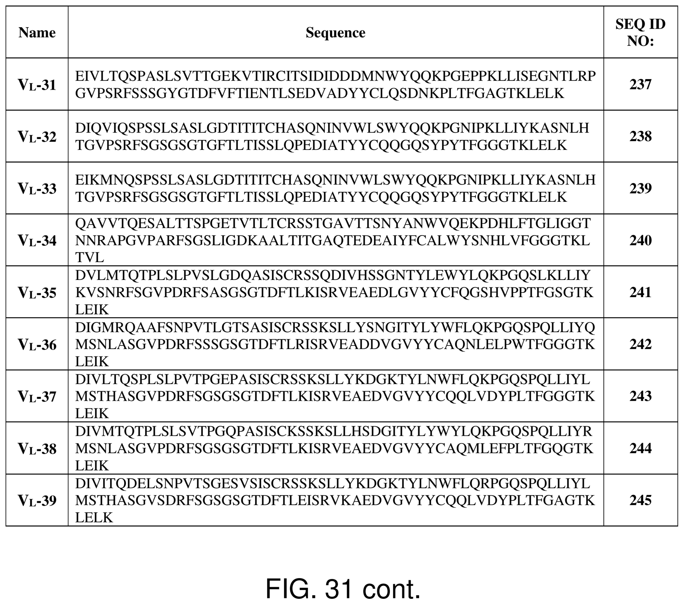

In some embodiments, the light chain variable region of any of the anti-Gal3 antibodies or binding fragments thereof disclosed herein comprises an amino acid sequence having at least 75%, 80%, 85%, 90%, 95%, or 100% sequence identity to any sequence according to **SEQ ID NOs: 207-245.** In some embodiments, the light chain variable region of any of the anti-Gal3 antibodies or binding fragments thereof disclosed herein is selected from the group consisting of **SEQ ID NOs: 207-245.** In some embodiments, exemplary V_{L} are depicted in **FIG. 31****.**

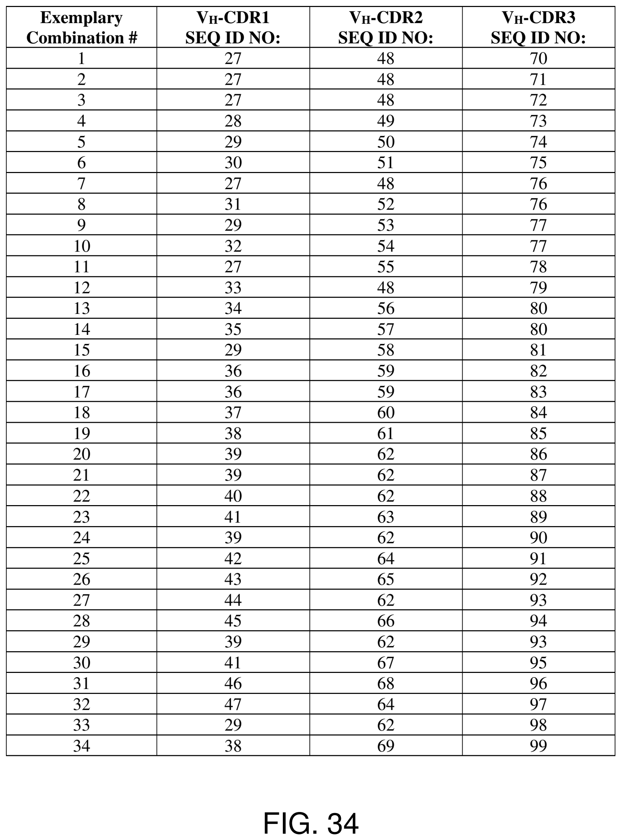

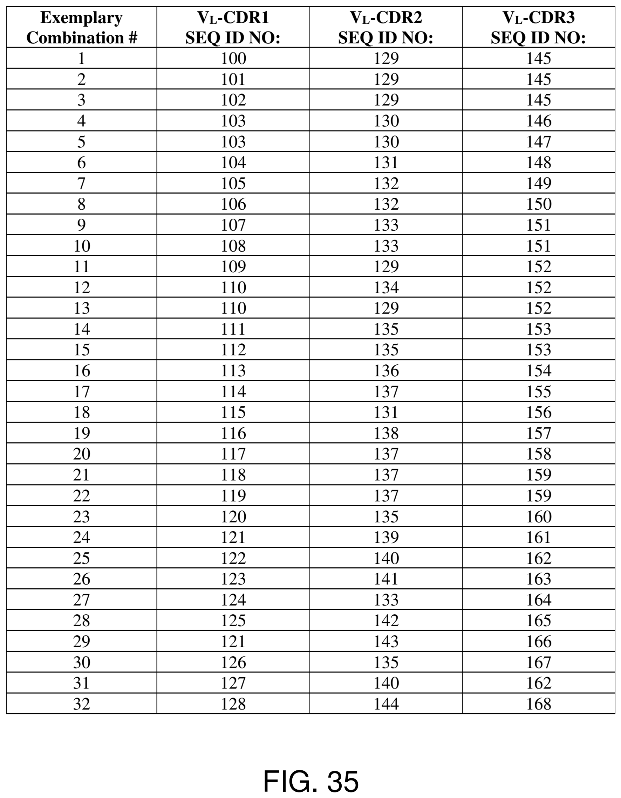

In some embodiments, the anti-Gal3 antibody or binding fragment thereof comprises: a) the V_{H}-CDR1, V_{H}-CDR2, V_{H}-CDR3 of the V_{H}-CDR1, V_{H}-CDR2, V_{H}-CDR3 within **SEQ ID NO: 169** and the V_{L}-CDR1, V_{L}-CDR2, V_{L}-CDR3 of the V_{L}-CDR1, V_{L}-CDR2, V_{L}-CDR3 within **SEQ ID NO: 207;** b) the V_{H}-CDR1, V_{H}-CDR2, V_{H}-CDR3 of the V_{H}-CDR1, V_{H}-CDR2, V_{H}-CDR3 within **SEQ ID NO: 170** and the V_{L}-CDR1, V_{L}-CDR2, V_{L}-CDR3 of the V_{L}-CDR1, V_{L}-CDR2, V_{L}-CDR3 within **SEQ ID NO: 208;** c) the V_{H}-CDR1, V_{H}-CDR2, V_{H}-CDR3 of the V_{H}-CDR1, V_{H}-CDR2, V_{H}-CDR3 within **SEQ ID NO: 171** and the V_{L}-CDR1, V_{L}-CDR2, V_{L}-CDR3 of the V_{L}-CDR1, V_{L}-CDR2, V_{L}-CDR3 within **SEQ ID NO: 209;** d) the V_{H}-CDR1, V_{H}-CDR2, V_{H}-CDR3 of the V_{H}-CDR1, V_{H}-CDR2, V_{H}-CDR3 within **SEQ ID NO: 172** and the V_{L}-CDR1, V_{L}-CDR2, V_{L}-CDR3 of the V_{L}-CDR1, V_{L}-CDR2, V_{L}-CDR3 within **SEQ ID NO: 210;** e) the V_{H}-CDR1, V_{H}-CDR2, V_{H}-CDR3 of the V_{H}-CDR1, V_{H}-CDR2, V_{H}-CDR3 within **SEQ ID NO: 173** and the V_{L}-CDR1, V_{L}-CDR2, V_{L}-CDR3 of the V_{L}-CDR1, V_{L}-CDR2, V_{L}-CDR3 within **SEQ ID NO: 211;** f) the V_{H}-CDR1, V_{H}-CDR2, V_{H}-CDR3 of the V_{H}-CDR1, V_{H}-CDR2, V_{H}-CDR3 within **SEQ ID NO: 174** and the V_{L}-CDR1, V_{L}-CDR2, V_{L}-CDR3 of the V_{L}-CDR1, V_{L}-CDR2, V_{L}-CDR3 within **SEQ ID NO: 212;** g) the V_{H}-CDR1, V_{H}-CDR2, V_{H}-CDR3 of the V_{H}-CDR1, V_{H}-CDR2, V_{H}-CDR3 within **SEQ ID NO: 175** and the V_{L}-CDR1, V_{L}-CDR2, V_{L}-CDR3 of the V_{L}-CDR1, V_{L}-CDR2, V_{L}-CDR3 within **SEQ ID NO: 213;** h) the V_{H}-CDR1, V_{H}-CDR2, V_{H}-CDR3 of the V_{H}-CDR1, V_{H}-CDR2, V_{H}-CDR3 within **SEQ ID NO: 176** and the V_{L}-CDR1, V_{L}-CDR2, V_{L}-CDR3 of the V_{L}-CDR1, V_{L}-CDR2, V_{L}-CDR3 within **SEQ ID NO: 214;** i) the V_{H}-CDR1, V_{H}-CDR2, V_{H}-CDR3 of the V_{H}-CDR1, V_{H}-CDR2, V_{H}-CDR3 within **SEQ ID NO: 177** and the V_{L}-CDR1, V_{L}-CDR2, V_{L}-CDR3 of the V_{L}-CDR1, V_{L}-CDR2, V_{L}-CDR3 within **SEQ ID NO: 215; j)** the V_{H}-CDR1, V_{H}-CDR2, V_{H}-CDR3 of the V_{H}-CDR1, V_{H}-CDR2, V_{H}-CDR3 within **SEQ ID NO: 178** and the V_{L}-CDR1, V_{L}-CDR2, V_{L}-CDR3 of the V_{L}-CDR1, V_{L}-CDR2, V_{L}-CDR3 within **SEQ ID NO: 216;** k) the V_{H}-CDR1, V_{H}-CDR2, V_{H}-CDR3 of the V_{H}-CDR1, V_{H}-CDR2, V_{H}-CDR3 within **SEQ ID NO: 179** and the V_{L}-CDR1, V_{L}-CDR2, V_{L}-CDR3 of the V_{L}-CDR1, V_{L}-CDR2, V_{L}-CDR3 within **SEQ ID NO: 217;** 1) the V_{H}-CDR1, V_{H}-CDR2, V_{H}-CDR3 of the V_{H}-CDR1, V_{H}-CDR2, V_{H}-CDR3 within SEQ **ID NO: 180** and the V_{L}-CDR1, V_{L}-CDR2, V_{L}-CDR3 of the V_{L}-CDR1, V_{L}-CDR2, V_{L}-CDR3 within **SEQ ID NO: 218;** m) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 219;** n) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 220;** o) the V_{H}-CDR1, V_{H}-CDR2, V_{H}-CDR3 of the V_{H}-CDR1, V_{H}-CDR2, V_{H}-CDR3 within **SEQ ID NO: 182** and the V_{L}-CDR1, V_{L}-CDR2, V_{L}-CDR3 of the V_{L}-CDR1, V_{L}-CDR2, V_{L}-CDR3 within **SEQ ID NO: 221;** p) the V_{H}-CDR1, V_{H}-CDR2, V_{H}-CDR3 of the V_{H}-CDR1, V_{H}-CDR2, V_{H}-CDR3 within **SEQ ID NO: 183** and the V_{L}-CDR1, V_{L}-CDR2, V_{L}-CDR3 of the V_{L}-CDR1, V_{L}-CDR2, V_{L}-CDR3 within **SEQ ID NO: 222;** q) the V_{H}-CDR1, V_{H}-CDR2, V_{H}-CDR3 of the V_{H}-CDR1, V_{H}-CDR2, V_{H}-CDR3 within **SEQ ID NO: 184** and the V_{L}-CDR1, V_{L}-CDR2, V_{L}-CDR3 of the V_{L}-CDR1, V_{L}-CDR2, V_{L}-CDR3 within **SEQ ID NO: 223;** r) the V_{H}-CDR1, V_{H}-CDR2, V_{H}-CDR3 of the V_{H}-CDR1, V_{H}-CDR2, V_{H}-CDR3 within **SEQ ID NO: 185** and the V_{L}-CDR1, V_{L}-CDR2, V_{L}-CDR3 of the V_{L}-CDR1, V_{L}-CDR2, V_{L}-CDR3 within **SEQ ID NO: 224;** s) the V_{H}-CDR1, V_{H}-CDR2, V_{H}-CDR3 of the V_{H}-CDR1, V_{H}-CDR2, V_{H}-CDR3 within **SEQ ID NO: 186** and the V_{L}-CDR1, V_{L}-CDR2, V_{L}-CDR3 of the V_{L}-CDR1, V_{L}-CDR2, V_{L}-CDR3 within **SEQ ID NO: 225;** t) the V_{H}-CDR1, V_{H}-CDR2, V_{H}-CDR3 of the V_{H}-CDR1, V_{H}-CDR2, V_{H}-CDR3 within **SEQ ID NO: 187** and the V_{L}-CDR1, V_{L}-CDR2, V_{L}-CDR3 of the V_{L}-CDR1, V_{L}-CDR2, V_{L}-CDR3 within **SEQ ID NO: 226;** u) the V_{H}-CDR1, V_{H}-CDR2, V_{H}-CDR3 of the V_{H}-CDR1, V_{H}-CDR2, V_{H}-CDR3 within **SEQ ID NO: 188** and the V_{L}-CDR1, V_{L}-CDR2, V_{L}-CDR3 of the V_{L}-CDR1, V_{L}-CDR2, V_{L}-CDR3 within **SEQ ID NO: 227;** v) the V_{H}-CDR1, V_{H}-CDR2, V_{H}-CDR3 of the V_{H}-CDR1, V_{H}-CDR2, V_{H}-CDR3 within **SEQ ID NO: 189** and the V_{L}-CDR1, V_{L}-CDR2, V_{L}-CDR3 of the V_{L}-CDR1, V_{L}-CDR2, V_{L}-CDR3 within **SEQ ID NO: 228;** w) the V_{H}-CDR1, V_{H}-CDR2, V_{H}-CDR3 of the V_{H}-CDR1, V_{H}-CDR2, V_{H}-CDR3 within **SEQ ID NO: 190** and the V_{L}-CDR1, V_{L}-CDR2, V_{L}-CDR3 of the V_{L}-CDR1, V_{L}-CDR2, V_{L}-CDR3 within **SEQ ID NO: 229;** x) the V_{H}-CDR1, V_{H}-CDR2, V_{H}-CDR3 of the V_{H}-CDR1, V_{H}-CDR2, V_{H}-CDR3 within **SEQ ID NO: 191** and the V_{L}-CDR1, V_{L}-CDR2, V_{L}-CDR3 of the V_{L}-CDR1, V_{L}-CDR2, V_{L}-CDR3 within **SEQ ID NO: 230;** y) the V_{H}-CDR1, V_{H}-CDR2, V_{H}-CDR3 of the V_{H}-CDR1, V_{H}-CDR2, V_{H}-CDR3 within **SEQ ID NO: 192** and the V_{L}-CDR1, V_{L}-CDR2, V_{L}-CDR3 of the V_{L}-CDR1, V_{L}-CDR2, V_{L}-CDR3 within **SEQ ID NO: 231;** z) the V_{H}-CDR1, V_{H}-CDR2, V_{H}-CDR3 of the V_{H}-CDR1, V_{H}-CDR2, V_{H}-CDR3 within **SEQ ID NO: 193** and the V_{L}-CDR1, V_{L}-CDR2, V_{L}-CDR3 of the V_{L}-CDR1, V_{L}-CDR2, V_{L}-CDR3 within **SEQ ID NO: 232;** aa) the V_{H}-CDR1, V_{H}-CDR2, V_{H}-CDR3 of the V_{H}-CDR1, V_{H}-CDR2, V_{H}-CDR3 within **SEQ ID NO: 194** and the V_{L}-CDR1, V_{L}-CDR2, V_{L}-CDR3 of the V_{L}-CDR1, V_{L}-CDR2, V_{L}-CDR3 within **SEQ ID NO: 233;** ab) the V_{H}-CDR1, V_{H}-CDR2, V_{H}-CDR3 of the V_{H}-CDR1, V_{H}-CDR2, V_{H}-CDR3 within **SEQ ID NO: 195** and the V_{L}-CDR1, V_{L}-CDR2, V_{L}-CDR3 of the V_{L}-CDR1, V_{L}-CDR2, V_{L}-CDR3 within **SEQ ID NO: 234;** ac) the V_{H}-CDR1, V_{H}-CDR2, V_{H}-CDR3 of the V_{H}-CDR1, V_{H}-CDR2, V_{H}-CDR3 within **SEQ ID NO: 196** and the V_{L}-CDR1, V_{L}-CDR2, V_{L}-CDR3 of the V_{L}-CDR1, V_{L}-CDR2, V_{L}-CDR3 within **SEQ ID NO: 235;** ad) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 236;** ae) the V_{H}-CDR1, V_{H}-CDR2, V_{H}-CDR3 of the V_{H}-CDR1, V_{H}-CDR2, V_{H}-CDR3 within **SEQ ID NO: 198** and the V_{L}-CDR1, V_{L}-CDR2, V_{L}-CDR3 of the V_{L}-CDR1, V_{L}-CDR2, V_{L}-CDR3 within **SEQ ID NO: 237;** af) the V_{H}-CDR1, V_{H}-CDR2, V_{H}-CDR3 of the V_{H}-CDR1, V_{H}-CDR2, V_{H}-CDR3 within **SEQ ID NO: 199** and the V_{L}-CDR1, V_{L}-CDR2, V_{L}-CDR3 of the V_{L}-CDR1, V_{L}-CDR2, V_{L}-CDR3 within **SEQ ID NO: 238;** ag) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 239;** ah) the V_{H}-CDR1, V_{H}-CDR2, V_{H}-CDR3 of the V_{H}-CDR1, V_{H}-CDR2, V_{H}-CDR3 within **SEQ ID NO: 200** and the V_{L}-CDR1, V_{L}-CDR2, V_{L}-CDR3 of the V_{L}-CDR1, V_{L}-CDR2, V_{L}-CDR3 within **SEQ ID NO: 240;** ai) the V_{H}-CDR1, V_{H}-CDR2, V_{H}-CDR3 of the V_{H}-CDR1, V_{H}-CDR2, V_{H}-CDR3 within **SEQ ID NO: 201** and the V_{L}-CDR1, V_{L}-CDR2, V_{L}-CDR3 of the V_{L}-CDR1, V_{L}-CDR2, V_{L}-CDR3 within **SEQ ID NO: 241;** aj) the V_{H}-CDR1, V_{H}-CDR2, V_{H}-CDR3 of the V_{H}-CDR1, V_{H}-CDR2, V_{H}-CDR3 within **SEQ ID NO: 202** and the V_{L}-CDR1, V_{L}-CDR2, V_{L}-CDR3 of the V_{L}-CDR1, V_{L}-CDR2, V_{L}-CDR3 within **SEQ ID NO: 242;** ak) the V_{H}-CDR1, V_{H}-CDR2, V_{H}-CDR3 of the V_{H}-CDR1, V_{H}-CDR2, V_{H}-CDR3 within **SEQ ID NO: 203** and the V_{L}-CDR1, V_{L}-CDR2, V_{L}-CDR3 of the V_{L}-CDR1, V_{L}-CDR2, V_{L}-CDR3 within **SEQ ID NO: 240;** al) the V_{H}-CDR1, V_{H}-CDR2, V_{H}-CDR3 of the V_{H}-CDR1, V_{H}-CDR2, V_{H}-CDR3 within **SEQ ID NO: 204** and the V_{L}-CDR1, V_{L}-CDR2, V_{L}-CDR3 of the V_{L}-CDR1, V_{L}-CDR2, V_{L}-CDR3 within **SEQ ID NO: 243;** am) the V_{H}-CDR1, V_{H}-CDR2, V_{H}-CDR3 of the V_{H}-CDR1, V_{H}-CDR2, V_{H}-CDR3 within **SEQ ID NO: 205** and the V_{L}-CDR1, V_{L}-CDR2, V_{L}-CDR3 of the V_{L}-CDR1, V_{L}-CDR2, V_{L}-CDR3 within **SEQ ID NO: 244;** or an) the V_{H}-CDR1, V_{H}-CDR2, V_{H}-CDR3 of the V_{H}-CDR1, V_{H}-CDR2, V_{H}-CDR3 within **SEQ ID NO: 206** and the V_{L}-CDR1, V_{L}-CDR2, V_{L}-CDR3 of the V_{L}-CDR1, V_{L}-CDR2, V_{L}-CDR3 within **SEQ ID NO: 245.** In some embodiments, exemplary combinations of heavy chain variable region CDRs are depicted in **FIG. 34****.** In some embodiments, exemplary combinations of light chain variable region CDRs are depicted in **FIG. 35****.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibody or binding fragment thereof comprises: a) the heavy chain variable region of **SEQ ID NO: 169** and the light chain variable region of **SEQ ID NO: 207;** b) the heavy chain variable region of **SEQ ID NO: 170** and the light chain variable region of **SEQ ID NO: 208;** c) the heavy chain variable region of **SEQ ID NO: 171** and the light chain variable region of **SEQ ID NO: 209;** d) the heavy chain variable region of **SEQ ID NO: 172** and the light chain variable region of **SEQ ID NO: 210;** e) the heavy chain variable region of **SEQ ID NO: 173** and the light chain variable region of **SEQ ID NO: 211;** f) the heavy chain variable region of **SEQ ID NO: 174** and the light chain variable region of **SEQ ID NO: 212;** g) the heavy chain variable region of **SEQ ID NO: 175** and the light chain variable region of **SEQ ID NO: 213;** h) the heavy chain variable region of **SEQ ID NO: 176** and the light chain variable region of **SEQ ID NO: 214;** i) the heavy chain variable region of **SEQ ID NO: 177** and the light chain variable region of **SEQ ID NO: 215;** j) the heavy chain variable region of **SEQ ID NO: 178** and the light chain variable region of **SEQ ID NO: 216;** k) the heavy chain variable region of **SEQ ID NO: 179** and the light chain variable region of **SEQ ID NO: 217;** 1) the heavy chain variable region of **SEQ ID NO: 180** and the light chain variable region of **SEQ ID NO: 218;** m) the heavy chain variable region of **SEQ ID NO: 181** and the light chain variable region of **SEQ ID NO: 219;** n) the heavy chain variable region of **SEQ ID NO: 181** and the light chain variable region of **SEQ ID NO: 220;** o) the heavy chain variable region of **SEQ ID NO: 182** and the light chain variable region of **SEQ ID NO: 221;** p) the heavy chain variable region of **SEQ ID NO: 183** and the light chain variable region of **SEQ ID NO: 222;** q) the heavy chain variable region of **SEQ ID NO: 184** and the light chain variable region of **SEQ ID NO: 223;** r) the heavy chain variable region of **SEQ ID NO: 185** and the light chain variable region of **SEQ ID NO: 224;** s) the heavy chain variable region of **SEQ ID NO: 186** and the light chain variable region of **SEQ ID NO: 225;** t) the heavy chain variable region of **SEQ ID NO: 187** and the light chain variable region of **SEQ ID NO: 226;** u) the heavy chain variable region of **SEQ ID NO: 188** and the light chain variable region of **SEQ ID NO: 227;** v) the heavy chain variable region of **SEQ ID NO: 189** and the light chain variable region of **SEQ ID NO: 228;** w) the heavy chain variable region of **SEQ ID NO: 190** and the light chain variable region of **SEQ ID NO: 229;** x) the heavy chain variable region of **SEQ ID NO: 191** and the light chain variable region of **SEQ ID NO: 230;** y) the heavy chain variable region of **SEQ ID NO: 192** and the light chain variable region of **SEQ ID NO: 231;** z) the heavy chain variable region of **SEQ ID NO: 193** and the light chain variable region of **SEQ ID NO: 232;** aa) the heavy chain variable region of **SEQ ID NO: 194** and the light chain variable region of **SEQ ID NO: 233;** ab) the heavy chain variable region of **SEQ ID NO: 195** and the light chain variable region of **SEQ ID NO: 234;** ac) the heavy chain variable region of **SEQ ID NO: 196** and the light chain variable region of **SEQ ID NO: 235;** ad) the heavy chain variable region of **SEQ ID NO: 197** and the light chain variable region of **SEQ ID NO: 236;** ae) the heavy chain variable region of **SEQ ID NO: 198** and the light chain variable region of **SEQ ID NO: 237;** af) the heavy chain variable region of **SEQ ID NO: 199** and the light chain variable region of **SEQ ID NO: 238;** ag) the heavy chain variable region of **SEQ ID NO: 197** and the light chain variable region of **SEQ ID NO: 239;** ah) the heavy chain variable region of **SEQ ID NO: 200** and the light chain variable region of **SEQ ID NO: 240;** ai) the heavy chain variable region of **SEQ ID NO: 201** and the light chain variable region of **SEQ ID NO: 241;** aj) the heavy chain variable region of **SEQ ID NO: 202** and the light chain variable region of **SEQ ID NO: 242;** ak) the heavy chain variable region of **SEQ ID NO: 203** and the light chain variable region of **SEQ ID NO: 240;** al) the heavy chain variable region of **SEQ ID NO: 204** and the light chain variable region of **SEQ ID NO: 243;** am) the heavy chain variable region of **SEQ ID NO: 205** and the light chain variable region of **SEQ ID NO: 244;** or an) the heavy chain variable region of **SEQ ID NO: 206** and the light chain variable region of **SEQ ID NO: 245.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

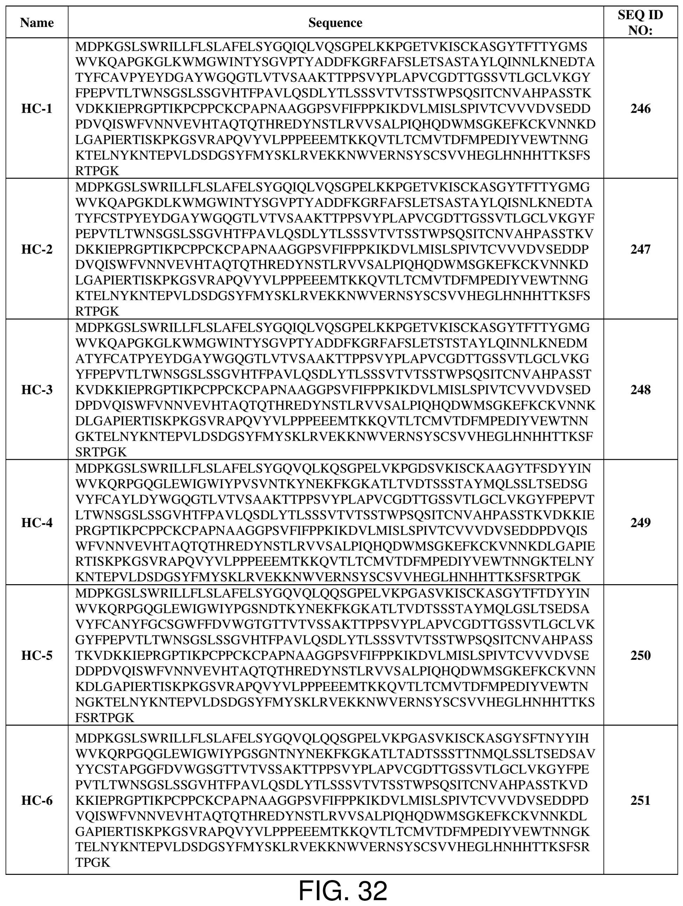

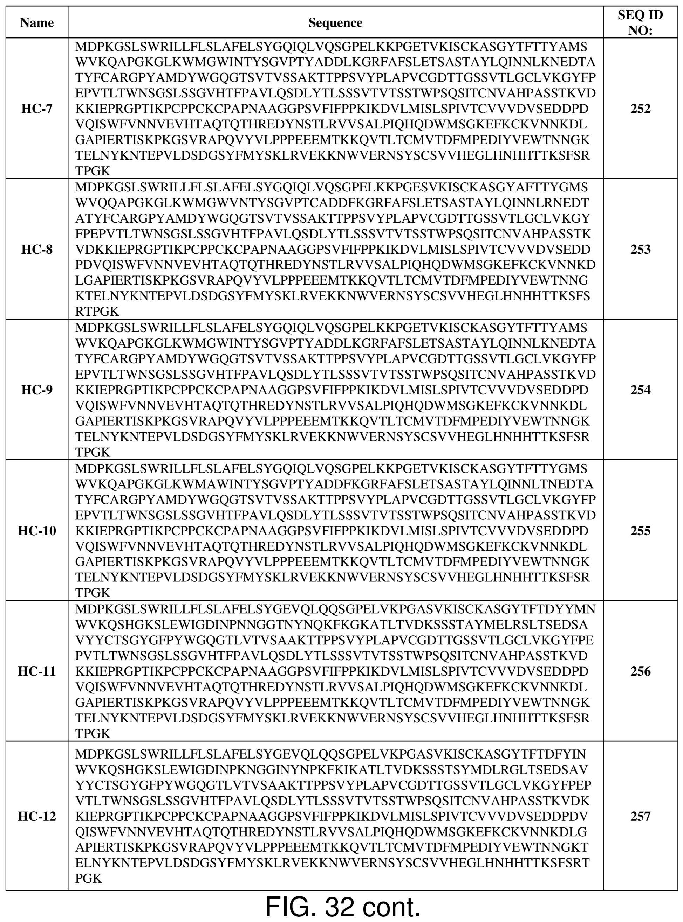

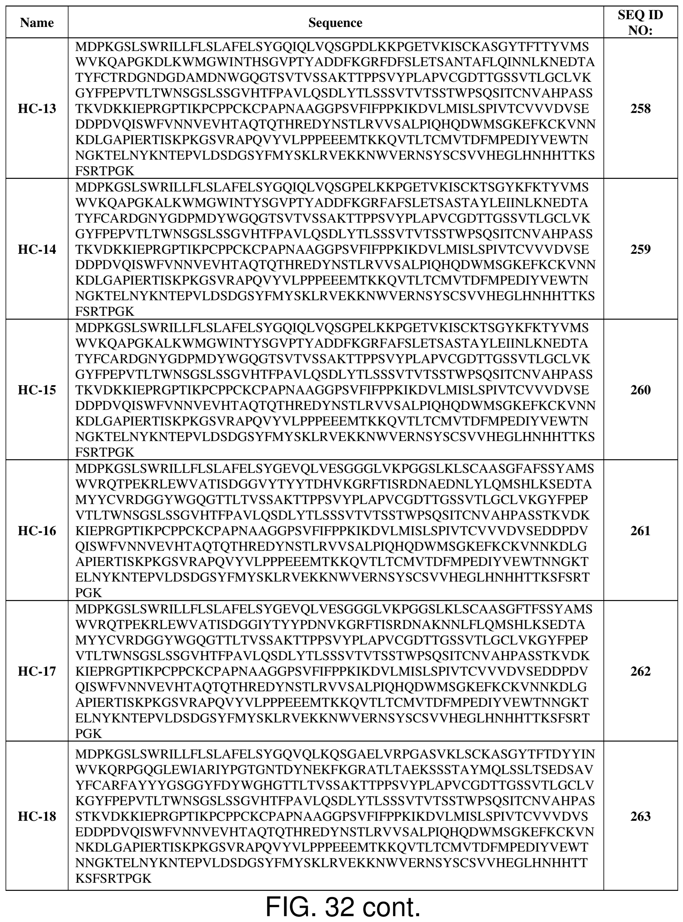

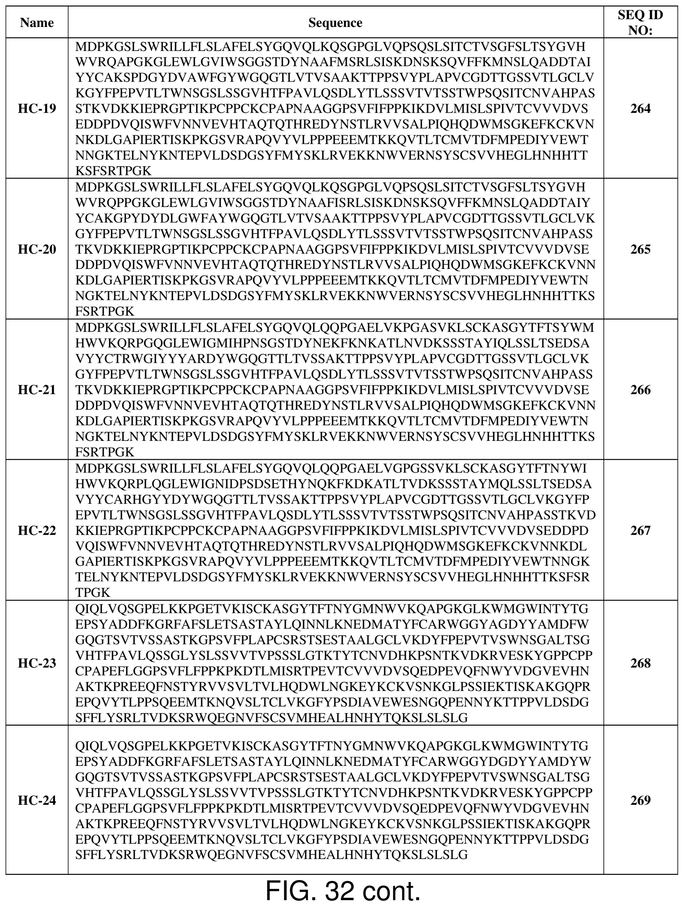

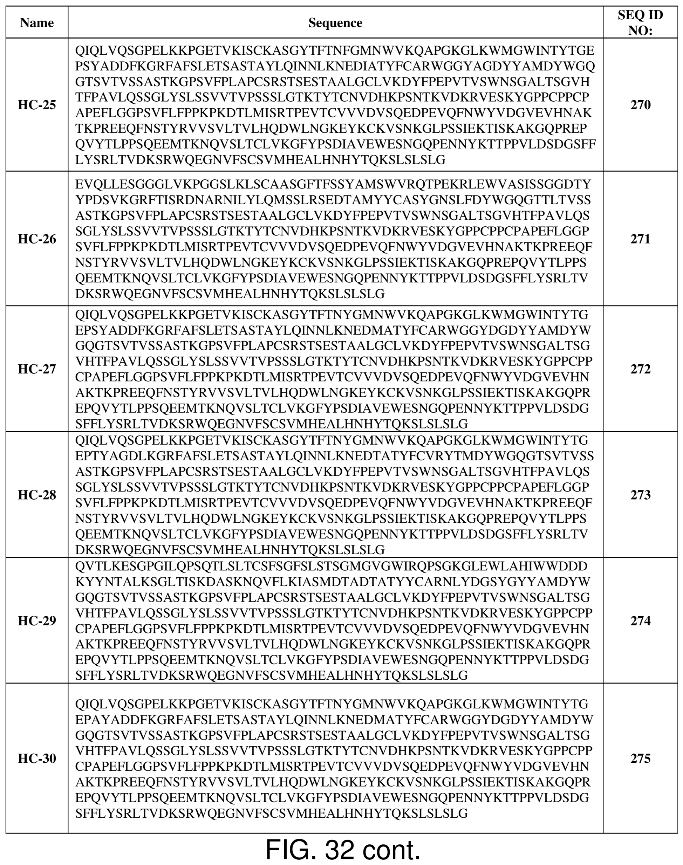

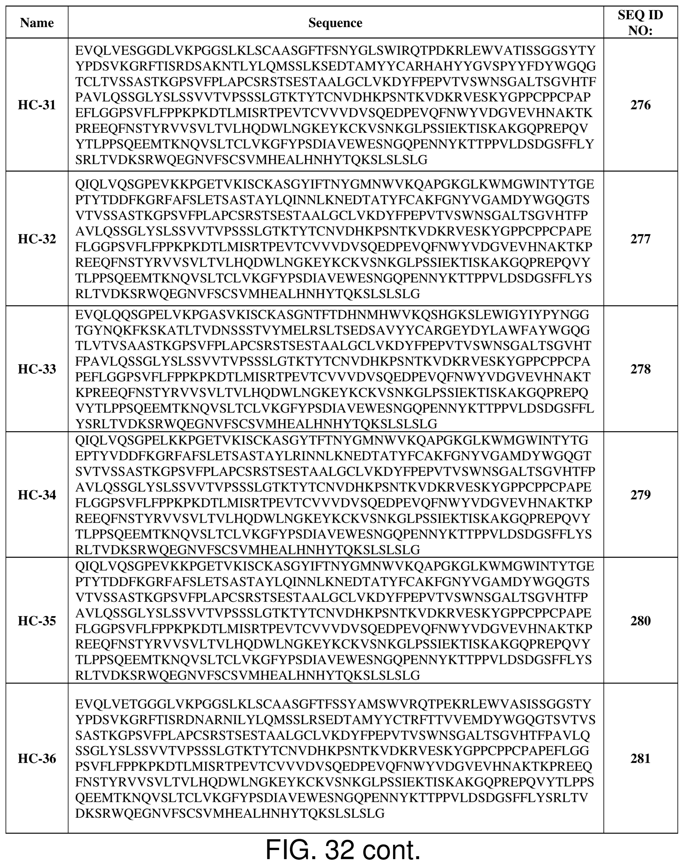

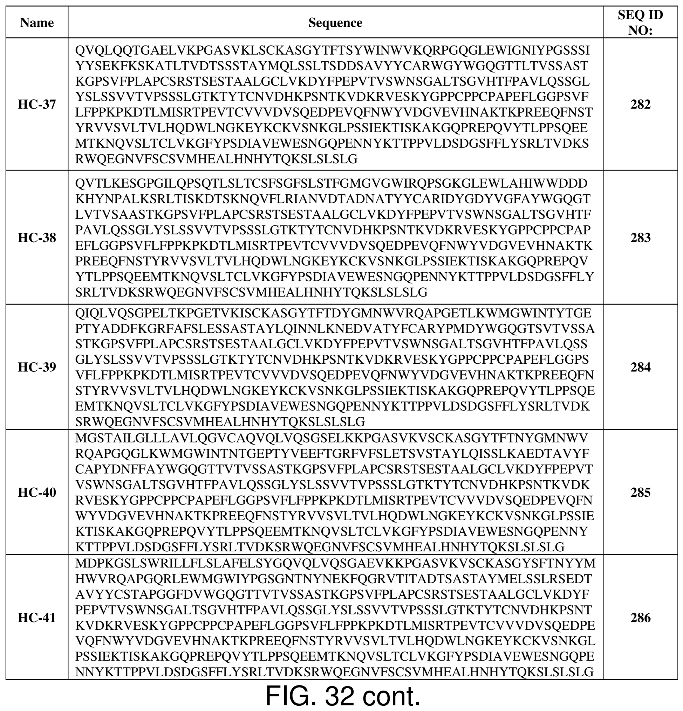

In some embodiments, the anti-Gal3 antibody or binding fragment thereof comprises the heavy chain (HC) sequence of any one of **SEQ ID NOs: 246-286.** In some embodiments, exemplary HC sequences are depicted in **FIG. 32****.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

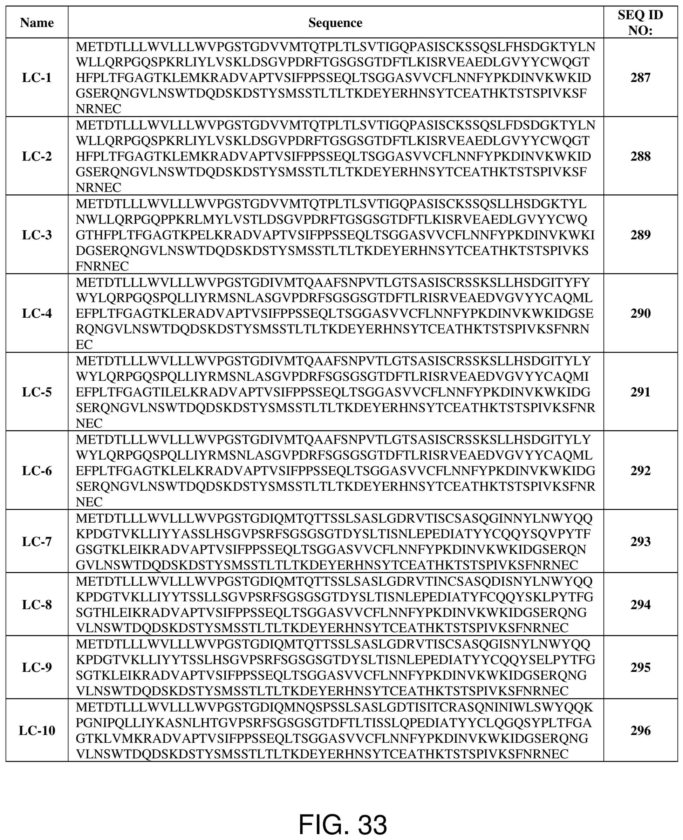

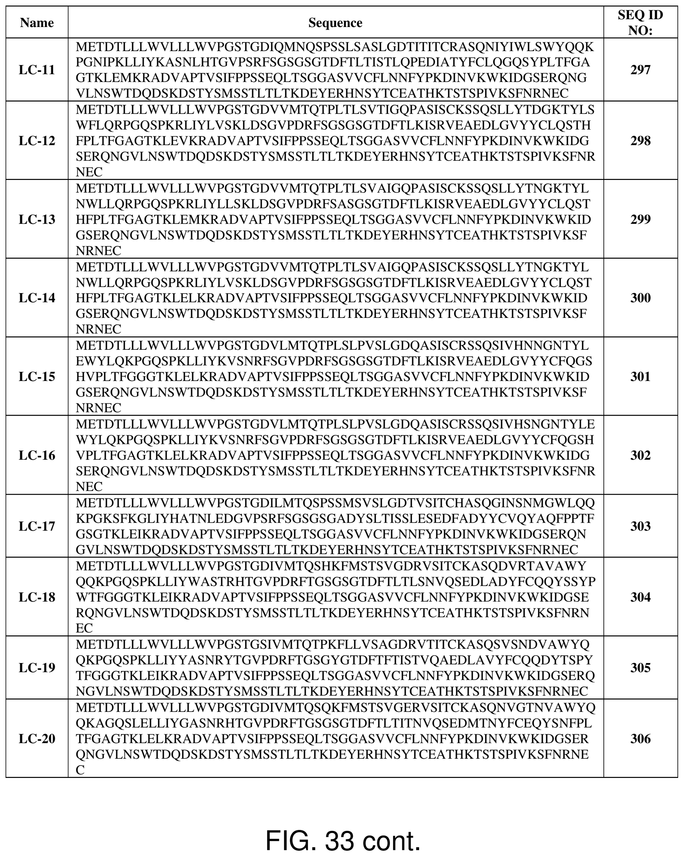

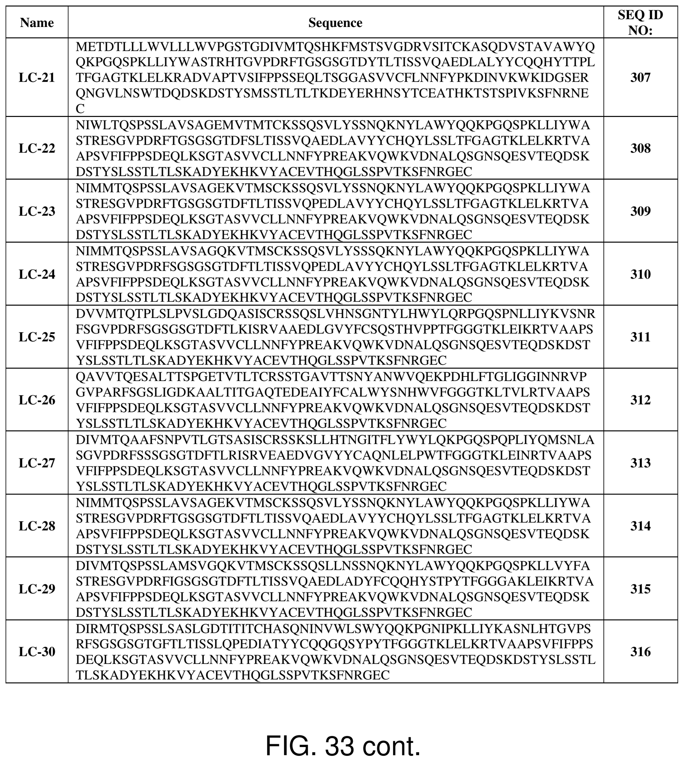

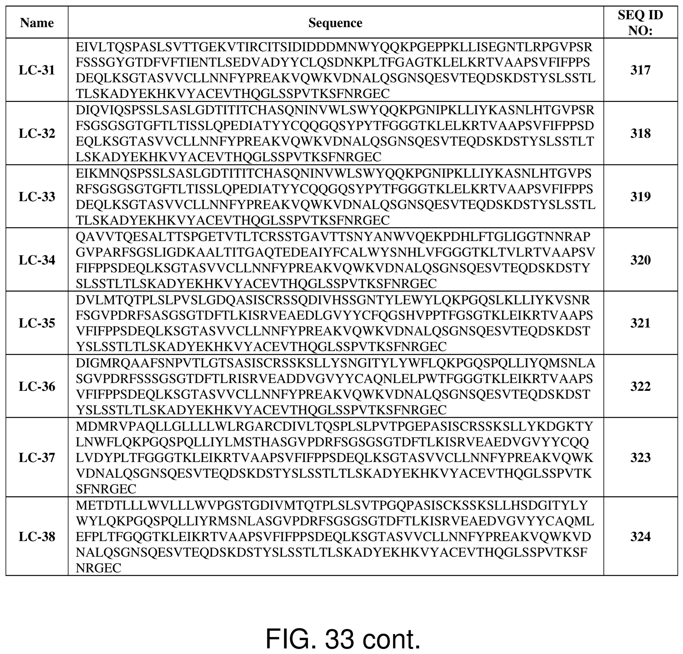

In some embodiments, the anti-Gal3 antibody or binding fragment thereof comprises the light chain (LC) sequence of any one of **SEQ ID NOs: 287-324.** In some embodiments, exemplary HC sequence are depicted in **FIG. 33****.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

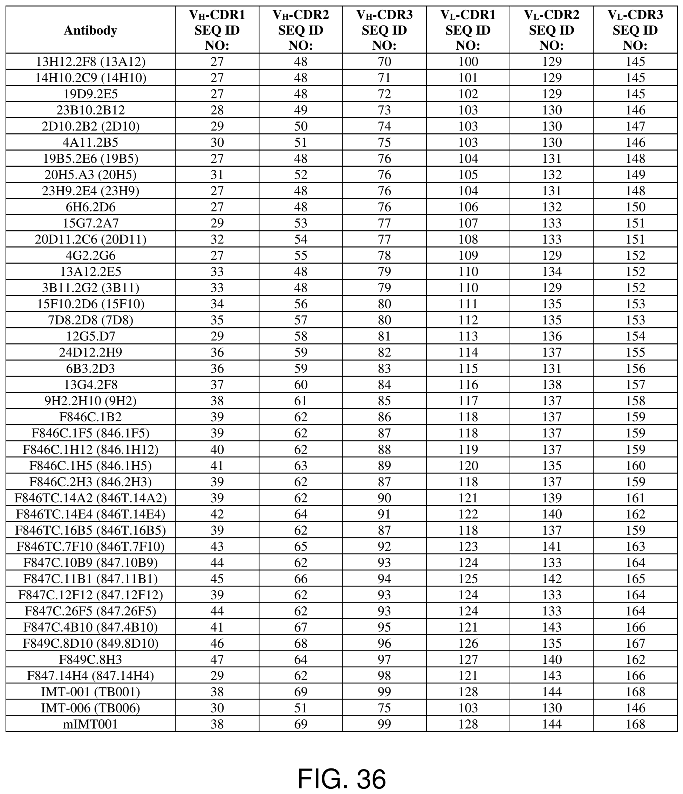

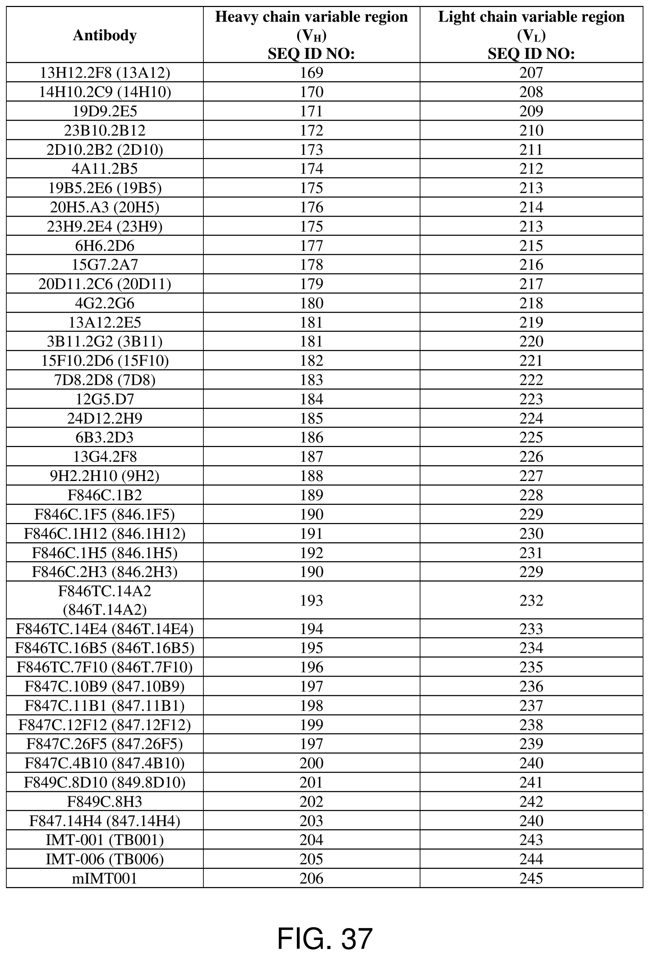

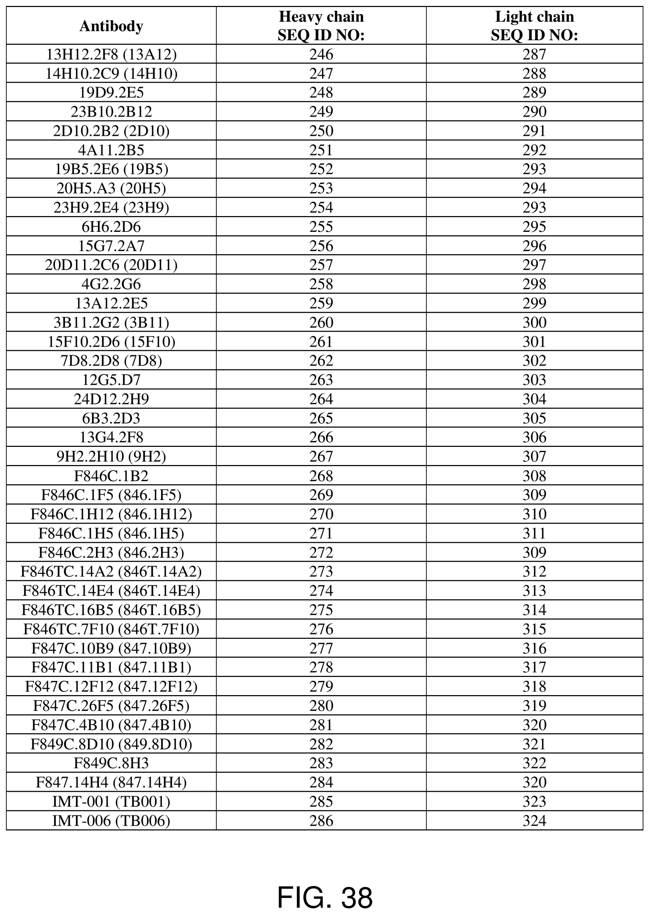

In some embodiments, the anti-Gal3 antibody or binding fragment thereof is selected from the group consisting of 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, F847.14H4 (847.14H4), mIMT001, IMT-001 (TB0001), and IMT-006 (TB006), or a binding fragment thereof. In some embodiments, the heavy and light chain CDRs associated with each of the foregoing antibodies are depicted in **FIG. 36****.** In some embodiments, the V_{H} and V_{L} associated with each of the foregoing antibodies are depicted in **FIG. 37****.** In some embodiments, the HC and LC associated with each of the foregoing antibodies are depicted in **FIG. 38****.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

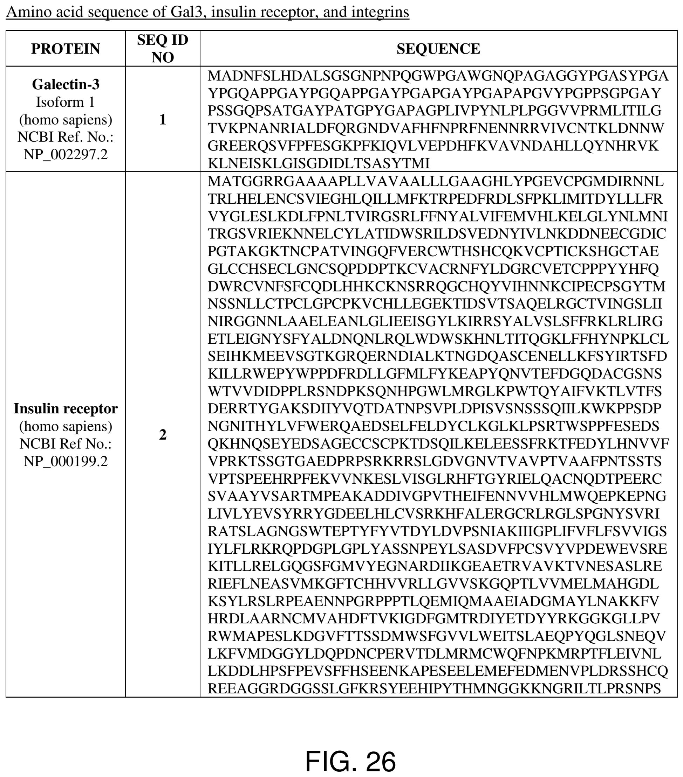

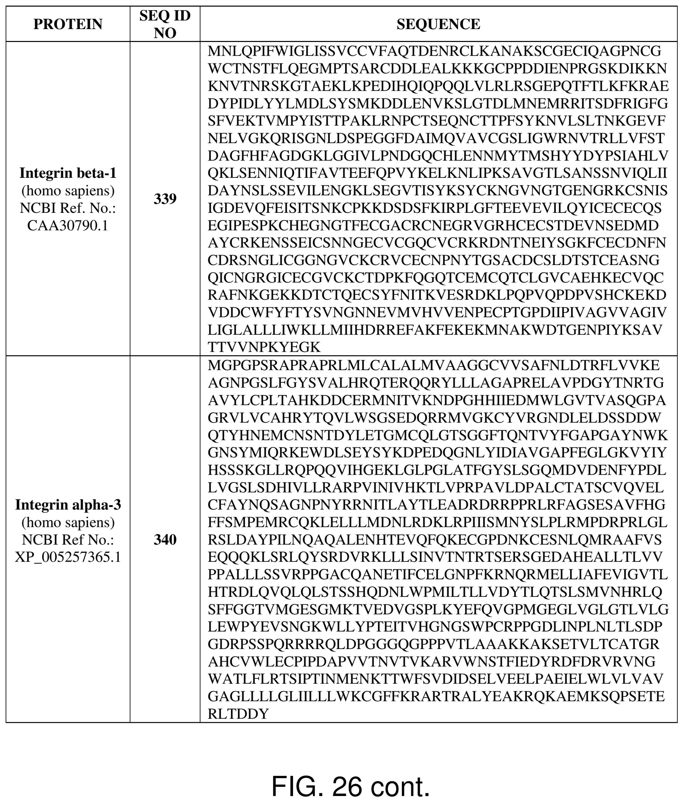

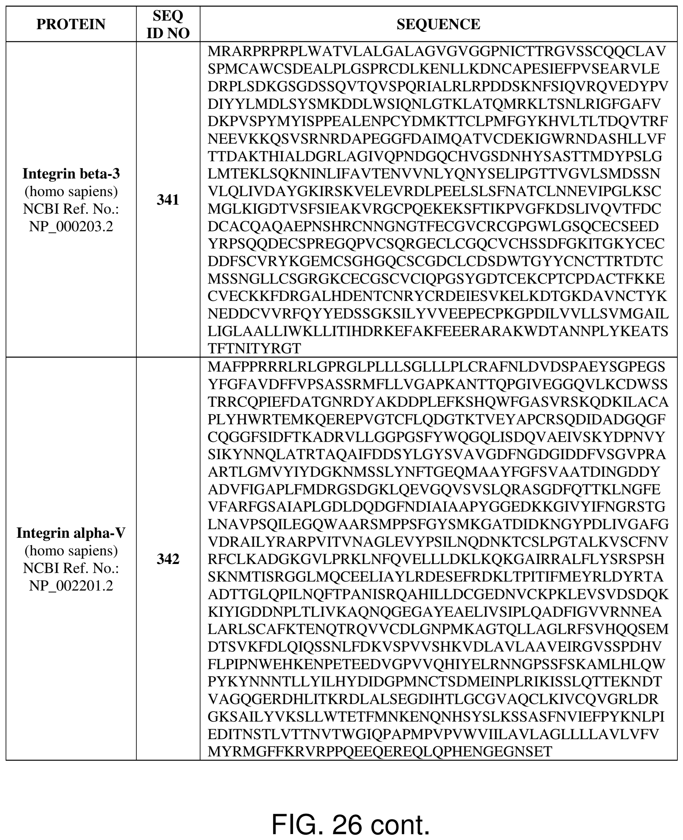

In some embodiments, the anti-Gal3 antibody or binding fragment thereof binds to specific epitopes within a Gal3 protein. In some embodiments, the anti-Gal3 antibody or binding fragment thereof binds to a specific epitope within a Gal3 protein having an amino acid sequence according to **SEQ ID NO: 1,** provided in **FIG. 26****.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

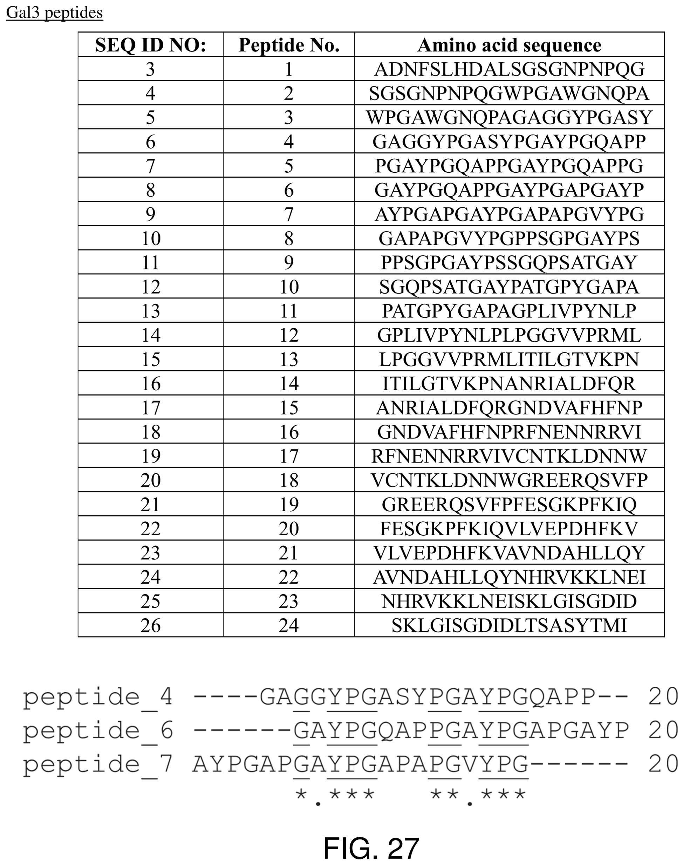

In some embodiments, the anti-Gal3 antibody or binding fragment thereof may bind to at least 1, 2, 3, 4, 5, 6, 10, 15, or 20 amino acid residues within a peptide illustrated in **FIG. 27****.** In some embodiments, the anti-Gal3 antibody or binding fragment thereof may bind to at least 1, 2, 3, 4, 5, 6, 10, 15, or 20 amino acid residues within amino acid residues 1-20 of **SEQ ID NO: 1.** In some embodiments, the anti-Gal3 antibody or binding fragment thereof may bind to at least 1, 2, 3, 4, 5, 6, 10, 15, or 20 amino acid residues within amino acid residues 31-50 of **SEQ ID NO: 1.** In some embodiments, the anti-Gal3 antibody or binding fragment thereof may bind to at least 1, 2, 3, 4, 5, 6, 10, 15, or 20 amino acid residues within amino acid residues 51-70 of **SEQ ID NO: 1.** In some embodiments, the anti-Gal3 antibody or binding fragment thereof may bind to at least 1, 2, 3, 4, 5, 6, 10, 15, or 20 amino acid residues within amino acid residues 61-80 of **SEQ ID NO: 1.** In some embodiments, any of the anti-Gal3 antibodies or binding fragments thereof or any arrangement of any of the anti-Gal3 antibodies or binding fragments provided herein may be substituted with an antigen binding molecule that binds to Gal3.

In some embodiments, the anti-Gal3 antibody or binding fragment thereof may bind to at least 1, 2, 3, 4, 5, 6, 7, 8, 9, 10, 11, 12, 13, 14, 15, 16, 17, 18, 19, or 20 amino acid residues within Peptide 1 **(SEQ ID NO: 3),** Peptide 2 **(SEQ ID NO: 4),** Peptide 3 **(SEQ ID NO: 5),** Peptide 4 **(SEQ ID NO: 6),** Peptide 5 **(SEQ ID NO: 7),** Peptide 6 **(SEQ ID NO: 8),** Peptide 7 **(SEQ ID NO: 9),** Peptide 8 **(SEQ ID NO: 10),** or Peptide 17 **(SEQ ID NO: 19)** or any combination thereof. In some embodiments, the anti-Gal3 or binding fragment thereof may bind to at least 1, 2, 3, 4, 5, 6, 7, 8, 9, 10, 11, 12, 13, 14, 15, 16, 17, 18, 19, or 20 amino acid residues within Peptide 6 **(SEQ ID NO: 8).** In some embodiments, any of the anti-Gal3 antibodies or binding fragments thereof or any arrangement of any of the anti-Gal3 antibodies or binding fragments provided herein may be substituted with an antigen binding molecule that binds to Gal3.

Some exemplary antibodies that bind to Peptide 1 **(SEQ ID NO: 3)** are 6H6.2D6, 20H5.A3, 20D11.2C6, 19B5.2E6, 15G7.2A7, 23H9.2E4, F846C.1H5, F846TC.14A2, F846TC.7F10, F847C.10B9, F847C.12F12, F847C.26F5, and F847C.4B10.

Some exemplary antibodies that bind to Peptide 2 **(SEQ ID NO: 4)** are 15F10.2D6, 7D8.2D8, F846TC.14E4, F849C.8D10, and F849C.8H3.

Some exemplary antibodies that bind to Peptide 3 **(SEQ ID NO: 5)** are 15F10.2D6, 7D8.2D8, and F849C.8D10.

Some exemplary antibodies that bind to Peptide 4 **(SEQ ID NO: 6)** are 4G2.2G6, 3B11.2G2, 13A12.2E5 and 15F10.2D6.

Some exemplary antibodies that bind to Peptide 5 **(SEQ ID NO: 7)** are IMT001 (TB001), F846C.1B2 and F846C.1H12.

Some exemplary antibodies that bind to Peptide 6 **(SEQ ID NO: 8)** are IMT001 (TB001), IMT006 (TB006), 13H12.2F8, 19D9.2E5, 2D10.2B2, 4A11.2B5, 3B11.2D2, 13A12.2E5, 14H10.2C9, 23H9.2E4, F846C.1B2, F846C.1F5, F846C.1H12, F846C.1H12, F846C.2H3, and F846TC.16B5.

Some exemplary antibodies that bind to Peptide 7 **(SEQ ID NO: 9)** are 6H6.2D6, 20H5.A3, 20D11.2C6, 13H12.2F8, 19B5.2E6, 23H9.2E4, 15G7.2A7, 19D9.2E5, 14H10.2C9, 7D8.2D8, 15F10.2D6, 14H10.2C9, F846C.1B2, F846TC.14A2, F847C.10B9, F847C.12F12, and F847C.26F5.

Some exemplary antibodies that bind to Peptide 8 **(SEQ ID NO: 10)** are 23H9.2E4 and F846TC.14A2.

Some exemplary antibodies that bind to Peptide 17 **(SEQ ID NO: 19)** are 7D8.2D8, F846C.1F5, F846C.1H12, F846TC.16B5, F847C.11B1, and F849C.8H3.

In some embodiments, the anti-Gal3 antibody or binding fragment thereof are epitope binned. Exemplary binning processes are detailed in Examples 3 and 18. In some embodiments, antibody TB001 is categorized into bin 1. In some embodiments, antibody 19D9.2E5 is categorized into bin 2. In some embodiments, antibodies TB006, 4A11.2B5, 23B10.2B12, 19B5.2E6, 20H5.A3, 23H9.2E4, and 2D10.2B2 are categorized into bin 3. In some embodiments, antibody 6H6.2D6 is categorized into bin 4. In some embodiments, antibodies 15G7.2A7, 20D11.2C6 is categorized into bin 5. In some embodiments, antibody 4G2.2G6 is categorized into bin 6. In some embodiments, antibodies 13A12.2E5 and 3B11.2G2 are categorized into bin 7. In some embodiments, antibodies 14H10.2C9, 15F10.2D6, 7D8.2D8, F846TC.14E4, F846TC.7F10, and F849C.8D10 are categorized into bin 8. In some embodiments, antibody 6F7.C4 is categorized into bin 9. In some embodiments, antibody 12G5.D7 is categorized into bin 10. In some embodiments, antibody 6B3.2D3 is categorized into bin 11. In some embodiments, antibodies 9H2.2H10 and 13G4.2F8 are categorized into bin 12. In some embodiments, antibodies F846C.1B2, F846C.1F5, F846C.1H12, F846C.2H3, and F846TC.16B5 are categorized into bin 17. In some embodiments, antibody F847C.10B9, F847C.12F12, and F847C.26F5 are categorized into bin 49.

Also disclosed herein in some embodiments are antibodies that bind to human Gal3 and competes with an anti-Gal3 antibody or binding fragment thereof for binding to human Gal3. In some embodiments, the anti-Gal3 antibody or binding fragment thereof that is competed against is any one of the anti-Gal3 antibodies disclosed herein. In some embodiments, the anti-Gal3 antibody or binding fragment thereof that is competed against binds to Peptide 1 **(SEQ ID NO: 3),** Peptide 2 **(SEQ ID NO: 4),** Peptide 3 **(SEQ ID NO: 5),** Peptide 4 **(SEQ ID NO: 6),** Peptide 5 **(SEQ ID NO: 7),** Peptide 6 **(SEQ ID NO: 8),** Peptide 7 **(SEQ ID NO: 9),** Peptide 8 **(SEQ ID NO: 10),** Peptide 17 **(SEQ ID NO: 19),** or any combination thereof. In some embodiments, the anti-Gal3 antibody or binding fragment thereof that is competed against belongs to bin 1, 2, 3, 4, 5, 6, 7, 8, 9, 10, 11, 12, 17, or 49. In some embodiments, the anti-Gal3 antibody or binding fragment thereof that is competed against is selected from the group consisting of: 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, 7D8.2D8, and 15F10.2D6, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT001(TB001), and IMT006 (TB006), or a binding fragment thereof. In some embodiments, the antibody that binds to human Gal3 and competes with an anti-Gal3 antibody may be substituted with an antigen binding molecule that binds to Gal3. In some embodiments, the anti-Gal3 antibody or binding fragment thereof that is competed against may be substituted with an antigen binding molecule that binds to Gal3.

In some instances, the anti-Gal3 antibody or binding fragment thereof comprises a humanized antibody or binding fragment thereof. In other instances, the anti-Gal3 antibody or binding fragment thereof comprises a chimeric antibody or binding fragment thereof. In some cases, the anti-Gal3 antibody comprises a full-length antibody or a binding fragment thereof. In some cases, the anti-Gal3 antibody or binding fragment thereof comprises a bispecific antibody or a binding fragment thereof. In some cases, the anti-Gal3 antibody or binding fragment thereof comprises a monovalent Fab', a divalent Fab2, a single-chain variable fragment (scFv), a diabody, a minibody, a nanobody, a single-domain antibody (sdAb), or a camelid antibody or binding fragment thereof.

In some instances, the anti-Gal3 antibody or binding fragment thereof is a bispecific antibody or binding fragment thereof. Exemplary bispecific antibody formats include, but are not limited to, Knobs-into-Holes (KiH), Asymmetric Re-engineering Technology-immunoglobulin (ART-Ig), Triomab quadroma, bispecific monoclonal antibody (BiMAb, BsmAb, BsAb, bsMab, BS-Mab, or Bi-MAb), Azymetric, Biclonics, Fab-scFv-Fc, Two-in-one/Dual Action Fab (DAF), FinomAb, scFv-Fc-(Fab)-fusion, Dock-aNd-Lock (DNL), Tandem diAbody (TandAb), Dual-affinity-ReTargeting (DART), nanobody, triplebody, tandems scFv (taFv), triple heads, tandem dAb/VHH, triple dAb/VHH, or tetravalent dAb/VHH. In some cases, the anti-Gal3 antibody or binding fragment thereof is a bispecific antibody or binding fragment thereof comprising a bispecific antibody format illustrated in Brinkmann and Kontermann, "The making of bispecific antibodies," MABS 9(2): 182-212 (2017).

In some embodiments, the anti-Gal3 antibody or binding fragment thereof can comprise an IgM, IgG (e.g., IgG1, IgG2, IgG3, or IgG4), IgA, or IgE framework. The IgG framework can be IgG1, IgG2, IgG3 or IgG4. In some cases, the anti-Gal3 antibody or binding fragment thereof comprises an IgG1 framework. In some cases, the anti-Gal3 antibody or binding fragment thereof comprises an IgG2 framework. In some cases, the anti-Gal3 antibody or binding fragment thereof comprises an IgG4 framework. The anti-Gal3 antibody or binding fragment thereof can further comprise a Fc mutation.

In some embodiments, the Fc region comprises one or more mutations that modulate Fc receptor interactions, e.g., to enhance effector functions such as ADCC and/or CDC. In such instances, exemplary residues when mutated modulate effector functions include S239, K326, A330, 1332, or E333, in which the residue position correspond to IgG1 and the residue numbering is in accordance to Kabat numbering (EU index of Kabat et al 1991 Sequences of Proteins of Immunological Interest). In some instances, the one or more mutations comprise S239D, K326W, A330L, I332E, E333A, E333S, or a combination thereof. In some cases, the one or more mutations comprise S239D, I332E, or a combination thereof. In some cases, the one or more mutations comprise S239D, A330L, I332E, or a combination thereof. In some cases, the one or more mutations comprise K326W, E333S, or a combination thereof. In some cases, the mutation comprises E333A.

### Methods of Use

Disclosed herein in some embodiments are methods that involve an anti-Gal3 antibody or binding fragment thereof that binds to Gal3 and disrupts an interaction between Gal3 and another protein. In some embodiments, this can be a direct obstruction of the interaction zone between Gal3 and the other protein, or an indirect alteration, such as a binding that results in a conformational change of Gal3, so that it no longer binds or is active with the other protein. In some embodiments, it can also result by binding to a first section of Gal3, where some other part of the antibody or binding fragment thereof obstructs or alters the interaction between Gal3 and the other protein. In some embodiments, the first section of Gal3 is the N-terminal domain of Gal3, the tandem repeat domain (TRD) of Gal3, or the C-terminal domain of Gal3. In some embodiments, the antibody or binding fragment thereof that binds to Gal3 does not bind to the C-terminal domain of Gal3. In some embodiments, any of the methods disclosed herein involving an anti-Gal3 antibody or binding fragment can be performed with an antigen binding molecule that binds to Gal3.

Disclosed herein are methods of disrupting an interaction between Gal3 and insulin receptor or an integrin, or both. In some embodiments, the methods comprise contacting an interaction site or surface on Gal3 (where Gal3 interacts with a) the insulin receptor or b) the integrin, or c) both) with an anti-Gal3 antibody or binding fragment thereof that selectively binds to Gal3 and disrupts the interaction between Gal3 and the insulin receptor or the integrin, or both. In some embodiments, the Gal3 is expressed by a cell or a plurality of cells. In some embodiments, the Gal3 is secreted by a cell or a plurality of cells. In some embodiments, the insulin receptor or the integrin, or both, is expressed by a cell or a plurality of cells. In some instances, the method comprises contacting a plurality of cells expressing Gal3 and a plurality of cells expressing insulin receptor or an integrin, or both, with an antibody or binding fragment thereof of the disclosure. In some instances, the method comprises contacting Gal3 secreted by a plurality of cells and a plurality of cells expressing insulin receptor or integrin, or both, with an antibody or binding fragment thereof of the disclosure. In some embodiments, the anti-Gal3 antibody or binding fragment thereof binds to an N-terminal domain of Gal3. In some embodiments, the interaction is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the anti-Gal3 antibody or binding fragment thereof. In some embodiments, the anti-Gal3 antibody or binding fragment thereof is any one of the anti-Gal3 antibodies or binding fragments thereof disclosed herein. In some embodiments, any of the methods disclosed herein involving an anti-Gal3 antibody or binding fragment can be performed with an antigen binding molecule that binds to Gal3.

Also disclosed herein in some embodiments are methods of diagnosing a disease or disorder in a subject, or a symptom thereof. In some embodiments, the disease or disorder is diabetes mellitus. In some embodiments, the disease or disorder is insulin-dependent diabetes mellitus. In some embodiments, the disease or disorder is insulin-independent diabetes mellitus. In some embodiments, the disease or disorder is Type I diabetes mellitus. In some embodiments, the disease or disorder is Type II diabetes mellitus. In some embodiments, the disease or disorder is inflammatory bowel disease. In some embodiments, the disease or disorder is non-alcoholic fatty liver disease. In some embodiments, the disease or disorder is non-alcoholic steatohepatitis. In some embodiments, the methods comprise contacting the subject, or a part of the subject (e.g. tissue, blood/serum) with an anti-Gal3 antibody or binding fragment thereof. In some embodiments, the anti-Gal3 antibody or binding fragment thereof is conjugated to a detectable moiety. In some embodiments, any of the methods disclosed herein involving an anti-Gal3 antibody or binding fragment can be performed with an antigen binding molecule that binds to Gal3.

In some embodiments of any of the methods disclosed herein, the insulin receptor comprises the sequence of **SEQ ID NO: 2.** In some embodiments, the Gal3-INSR interaction can be reduced to less than 80%, less than 75%, less than 70%, less than 60%, less than 59%, less than 50%, less than 40%, less than 34%, less than 30%, less than 20%, less than 14%, less than 10%, less than 7%, less than 5%, less than 4%, or less than 1%.

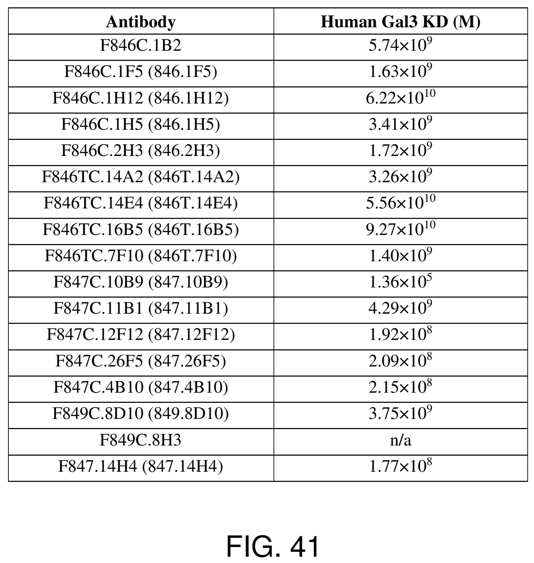

In some instances, the anti-Gal3 antibody binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM. In some instances, the anti-Gal3 antibody binds to Gal3 with a KD of less than 1 nM. In some instances, the anti-Gal3 antibody binds to Gal3 with a KD of less than 1.2 nM. In some instances, the anti-Gal3 antibody binds to Gal3 with a KD of less than 2 nM. In some instances, the anti-Gal3 antibody binds to Gal3 with a KD of less than 5 nM. In some instances, the anti-Gal3 antibody binds to Gal3 with a KD of less than 10 nM. In some instances, the anti-Gal3 antibody binds to Gal3 with a KD of less than 13.5 nM. In some instances, the anti-Gal3 antibody binds to Gal3 with a KD of less than 15 nM. In some instances, the anti-Gal3 antibody binds to Gal3 with a KD of less than 20 nM. In some instances, the anti-Gal3 antibody binds to Gal3 with a KD of less than 25 nM. In some instances, the anti-Gal3 antibody binds to Gal3 with a KD of less than 30 nM. KD values of Gal3 binding of exemplary anti-Gal3 antibodies are provided in **FIG. 41****.** In some embodiments, any of the methods disclosed herein involving an anti-Gal3 antibody or binding fragment can be performed with an antigen binding molecule that binds to Gal3.

In some embodiments of any of the methods disclosed herein, the integrin is selected from integrin alpha-1 (ITGa1), integrin alpha-2 (ITGa2), integrin alpha-3 (ITGa3), integrin alpha-4 (ITGa4), integrin alpha-5 (ITGa5), integrin alpha-6 (ITGa6), integrin alpha-7 (ITGa7), integrin alpha-8 (ITGa8), integrin alpha-9 (ITGa9), integrin alpha-10 (ITGa10), integrin alpha-11 (ITGa11), integrin alpha-D (ITGaD), integrin alpha-E (ITGaE), integrin alpha-L (ITGaL), integrin alpha-M (ITGaM), integrin alpha-V (ITGaV), integrin alpha-2B (ITGa2B), integrin alpha-2X (ITGa2X), integrin beta-1 (ITGb1), integrin beta-2 (ITGb2), integrin beta-3 (ITGb3), integrin beta-4 (ITGb4), integrin beta-5 (ITGb5), integrin beta-6 (ITGb6), integrin beta-7 (ITGb7), integrin beta-8 (ITGb8), or any combination thereof. In some embodiments, the integrin is selected from integrin beta-1, integrin alpha-3, integrin beta-3, or integrin alpha-V, or any combination thereof. In some embodiments, the integrin comprises the sequence of **SEQ ID NOs: 339-342.** In some embodiments, disrupting the interaction between Gal3 and the integrin reduces lymphocyte adhesion. In some embodiments, the lymphocyte adhesion is reduced by at least 50%, 55%, 60%, 65%, 70%, 75%, 80%, 85%, 90%, 95%, 96%, 97%, 98%, or 99%, or any percentage within a range defined by any two aforementioned percentages.

As applied to any of the methods of use disclosed herein, in some embodiments, the anti-Gal3 antibody or binding fragment thereof binds to one or more peptides **of SEQ ID NOs: 3-26.** In some embodiments, the anti-Gal3 antibody or binding fragment thereof binds to an epitope present within a region of Gal3 defined by Peptide 1 (ADNFSLHDALSGSGNPNPQG; **SEQ ID NO: 3),** Peptide 4 (GAGGYPGASYPGAYPGQAPP; **SEQ ID NO: 6),** Peptide 6 (GAYPGQAPPGAYPGAPGAYP; **SEQ ID NO: 8),** Peptide 7 (AYPGAPGAYPGAPAPGVYPG; **SEQ ID NO: 9),** or a combination thereof. In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof binds to an epitope of Gal3 that includes a motif of GxYPG, where x is the amino acids alanine (A), glycine (G), or valine (V). In some embodiments, an anti-Gal3 antibody as described herein binds to an epitope of Gal3 that includes two GxYPG motifs separated by three amino acids, where x is A, G, or V. In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof comprises (1) a light chain variable region comprising a V_{L}-CDR1, a V_{L}-CDR2, and a V_{L}-CDR3 and (2) a heavy chain variable region comprising a V_{H}-CDR1, a V_{H}-CDR2, and a V_{H}-CDR3. In some embodiments, the V_{L}-CDR1 comprises an amino acid sequence having at least 60%, at least 70%, at least 80%, at least 90%, or 100% sequence identity to any amino acid sequence according to **SEQ ID NOs: 100-128.** In some embodiments, the V_{L}-CDR2 comprises an amino acid sequence having at least 60%, at least 70%, at least 80%, at least 90%, or 100% sequence identity to any amino acid sequence according to **SEQ ID NOs: 129-144.** In some embodiments, the V_{L}-CDR3 comprises an amino acid sequence having at least 60%, at least 70%, at least 80%, at least 90%, or 100% sequence identity to any amino acid sequence according to **SEQ ID NOs: 145-168.** In some embodiments, the V_{H}-CDR1 comprises an amino acid sequence having at least 60%, at least 70%, at least 80%, at least 90%, or 100% sequence identity to any amino acid sequence according to **SEQ ID NOs: 27-47.** In some embodiments, the V_{H}-CDR2 comprises an amino acid sequence having at least 60%, at least 70%, at least 80%, at least 90%, or 100% sequence identity to any amino acid sequence according to **SEQ ID NOs: 48-69.** In some embodiments, the V_{H}-CDR3 comprises an amino acid sequence having at least 60%, at least 70%, at least 80%, at least 90%, or 100% sequence identity to any amino acid sequence according to **SEQ ID NOs: 70-99.** In some embodiments, any of the methods disclosed herein involving an anti-Gal3 antibody or binding fragment can be performed with an antigen binding molecule that binds to Gal3.

As applied to any of the methods of use disclosed herein, in some embodiments, exemplary V_{H}-CDR1 sequences are depicted in FIG. 28A. In some embodiments, exemplary V_{H}-CDR2 sequences are depicted in FIG. 28B. In some embodiments, exemplary V_{H}-CDR3 sequences are depicted in FIG. 28C. In some embodiments, exemplary V_{L}-CDR1 sequences are depicted in FIG. 29A. In some embodiments, exemplary V_{L}-CDR2 sequences are depicted in FIG. 29B. In some embodiments, exemplary V_{L}-CDR3 sequences are depicted in FIG. 29C.

As applied to any of the methods of use disclosed herein, in some embodiments, the heavy chain variable region of any of the anti-Gal3 antibodies or binding fragments thereof disclosed herein comprises an amino acid sequence having at least 75%, 80%, 85%, 90%, 95%, or 100% sequence identity to any sequence according to **SEQ ID NOs: 169-206.** In some embodiments, the heavy chain variable region of any of the anti-Gal3 antibodies or binding fragments thereof disclosed herein is selected from the group consisting of **SEQ ID NOs: 169-206.** In some embodiments, exemplary V_{H} are depicted in **FIG. 30****.** In some embodiments, any of the methods disclosed herein involving an anti-Gal3 antibody or binding fragment can be performed with an antigen binding molecule that binds to Gal3.

As applied to any of the methods of use disclosed herein, in some embodiments, the light chain variable region of any of the anti-Gal3 antibodies or binding fragments thereof disclosed herein comprises an amino acid sequence having at least 75%, 80%, 85%, 90%, 95%, or 100% sequence identity to any sequence according to **SEQ ID NOs: 207-245.** In some embodiments, the light chain variable region of any of the anti-Gal3 antibodies or binding fragments thereof disclosed herein is selected from the group consisting of **SEQ ID NOs: 207-245.** In some embodiments, exemplary V_{L} are depicted in **FIG. 31****.** In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof comprises: a) the V_{H}-CDR1, V_{H}-CDR2, V_{H}-CDR3 of the V_{H}-CDR1, V_{H}-CDR2, V_{H}-CDR3 within **SEQ ID NO: 169** and the V_{L}-CDR1, V_{L}-CDR2, V_{L}-CDR3 of the V_{L}-CDR1, V_{L}-CDR2, V_{L}-CDR3 within **SEQ ID NO: 207;** b) the V_{H}-CDR1, V_{H}-CDR2, V_{H}-CDR3 of the V_{H}-CDR1, V_{H}-CDR2, V_{H}-CDR3 within **SEQ ID NO: 170** and the V_{L}-CDR1, V_{L}-CDR2, V_{L}-CDR3 of the V_{L}-CDR1, V_{L}-CDR2, V_{L}-CDR3 within **SEQ ID NO: 208;** c) the V_{H}-CDR1, V_{H}-CDR2, V_{H}-CDR3 of the V_{H}-CDR1, V_{H}-CDR2, V_{H}-CDR3 within **SEQ ID NO: 171** and the V_{L}-CDR1, V_{L}-CDR2, V_{L}-CDR3 of the V_{L}-CDR1, V_{L}-CDR2, V_{L}-CDR3 within **SEQ ID NO: 209;** d) the V_{H}-CDR1, V_{H}-CDR2, V_{H}-CDR3 of the V_{H}-CDR1, V_{H}-CDR2, V_{H}-CDR3 within **SEQ ID NO: 172** and the V_{L}-CDR1, V_{L}-CDR2, V_{L}-CDR3 of the V_{L}-CDR1, V_{L}-CDR2, V_{L}-CDR3 within **SEQ ID NO: 210;** e) the V_{H}-CDR1, V_{H}-CDR2, V_{H}-CDR3 of the V_{H}-CDR1, V_{H}-CDR2, V_{H}-CDR3 within **SEQ ID NO: 173** and the V_{L}-CDR1, V_{L}-CDR2, V_{L}-CDR3 of the V_{L}-CDR1, V_{L}-CDR2, V_{L}-CDR3 within **SEQ ID NO: 211;** f) the V_{H}-CDR1, V_{H}-CDR2, V_{H}-CDR3 of the V_{H}-CDR1, V_{H}-CDR2, V_{H}-CDR3 within **SEQ ID NO: 174** and the V_{L}-CDR1, V_{L}-CDR2, V_{L}-CDR3 of the V_{L}-CDR1, V_{L}-CDR2, V_{L}-CDR3 within **SEQ ID NO: 212;** g) the V_{H}-CDR1, V_{H}-CDR2, V_{H}-CDR3 of the V_{H}-CDR1, V_{H}-CDR2, V_{H}-CDR3 within **SEQ ID NO: 175** and the V_{L}-CDR1, V_{L}-CDR2, V_{L}-CDR3 of the V_{L}-CDR1, V_{L}-CDR2, V_{L}-CDR3 within **SEQ ID NO: 213;** h) the V_{H}-CDR1, V_{H}-CDR2, V_{H}-CDR3 of the V_{H}-CDR1, V_{H}-CDR2, V_{H}-CDR3 within **SEQ ID NO: 176** and the V_{L}-CDR1, V_{L}-CDR2, V_{L}-CDR3 of the V_{L}-CDR1, V_{L}-CDR2, V_{L}-CDR3 within **SEQ ID NO: 214;** i) the V_{H}-CDR1, V_{H}-CDR2, V_{H}-CDR3 of the V_{H}-CDR1, V_{H}-CDR2, V_{H}-CDR3 within **SEQ ID NO: 177** and the V_{L}-CDR1, V_{L}-CDR2, V_{L}-CDR3 of the V_{L}-CDR1, V_{L}-CDR2, V_{L}-CDR3 within **SEQ ID NO: 215;** j) the V_{H}-CDR1, V_{H}-CDR2, V_{H}-CDR3 of the V_{H}-CDR1, V_{H}-CDR2, V_{H}-CDR3 within **SEQ ID NO: 178** and the V_{L}-CDR1, V_{L}-CDR2, V_{L}-CDR3 of the V_{L}-CDR1, V_{L}-CDR2, V_{L}-CDR3 within **SEQ ID NO: 216;** k) the V_{H}-CDR1, V_{H}-CDR2, V_{H}-CDR3 of the V_{H}-CDR1, V_{H}-CDR2, V_{H}-CDR3 within **SEQ ID NO: 179** and the V_{L}-CDR1, V_{L}-CDR2, V_{L}-CDR3 of the V_{L}-CDR1, V_{L}-CDR2, V_{L}-CDR3 within **SEQ ID NO: 217;** 1) the V_{H}-CDR1, V_{H}-CDR2, V_{H}-CDR3 of the V_{H}-CDR1, V_{H}-CDR2, V_{H}-CDR3 within **SEQ ID NO: 180** and the V_{L}-CDR1, V_{L}-CDR2, V_{L}-CDR3 of the V_{L}-CDR1, V_{L}-CDR2, V_{L}-CDR3 within **SEQ ID NO: 218;** m) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 219;** n) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 220;** o) the V_{H}-CDR1, V_{H}-CDR2, V_{H}-CDR3 of the V_{H}-CDR1, V_{H}-CDR2, V_{H}-CDR3 within **SEQ ID NO: 182** and the V_{L}-CDR1, V_{L}-CDR2, V_{L}-CDR3 of the V_{L}-CDR1, V_{L}-CDR2, V_{L}-CDR3 within **SEQ ID NO: 221;** p) the V_{H}-CDR1, V_{H}-CDR2, V_{H}-CDR3 of the V_{H}-CDR1, V_{H}-CDR2, V_{H}-CDR3 within **SEQ ID NO: 183** and the V_{L}-CDR1, V_{L}-CDR2, V_{L}-CDR3 of the V_{L}-CDR1, V_{L}-CDR2, V_{L}-CDR3 within **SEQ ID NO: 222;** q) the V_{H}-CDR1, V_{H}-CDR2, V_{H}-CDR3 of the V_{H}-CDR1, V_{H}-CDR2, V_{H}-CDR3 within **SEQ ID NO: 184** and the V_{L}-CDR1, V_{L}-CDR2, V_{L}-CDR3 of the V_{L}-CDR1, V_{L}-CDR2, V_{L}-CDR3 within **SEQ ID NO: 223;** r) the V_{H}-CDR1, V_{H}-CDR2, V_{H}-CDR3 of the V_{H}-CDR1, V_{H}-CDR2, V_{H}-CDR3 within **SEQ ID NO: 185** and the V_{L}-CDR1, V_{L}-CDR2, V_{L}-CDR3 of the V_{L}-CDR1, V_{L}-CDR2, V_{L}-CDR3 within **SEQ ID NO: 224;** s) the V_{H}-CDR1, V_{H}-CDR2, V_{H}-CDR3 of the V_{H}-CDR1, V_{H}-CDR2, V_{H}-CDR3 within **SEQ ID NO: 186** and the V_{L}-CDR1, V_{L}-CDR2, V_{L}-CDR3 of the V_{L}-CDR1, V_{L}-CDR2, V_{L}-CDR3 within **SEQ ID NO: 225;** t) the V_{H}-CDR1, V_{H}-CDR2, V_{H}-CDR3 of the V_{H}-CDR1, V_{H}-CDR2, V_{H}-CDR3 within **SEQ ID NO: 187** and the V_{L}-CDR1, V_{L}-CDR2, V_{L}-CDR3 of the V_{L}-CDR1, V_{L}-CDR2, V_{L}-CDR3 within **SEQ ID NO: 226;** u) the V_{H}-CDR1, V_{H}-CDR2, V_{H}-CDR3 of the V_{H}-CDR1, V_{H}-CDR2, V_{H}-CDR3 within **SEQ ID NO: 188** and the V_{L}-CDR1, V_{L}-CDR2, V_{L}-CDR3 of the V_{L}-CDR1, V_{L}-CDR2, V_{L}-CDR3 within **SEQ ID NO: 227;** v) the V_{H}-CDR1, V_{H}-CDR2, V_{H}-CDR3 of the V_{H}-CDR1, V_{H}-CDR2, V_{H}-CDR3 within **SEQ ID NO: 189** and the V_{L}-CDR1, V_{L}-CDR2, V_{L}-CDR3 of the V_{L}-CDR1, V_{L}-CDR2, V_{L}-CDR3 within **SEQ ID NO: 228;** w) the V_{H}-CDR1, V_{H}-CDR2, V_{H}-CDR3 of the V_{H}-CDR1, V_{H}-CDR2, V_{H}-CDR3 within **SEQ ID NO: 190** and the V_{L}-CDR1, V_{L}-CDR2, V_{L}-CDR3 of the V_{L}-CDR1, V_{L}-CDR2, V_{L}-CDR3 within **SEQ ID NO: 229;** x) the V_{H}-CDR1, V_{H}-CDR2, V_{H}-CDR3 of the V_{H}-CDR1, V_{H}-CDR2, V_{H}-CDR3 within **SEQ ID NO: 191** and the V_{L}-CDR1, V_{L}-CDR2, V_{L}-CDR3 of the V_{L}-CDR1, V_{L}-CDR2, V_{L}-CDR3 within **SEQ ID NO: 230;** y) the V_{H}-CDR1, V_{H}-CDR2, V_{H}-CDR3 of the V_{H}-CDR1, V_{H}-CDR2, V_{H}-CDR3 within **SEQ ID NO: 192** and the V_{L}-CDR1, V_{L}-CDR2, V_{L}-CDR3 of the V_{L}-CDR1, V_{L}-CDR2, V_{L}-CDR3 within **SEQ ID NO: 231;** z) the V_{H}-CDR1, V_{H}-CDR2, V_{H}-CDR3 of the V_{H}-CDR1, V_{H}-CDR2, V_{H}-CDR3 within **SEQ ID NO: 193** and the V_{L}-CDR1, V_{L}-CDR2, V_{L}-CDR3 of the V_{L}-CDR1, V_{L}-CDR2, V_{L}-CDR3 within **SEQ ID NO: 232;** aa) the V_{H}-CDR1, V_{H}-CDR2, V_{H}-CDR3 of the V_{H}-CDR1, V_{H}-CDR2, V_{H}-CDR3 within **SEQ ID NO: 194** and the V_{L}-CDR1, V_{L}-CDR2, V_{L}-CDR3 of the V_{L}-CDR1, V_{L}-CDR2, V_{L}-CDR3 within **SEQ ID NO: 233;** ab) the V_{H}-CDR1, V_{H}-CDR2, V_{H}-CDR3 of the V_{H}-CDR1, V_{H}-CDR2, V_{H}-CDR3 within **SEQ ID NO: 195** and the V_{L}-CDR1, V_{L}-CDR2, V_{L}-CDR3 of the V_{L}-CDR1, V_{L}-CDR2, V_{L}-CDR3 within **SEQ ID NO: 234;** ac) the V_{H}-CDR1, V_{H}-CDR2, V_{H}-CDR3 of the V_{H}-CDR1, V_{H}-CDR2, V_{H}-CDR3 within **SEQ ID NO: 196** and the V_{L}-CDR1, V_{L}-CDR2, V_{L}-CDR3 of the V_{L}-CDR1, V_{L}-CDR2, V_{L}-CDR3 within **SEQ ID NO: 235;** ad) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 236;** ae) the V_{H}-CDR1, V_{H}-CDR2, V_{H}-CDR3 of the V_{H}-CDR1, V_{H}-CDR2, V_{H}-CDR3 within **SEQ ID NO: 198** and the V_{L}-CDR1, V_{L}-CDR2, V_{L}-CDR3 of the V_{L}-CDR1, V_{L}-CDR2, V_{L}-CDR3 within **SEQ ID NO: 237;** af) the V_{H}-CDR1, V_{H}-CDR2, V_{H}-CDR3 of the V_{H}-CDR1, V_{H}-CDR2, V_{H}-CDR3 within **SEQ ID NO: 199** and the V_{L}-CDR1, V_{L}-CDR2, V_{L}-CDR3 of the V_{L}-CDR1, V_{L}-CDR2, V_{L}-CDR3 within **SEQ ID NO: 238;** ag) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 239;** ah) the V_{H}-CDR1, V_{H}-CDR2, V_{H}-CDR3 of the V_{H}-CDR1, V_{H}-CDR2, V_{H}-CDR3 within **SEQ ID NO: 200** and the V_{L}-CDR1, V_{L}-CDR2, V_{L}-CDR3 of the V_{L}-CDR1, V_{L}-CDR2, V_{L}-CDR3 within **SEQ ID NO: 240;** ai) the V_{H}-CDR1, V_{H}-CDR2, V_{H}-CDR3 of the V_{H}-CDR1, V_{H}-CDR2, V_{H}-CDR3 within **SEQ ID NO:** 201 and the V_{L}-CDR1, V_{L}-CDR2, V_{L}-CDR3 of the V_{L}-CDR1, V_{L}-CDR2, V_{L}-CDR3 within **SEQ ID NO: 241;** aj) the V_{H}-CDR1, V_{H}-CDR2, V_{H}-CDR3 of the V_{H}-CDR1, V_{H}-CDR2, V_{H}-CDR3 within **SEQ ID NO: 202** and the V_{L}-CDR1, V_{L}-CDR2, V_{L}-CDR3 of the V_{L}-CDR1, V_{L}-CDR2, V_{L}-CDR3 within **SEQ ID NO: 242;** ak) the V_{H}-CDR1, V_{H}-CDR2, V_{H}-CDR3 of the V_{H}-CDR1, V_{H}-CDR2, V_{H}-CDR3 within **SEQ ID NO: 203** and the V_{L}-CDR1, V_{L}-CDR2, V_{L}-CDR3 of the V_{L}-CDR1, V_{L}-CDR2, V_{L}-CDR3 within **SEQ ID NO: 240;** al) the V_{H}-CDR1, V_{H}-CDR2, V_{H}-CDR3 of the V_{H}-CDR1, V_{H}-CDR2, V_{H}-CDR3 within **SEQ ID NO: 204** and the V_{L}-CDR1, V_{L}-CDR2, V_{L}-CDR3 of the V_{L}-CDR1, V_{L}-CDR2, V_{L}-CDR3 within **SEQ ID NO: 243;** am) the V_{H}-CDR1, V_{H}-CDR2, V_{H}-CDR3 of the V_{H}-CDR1, V_{H}-CDR2, V_{H}-CDR3 within **SEQ ID NO: 205** and the V_{L}-CDR1, V_{L}-CDR2, V_{L}-CDR3 of the V_{L}-CDR1, V_{L}-CDR2, V_{L}-CDR3 within **SEQ ID** NO: 244; or an) the V_{H}-CDR1, V_{H}-CDR2, V_{H}-CDR3 of the V_{H}-CDR1, V_{H}-CDR2, V_{H}-CDR3 within **SEQ ID NO: 206** and the V_{L}-CDR1, V_{L}-CDR2, V_{L}-CDR3 of the V_{L}-CDR1, V_{L}-CDR2, V_{L}-CDR3 within **SEQ ID NO: 245.** In some embodiments, exemplary combinations of heavy chain variable region CDRs are depicted in **FIG. 34****.** In some embodiments, exemplary combinations of light chain variable region CDRs are depicted in **FIG. 35****.** In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof comprises: a) the heavy chain variable region of **SEQ ID NO: 169** and the light chain variable region of **SEQ ID NO: 207;** b) the heavy chain variable region of **SEQ ID NO: 170** and the light chain variable region of **SEQ ID NO: 208;** c) the heavy chain variable region of **SEQ ID NO: 171** and the light chain variable region of **SEQ ID NO: 209;** d) the heavy chain variable region of **SEQ ID NO: 172** and the light chain variable region of **SEQ ID NO: 210;** e) the heavy chain variable region of **SEQ ID NO: 173** and the light chain variable region of **SEQ ID NO: 211;** f) the heavy chain variable region of **SEQ ID NO: 174** and the light chain variable region of **SEQ ID NO: 212;** g) the heavy chain variable region of **SEQ ID NO: 175** and the light chain variable region of **SEQ ID NO: 213;** h) the heavy chain variable region of **SEQ ID NO: 176** and the light chain variable region of **SEQ ID NO: 214;** i) the heavy chain variable region of **SEQ ID NO: 177** and the light chain variable region of **SEQ ID NO: 215;** j) the heavy chain variable region of **SEQ ID NO: 178** and the light chain variable region of **SEQ ID NO: 216;** k) the heavy chain variable region of **SEQ ID NO: 179** and the light chain variable region of **SEQ ID NO: 217;** l) the heavy chain variable region of **SEQ ID NO: 180** and the light chain variable region of **SEQ ID NO: 218;** m) the heavy chain variable region of **SEQ ID NO: 181** and the light chain variable region of **SEQ ID NO: 219;** n) the heavy chain variable region of **SEQ ID NO: 181** and the light chain variable region of **SEQ ID NO: 220;** o) the heavy chain variable region of **SEQ ID NO: 182** and the light chain variable region of **SEQ ID NO: 221;** p) the heavy chain variable region of **SEQ ID NO: 183** and the light chain variable region of **SEQ ID NO: 222;** q) the heavy chain variable region of **SEQ ID NO: 184** and the light chain variable region of **SEQ ID NO: 223;** r) the heavy chain variable region of **SEQ ID NO: 185** and the light chain variable region of **SEQ ID NO: 224;** s) the heavy chain variable region of **SEQ ID NO: 186** and the light chain variable region of **SEQ ID NO: 225;** t) the heavy chain variable region of **SEQ ID NO: 187** and the light chain variable region of **SEQ ID NO: 226;** u) the heavy chain variable region of **SEQ ID NO: 188** and the light chain variable region of **SEQ ID NO: 227;** v) the heavy chain variable region of **SEQ ID NO: 189** and the light chain variable region of **SEQ ID NO: 228;** w) the heavy chain variable region of **SEQ ID NO: 190** and the light chain variable region of **SEQ ID NO: 229;** x) the heavy chain variable region of **SEQ ID NO: 191** and the light chain variable region of **SEQ ID NO: 230;** y) the heavy chain variable region of **SEQ ID NO: 192** and the light chain variable region of **SEQ ID NO: 231;** z) the heavy chain variable region of **SEQ ID NO: 193** and the light chain variable region of **SEQ ID NO: 232;** aa) the heavy chain variable region of **SEQ ID NO: 194** and the light chain variable region of **SEQ ID NO: 233;** ab) the heavy chain variable region of **SEQ ID NO: 195** and the light chain variable region of **SEQ ID NO: 234;** ac) the heavy chain variable region of **SEQ ID NO: 196** and the light chain variable region of **SEQ ID NO: 235;** ad) the heavy chain variable region of **SEQ ID NO: 197** and the light chain variable region of **SEQ ID NO: 236;** ae) the heavy chain variable region of **SEQ ID NO: 198** and the light chain variable region of **SEQ ID NO: 237;** af) the heavy chain variable region of **SEQ ID NO: 199** and the light chain variable region of **SEQ ID NO: 238;** ag) the heavy chain variable region of **SEQ ID NO: 197** and the light chain variable region of **SEQ ID NO: 239;** ah) the heavy chain variable region of **SEQ ID NO: 200** and the light chain variable region of **SEQ ID NO: 240;** ai) the heavy chain variable region of **SEQ ID NO: 201** and the light chain variable region of **SEQ ID NO: 241;** aj) the heavy chain variable region of **SEQ ID NO: 202** and the light chain variable region of **SEQ ID NO: 242;** ak) the heavy chain variable region of **SEQ ID NO: 203** and the light chain variable region of **SEQ ID NO: 240;** al) the heavy chain variable region of **SEQ ID NO: 204** and the light chain variable region of **SEQ ID NO: 243;** am) the heavy chain variable region of **SEQ ID NO: 205** and the light chain variable region of **SEQ ID NO: 244;** or an) the heavy chain variable region of **SEQ ID NO: 206** and the light chain variable region of **SEQ ID NO: 245.** In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof comprises the heavy chain (HC) sequence of any one of **SEQ ID NOs: 246-286.** In some embodiments, exemplary HC sequences are depicted in **FIG. 32****.** In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof comprises the light chain (LC) sequence of any one of **SEQ ID NOs: 287-324.** In some embodiments, exemplary HC sequence are depicted in **FIG. 33****.** In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof is selected from the group consisting of 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT-001 (TB001), and IMT-006 (TB006), or a binding fragment thereof. In some embodiments, the heavy and light chain CDRs associated with each of the foregoing antibodies are depicted in **FIG. 36****.** In some embodiments, the V_{H} and V_{L} associated with each of the foregoing antibodies are depicted in **FIG. 37****.** In some embodiments, the HC and LC associated with each of the foregoing antibodies are depicted in **FIG. 38****.**

As applied to any of the methods of use disclosed herein, in some embodiments, the anti-Gal3 antibody or binding fragment thereof comprises a payload. In some embodiments, the payload is conjugated to the anti-Gal3 antibody or binding fragment thereof. In some embodiments, the payload is a cytotoxic payload, microtubule disrupting agent, DNA modifying agent, Akt inhibitor, polymerase inhibitor, detectable moiety, immunomodulatory agent, immune modulator, immunotoxin, nucleic acid polymer, aptamer, peptide, or any combination thereof. In some embodiments, the payload is a detectable moiety. In some embodiments, any of the methods disclosed herein involving an anti-Gal3 antibody or binding fragment can be performed with an antigen binding molecule that binds to Gal3.

As applied to any of the methods of use disclosed herein, in some embodiments, the anti-Gal3 antibody or binding fragment thereof is or comprises a humanized antibody. In some embodiments, the anti-Gal3 antibody or binding fragment thereof is or comprises a full-length antibody or a binding fragment thereof. In some embodiments, the anti-Gal3 antibody or binding fragment thereof is or comprises a bispecific antibody or a binding fragment thereof. In some embodiments, the anti-Gal3-antibody or binding fragment thereof is or comprises a monovalent Fab', a divalent Fab2, a single-chain variable fragment (scFv), a diabody, a minibody, a nanobody, a single-domain antibody (sdAb), or a camelid antibody, or binding fragment thereof. In some embodiments, the anti-Gal3 antibody or binding fragment thereof is or comprises an IgG framework. In some embodiments, the anti-Gal3 antibody or binding fragment thereof is or comprises an IgG1, IgG2, or IgG4 framework. In some embodiments, any of the methods disclosed herein involving an anti-Gal3 antibody or binding fragment can be performed with an antigen binding molecule that binds to Gal3.

In some instances, an anti-Gal3 antibody or binding fragment thereof as described herein may be used to treat a disease or disorder in a subject, or a symptom thereof. In some cases, the disease or disorder is diabetes mellitus. In some cases, the disease or disorder is insulin-dependent diabetes mellitus. In some cases, the disease or disorder is insulin-independent diabetes mellitus. In some cases, the disease or disorder is Type I diabetes mellitus. In some cases, the disease or disorder is Type II diabetes mellitus. In some cases, the disease or disorder is inflammatory bowel disease. In some cases, the disease or disorder is non-alcoholic fatty liver disease (NAFLD). In some cases, the disease or disorder is non-alcoholic steatohepatitis (NASH). In some embodiments, any of the methods disclosed herein involving an anti-Gal3 antibody or binding fragment can be performed with an antigen binding molecule that binds to Gal3.

In some cases, the methods provide for treatment of a disease or disorder in a subject. In some cases, the methods include administering an anti-Gal3 antibody or binding fragment thereof to a subject having, suspected of having, or at risk of developing a disease or disorder as described herein. In some embodiments, the methods comprise administering an anti-Gal3 antibody or binding fragment thereof as described herein to a subject having, suspected of having, or at risk of developing diabetes mellitus. In some embodiments, the methods comprise administering an anti-Gal3 antibody or binding fragment thereof to a subject having, suspected of having, or at risk of developing IBD. In some embodiments, the methods comprise administering an anti-Gal3 antibody or binding fragment thereof as described herein to a subject having, suspected of having, or at risk of developing NAFLD. In some embodiments, the methods comprise administering an anti-Gal3 antibody or binding fragment thereof as described herein to a subject having, suspected of having, or at risk of developing NASH. In some embodiments, any of the methods disclosed herein involving an anti-Gal3 antibody or binding fragment can be performed with an antigen binding molecule that binds to Gal3.

In some aspects, the methods provide for treatment of a symptom associated with diabetes in a subject. In some embodiments, the methods provide for decreasing glucose tolerance in a subject in need thereof, comprising administering to the subject an anti-Gal3 antibody or binding fragment thereof as described herein. In some embodiments, administration of the anti-Gal3 antibody or binding fragment thereof decreases glucose tolerance in the subject by at least 50%, 55%, 60%, 65%, 70%, 75%, 80%, 85%, 90%, 95%, or 99%, or any percentage within a range defined by any two of the aforementioned percentages. In some embodiments, the methods provide for decreasing insulin resistance in a subject in need thereof, comprising administering to the subject an anti-Gal3 antibody or binding fragment thereof as described herein. In some embodiments, administration of the anti-Gal3 antibody or binding fragment thereof decreases insulin resistance in the subject by at least 50%, 55%, 60%, 65%, 70%, 75%, 80%, 85%. 90%, 95%, or 99%, or any percentage within a range defined by any two of the aforementioned percentages. In some embodiments, the methods provide for reducing weight gain in a subject in need thereof, comprising administering to the subject an anti-Gal3 antibody or binding fragment thereof as described herein. In some embodiments, administration of the anti-Gal3 antibody or binding fragment thereof reduces weight gain in the subject by at least 50%, 55%, 60%, 65%, 70%, 75%, 80%, 85%. 90%, 95%, or 99%, or any percentage within a range defined by any two of the aforementioned percentages. In some embodiments, the methods provide for reducing liver steatosis in a subject in need thereof, comprising administering to the subject an anti-Gal3 antibody or binding fragment thereof as described herein. In some embodiments, administration of the anti-Gal3 antibody or binding fragment thereof reduces liver steatosis in the subject by at least 50%, 55%, 60%, 65%, 70%, 75%, 80%, 85%. 90%, 95%, or 99%, or any percentage within a range defined by any two of the aforementioned percentages. In some embodiments, any of the methods disclosed herein involving an anti-Gal3 antibody or binding fragment can be performed with an antigen binding molecule that binds to Gal3.

Disclosed herein are methods of treating diabetes mellitus in a subject in need thereof. In some embodiments, the methods comprise administering to the subject an anti-Gal3 antibody or binding fragment thereof that selectively binds to Gal3 and disrupts an interaction between Gal3 and insulin receptor or an integrin, or both, thereby treating diabetes mellitus in the subject. In some embodiments, the diabetes mellitus is insulin-dependent diabetes mellitus. In some embodiments, the diabetes mellitus is insulin-independent diabetes mellitus. In some embodiments, the diabetes mellitus is Type I diabetes mellitus. In some embodiments, the diabetes mellitus is Type II diabetes mellitus. In some embodiments, the treating comprises decreasing glucose tolerance in the subject in need thereof. In some embodiments, the treating comprises decreasing insulin sensitivity in the subject in need thereof. In some embodiments, the treating comprises reducing weight gain in the subject in need thereof. In some embodiments, the treating comprises reducing liver steatosis in the subject in need thereof. In some embodiments, the methods further comprise selecting the subject as having diabetes mellitus or at risk of contracting diabetes mellitus prior to the administering step. In some embodiments, the methods further comprise detecting an amelioration of symptoms associated with diabetes mellitus in the subject after the administering step. In some embodiments, the insulin receptor comprises the sequence of **SEQ ID NO: 2.** In some embodiments, the integrin is selected from ITGa1, ITGa2, ITGa3, ITGa4, ITGa5, ITGa6, ITGa7, ITGa8, ITGa9, ITGa10, ITGa11, ITGaD, ITGaE, ITGaL, ITGaM, ITGaV, ITGa2B, ITGa2X, ITGb1, ITGb2, ITGb3, ITGb4, ITGb5, ITGb6, ITGb7, ITGb8, or any combination thereof. In some embodiments, the integrin comprises a sequence selected from **SEQ ID NO: 339-342.** In some embodiments, the anti-Gal3 antibody or binding fragment thereof is administered with one or more additional therapeutic compositions. In some embodiments, the one or more additional therapeutic compositions comprises insulin, an insulin derivative or mimetic thereof, insulin aspart, insulin glulisine, insulin lispro, insulin isophane, insulin degludec, insulin detemir, insulin zinc, insulin glargine, vanadium, biguanides, metformin, phenformin, buformin, thiazolidinediones, rosiglitazone, pioglitazone, troglitazone, tolimidone, sulfonylureas, tolbutamide, acetohexamide, tolazamide, chlorpropamide, glipizide, glibenclamide, glimepiride, gliclazide, glyclopyramide, gliquidone, meglitinides, repaglinide, nateglinide, alpha-glucosidase inhibitors, miglitol, acarbose, voglibose, incretins, glucagon-like peptide 1, glucagon-like peptide agonists, exenatide, liraglutide, taspoglutide, lixisenatide, semaglutide, dulaglutide, gastric inhibitory peptide, dipeptidyl peptidase-4 inhibitors, vildagliptin, sitagliptin, saxagliptin, linagliptin, alogliptin, septagliptin, teneligpliptin, gemigliptin, pramlintide, dapagliflozin, canagliflozin, empagliflozin, or remogliflozin, or any combination thereof. In some embodiments, any of the methods disclosed herein involving an anti-Gal3 antibody or binding fragment can be performed with an antigen binding molecule that binds to Gal3.

Disclosed herein are methods of treating inflammatory bowel syndrome in a subject in need thereof. In some embodiments, the methods comprise administering to the subject an anti-Gal3 antibody or binding fragment thereof that selectively binds to Gal3 and disrupts an interaction between Gal3 and an integrin, thereby treating inflammatory bowel syndrome in the subject. In some embodiments, the inflammatory bowel syndrome is ulcerative colitis or Crohn's disease, or both. In some embodiments, the methods further comprise selecting the subject as having inflammatory bowel syndrome or at risk of contracting inflammatory bowel syndrome prior to the administering step. In some embodiments, the methods further comprise detecting an amelioration of symptoms associated with inflammatory bowel syndrome in the subject after the administering step. In some embodiments, the disruption of the interaction between Gal3 and the integrin reduces lymphocyte adhesion or activity, or both, in the subject. In some embodiments, the lymphocyte adhesion or activity, or both, is reduced by at least 50%, 55%, 60%, 65%, 70%, 75%, 80%, 85%, 90%, 95%, 96%, 97%, 98%, or 99%, or any percentage within a range defined by any two aforementioned percentages. In some embodiments, the integrin is selected from ITGa1, ITGa2, ITGa3, ITGa4, ITGa5, ITGa6, ITGa7, ITGa8, ITGa9, ITGa10, ITGa11, ITGaD, ITGaE, ITGaL, ITGaM, ITGaV, ITGa2B, ITGa2X, ITGb1, ITGb2, ITGb3, ITGb4, ITGb5, ITGb6, ITGb7, ITGb8, or any combination thereof. In some embodiments, the integrin comprises a sequence selected from **SEQ ID NO: 339-342.** In some embodiments, the anti-Gal3 antibody or binding fragment thereof is administered with one or more additional therapeutic compositions. In some embodiments, the one or more additional therapeutic compositions comprises mesalazine, immunosuppressants, prednisone, TNF inhibitors, azathioprine, methotrexate, 6-mercaptopurine, or rifaximin, or any combination thereof. In some embodiments, any of the methods disclosed herein involving an anti-Gal3 antibody or binding fragment can be performed with an antigen binding molecule that binds to Gal3.

Disclosed herein are methods of treating non-alcoholic fatty liver disease in a subject in need thereof. In some embodiments, the methods comprise administering to the subject an anti-Gal3 antibody or binding fragment thereof that selectively binds to Gal3 and disrupts an interaction between Gal3 and insulin receptor, thereby treating NAFLD in the subject. In some embodiments, any of the methods disclosed herein involving an anti-Gal3 antibody or binding fragment can be performed with an antigen binding molecule that binds to Gal3.

Disclosed herein are methods of treating non-alcoholic steatohepatitis in a subject in need thereof. In some embodiments, the methods comprise administering to the subject an anti-Gal3 antibody or binding fragment thereof that selectively binds to Gal3 and disrupts an interaction between Gal3 and insulin receptor, thereby treating NASH in the subject. In some embodiments, any of the methods disclosed herein involving an anti-Gal3 antibody or binding fragment can be performed with an antigen binding molecule that binds to Gal3.

In some embodiments of any of the methods disclosed herein, in some embodiments, the interaction between Gal3 and the insulin receptor or the integrin, or both, is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the anti-Gal3 antibody or binding fragment thereof.

As applied to any of the methods of treatment disclosed herein, in some embodiments, the anti-Gal3 antibody or binding fragment thereof binds to an N-terminal domain of Gal3. In some embodiments, the anti-Gal3 antibody or binding fragment thereof binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM.

As applied to any of the methods of treatment disclosed herein, in some embodiments, the anti-Gal3 antibody or binding fragment thereof binds to one or more peptides of **SEQ ID NOs: 3-26.** In some embodiments, the anti-Gal3 antibody or binding fragment thereof binds to an epitope present within a region of Gal3 defined by Peptide 1 (ADNFSLHDALSGSGNPNPQG; **SEQ ID NO: 3**), Peptide 4 (GAGGYPGASYPGAYPGQAPP; **SEQ ID NO: 6**), Peptide 6 (GAYPGQAPPGAYPGAPGAYP; **SEQ ID NO: 8**), Peptide 7 (AYPGAPGAYPGAPAPGVYPG; **SEQ ID NO: 9**), or a combination thereof. In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof binds to an epitope of Gal3 that includes a motif of GxYPG, where x is the amino acids alanine (A), glycine (G), or valine (V). In some embodiments, an anti-Gal3 antibody as described herein binds to an epitope of Gal3 that includes two GxYPG motifs separated by three amino acids, where x is A, G, or V. In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof comprises (1) a light chain variable region comprising a V_{L}-CDR1, a V_{L}-CDR2, and a V_{L}-CDR3 and (2) a heavy chain variable region comprising a V_{H}-CDR1, a V_{H}-CDR2, and a V_{H}-CDR3. In some embodiments, the V_{L}-CDR1 comprises an amino acid sequence having at least 60%, at least 70%, at least 80%, at least 90%, or 100% sequence identity to any amino acid sequence according to **SEQ ID NOs: 100-128.** In some embodiments, the V_{L}-CDR2 comprises an amino acid sequence having at least 60%, at least 70%, at least 80%, at least 90%, or 100% sequence identity to any amino acid sequence according to **SEQ ID NOs: 129-144.** In some embodiments, the V_{L}-CDR3 comprises an amino acid sequence having at least 60%, at least 70%, at least 80%, at least 90%, or 100% sequence identity to any amino acid sequence according to **SEQ ID NOs: 145-168.** In some embodiments, the V_{H}-CDR1 comprises an amino acid sequence having at least 60%, at least 70%, at least 80%, at least 90%, or 100% sequence identity to any amino acid sequence according to **SEQ ID NOs: 27-47.** In some embodiments, the V_{H}-CDR2 comprises an amino acid sequence having at least 60%, at least 70%, at least 80%, at least 90%, or 100% sequence identity to any amino acid sequence according to **SEQ ID NOs: 48-69.** In some embodiments, the V_{H}-CDR3 comprises an amino acid sequence having at least 60%, at least 70%, at least 80%, at least 90%, or 100% sequence identity to any amino acid sequence according to **SEQ ID NOs: 70-99.**

As applied to any of the methods of treatment disclosed herein, in some embodiments, exemplary V_{H}-CDR1 sequences are depicted in **FIG. 28A****.** In some embodiments, exemplary V_{H}-CDR2 sequences are depicted in **FIG. 28B****.** In some embodiments, exemplary V_{H}-CDR3 sequences are depicted in **FIG. 28C****.** In some embodiments, exemplary V_{L}-CDR1 sequences are depicted in **FIG. 29A****.** In some embodiments, exemplary V_{L}-CDR2 sequences are depicted in **FIG. 29B****.** In some embodiments, exemplary V_{L}-CDR3 sequences are depicted in **FIG. 29C****.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the heavy chain variable region of any of the anti-Gal3 antibodies or binding fragments thereof disclosed herein comprises an amino acid sequence having at least 75%, 80%, 85%, 90%, 95%, or 100% sequence identity to any sequence according to **SEQ ID NOs: 169-206.** In some embodiments, the heavy chain variable region of any of the anti-Gal3 antibodies or binding fragments thereof disclosed herein is selected from the group consisting of **SEQ ID NOs: 169-206.** In some embodiments, exemplary V_{H} are depicted in **FIG. 30****.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the light chain variable region of any of the anti-Gal3 antibodies or binding fragments thereof disclosed herein comprises an amino acid sequence having at least 75%, 80%, 85%, 90%, 95%, or 100% sequence identity to any sequence according to **SEQ ID NOs: 207-245.** In some embodiments, the light chain variable region of any of the anti-Gal3 antibodies or binding fragments thereof disclosed herein is selected from the group consisting of **SEQ ID NOs: 207-245.** In some embodiments, exemplary V_{L} are depicted in **FIG. 31****.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof comprises: a) the V_{H}-CDR1, V_{H}-CDR2, V_{H}-CDR3 of the V_{H}-CDR1, V_{H}-CDR2, V_{H}-CDR3 within **SEQ ID NO: 169** and the V_{L}-CDR1, V_{L}-CDR2, V_{L}-CDR3 of the V_{L}-CDR1, V_{L}-CDR2, V_{L}-CDR3 within **SEQ ID NO: 207;** b) the V_{H}-CDR1, V_{H}-CDR2, V_{H}-CDR3 of the V_{H}-CDR1, V_{H}-CDR2, V_{H}-CDR3 within **SEQ ID NO: 170** and the V_{L}-CDR1, V_{L}-CDR2, V_{L}-CDR3 of the V_{L}-CDR1, V_{L}-CDR2, V_{L}-CDR3 within **SEQ ID NO: 208;** c) the V_{H}-CDR1, V_{H}-CDR2, V_{H}-CDR3 of the V_{H}-CDR1, V_{H}-CDR2, V_{H}-CDR3 within **SEQ ID NO: 171** and the V_{L}-CDR1, V_{L}-CDR2, V_{L}-CDR3 of the V_{L}-CDR1, V_{L}-CDR2, V_{L}-CDR3 within **SEQ ID NO: 209;** d) the V_{H}-CDR1, V_{H}-CDR2, V_{H}-CDR3 of the V_{H}-CDR1, V_{H}-CDR2, V_{H}-CDR3 within **SEQ ID NO: 172** and the V_{L}-CDR1, V_{L}-CDR2, V_{L}-CDR3 of the V_{L}-CDR1, V_{L}-CDR2, V_{L}-CDR3 within **SEQ ID NO: 210;** e) the V_{H}-CDR1, V_{H}-CDR2, V_{H}-CDR3 of the V_{H}-CDR1, V_{H}-CDR2, V_{H}-CDR3 within **SEQ ID NO: 173** and the V_{L}-CDR1, V_{L}-CDR2, V_{L}-CDR3 of the V_{L}-CDR1, V_{L}-CDR2, V_{L}-CDR3 within **SEQ ID NO: 211;** f) the V_{H}-CDR1, V_{H}-CDR2, V_{H}-CDR3 of the V_{H}-CDR1, V_{H}-CDR2, V_{H}-CDR3 within **SEQ ID NO: 174** and the V_{L}-CDR1, V_{L}-CDR2, V_{L}-CDR3 of the V_{L}-CDR1, V_{L}-CDR2, V_{L}-CDR3 within **SEQ ID NO: 212;** g) the V_{H}-CDR1, V_{H}-CDR2, V_{H}-CDR3 of the V_{H}-CDR1, V_{H}-CDR2, V_{H}-CDR3 within **SEQ ID NO: 175** and the V_{L}-CDR1, V_{L}-CDR2, V_{L}-CDR3 of the V_{L}-CDR1, V_{L}-CDR2, V_{L}-CDR3 within **SEQ ID NO: 213;** h) the V_{H}-CDR1, V_{H}-CDR2, V_{H}-CDR3 of the V_{H}-CDR1, V_{H}-CDR2, V_{H}-CDR3 within **SEQ ID NO: 176** and the V_{L}-CDR1, V_{L}-CDR2, V_{L}-CDR3 of the V_{L}-CDR1, V_{L}-CDR2, V_{L}-CDR3 within **SEQ ID NO: 214;** i) the V_{H}-CDR1, V_{H}-CDR2, V_{H}-CDR3 of the V_{H}-CDR1, V_{H}-CDR2, V_{H}-CDR3 within **SEQ ID NO: 177** and the V_{L}-CDR1, V_{L}-CDR2, V_{L}-CDR3 of the V_{L}-CDR1, V_{L}-CDR2, V_{L}-CDR3 within **SEQ ID NO: 215;** j) the V_{H}-CDR1, V_{H}-CDR2, V_{H}-CDR3 of the V_{H}-CDR1, V_{H}-CDR2, V_{H}-CDR3 within **SEQ ID NO: 178** and the V_{L}-CDR1, V_{L}-CDR2, V_{L}-CDR3 of the V_{L}-CDR1, V_{L}-CDR2, V_{L}-CDR3 within **SEQ ID NO: 216;** k) the V_{H}-CDR1, V_{H}-CDR2, V_{H}-CDR3 of the V_{H}-CDR1, V_{H}-CDR2, V_{H}-CDR3 within **SEQ ID NO: 179** and the V_{L}-CDR1, V_{L}-CDR2, V_{L}-CDR3 of the V_{L}-CDR1, V_{L}-CDR2, V_{L}-CDR3 within **SEQ ID NO: 217;** 1) the V_{H}-CDR1, V_{H}-CDR2, V_{H}-CDR3 of the V_{H}-CDR1, V_{H}-CDR2, V_{H}-CDR3 within **SEQ ID NO: 180** and the V_{L}-CDR1, V_{L}-CDR2, V_{L}-CDR3 of the V_{L}-CDR1, V_{L}-CDR2, V_{L}-CDR3 within **SEQ ID NO: 218;** m) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 219;** n) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 220;** o) the V_{H}-CDR1, V_{H}-CDR2, V_{H}-CDR3 of the V_{H}-CDR1, V_{H}-CDR2, V_{H}-CDR3 within **SEQ ID NO: 182** and the V_{L}-CDR1, V_{L}-CDR2, V_{L}-CDR3 of the V_{L}-CDR1, V_{L}-CDR2, V_{L}-CDR3 within **SEQ ID NO: 221;** p) the V_{H}-CDR1, V_{H}-CDR2, V_{H}-CDR3 of the V_{H}-CDR1, V_{H}-CDR2, V_{H}-CDR3 within **SEQ ID NO: 183** and the V_{L}-CDR1, V_{L}-CDR2, V_{L}-CDR3 of the V_{L}-CDR1, V_{L}-CDR2, V_{L}-CDR3 within **SEQ ID NO: 222;** q) the V_{H}-CDR1, V_{H}-CDR2, V_{H}-CDR3 of the V_{H}-CDR1, V_{H}-CDR2, V_{H}-CDR3 within **SEQ ID NO: 184** and the V_{L}-CDR1, V_{L}-CDR2, V_{L}-CDR3 of the V_{L}-CDR1, V_{L}-CDR2, V_{L}-CDR3 within **SEQ ID NO: 223;** r) the V_{H}-CDR1, V_{H}-CDR2, V_{H}-CDR3 of the V_{H}-CDR1, V_{H}-CDR2, V_{H}-CDR3 within **SEQ ID NO: 185** and the V_{L}-CDR1, V_{L}-CDR2, V_{L}-CDR3 of the V_{L}-CDR1, V_{L}-CDR2, V_{L}-CDR3 within **SEQ ID NO: 224;** s) the V_{H}-CDR1, V_{H}-CDR2, V_{H}-CDR3 of the V_{H}-CDR1, V_{H}-CDR2, V_{H}-CDR3 within **SEQ ID NO: 186** and the V_{L}-CDR1, V_{L}-CDR2, V_{L}-CDR3 of the V_{L}-CDR1, V_{L}-CDR2, V_{L}-CDR3 within **SEQ ID NO: 225;** t) the V_{H}-CDR1, V_{H}-CDR2, V_{H}-CDR3 of the V_{H}-CDR1, V_{H}-CDR2, V_{H}-CDR3 within **SEQ ID NO: 187** and the V_{L}-CDR1, V_{L}-CDR2, V_{L}-CDR3 of the V_{L}-CDR1, V_{L}-CDR2, V_{L}-CDR3 within **SEQ ID NO: 226;** u) the V_{H}-CDR1, V_{H}-CDR2, V_{H}-CDR3 of the V_{H}-CDR1, V_{H}-CDR2, V_{H}-CDR3 within **SEQ ID NO: 188** and the V_{L}-CDR1, V_{L}-CDR2, V_{L}-CDR3 of the V_{L}-CDR1, V_{L}-CDR2, V_{L}-CDR3 within **SEQ ID NO: 227;** v) the V_{H}-CDR1, V_{H}-CDR2, V_{H}-CDR3 of the V_{H}-CDR1, V_{H}-CDR2, V_{H}-CDR3 within **SEQ ID NO: 189** and the V_{L}-CDR1, V_{L}-CDR2, V_{L}-CDR3 of the V_{L}-CDR1, V_{L}-CDR2, V_{L}-CDR3 within **SEQ ID NO: 228;** w) the V_{H}-CDR1, V_{H}-CDR2, V_{H}-CDR3 of the V_{H}-CDR1, V_{H}-CDR2, V_{H}-CDR3 within **SEQ ID NO: 190** and the V_{L}-CDR1, V_{L}-CDR2, V_{L}-CDR3 of the V_{L}-CDR1, V_{L}-CDR2, V_{L}-CDR3 within **SEQ ID NO: 229;** x) the V_{H}-CDR1, V_{H}-CDR2, V_{H}-CDR3 of the V_{H}-CDR1, V_{H}-CDR2, V_{H}-CDR3 within **SEQ ID NO: 191** and the V_{L}-CDR1, V_{L}-CDR2, V_{L}-CDR3 of the V_{L}-CDR1, V_{L}-CDR2, V_{L}-CDR3 within **SEQ ID NO: 230;** y) the V_{H}-CDR1, V_{H}-CDR2, V_{H}-CDR3 of the V_{H}-CDR1, V_{H}-CDR2, V_{H}-CDR3 within **SEQ ID NO: 192** and the V_{L}-CDR1, V_{L}-CDR2, V_{L}-CDR3 of the V_{L}-CDR1, V_{L}-CDR2, V_{L}-CDR3 within **SEQ ID NO: 231;** z) the V_{H}-CDR1, V_{H}-CDR2, V_{H}-CDR3 of the V_{H}-CDR1, V_{H}-CDR2, V_{H}-CDR3 within **SEQ ID NO: 193** and the V_{L}-CDR1, V_{L}-CDR2, V_{L}-CDR3 of the V_{L}-CDR1, V_{L}-CDR2, V_{L}-CDR3 within **SEQ ID NO: 232;** aa) the V_{H}-CDR1, V_{H}-CDR2, V_{H}-CDR3 of the V_{H}-CDR1, V_{H}-CDR2, V_{H}-CDR3 within **SEQ ID NO: 194** and the V_{L}-CDR1, V_{L}-CDR2, V_{L}-CDR3 of the V_{L}-CDR1, V_{L}-CDR2, V_{L}-CDR3 within **SEQ ID NO: 233;** ab) the V_{H}-CDR1, V_{H}-CDR2, V_{H}-CDR3 of the V_{H}-CDR1, V_{H}-CDR2, V_{H}-CDR3 within **SEQ ID NO: 195** and the V_{L}-CDR1, V_{L}-CDR2, V_{L}-CDR3 of the V_{L}-CDR1, V_{L}-CDR2, V_{L}-CDR3 within **SEQ ID NO: 234;** ac) the V_{H}-CDR1, V_{H}-CDR2, V_{H}-CDR3 of the V_{H}-CDR1, V_{H}-CDR2, V_{H}-CDR3 within **SEQ ID NO: 196** and the V_{L}-CDR1, V_{L}-CDR2, V_{L}-CDR3 of the V_{L}-CDR1, V_{L}-CDR2, V_{L}-CDR3 within **SEQ ID NO: 235;** ad) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 236;** ae) the V_{H}-CDR1, V_{H}-CDR2, V_{H}-CDR3 of the V_{H}-CDR1, V_{H}-CDR2, V_{H}-CDR3 within **SEQ ID NO: 198** and the V_{L}-CDR1, V_{L}-CDR2, V_{L}-CDR3 of the V_{L}-CDR1, V_{L}-CDR2, V_{L}-CDR3 within **SEQ ID NO: 237;** af) the V_{L}-CDR1, V_{H}-CDR2, V_{H}-CDR3 of the V_{H}-CDR1, V_{H}-CDR2, V_{H}-CDR3 within **SEQ ID NO: 199** and the V_{L}-CDR1, V_{L}-CDR2, V_{L}-CDR3 of the V_{L}-CDR1, V_{L}-CDR2, V_{L}-CDR3 within **SEQ ID NO: 238;** ag) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 239;** ah) the V_{H}-CDR1, V_{H}-CDR2, V_{H}-CDR3 of the V_{H}-CDR1, V_{H}-CDR2, V_{H}-CDR3 within **SEQ ID NO: 200** and the V_{L}-CDR1, V_{L}-CDR2, V_{L}-CDR3 of the V_{L}-CDR1, V_{L}-CDR2, V_{L}-CDR3 within **SEQ ID NO: 240;** ai) the V_{H}-CDR1, V_{H}-CDR2, V_{H}-CDR3 of the V_{H}-CDR1, V_{H}-CDR2, V_{H}-CDR3 within **SEQ ID NO: 201** and the V_{L}-CDR1, V_{L}-CDR2, V_{L}-CDR3 of the V_{L}-CDR1, V_{L}-CDR2, V_{L}-CDR3 within **SEQ ID NO: 241;** aj) the V_{H}-CDR1, V_{H}-CDR2, V_{H}-CDR3 of the V_{H}-CDR1, V_{H}-CDR2, V_{H}-CDR3 within **SEQ ID NO: 202** and the V_{L}-CDR1, V_{L}-CDR2, V_{L}-CDR3 of the V_{L}-CDR1, V_{L}-CDR2, V_{L}-CDR3 within **SEQ ID NO: 242;** ak) the V_{H}-CDR1, V_{H}-CDR2, V_{H}-CDR3 of the V_{H}-CDR1, V_{H}-CDR2, V_{H}-CDR3 within **SEQ ID NO: 203** and the V_{L}-CDR1, V_{L}-CDR2, V_{L}-CDR3 of the V_{L}-CDR1, V_{L}-CDR2, V_{L}-CDR3 within **SEQ ID NO: 240;** al) the V_{H}-CDR1, V_{H}-CDR2, V_{H}-CDR3 of the V_{H}-CDR1, V_{H}-CDR2, V_{H}-CDR3 within **SEQ ID NO: 204** and the V_{L}-CDR1, V_{L}-CDR2, V_{L}-CDR3 of the V_{L}-CDR1, V_{L}-CDR2, V_{L}-CDR3 within **SEQ ID NO: 243;** am) the V_{H}-CDR1, V_{H}-CDR2, V_{H}-CDR3 of the V_{H}-CDR1, V_{H}-CDR2, V_{H}-CDR3 within **SEQ ID NO: 205** and the V_{L}-CDR1, V_{L}-CDR2, V_{L}-CDR3 of the V_{L}-CDR1, V_{L}-CDR2, V_{L}-CDR3 within **SEQ ID NO: 244;** or an) the V_{H}-CDR1, V_{H}-CDR2, V_{H}-CDR3 of the V_{H}-CDR1, V_{H}-CDR2, V_{H}-CDR3 within **SEQ ID NO: 206** and the V_{L}-CDR1, V_{L}-CDR2, V_{L}-CDR3 of the V_{L}-CDR1, V_{L}-CDR2, V_{L}-CDR3 within **SEQ ID NO: 245.** In some embodiments, exemplary combinations of heavy chain variable region CDRs are depicted in **FIG. 34****.** In some embodiments, exemplary combinations of light chain variable region CDRs are depicted in **FIG. 35****.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof comprises: a) the heavy chain variable region of **SEQ ID NO: 169** and the light chain variable region of **SEQ ID NO: 207;** b) the heavy chain variable region of **SEQ ID NO: 170** and the light chain variable region of **SEQ ID NO: 208;** c) the heavy chain variable region of **SEQ ID NO: 171** and the light chain variable region of **SEQ ID NO: 209;** d) the heavy chain variable region of **SEQ ID NO: 172** and the light chain variable region of **SEQ ID NO: 210;** e) the heavy chain variable region of **SEQ ID NO: 173** and the light chain variable region of **SEQ ID NO: 211;** f) the heavy chain variable region of **SEQ ID NO: 174** and the light chain variable region of **SEQ ID NO: 212;** g) the heavy chain variable region of **SEQ ID NO: 175** and the light chain variable region of **SEQ ID NO: 213;** h) the heavy chain variable region of **SEQ ID NO: 176** and the light chain variable region of **SEQ ID NO: 214;** i) the heavy chain variable region of **SEQ ID NO: 177** and the light chain variable region of **SEQ ID NO: 215;** j) the heavy chain variable region of **SEQ ID NO: 178** and the light chain variable region of **SEQ ID NO: 216;** k) the heavy chain variable region of **SEQ ID NO: 179** and the light chain variable region of **SEQ ID NO: 217;** l) the heavy chain variable region of **SEQ ID NO: 180** and the light chain variable region of **SEQ ID NO: 218;** m) the heavy chain variable region of **SEQ ID NO: 181** and the light chain variable region of **SEQ ID NO: 219;** n) the heavy chain variable region of **SEQ ID NO: 181** and the light chain variable region of **SEQ ID NO: 220;** o) the heavy chain variable region of **SEQ ID NO: 182** and the light chain variable region of **SEQ ID NO: 221;** p) the heavy chain variable region of **SEQ ID NO: 183** and the light chain variable region of **SEQ ID NO: 222;** q) the heavy chain variable region of **SEQ ID NO: 184** and the light chain variable region of **SEQ ID NO: 223;** r) the heavy chain variable region of **SEQ ID NO: 185** and the light chain variable region of **SEQ ID NO: 224;** s) the heavy chain variable region of **SEQ ID NO: 186** and the light chain variable region of **SEQ ID NO: 225;** t) the heavy chain variable region of **SEQ ID NO: 187** and the light chain variable region of **SEQ ID NO: 226;** u) the heavy chain variable region of **SEQ ID NO: 188** and the light chain variable region of **SEQ ID NO: 227;** v) the heavy chain variable region of **SEQ ID NO: 189** and the light chain variable region of **SEQ ID NO: 228;** w) the heavy chain variable region of **SEQ ID NO: 190** and the light chain variable region of **SEQ ID NO: 229;** x) the heavy chain variable region of **SEQ ID NO: 191** and the light chain variable region of **SEQ ID NO: 230;** y) the heavy chain variable region of **SEQ ID NO: 192** and the light chain variable region of **SEQ ID NO: 231;** z) the heavy chain variable region of **SEQ ID NO: 193** and the light chain variable region of **SEQ ID NO: 232;** aa) the heavy chain variable region of **SEQ ID NO: 194** and the light chain variable region of **SEQ ID NO: 233;** ab) the heavy chain variable region of **SEQ ID NO: 195** and the light chain variable region of **SEQ ID NO: 234;** ac) the heavy chain variable region of **SEQ ID NO: 196** and the light chain variable region of **SEQ ID NO: 235;** ad) the heavy chain variable region of **SEQ ID NO: 197** and the light chain variable region of **SEQ ID NO: 236;** ae) the heavy chain variable region of **SEQ ID NO: 198** and the light chain variable region of **SEQ ID NO: 237;** af) the heavy chain variable region of **SEQ ID NO: 199** and the light chain variable region of **SEQ ID NO: 238;** ag) the heavy chain variable region of **SEQ ID NO: 197** and the light chain variable region of **SEQ ID NO: 239;** ah) the heavy chain variable region of **SEQ ID NO: 200** and the light chain variable region of **SEQ ID NO: 240;** ai) the heavy chain variable region of **SEQ ID NO: 201** and the light chain variable region of **SEQ ID NO: 241;** aj) the heavy chain variable region of **SEQ ID NO: 202** and the light chain variable region of **SEQ ID NO: 242;** ak) the heavy chain variable region of **SEQ ID NO: 203** and the light chain variable region of **SEQ ID NO: 240;** al) the heavy chain variable region of **SEQ ID NO: 204** and the light chain variable region of **SEQ ID NO: 243;** am) the heavy chain variable region of **SEQ ID NO: 205** and the light chain variable region of **SEQ ID NO: 244;** or an) the heavy chain variable region of **SEQ ID NO: 206** and the light chain variable region of **SEQ ID NO: 245.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof comprises the heavy chain (HC) sequence of any one of **SEQ ID NOs: 246-286.** In some embodiments, exemplary HC sequences are depicted in **FIG. 32****.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof comprises the light chain (LC) sequence of any one of **SEQ ID NOs: 287-324.** In some embodiments, exemplary HC sequence are depicted in **FIG. 33****.** In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof is selected from the group consisting of 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT-001 (TB001), and IMT-006 (TB006), or a binding fragment thereof. In some embodiments, the heavy and light chain CDRs associated with each of the foregoing antibodies are depicted in **FIG. 36****.** In some embodiments, the V_{H} and V_{L} associated with each of the foregoing antibodies are depicted in **FIG. 37****.** In some embodiments, the HC and LC associated with each of the foregoing antibodies are depicted in

### FIG. 38.

As applied to any of the methods of treatment disclosed herein, in some embodiments, in some embodiments, the anti-Gal3 antibody or binding fragment thereof is administered enterally, orally, intranasally, parenterally, intracranially, subcutaneously, intramuscularly, intradermally, or intravenously, or any combination thereof.

As applied to any of the methods of treatment disclosed herein, in some embodiments, the anti-Gal3 antibody or binding fragment thereof comprises a payload. In some embodiments, the payload is conjugated to the anti-Gal3 antibody or binding fragment thereof. In some embodiments, the payload is a cytotoxic payload, microtubule disrupting agent, DNA modifying agent, Akt inhibitor, polymerase inhibitor, detectable moiety, immunomodulatory agent, immune modulator, immunotoxin, nucleic acid polymer, aptamer, peptide, or any combination thereof. In some embodiments, the payload is a detectable moiety.

As applied to any of the methods of treatment disclosed herein, in some embodiments, the anti-Gal3 antibody or binding fragment thereof is or comprises a humanized antibody. In some embodiments, the anti-Gal3 antibody or binding fragment thereof is or comprises a full-length antibody or a binding fragment thereof. In some embodiments, the anti-Gal3 antibody or binding fragment thereof is or comprises a bispecific antibody or a binding fragment thereof. In some embodiments, the anti-Gal3-antibody or binding fragment thereof is or comprises a monovalent Fab', a divalent Fab2, a single-chain variable fragment (scFv), a diabody, a minibody, a nanobody, a single-domain antibody (sdAb), or a camelid antibody, or binding fragment thereof. In some embodiments, the anti-Gal3 antibody or binding fragment thereof is or comprises an IgG framework. In some embodiments, the anti-Gal3 antibody or binding fragment thereof is or comprises an IgG1, IgG2, or IgG4 framework. In some embodiments, any of the methods disclosed herein involving an anti-Gal3 antibody or binding fragment can be performed with an antigen binding molecule that binds to Gal3.

As applied to any of the methods of treatment disclosed herein, in some embodiments, the anti-Gal3 antibody or binding fragment thereof is formulated for systemic administration. In some embodiments, the anti-Gal3 antibody or binding fragment thereof is formulated for parenteral administration. In some embodiments, more than one anti-Gal3 antibody or binding fragment is administered. In some embodiments, when more than one anti-Gal3 antibody or binding fragment is administered, the more than one anti-Gal3 antibodies or binding fragments thereof may be selected from the anti-Gal3 antibodies or binding fragments thereof disclosed herein. In some embodiments, any of the methods disclosed herein involving an anti-Gal3 antibody or binding fragment can be performed with an antigen binding molecule that binds to Gal3.

As applied to any of the methods of use or treatment disclosed herein, the subject is a mammal. In some embodiments, the mammal is a human, cat, dog, mouse, rat, hamster, rodent, pig, cow, horse, sheep, or goat. In some embodiments, the mammal is a human. In some embodiments, the subject has Type I diabetes or Type II diabetes.

### Antibody Production

In some cases, anti-Gal3 antibodies or binding fragments thereof are raised by standard protocol by injecting a production animal with an antigenic composition. See, e.g., Harlow and Lane, Antibodies: A Laboratory Manual, Cold Spring Harbor Laboratory, 1988. When utilizing an entire protein, or a larger section of the protein, antibodies may be raised by immunizing the production animal with the protein and a suitable adjuvant (e.g., Freund's, Freund's complete, oil-in-water emulsions, etc.). When a smaller peptide is utilized, it is advantageous to conjugate the peptide with a larger molecule to make an immunostimulatory conjugate. Commonly utilized conjugate proteins that are commercially available for such use include bovine serum albumin (BSA) and keyhole limpet hemocyanin (KLH). In order to raise antibodies to particular epitopes, peptides derived from the full sequence may be utilized. Alternatively, in order to generate antibodies to relatively short peptide portions of the protein target, a superior immune response may be elicited if the polypeptide is joined to a carrier protein, such as ovalbumin, BSA or KLH.

Polyclonal or monoclonal anti-Gal3 antibodies or binding fragments thereof can be produced from animals which have been genetically altered to produce human immunoglobulins. A transgenic animal can be produced by initially producing a "knock-out" animal which does not produce the animal's natural antibodies, and stably transforming the animal with a human antibody locus (e.g., by the use of a human artificial chromosome). In such cases, only human antibodies are then made by the animal. Techniques for generating such animals, and deriving antibodies therefrom, are described in U.S. Pat. Nos. 6,162,963 and 6,150,584, each incorporated fully herein by reference in its entirety. Such antibodies can be referred to as human xenogenic antibodies.

Alternatively, anti-Gal3 antibodies or binding fragments thereof can be produced from phage libraries containing human variable regions. See U.S. Pat. No. 6,174,708, incorporated fully herein by reference in its entirety.

In some aspects of any of the embodiments disclosed herein, an anti-Gal3 antibody or binding fragment thereof is produced by a hybridoma.

For monoclonal anti-Gal3 antibodies, hybridomas may be formed by isolating the stimulated immune cells, such as those from the spleen of the inoculated animal. These cells can then be fused to immortalized cells, such as myeloma cells or transformed cells, which are capable of replicating indefinitely in cell culture, thereby producing an immortal, immunoglobulin-secreting cell line. The immortal cell line utilized can be selected to be deficient in enzymes necessary for the utilization of certain nutrients. Many such cell lines (such as myelomas) are known to those skilled in the art, and include, for example: thymidine kinase (TK) or hypoxanthine-guanine phosphoriboxyl transferase (HGPRT). These deficiencies allow selection for fused cells according to their ability to grow on, for example, hypoxanthine aminopterinthymidine medium (HAT).

In addition, the anti-Gal3 antibody or binding fragment thereof may be produced by genetic engineering.

Anti-Gal3 antibodies or binding fragments thereof disclosed herein can have a reduced propensity to induce an undesired immune response in humans, for example, anaphylactic shock, and can also exhibit a reduced propensity for priming an immune response which would prevent repeated dosage with an antibody therapeutic or imaging agent (e.g., the human-anti-murine-antibody "HAMA" response). Such anti-Gal3 antibodies or binding fragments thereof include, but are not limited to, humanized, chimeric, or xenogenic human anti-Gal3 antibodies or binding fragments thereof.

Chimeric anti-Gal3 antibodies or binding fragments thereof can be made, for example, by recombinant means by combining the murine variable light and heavy chain regions (VK and VH), obtained from a murine (or other animal-derived) hybridoma clone, with the human constant light and heavy chain regions, in order to produce an antibody with predominantly human domains. The production of such chimeric antibodies is well known in the art and may be achieved by standard means (as described, e.g., in U.S. Pat. No. 5,624,659, incorporated fully herein by reference).

The term "humanized" as applies to a non-human (e.g. rodent or primate) antibodies are hybrid immunoglobulins, immunoglobulin chains or fragments thereof which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, rabbit or primate having the desired specificity, affinity and capacity. In some instances, Fv framework region (FR) residues of the human immunoglobulin are replaced by corresponding non-human residues. Furthermore, the humanized antibody may comprise residues which are found neither in the recipient antibody nor in the imported CDR or framework sequences. These modifications are made to further refine and optimize antibody performance and minimize immunogenicity when introduced into a human body. In some examples, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody may also comprise at least a portion of an immunoglobulin constant region (Fc), typically that of a human immunoglobulin.

Humanized antibodies can be engineered to contain human-like immunoglobulin domains and incorporate only the complementarity-determining regions of the animal-derived antibody. This can be accomplished by carefully examining the sequence of the hyper-variable loops of the variable regions of a monoclonal antigen binding unit or monoclonal antibody and fitting them to the structure of a human antigen binding unit or human antibody chains. See, e.g., U.S. Pat. No. 6,187,287, incorporated fully herein by reference.

Methods for humanizing non-human antibodies are well known in the art. "Humanized" antibodies are antibodies in which at least part of the sequence has been altered from its initial form to render it more like human immunoglobulins. In some versions, the heavy (H) chain and light (L) chain constant (C) regions are replaced with human sequence. This can be a fusion polypeptide comprising a variable (V) region and a heterologous immunoglobulin C region. In some versions, the complementarity determining regions (CDRs) comprise non-human antibody sequences, while the V framework regions have also been converted to human sequences. See, for example, EP 0329400. In some versions, V regions are humanized by designing consensus sequences of human and mouse V regions and converting residues outside the CDRs that are different between the consensus sequences.

In principle, a framework sequence from a humanized antibody can serve as the template for CDR grafting; however, it has been demonstrated that straight CDR replacement into such a framework can lead to significant loss of binding affinity to the antigen. Glaser et al. (1992) J. Immunol. 149:2606; Tempest et al. (1992) Biotechnology 9:266; and Shalaby et al. (1992) J. Exp. Med. 17:217. The more homologous a human antibody (HuAb) is to the original murine antibody (muAb), the less likely that the human framework will introduce distortions into the murine CDRs that could reduce affinity. Based on a sequence homology search against an antibody sequence database, the HuAb IC4 provides good framework homology to muM4TS.22, although other highly homologous HuAbs would be suitable as well, especially kappa L chains from human subgroup I or H chains from human subgroup III. Kabat et al. (1987). Various computer programs such as ENCAD (Levitt et al. (1983) J. Mol. Biol. 168:595) are available to predict the ideal sequence for the V region. The disclosure thus encompasses HuAbs with different variable (V) regions. It is within the skill of one in the art to determine suitable V region sequences and to optimize these sequences. Methods for obtaining antibodies with reduced immunogenicity are also described in U.S. Pat. No. 5,270,202 and EP 699,755, each hereby incorporated by reference in its entirety.

Humanized antibodies can be prepared by a process of analysis of the parental sequences and various conceptual humanized products using three dimensional models of the parental and humanized sequences. Three-dimensional immunoglobulin models are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved.

A process for humanization of subject antigen binding units can be as follows. The best-fit germline acceptor heavy and light chain variable regions are selected based on homology, canonical structure and physical properties of the human antibody germlines for grafting. Computer modeling of mVH/VL versus grafted hVH/VL is performed and prototype humanized antibody sequence is generated. If modeling indicated a need for framework back-mutations, second variant with indicated FW changes is generated. DNA fragments encoding the selected germline frameworks and murine CDRs are synthesized. The synthesized DNA fragments are subcloned into IgG expression vectors and sequences are confirmed by DNA sequencing. The humanized antibodies are expressed in cells, such as 293F and the proteins are tested, for example in MDM phagocytosis assays and antigen binding assays. The humanized antigen binding units are compared with parental antigen binding units in antigen binding affinity, for example, by FACS on cells expressing the target antigen. If the affinity is greater than 2-fold lower than parental antigen binding unit, a second round of humanized variants can be generated and tested as described above.

As noted above, an anti-Gal3 antibody or binding fragment thereof can be either "monovalent" or "multivalent." Whereas the former has one binding site per antigen-binding unit, the latter contains multiple binding sites capable of binding to more than one antigen of the same or different kind. Depending on the number of binding sites, antigen binding units may be bivalent (having two antigen-binding sites), trivalent (having three antigen-binding sites), tetravalent (having four antigen-binding sites), and so on.

Multivalent anti-Gal3 antibodies or binding fragments thereof can be further classified on the basis of their binding specificities. A "monospecific" anti-Gal3 antibody or binding fragment thereof is a molecule capable of binding to one or more antigens of the same kind. A "multispecific" anti-Gal3 antibody or binding fragment thereof is a molecule having binding specificities for at least two different antigens. While such molecules normally will only bind two distinct antigens (i.e. bispecific anti-Gal3 antibodies), antibodies with additional specificities such as trispecific antibodies are encompassed by this expression when used herein. This disclosure further provides multispecific anti-Gal3 antibodies. Multispecific anti-Gal3 antibodies or binding fragments thereof are multivalent molecules capable of binding to at least two distinct antigens, e.g., bispecific and trispecific molecules exhibiting binding specificities to two and three distinct antigens, respectively.

In some embodiments, the methods further provide for screening for or identifying antibodies or binding fragments thereof capable of disrupting an interaction between Gal3 and insulin receptor or an integrin, or both. A non-limiting example of such a method is described in **Example 1.** In some aspects, the method may comprise: (a) contacting Gal3 protein with an antibody or binding fragment thereof that selectively binds to Gal3, thereby forming a Gal3-antibody complex; (b) contacting the Gal3-antibody complex with the insulin receptor or the integrin, or both; (c) removing unbound insulin receptor or integrin, or both; and (d) detecting the insulin receptor or integrin, or both, bound to the Gal3-antibody complex, wherein the antibody or binding fragment thereof is capable of disrupting an interaction of Gal3 and insulin receptor or the integrin, or both, when insulin receptor or the integrin, or both, is not detected in (d). In some cases, the method comprises an immunoassay. In some cases, the immunoassay is an enzyme-linked immunosorbent assay (ELISA).

### Polynucleotides and Vectors

In some embodiments, the present disclosure provides isolated nucleic acids encoding any of the anti-Gal3 antibodies or binding fragments thereof disclosed herein. In another embodiment, the present disclosure provides vectors comprising a nucleic acid sequence encoding any anti-Gal3 antibody or binding fragment thereof disclosed herein. In some embodiments, this disclosure provides isolated nucleic acids that encode a light-chain CDR and a heavy-chain CDR of an anti-Gal3 antibody or binding fragment thereof disclosed herein.

In some embodiments are nucleic acids that encode for heavy chain CDR1 peptide sequences selected from **SEQ ID NOs: 27-47.** In some embodiments are nucleic acids that encode for heavy chain CDR2 peptide sequences selected from **SEQ ID NOs: 48-69.** In some embodiments are nucleic acids that encode for heavy chain CDR3 peptide sequences selected from **SEQ ID NOs: 70-99.** In some embodiments are nucleic acids that encode for light chain CDR1 peptide sequences selected from **SEQ ID NOs: 100-128.** In some embodiments are nucleic acids that encode for light chain CDR2 peptide sequences selected from **SEQ ID NOs: 129-144.** In some embodiments are nucleic acids that encode for light chain CDR3 peptide sequences selected from **SEQ ID NOs: 145-168.** In some embodiments are nucleic acids that encode for heavy chain variable region peptide sequences selected from **SEQ ID NOs: 169-206.** In some embodiments are nucleic acids that encode for light chain variable region peptide sequences selected from **SEQ ID NOs: 207-245.** In some embodiments are nucleic acids that encode for heavy chain peptide sequences selected from **SEQ ID NOs: 246-286.** In some embodiments are nucleic acids that encode for light chain peptide sequences selected from **SEQ ID NOs: 287-324.** In some embodiments are nucleic acids that encode for any of the peptide sequences represented in **FIGs. 28A-33****.**

Any one of the anti-Gal3 antibodies or binding fragments thereof described herein can be prepared by recombinant DNA technology, synthetic chemistry techniques, or a combination thereof. For instance, sequences encoding the desired components of the anti-Gal3 antibodies, including light chain CDRs and heavy chain CDRs are typically assembled cloned into an expression vector using standard molecular techniques know in the art. These sequences may be assembled from other vectors encoding the desired protein sequence, from PCR-generated fragments using respective template nucleic acids, or by assembly of synthetic oligonucleotides encoding the desired sequences. Expression systems can be created by transfecting a suitable cell with an expressing vector which comprises an anti-Gal3 antibody of interest or binding fragment thereof.

Nucleotide sequences corresponding to various regions of light or heavy chains of an existing antibody can be readily obtained and sequenced using convention techniques including but not limited to hybridization, PCR, and DNA sequencing. Hybridoma cells that produce monoclonal antibodies serve as a preferred source of antibody nucleotide sequences. A vast number of hybridoma cells producing an array of monoclonal antibodies may be obtained from public or private repositories. The largest depository agent is American Type Culture Collection, which offers a diverse collection of well-characterized hybridoma cell lines. Alternatively, antibody nucleotides can be obtained from immunized or non-immunized rodents or humans, and form organs such as spleen and peripheral blood lymphocytes. Specific techniques applicable for extracting and synthesizing antibody nucleotides are described in Orlandi et al.(1989) Proc. Natl. Acad. Sci. U.S.A. 86: 3833-3837; Larrick et al. (1989) Biochem. Biophys. Res. Commun. 160:1250-1255; Sastry et al. (1989) Proc. Natl. Acad. Sci., U.S.A. 86: 5728-5732; and U.S. Pat. No. 5,969,108.

Polynucleotides encoding anti-Gal3 antibodies or binding fragments thereof can also be modified, for example, by substituting the coding sequence for human heavy and light chain constant regions in place of the homologous non-human sequences. In that manner, chimeric antibodies are prepared that retain the binding specificity of the original anti-Gal3 antibody or binding fragment thereof.

Also disclosed herein are methods of producing an anti-Gal3 antibody or binding fragment thereof. In some embodiments, the methods comprise expressing a nucleic acid that encodes for the anti-Gal3 antibody or binding fragment thereof in a cell and isolating the expressed anti-Gal3 antibody or binding fragment thereof from the cell. In some embodiments, the methods further comprise concentrating the anti-Gal3 antibody or binding fragment thereof to a desired concentration. In some embodiments, the cell is a mammalian cell, inset cell, or bacterial cell. In some embodiments, the anti-Gal3 antibody or binding fragment thereof is any one of the anti-Gal3 antibodies or binding fragments disclosed herein. Specific procedures of expressing antibodies in a cell and isolation of the expressed antibodies are conventionally known and can be practiced by one skilled in the art.

### Host Cells

In some embodiments, the present disclosure provides host cells expressing any one of the anti-Gal3 antibodies or binding fragments thereof disclosed herein. A subject host cell typically comprises a nucleic acid encoding any one of the anti-Gal3 antibodies or binding fragments thereof disclosed herein.

The disclosure provides host cells transfected with the polynucleotides, vectors, or a library of the vectors described above. The vectors can be introduced into a suitable prokaryotic or eukaryotic cell by any of a number of appropriate means, including electroporation, microprojectile bombardment; lipofection, infection (where the vector is coupled to an infectious agent), transfection employing calcium chloride, rubidium chloride, calcium phosphate, DEAE-dextran, or other substances. The choice of the means for introducing vectors will often depend on features of the host cell.

For most animal cells, any of the above-mentioned methods is suitable for vector delivery. Preferred animal cells are vertebrate cells, preferably mammalian cells, capable of expressing exogenously introduced gene products in large quantity, e.g. at the milligram level. Non-limiting examples of preferred cells are NIH3T3 cells, COS, HeLa, and CHO cells.

Once introduced into a suitable host cell, expression of the anti-Gal3 antibodies or binding fragments thereof can be determined using any nucleic acid or protein assay known in the art. For example, the presence of transcribed mRNA of light chain CDRs or heavy chain CDRs, or the anti-Gal3 antibody or binding fragment thereof can be detected and/or quantified by conventional hybridization assays (e.g. Northern blot analysis), amplification procedures (e.g. RT-PCR), SAGE (U.S. Pat. No. 5,695,937), and array-based technologies (see e.g. U.S. Pat. Nos. 5,405,783, 5,412,087 and 5,445,934), using probes complementary to any region of a polynucleotide that encodes the anti-Gal3 antibody or binding fragment thereof.

Expression of the vector can also be determined by examining the expressed anti-Gal3 antibody or binding fragment thereof. A variety of techniques are available in the art for protein analysis. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

### Payload

In some embodiments, any anti-Gal3 antibody disclosed herein further comprises a payload. In some cases, the payload comprises a small molecule, a protein or functional fragment thereof, a peptide, or a nucleic acid polymer.

In some cases, the number of payloads conjugated to the anti-Gal3 antibody (e.g., the drug-to-antibody ratio or DAR) is about 1:1, one payload to one anti-Gal3 antibody. In some cases, the ratio of the payloads to the anti-Gal3 antibody is about 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, or 20:1. In some cases, the ratio of the payloads to the anti-Gal3 antibody is about 2:1. In some cases, the ratio of the payloads to the anti-Gal3 antibody is about 3:1. In some cases, the ratio of the payloads to the anti-Gal3 antibody is about 4:1. In some cases, the ratio of the payloads to the anti-Gal3 antibody is about 6:1. In some cases, the ratio of the payloads to the anti-Gal3 antibody is about 8:1. In some cases, the ratio of the payloads to the anti-Gal3 antibody is about 12:1.

In some embodiment, the payload is a small molecule. In some instances, the small molecule is a cytotoxic payload. Exemplary cytotoxic payloads include, but are not limited to, microtubule disrupting agents, DNA modifying agents, or Akt inhibitors.

In some embodiments, the payload comprises a microtubule disrupting agent. Exemplary microtubule disrupting agents include, but are not limited to, 2-methoxyestradiol, auristatin, chalcones, colchicine, combretastatin, cryptophycin, dictyostatin, discodermolide, dolastain, eleutherobin, epothilone, halichondrin, laulimalide, maytansine, noscapinoid, paclitaxel, peloruside, phomopsin, podophyllotoxin, rhizoxin, spongistatin, taxane, tubulysin, vinca alkaloid, vinorelbine, or derivatives or analogs thereof.

In some embodiments, the maytansine is a maytansinoid. In some embodiments, the maytansinoid is DM1, DM4, or ansamitocin. In some embodiments, the maytansinoid is DM1. In some embodiments, the maytansinoid is DM4. In some embodiments, the maytansinoid is ansamitocin. In some embodiments, the maytansinoid is a maytansionid derivative or analog such as described in U.S. Patent Nos. 5208020, 5416064, 7276497, and 6716821 or U.S. Publication Nos. 2013029900 and US20130323268.

In some embodiments, the payload is a dolastatin, or a derivative or analog thereof. In some embodiments, the dolastatin is dolastatin 10 or dolastatin 15, or derivatives or analogs thereof. In some embodiments, the dolastatin 10 analog is auristatin, soblidotin, symplostatin 1, or symplostatin 3. In some embodiments, the dolastatin 15 analog is cemadotin or tasidotin.

In some embodiments, the dolastatin 10 analog is auristatin or an auristatin derivative. In some embodiments, the auristatin or auristatin derivative is auristatin E (AE), auristatin F (AF), auristatin E5-benzoylvaleric acid ester (AEVB), monomethyl auristatin E (MMAE), monomethyl auristatin F (MMAF), or monomethyl auristatin D (MMAD), auristatin PE, or auristatin PYE. In some embodiments, the auristatin derivative is monomethyl auristatin E (MMAE). In some embodiments, the auristatin derivative is monomethyl auristatin F (MMAF). In some embodiments, the auristatin is an auristatin derivative or analog such as described in U.S. Patent No. 6884869, 7659241, 7498298, 7964566, 7750116, 8288352, 8703714, and 8871720.

In some embodiments, the payload comprises a DNA modifying agent. In some embodiments, the DNA modifying agent comprises DNA cleavers, DNA intercalators, DNA transcription inhibitors, or DNA cross-linkers. In some instances, the DNA cleaver comprises bleomycine A2, calicheamicin, or derivatives or analogs thereof. In some instances, the DNA intercalator comprises doxorubicin, epirubicin, PNU-159682, duocarmycin, pyrrolobenzodiazepine, oligomycin C, daunorubicin, valrubicin, topotecan, or derivatives or analogs thereof. In some instances, the DNA transcription inhibitor comprises dactinomycin. In some instances, the DNA cross-linker comprises mitomycin C.

In some embodiments, the DNA modifying agent comprises amsacrine, anthracycline, camptothecin, doxorubicin, duocarmycin, enediyne, etoposide, indolinobenzodiazepine, netropsin, teniposide, or derivatives or analogs thereof.

In some embodiments, the anthracycline is doxorubicin, daunorubicin, epirubicin, idarubicin, mitomycin-C, dactinomycin, mithramycin, nemorubicin, pixantrone, sabarubicin, or valrubicin.

In some embodiments, the analog of camptothecin is topotecan, irinotecan, silatecan, cositecan, exatecan, lurtotecan, gimatecan, belotecan, rubitecan, or SN-38.

In some embodiments, the duocarmycin is duocarmycin A, duocarmycin B1, duocarmycin B2, duocarmycin C1, duocarmycin C2, duocarmycin D, duocarmycin SA, or CC-1065. In some embodiments, the enediyne is a calicheamicin, esperamicin, or dynemicin A.

In some embodiments, the pyrrolobenzodiazepine is anthramycin, abbeymycin, chicamycin, DC-81, mazethramycin, neothramycins A, neothramycin B, porothramycin, prothracarcin, sibanomicin (DC-102), sibiromycin, or tomaymycin. In some embodiments, the pyrrolobenzodiazepine is a tomaymycin derivative, such as described in U.S. Patent Nos. 8404678 and 8163736. In some embodiments, the pyrrolobenzodiazepine is such as described in U.S. Patent Nos. 8426402, 8802667, 8809320, 6562806, 6608192, 7704924, 7067511, US7612062, 7244724, 7528126, 7049311, 8633185, 8501934, and 8697688 and U.S. Publication No. US20140294868.

In some embodiments, the pyrrolobenzodiazepine is a pyrrolobenzodiazepine dimer. In some embodiments, the PBD dimer is a symmetric dimer. Examples of symmetric PBD dimers include, but are not limited to, SJG-136 (SG-2000), ZC-423 (SG2285), SJG-720, SJG-738, ZC-207 (SG2202), and DSB-120. In some embodiments, the PBD dimer is an unsymmetrical dimer. Examples of unsymmetrical PBD dimers include, but are not limited to, SJG-136 derivatives such as described in U.S. Patent Nos. 8697688 and 9242013 and U.S. Publication No. 20140286970.

In some embodiments, the payload comprises an Akt inhibitor. In some cases, the Akt inhibitor comprises ipatasertib (GDC-0068) or derivatives thereof.

In some embodiments, the payload comprises a polymerase inhibitor, including, but not limited to polymerase II inhibitors such as a-amanitin, and poly(ADP-ribose) polymerase (PARP) inhibitors. Exemplary PARP inhibitors include, but are not limited to Iniparib (BSI 201), Talazoparib (BMN-673), Olaparib (AZD-2281), Olaparib, Rucaparib (AG014699, PF-01367338), Veliparib (ABT-888), CEP 9722, MK 4827, BGB-290, or 3-aminobenzamide.

In some embodiments, the payload comprises a detectable moiety. As used herein, a "detectable moiety" may comprise an atom, molecule, or compound that is useful in diagnosing, detecting or visualizing a location and/or quantity of a target molecule, cell, tissue, organ, and the like. Detectable moieties that can be used in accordance with the embodiments herein include, but are not limited to, radioactive substances (e.g. radioisotopes, radionuclides, radiolabels or radiotracers), dyes, contrast agents, fluorescent compounds or molecules, bioluminescent compounds or molecules, enzyme and enhancing agents (e.g. paramagnetic ions), or specific binding moieties such as streptavidin, avidin, or biotin. In addition, some nanoparticles, for example quantum dots or metal nanoparticles can be suitable for use as a detectable moiety.

Exemplary radioactive substances that can be used as detectable moieties in accordance with the embodiments herein include, but are not limited to, ¹⁸F, ¹⁸F-FAC, ³²P, ³³P, ⁴⁵Ti, ⁴⁷Sc, ⁵²Fe, ⁵⁹Fe, ⁶²Cu, ⁶⁴Cu, ⁶⁷Cu, ⁶⁷Ga, ⁶⁸Ga, ⁷⁵Sc, ⁷⁷As, ⁸⁶Y, ⁹⁰Y, ⁸⁹Sr, ⁸⁹Zr, ⁹⁴Tc, ⁹⁴Tc, ⁹⁹mTc, ⁹⁹Mo, ¹⁰⁵Pd, ¹⁰⁵Rh, ¹¹¹Ag, ¹¹¹In, ¹²³I, ¹²⁴I, ¹²⁵I, ¹³¹I, ¹⁴²Pr, ¹⁴³Pr, ¹⁴⁹Pm, ¹⁵³Sm, ¹⁵⁴⁻¹⁵⁸Gd, ¹⁶¹Tb, ¹⁶⁶Dy, ¹⁶⁶Ho, ¹⁶⁹Er, ¹⁷⁵Lu, ¹⁷⁷Lu, ¹⁸⁶Re, ¹⁸⁸Re, ¹⁸⁹Re, ¹⁹⁴Ir, ¹⁹⁸Au, ¹⁹⁹Au, ²¹¹At, ²¹¹Pb, ²¹²Bi, ²¹²Pb, ²¹³Bi, ²²³Ra and ²²⁵Ac. Exemplary paramagnetic ions substances that can be used as detectable markers include, but are not limited to ions of transition and lanthanide metals (e.g. metals having atomic numbers of 6 to 9, 21-29, 42, 43, 44, or 57-71). These metals include ions of Cr, V, Mn, Fe, Co, Ni, Cu, La, Ce, Pr, Nd, Pm, Sm, Eu, Gd, Tb, Dy, Ho, Er, Tm, Yb and Lu.

When the detectable marker is a radioactive metal or paramagnetic ion, in some embodiments, the marker can be reacted with a reagent having a long tail with one or more chelating groups attached to the long tail for binding these ions. The long tail can be a polymer such as a polylysine, polysaccharide, or other derivatized or derivatizable chain having pendant groups to which may be bound to a chelating group for binding the ions. Examples of chelating groups that may be used according to the embodiments herein include, but are not limited to, ethylenediaminetetraacetic acid (EDTA), diethylenetriaminepentaacetic acid (DTPA), DOTA, NOTA, NOGADA, NETA, deferoxamine (DfO), porphyrins, polyamines, crown ethers, bis-thiosemicarbazones, polyoximes, and like groups. The chelate can be linked to the antigen binding construct by a group which allows formation of a bond to the molecule with minimal loss of immunoreactivity and minimal aggregation and/or internal cross-linking. The same chelates, when complexed with non-radioactive metals, such as manganese, iron and gadolinium are useful for MRI, when used along with the antigen binding constructs and carriers described herein. Macrocyclic chelates such as NOTA, NOGADA, DOTA, and TETA are of use with a variety of metals and radiometals including, but not limited to, radionuclides of gallium, yttrium and copper, respectively. Other ring-type chelates such as macrocyclic polyethers, which are of interest for stably binding radionuclides, such as Radium-223 for RAIT may be used. In certain embodiments, chelating moieties may be used to attach a PET imaging agent, such as an Aluminum-¹⁸F complex, to a targeting molecule for use in PET analysis.

Exemplary contrast agents that can be used as detectable moieties in accordance with the embodiments of the disclosure include, but are not limited to, barium, diatrizoate, ethiodized oil, gallium citrate, iocarmic acid, iocetamic acid, iodamide, iodipamide, iodoxamic acid, iogulamide, iohexyl, iopamidol, iopanoic acid, ioprocemic acid, iosefamic acid, ioseric acid, iosulamide meglumine, iosemetic acid, iotasul, iotetric acid, iothalamic acid, iotroxic acid, ioxaglic acid, ioxotrizoic acid, ipodate, meglumine, metrizamide, metrizoate, propyliodone, thallous chloride, or combinations thereof.

Bioluminescent and fluorescent compounds or molecules and dyes that can be used as detectable moieties in accordance with the embodiments of the disclosure include, but are not limited to, fluorescein, fluorescein isothiocyanate (FITC), OREGON GREEN^{™}, rhodamine, Texas red, tetrarhodimine isothiocynate (TRITC), Cy3, Cy5, and the like), fluorescent markers (e.g., green fluorescent protein (GFP), phycoerythrin, and the like), autoquenched fluorescent compounds that are activated by tumor-associated proteases, enzymes (e.g., luciferase, horseradish peroxidase, alkaline phosphatase, and the like), nanoparticles, biotin, digoxigenin or combinations thereof.

Enzymes that can be used as detectable moieties in accordance with the embodiments of the disclosure include, but are not limited to, horseradish peroxidase, alkaline phosphatase, acid phosphatase, glucose oxidase, β-galactosidase, β-glucoronidase or β-lactamase. Such enzymes may be used in combination with a chromogen, a fluorogenic compound or a luminogenic compound to generate a detectable signal.

In some embodiments, the payload is a nanoparticle. The term "nanoparticle" refers to a microscopic particle whose size is measured in nanometers, e.g., a particle with at least one dimension less than about 100 nm. Nanoparticles can be used as detectable substances because they are small enough to scatter visible light rather than absorb it. For example, gold nanoparticles possess significant visible light extinction properties and appear deep red to black in solution. As a result, compositions comprising antigen binding constructs conjugated to nanoparticles can be used for the in vivo imaging of T-cells in a subject. At the small end of the size range, nanoparticles are often referred to as clusters. Metal, dielectric, and semiconductor nanoparticles have been formed, as well as hybrid structures (e.g. core-shell nanoparticles). Nanospheres, nanorods, and nanocups are just a few of the shapes that have been grown. Semiconductor quantum dots and nanocrystals are examples of additional types of nanoparticles. Such nanoscale particles can be used as payloads to be conjugated to any one of the anti-Gal3 antibodies disclosed herein.

In some embodiments, the payload comprises an immunomodulatory agent. Useful immunomodulatory agents include anti-hormones that block hormone action on tumors and immunosuppressive agents that suppress cytokine production, down-regulate self-antigen expression, or mask MHC antigens. Representative anti-hormones include anti-estrogens including, for example, tamoxifen, raloxifene, aromatase inhibiting 4(5)-imidazoles, 4-hydroxytamoxifen, trioxifene, keoxifene, LY 117018, onapnstone, and toremifene; and antiandrogens such as flutamide, nilutamide, bicalutamide, leuprolide, and goserelin; and anti-adrenal agents. Illustrative immunosuppressive agents include, but are not limited to 2-amino-6-aryl-5-substituted pyrimidines, azathioprine, cyclophosphamide, bromocryptine, danazol, dapsone, glutaraldehyde, anti-idiotypic antibodies for MHC antigens and MHC fragments, cyclosporin A, steroids such as glucocorticosteroids, streptokinase, or rapamycin.

In some embodiments, the payload comprises an immune modulator. Exemplary immune modulators include, but are not limited to, gancyclovier, etanercept, tacrolimus, sirolimus, voclosporin, cyclosporine, rapamycin, cyclophosphamide, azathioprine, mycophenolgate mofetil, methotrextrate, glucocorticoid and its analogs, xanthines, stem cell growth factors, lymphotoxins, hematopoietic factors, tumor necrosis factor (TNF) (*e.g.,* TNFα), interleukins (*e.g*., interleukin-1 (IL-1), IL-2, IL-3, IL-6, IL-10, IL-12, IL-18, and IL-21), colony stimulating factors (*e.g*., granulocyte-colony stimulating factor (G-CSF) and granulocyte macrophage-colony stimulating factor (GM-CSF)), interferons (*e.g*., interferons-alpha, interferon-beta, interferon-gamma), the stem cell growth factor designated "S1 factor," erythropoietin and thrombopoietin, or a combination thereof.

In some embodiments, the payload comprises an immunotoxin. Immunotoxins include, but are not limited to, ricin, radionuclides, pokeweed antiviral protein, Pseudomonas exotoxin A, diphtheria toxin, ricin A chain, fungal toxins such as restrictocin and phospholipase enzymes. See, generally, "Chimeric Toxins," Olsnes and Pihl, Pharmac. Ther. 15:355-381 (1981); and "Monoclonal Antibodies for Cancer Detection and Therapy," eds. Baldwin and Byers, pp. 159-179, 224-266, Academic Press (1985).

In some instances, the payload comprises a nucleic acid polymer. In such instances, the nucleic acid polymer comprises short interfering nucleic acid (siNA), short interfering RNA (siRNA), double-stranded RNA (dsRNA), micro-RNA (miRNA), short hairpin RNA (shRNA), an antisense oligonucleotide. In other instances, the nucleic acid polymer comprises an mRNA, encoding, e.g., a cytotoxic protein or peptide or an apoptotic triggering protein or peptide. Exemplary cytotoxic proteins or peptides include a bacterial cytotoxin such as an alpha-pore forming toxin (e.g., cytolysin A from E. coli), a beta-pore-forming toxin (e.g., α-Hemolysin, PVL-panton Valentine leukocidin, aerolysin, clostridial Epsilon-toxin, clostridium perfringens enterotoxin), binary toxins (anthrax toxin, edema toxin, C. botulinum C2 toxin, C spirofome toxin, C. perfringens iota toxin, C. difficile cyto-lethal toxins (A and B)), prion, parasporin, a cholesterol-dependent cytolysins (e.g., pneumolysin), a small pore-forming toxin (e.g., Gramicidin A), a cyanotoxin (e.g., microcystins, nodularins), a hemotoxin, a neurotoxin (e.g., botulinum neurotoxin), a cytotoxin, cholera toxin, diphtheria toxin, Pseudomonas exotoxin A, tetanus toxin, or an immunotoxin (idarubicin, ricin A, CRM9, Pokeweed antiviral protein, DT). Exemplary apoptotic triggering proteins or peptides include apoptotic protease activating factor-1 (Apaf-1), cytochrome-c, caspase initiator proteins (CASP2, CASP8, CASP9, CASP10), apoptosis inducing factor (AIF), p53, p73, p63, Bcl-2, Bax, granzyme B, poly-ADP ribose polymerase (PARP), and P 21-activated kinase 2 (PAK2). In additional instances, the nucleic acid polymer comprises a nucleic acid decoy. In some instances, the nucleic acid decoy is a mimic of protein-binding nucleic acids such as RNA-based protein-binding mimics. Exemplary nucleic acid decoys include transactivating region (TAR) decoy and Rev response element (RRE) decoy.

In some cases, the payload is an aptamer. Aptamers are small oligonucleotide or peptide molecules that bind to specific target molecules. Exemplary nucleic acid aptamers include DNA aptamers, RNA aptamers, or XNA aptamers which are RNA and/or DNA aptamers comprising one or more unnatural nucleotides. Exemplary nucleic acid aptamers include ARC19499 (Archemix Corp.), REG1 (Regado Biosciences), and ARC1905 (Ophthotech).

Nucleic acids in accordance with the embodiments described herein optionally include naturally occurring nucleic acids, or one or more nucleotide analogs or have a structure that otherwise differs from that of a naturally occurring nucleic acid. For example, 2'-modifications include halo, alkoxy, and allyloxy groups. In some embodiments, the 2'-OH group is replaced by a group selected from H, OR, R, halo, SH, SR, NH₂, NHR, NR₂ or CN, wherein R is C₁-C₆ alkyl, alkenyl, or alkynyl, and halo is F, Cl, Br, or I. Examples of modified linkages include phosphorothioate and 5'-N-phosphoramidite linkages.

Nucleic acids having a variety of different nucleotide analogs, modified backbones, or non-naturally occurring internucleoside linkages are utilized in accordance with the embodiments described herein. In some cases, nucleic acids include natural nucleosides (i.e., adenosine, thymidine, guanosine, cytidine, uridine, deoxyadenosine, deoxythymidine, deoxyguanosine, and deoxycytidine) or modified nucleosides. Examples of modified nucleotides include base modified nucleoside (e.g., aracytidine, inosine, isoguanosine, nebularine, pseudouridine, 2,6-diaminopurine, 2-aminopurine, 2-thiothymidine, 3-deaza-5-azacytidine, 2'-deoxyuridine, 3-nitorpyrrole, 4-methylindole, 4-thiouridine, 4-thiothymidine, 2-aminoadenosine, 2-thiothymidine, 2-thiouridine, 5-bromocytidine, 5-iodouridine, inosine, 6-azauridine, 6-chloropurine, 7-deazaadenosine, 7-deazaguanosine, 8-azaadenosine, 8-azidoadenosine, benzimidazole, M1-methyladenosine, pyrrolo-pyrimidine, 2-amino-6-chloropurine, 3-methyl adenosine, 5-propynylcytidine, 5-propynyluridine, 5-bromouridine, 5-fluorouridine, 5-methylcytidine, 7-deazaadenosine, 7-deazaguanosine, 8-oxoadenosine, 8-oxoguanosine, O(6)-methylguanine, and 2-thiocytidine), chemically or biologically modified bases (e.g., methylated bases), modified sugars (e.g., 2'-fluororibose, 2'-aminoribose, 2'-azidoribose, 2'-O-methylribose, L-enantiomeric nucleosides arabinose, and hexose), modified phosphate groups (e.g., phosphorothioates and 5'-N-phosphoramidite linkages), and combinations thereof. Natural and modified nucleotide monomers for the chemical synthesis of nucleic acids are readily available. In some cases, nucleic acids comprising such modifications display enhanced properties relative to nucleic acids consisting only of naturally occurring nucleotides. In some embodiments, nucleic acid modifications described herein are utilized to reduce and/or prevent digestion by nucleases (e.g. exonucleases, endonucleases, etc.). For example, the structure of a nucleic acid may be stabilized by including nucleotide analogs at the 3' end of one or both strands order to reduce digestion.

Different nucleotide modifications and/or backbone structures may exist at various positions in the nucleic acid. Such modifications include morpholinos, peptide nucleic acids (PNAs), methylphosphonate nucleotides, thiolphosphonate nucleotides, 2'-fluoro N3-P5'-phosphoramidites, 1', 5'- anhydrohexitol nucleic acids (HNAs), or a combination thereof.

Any of the anti-Gal3 antibodies disclosed herein may be conjugated to one or more (e.g. 1, 2, 3, 4, 5, 6, 7, 8, 9, or 10, or more) payloads described herein.

### Conjugation Chemistry

In some instances, the payload is conjugated to an anti-Gal3 antibody described herein by a native ligation. In some instances, the conjugation is as described in: Dawson, et al. "Synthesis of proteins by native chemical ligation," Science 1994, 266, 776-779; Dawson, et al. "Modulation of Reactivity in Native Chemical Ligation through the Use of Thiol Additives," J. Am. Chem. Soc. 1997, 119, 4325-4329; Hackeng, et al. "Protein synthesis by native chemical ligation: Expanded scope by using straightforward methodology.," Proc. Natl. Acad. Sci. USA 1999, 96, 10068-10073; or Wu, et al. "Building complex glycopeptides: Development of a cysteine-free native chemical ligation protocol," Angew. Chem. Int. Ed. 2006, 45, 4116-4125. In some instances, the conjugation is as described in U.S. Patent No. 8,936,910.

In some instances, the payload is conjugated to an anti-Gal3 antibody described herein by a site-directed method utilizing a "traceless" coupling technology (Philochem). In some instances, the "traceless" coupling technology utilizes an N-terminal 1,2-aminothiol group on the binding moiety which is then conjugate with a polynucleic acid molecule containing an aldehyde group. (*see* Casi et al., "Site-specific traceless coupling of potent cytotoxic drugs to recombinant antibodies for pharmacodelivery," JACS 134(13): 5887-5892 (2012))

In some instances, the payload is conjugated to an anti-Gal3 antibody described herein by a site-directed method utilizing an unnatural amino acid incorporated into the binding moiety. In some instances, the unnatural amino acid comprises p-acetylphenylalanine (pAcPhe). In some instances, the keto group of pAcPhe is selectively coupled to an alkoxy-amine derivatived conjugating moiety to form an oxime bond. (*see* Axup et al., "Synthesis of site-specific antibody-drug conjugates using unnatural amino acids," PNAS 109(40): 16101-16106 (2012)).

In some instances, the payload is conjugated to an anti-Gal3 antibody described herein by a site-directed method utilizing an enzyme-catalyzed process. In some instances, the site-directed method utilizes SMARTag^{™} technology (Redwood). In some instances, the SMARTag^{™} technology comprises generation of a formylglycine (FGly) residue from cysteine by formylglycine-generating enzyme (FGE) through an oxidation process under the presence of an aldehyde tag and the subsequent conjugation of FGly to an alkylhydraine-functionalized polynucleic acid molecule via hydrazino-Pictet-Spengler (HIPS) ligation. (*see* Wu et al., "Site-specific chemical modification of recombinant proteins produced in mammalian cells by using the genetically encoded aldehyde tag," PNAS 106(9): 3000-3005 (2009); Agarwal, et al., "A Pictet-Spengler ligation for protein chemical modification," PNAS 110(1): 46-51 (2013)).

In some instances, the enzyme-catalyzed process comprises microbial transglutaminase (mTG). In some cases, the payload is conjugated to the anti-Gal3 antibody utilizing a microbial transglutaminze catalyzed process. In some instances, mTG catalyzes the formation of a covalent bond between the amide side chain of a glutamine within the recognition sequence and a primary amine of a functionalized polynucleic acid molecule. In some instances, mTG is produced from *Streptomyces mobarensis.* (*see* Strop et al., "Location matters: site of conjugation modulates stability and pharmacokinetics of antibody drug conjugates," Chemistry and Biology 20(2) 161-167 (2013)).

In some instances, the payload is conjugated to an anti-Gal3 antibody by a method as described in PCT Publication No. WO2014/140317, which utilizes a sequence-specific transpeptidase and is hereby expressly incorporated by reference in its entirety.

In some instances, the payload is conjugated to an anti-Gal3 antibody described herein by a method as described in U.S. Patent Publication Nos. 2015/0105539 and 2015/0105540.

### Linker

In some instances, a linker described herein comprises a natural or synthetic polymer, consisting of long chains of branched or unbranched monomers, and/or cross-linked network of monomers in two or three dimensions. In some instances, the linker includes a polysaccharide, lignin, rubber, or polyalkylen oxide (e.g., polyethylene glycol).

In some instances, the linker includes, but is not limited to, alpha-, omega-dihydroxylpolyethyleneglycol, biodegradable lactone-based polymer, e.g. polyacrylic acid, polylactide acid (PLA), poly(glycolic acid) (PGA), polypropylene, polystyrene, polyolefin, polyamide, polycyanoacrylate, polyimide, polyethylenterephthalat (PET, PETG), polyethylene terephthalate (PETE), polytetramethylene glycol (PTG), or polyurethane as well as mixtures thereof. As used herein, a mixture refers to the use of different polymers within the same compound as well as in reference to block copolymers. In some cases, block copolymers are polymers wherein at least one section of a polymer is built up from monomers of another polymer. In some instances, the linker comprises polyalkylene oxide. In some instances, the linker comprises PEG. In some instances, the linker comprises polyethylene imide (PEI) or hydroxy ethyl starch (HES).

In some cases, the polyalkylene oxide (e.g., PEG) is a polydispers or monodispers compound. In some instances, polydispers material comprises disperse distribution of different molecular weight of the material, characterized by mean weight (weight average) size and dispersity. In some instances, the monodisperse PEG comprises one size of molecules. In some embodiments, the linker is poly- or monodispersed polyalkylene oxide (e.g., PEG) and the indicated molecular weight represents an average of the molecular weight of the polyalkylene oxide, e.g., PEG, molecules.

In some embodiments, the linker comprises a polyalkylene oxide (e.g., PEG) and the molecular weight of the polyalkylene oxide (e.g., PEG) is about 200, 300, 400, 500, 600, 700, 800, 900, 1000, 1100, 1200, 1300, 1400, 1450, 1500, 1600, 1700, 1800, 1900, 2000, 2100, 2200, 2300, 2400, 2500, 2600, 2700, 2800, 2900, 3000, 3250, 3350, 3500, 3750, 4000, 4250, 4500, 4600, 4750, 5000, 5500, 6000, 6500, 7000, 7500, 8000, 10,000, 12,000, 20,000, 35,000, 40,000, 50,000, 60,000, or 100,000 Da.

In some embodiments, the polyalkylene oxide (e.g., PEG) is a discrete PEG, in which the discrete PEG is a polymeric PEG comprising more than one repeating ethylene oxide units. In some instances, a discrete PEG (dPEG) comprises from 2 to 60, from 2 to 50, or from 2 to 48 repeating ethylene oxide units. In some instances, a dPEG comprises about 2, 3, 4, 5, 6, 7, 8, 9, 10, 11, 12, 13, 14, 15, 16, 17, 18, 19, 20, 22, 24, 26, 28, 30, 35, 40, 42, 48, 50 or more repeating ethylene oxide units. In some instances, a dPEG comprises about 2 or more repeating ethylene oxide units. In some cases, a dPEG is synthesized as a single molecular weight compound from pure (e.g., about 95%, 98%, 99%, or 99.5%) staring material in a step-wise fashion. In some cases, a dPEG has a specific molecular weight, rather than an average molecular weight.

In some instances, the linker is a discrete PEG, optionally comprising from 2 to 60, from 2 to 50, or from 2 to 48 repeating ethylene oxide units. In some cases, the linker comprises a dPEG comprising about 2, 3, 4, 5, 6, 7, 8, 9, 10, 11, 12, 13, 14, 15, 16, 17, 18, 19, 20, 22, 24, 26, 28, 30, 35, 40, 42, 48, 50 or more repeating ethylene oxide units.

In some embodiments, the linker is a polypeptide linker. In some instances, the polypeptide linker comprises at least 2, 3, 4, 5, 6, 7, 8, 10, 15, 20, 25, 30, 35, 40, 45, 50, 60, 70, 80, 90, 100, or more amino acid residues. In some instances, the polypeptide linker comprises at least 2, 3, 4, 5, 6, 7, 8, or more amino acid residues. In some instances, the polypeptide linker comprises at most 2, 3, 4, 5, 6, 7, 8, or less amino acid residues. In some cases, the polypeptide linker is a cleavable polypeptide linker (e.g., either enzymatically or chemically). In some cases, the polypeptide linker is a non-cleavable polypeptide linker. In some instances, the polypeptide linker comprises Val-Cit (valine-citrulline), Gly-Gly-Phe-Gly, Phe-Lys, Val-Lys, Gly-Phe-Lys, Phe-Phe-Lys, Ala-Lys, Val-Arg, Phe-Cit, Phe-Arg, Leu-Cit, Ile-Cit, Trp-Cit, Phe-Ala, Ala-Leu-Ala-Leu, or Gly-Phe-Leu-Gly. In some instances, the polypeptide linker comprises a peptide such as: Val-Cit (valine-citrulline), Gly-Gly-Phe-Gly, Phe-Lys, Val-Lys, Gly-Phe-Lys, Phe-Phe-Lys, Ala-Lys, Val-Arg, Phe-Cit, Phe-Arg, Leu-Cit, Ile-Cit, Trp-Cit, Phe-Ala, Ala-Leu-Ala-Leu, or Gly-Phe-Leu-Gly. In some cases, the polypeptide linker comprises L-amino acids, D-amino acids, or a mixture of both L- and D-amino acids.

In some instances, the linker comprises a homobifuctional linker. Exemplary homobifuctional linkers include, but are not limited to, Lomant's reagent dithiobis (succinimidylpropionate) DSP, 3'3'-dithiobis(sulfosuccinimidyl proprionate (DTSSP), disuccinimidyl suberate (DSS), bis(sulfosuccinimidyl)suberate (BS), disuccinimidyl tartrate (DST), disulfosuccinimidyl tartrate (sulfo DST), ethylene glycobis(succinimidylsuccinate) (EGS), disuccinimidyl glutarate (DSG), N,N'-disuccinimidyl carbonate (DSC), dimethyl adipimidate (DMA), dimethyl pimelimidate (DMP), dimethyl suberimidate (DMS), dimethyl-3,3'-dithiobispropionimidate (DTBP), 1,4-di-3'-(2'-pyridyldithio)propionamido)butane (DPDPB), bismaleimidohexane (BMH), aryl halide-containing compound (DFDNB), such as e.g. 1,5-difluoro-2,4-dinitrobenzene or 1,3-difluoro-4,6-dinitrobenzene, 4,4'-difluoro-3,3'-dinitrophenylsulfone (DFDNPS), bis-[β-(4-azidosalicylamido)ethyl]disulfide (BASED), formaldehyde, glutaraldehyde, 1,4-butanediol diglycidyl ether, adipic acid dihydrazide, carbohydrazide, o-toluidine, 3,3'-dimethylbenzidine, benzidine, α,α'-p-diaminodiphenyl, diiodo-p-xylene sulfonic acid, N,N'-ethylene-bis(iodoacetamide), or N,N'-hexamethylene-bis(iodoacetamide).

In some embodiments, the linker comprises a heterobifunctional linker. Exemplary heterobifunctional linker include, but are not limited to, amine-reactive and sulfhydryl cross-linkers such as N-succinimidyl 3-(2-pyridyldithio)propionate (sPDP), long-chain N-succinimidyl 3-(2-pyridyldithio)propionate (LC-sPDP), water-soluble-long-chain N-succinimidyl 3-(2-pyridyldithio) propionate (sulfo-LC-sPDP), succinimidyloxycarbonyl-α-methyl-α-(2-pyridyldithio)toluene (sMPT), sulfosuccinimidyl-6-[α-methyl-α-(2-pyridyldithio)toluamido]hexanoate (sulfo-LC-sMPT), succinimidyl-4-(N-maleimidomethyl)cyclohexane-1-carboxylate (sMCC), sulfosuccinimidyl-4-(N-maleimidomethyl)cyclohexane-1-carboxylate (sulfo-sMCC), m-maleimidobenzoyl-N-hydroxysuccinimide ester (MBs), m-maleimidobenzoyl-N-hydroxysulfosuccinimide ester (sulfo-MBs), N-succinimidyl(4-iodoacteyl)aminobenzoate (sIAB), sulfosuccinimidyl(4-iodoacteyl)aminobenzoate (sulfo-sIAB), succinimidyl-4-(p-maleimidophenyl)butyrate (sMPB), sulfosuccinimidyl-4-(p-maleimidophenyl)butyrate (sulfo-sMPB), N-(γ-maleimidobutyryloxy)succinimide ester (GMBs), N-(γ-maleimidobutyryloxy)sulfosuccinimide ester (sulfo-GMBs), succinimidyl 6-((iodoacetyl)amino)hexanoate (sIAX), succinimidyl 6-[6-(((iodoacetyl)amino)hexanoyl)amino]hexanoate (sIAXX), succinimidyl 4-(((iodoacetyl)amino)methyl)cyclohexane-1-carboxylate (sIAC), succinimidyl 6-((((4-iodoacetyl)amino)methyl)cyclohexane-1-carbonyl)amino) hexanoate (sIACX), p-nitrophenyl iodoacetate (NPIA), carbonyl-reactive and sulfhydryl-reactive cross-linkers such as 4-(4-N-maleimidophenyl)butyric acid hydrazide (MPBH), 4-(N-maleimidomethyl)cyclohexane-1-carboxyl-hydrazide-8 (M₂C₂H), 3-(2-pyridyldithio)propionyl hydrazide (PDPH), amine-reactive and photoreactive cross-linkers such as N-hydroxysuccinimidyl-4-azidosalicylic acid (NHs-AsA), N-hydroxysulfosuccinimidyl-4-azidosalicylic acid (sulfo-NHs-AsA), sulfosuccinimidyl-(4-azidosalicylamido)hexanoate (sulfo-NHs-LC-AsA), sulfosuccinimidyl-2-(ρ-azidosalicylamido)ethyl-1,3'-dithiopropionate (sAsD), N-hydroxysuccinimidyl-4-azidobenzoate (HsAB), N-hydroxysulfosuccinimidyl-4-azidobenzoate (sulfo-HsAB), N-succinimidyl-6-(4'-azido-2'-nitrophenylamino)hexanoate (sANPAH), sulfosuccinimidyl-6-(4'-azido-2'-nitrophenylamino)hexanoate (sulfo-sANPAH), N-5-azido-2-nitrobenzoyloxysuccinimide (ANB-NOs), sulfosuccinimidyl-2-(m-azido-o-nitrobenzamido)-ethyl-1,3'-dithiopropionate (sAND), N-succinimidyl-4(4-azidophenyl)1,3'-dithiopropionate (sADP), N-sulfosuccinimidyl(4-azidophenyl)-1,3'-dithiopropionate (sulfo-sADP), sulfosuccinimidyl 4-(ρ-azidophenyl)butyrate (sulfo-sAPB), sulfosuccinimidyl 2-(7-azido-4-methylcoumarin-3-acetamide)ethyl-1,3'-dithiopropionate (sAED), sulfosuccinimidyl 7-azido-4-methylcoumain-3-acetate (sulfo-sAMCA), ρ-nitrophenyl diazopyruvate (ρNPDP), ρ-nitrophenyl-2-diazo-3,3,3-trifluoropropionate (PNP-DTP), sulfhydryl-reactive and photoreactive cross-linkers such as1-(ρ-Azidosalicylamido)-4-(iodoacetamido)butane (AsIB), N-[4-(ρ-azidosalicylamido)butyl]-3'-(2'-pyridyldithio)propionamide (APDP), benzophenone-4-iodoacetamide, benzophenone-4-maleimide carbonyl-reactive and photoreactive cross-linkers such as ρ-azidobenzoyl hydrazide (ABH), carboxylate-reactive and photoreactive cross-linkers such as 4-(ρ-azidosalicylamido)butylamine (AsBA), and arginine-reactive and photoreactive cross-linkers such as ρ-azidophenyl glyoxal (APG).

In some embodiments, the linker comprises a benzoic acid group, or its derivatives thereof. In some instances, the benzoic acid group or its derivatives thereof comprise paraaminobenzoic acid (PABA). In some instances, the benzoic acid group or its derivatives thereof comprise gamma-aminobutyric acid (GABA).

In some embodiments, the linker comprises one or more of a maleimide group, a peptide moiety, and/or a benzoic acid group, in any combination. In some embodiments, the linker comprises a combination of a maleimide group, a peptide moiety, and/or a benzoic acid group. In some instances, the maleimide group is maleimidocaproyl (mc). In some instances, the peptide group is val-cit. In some instances, the benzoic acid group is PABA. In some instances, the linker comprises a mc-val-cit group. In some cases, the linker comprises a val-cit-PABA group. In additional cases, the linker comprises a mc-val-cit-PABA group.

In some embodiments, the linker is a self-immolative linker or a self-elimination linker. In some cases, the linker is a self-immolative linker. In other cases, the linker is a self-elimination linker (e.g., a cyclization self-elimination linker). In some instances, the linker comprises a linker described in U.S. Patent No. 9,089,614 or PCT Publication No. WO2015038426.

In some embodiments, the linker is a dendritic type linker. In some instances, the dendritic type linker comprises a branching, multifunctional linker moiety. In some instances, the dendritic type linker comprises PAMAM dendrimers.

In some embodiments, the linker is a traceless linker or a linker in which after cleavage does not leave behind a linker moiety (e.g., an atom or a linker group) to the antibody or payload. Exemplary traceless linkers include, but are not limited to, germanium linkers, silicium linkers, sulfur linkers, selenium linkers, nitrogen linkers, phosphorus linkers, boron linkers, chromium linkers, or phenylhydrazide linker. In some cases, the linker is a traceless aryl-triazene linker as described in Hejesen, et al., "A traceless aryl-triazene linker for DNA-directed chemistry," Org Biomol Chem 11(15): 2493-2497 (2013). In some instances, the linker is a traceless linker described in Blaney, et al., "Traceless solid-phase organic synthesis," Chem. Rev. 102: 2607-2024 (2002). In some instances, a linker is a traceless linker as described in U.S. Patent No. 6,821,783.

### Pharmaceutical Formulations

A pharmaceutical formulation for treating a disease as described herein can comprise an anti-Gal3 antibody or binding fragment thereof described *supra.* The anti-Gal3 antibody or binding fragment thereof can be formulated for systemic administration. Alternatively, the anti-Gal3 antibody or binding fragment thereof can be formulated for parenteral administration.

In some embodiments, an anti-Gal3 antibody or binding fragment thereof is formulated as a pharmaceutical composition for administration to a subject by, but not limited to, parenteral (e.g., intravenous, subcutaneous, intramuscular, intraarterial, intradermal, intraperitoneal, intravitreal, intracerebral, or intracerebroventricular), oral, intranasal, buccal, rectal, or transdermal administration routes. In some instances, the pharmaceutical composition describe herein is formulated for parenteral (e.g., intravenous, subcutaneous, intramuscular, intraarterial, intradermal, intraperitoneal, intravitreal, intracerebral, or intracerebroventricular) administration. In other instances, the pharmaceutical composition describe herein is formulated for systemic administration. In other instances, the pharmaceutical composition describe herein is formulated for oral administration. In still other instances, the pharmaceutical composition describe herein is formulated for intranasal administration.

In some instances, the pharmaceutical compositions further include pH adjusting agents or buffering agents which include acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

In some instances, the pharmaceutical compositions include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

In some instances, the pharmaceutical compositions further include diluent which are used to stabilize compounds because they can provide a more stable environment. Salts dissolved in buffered solutions (which also can provide pH control or maintenance) are utilized as diluents in the art, including, but not limited to a phosphate buffered saline solution. In certain instances, diluents increase bulk of the composition to facilitate compression or create sufficient bulk for homogenous blend for capsule filling. Such compounds can include e.g., lactose, starch, mannitol, sorbitol, dextrose, microcrystalline cellulose such as Avicel^{®}; dibasic calcium phosphate, dicalcium phosphate dihydrate; tricalcium phosphate, calcium phosphate; anhydrous lactose, spray-dried lactose; pregelatinized starch, compressible sugar, such as Di-Pac^{®} (Amstar); mannitol, hydroxypropylmethylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylose; powdered cellulose, calcium carbonate; glycine, kaolin; mannitol, sodium chloride; inositol, bentonite, and the like.

In some embodiments, the pharmaceutical formulations include, but are not limited to, aqueous liquid dispersions, self-emulsifying dispersions, solid solutions, liposomal dispersions, aerosols, solid dosage forms, powders, immediate release formulations, controlled release formulations, fast melt formulations, tablets, capsules, pills, delayed release formulations, extended release formulations, pulsatile release formulations, multiparticulate formulations (e.g., nanoparticle formulations), and mixed immediate and controlled release formulations.

In some instances, the pharmaceutical formulation can further comprise an additional therapeutic agent. Non-limiting examples of additional therapeutic agents include alpha-glucosidase inhibitors, including acarbose (Precose^{®}) and miglitol (Glyset^{®}); biguanides, including metformin-alogliptin (Kazano^{®}), metformin-canagliflozin (Invokamet^{®}), metformin-dapagliflozin (Xigduo^{®} XR), metformin-empagliflozin (Synjardy^{®}), metformin-glipizide, metformin-glyburide (Glucovance^{®}), metformin-linagliptin (Jentadueto^{®}), metformin-pioglitazone (Actoplus^{®}), metformin-repaglinide (PrandiMet^{®}), metformin-rosiglitazone (Avandamet^{®}), metformin-saxagliptin (Kombiglyze^{®} XR), and metformin-sitagliptin (Janumet^{®}); dopamine agonists, including Bromocriptine (Cycloset^{®}); Dipeptidyl peptidase-4 (DPP-4) inhibitors, including alogliptin (Nesina^{®}), alogliptin-metformin (Kazano^{®}), alogliptin-pioglitazone (Oseni^{®}), linagliptin (Tradjenta^{®}), linagliptin-empagliflozin (Glyxambi^{®}), linagliptin-metformin (Jentadueto^{®}), saxagliptin (Onglyza^{®}), saxagliptin-metformin (Kombiglyze^{®} XR), sitagliptin (Januvia^{®}), sitagliptin-metformin (Janumet^{®} and Janumet^{®} XR), and sitagliptin and simvastatin (Juvisync^{®}); Glucagon-like peptide-1 receptor agonists (GLP-1 receptor agonists), including albiglutide (Tanzeum^{®}), dulaglutide (Trulicity^{®}), exenatide (Byetta^{®}), exenatide extended-release (Bydureon^{®}), and liraglutide (Victoza^{®}), semaglutide (Ozempic^{®}); Meglitinides, including nateglinide (Starlix^{®}), repaglinide (Prandin^{®}), and repaglinide-metformin (Prandimet^{®}); Sodium-glucose transporter (SGLT) 2 inhibitors, including dapagliflozin (Farxiga^{®}), dapagliflozin-metformin (Xigduo^{®} XR), canagliflozin (Invokana^{®}), canagliflozin-metformin (Invokamet^{®}), empagliflozin (Jardiance^{®}), empagliflozin-linagliptin (Glyxambi^{®}), empagliflozin-metformin (Synjardy^{®}), and ertugliflozin (Steglatro^{®}); Sulfonylureas, including glimepiride (Amaryl^{®}), glimepiride-pioglitazone (Duetact^{®}), glimepiride-rosiglitazone (Avandaryl^{®}), gliclazide, glipizide (Glucotrol^{®}), glipizide-metformin (Metaglip^{®}), glyburide (DiaBeta^{®}, Glynase^{®}, Micronase^{®}), glyburide-metformin (Glucovance^{®}), chlorpropamide (Diabinese^{®}), tolazamide (Tolinase^{®}), and tolbutamide (Orinase^{®}, Tol-Tab^{®}); Thiazolidinediones, including rosiglitazone (Avandia^{®}), rosiglitazone-glimepiride (Avandaryl^{®}), rosiglitazone-metformin (Amaryl M^{®}), pioglitazone (Actos^{®}), pioglitazone-alogliptin (Oseni^{®}), pioglitazone-glimepiride (Duetact^{®}), pioglitazone-metformin (Actoplus Met^{®}, Actoplus Met^{®} XR).

### Therapeutic Regimens

In some embodiments, the anti-Gal3 antibodies or binding fragments thereof disclosed herein are administered for therapeutic applications. In some embodiments, the anti-Gal3 antibody or binding fragment thereof is administered once per day, twice per day, three times per day or more. The anti-Gal3 antibody or binding fragment thereof is administered daily, every day, every alternate day, five days a week, once a week, every other week, two weeks per month, three weeks per month, once a month, twice a month, three times per month, or more. The anti-Gal3 antibody is administered for at least 1 month, 2 months, 3 months, 4 months, 5 months, 6 months, 7 months, 8 months, 9 months, 10 months, 11 months, 12 months, 18 months, 2 years, 3 years, or more.

In the case wherein the patient's status does improve, upon the doctor's discretion the administration of the anti-Gal3 antibody or binding fragment thereof is given continuously; alternatively, the dose of the anti-Gal3 antibody or binding fragment thereof being administered is temporarily reduced or temporarily suspended for a certain length of time (i.e., a "drug holiday"). In some instances, the length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday is from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's condition has occurred, a maintenance dose is administered if necessary. Subsequently, the dosage or the frequency of administration, or both, can be reduced, as a function of the symptoms, to a level at which the treated disease, disorder, or condition is retained.

In some embodiments, the amount of a given agent that correspond to such an amount varies depending upon factors such as the particular compound, the severity of the disease, the identity (e.g., weight) of the subject or host in need of treatment, but nevertheless is routinely determined in a manner known in the art according to the particular circumstances surrounding the case, including, e.g., the specific agent being administered, the route of administration, and the subject or host being treated. In some instances, the desired dose is conveniently presented in a single dose or as divided doses administered simultaneously (or over a short period of time) or at appropriate intervals, for example as two, three, four or more sub-doses per day.

The foregoing ranges are merely suggestive, as the number of variables in regard to an individual treatment regime is large, and considerable excursions from these recommended values are not uncommon. Such dosages are altered depending on a number of variables, not limited to the activity of the compound used, the disease or condition to be treated, the mode of administration, the requirements of the individual subject, the severity of the disease or condition being treated, and the judgment of the practitioner.

In some embodiments, toxicity and therapeutic efficacy of such therapeutic regimens are determined by standard pharmaceutical procedures in cell cultures or experimental animals, including, but not limited to, the determination of the LD50 (the dose lethal to 50% of the population) and the ED50 (the dose therapeutically effective in 50% of the population). The dose ratio between the toxic and therapeutic effects is the therapeutic index and it is expressed as the ratio between LD50 and ED50. Compounds exhibiting high therapeutic indices are preferred. The data obtained from cell culture assays and animal studies are used in formulating a range of dosage for use in human. The dosage of such compounds lies preferably within a range of circulating concentrations that include the ED50 with minimal toxicity. The dosage varies within this range depending upon the dosage form employed and the route of administration utilized.

### Kit/Article of Manufacture

Disclosed herein, in certain embodiments, are kits and articles of manufacture for use with one or more of the compositions and methods described herein. Such kits include a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. In one embodiment, the containers are formed from a variety of materials such as glass or plastic.

The articles of manufacture provided herein contain packaging materials. Examples of pharmaceutical packaging materials include, but are not limited to, blister packs, bottles, tubes, bags, containers, bottles, and any packaging material suitable for a selected formulation and intended mode of administration and treatment.

For example, the container(s) include an anti-Gal3 antibody as disclosed herein, host cells for producing one or more antibodies described herein, and/or vectors comprising nucleic acid molecules that encode the antibodies described herein. Such kits optionally include an identifying description or label or instructions relating to its use in the methods described herein.

A kit typically includes labels listing contents and/or instructions for use, and package inserts with instructions for use. A set of instructions will also typically be included.

In one embodiment, a label is on or associated with the container. In one embodiment, a label is on a container when letters, numbers or other characters forming the label are attached, molded or etched into the container itself; a label is associated with a container when it is present within a receptacle or carrier that also holds the container, e.g., as a package insert. In one embodiment, a label is used to indicate that the contents are to be used for a specific therapeutic application. The label also indicates directions for use of the contents, such as in the methods described herein.

In certain embodiments, the pharmaceutical compositions are presented in a pack or dispenser device which contains one or more unit dosage forms containing a compound provided herein. The pack, for example, contains metal or plastic foil, such as a blister pack. In one embodiment, the pack or dispenser device is accompanied by instructions for administration. In one embodiment, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for prescription drugs, or the approved product insert. In one embodiment, compositions containing a compound provided herein formulated in a compatible pharmaceutical carrier are also prepared, placed in an appropriate container, and labeled for treatment of an indicated condition.

Some embodiments provided herein are described by way of the following provided numbered arrangements and also provided as possible combinations or overlapping embodiments:
1. A method of disrupting an interaction between galectin-3 (Gal3) and insulin receptor, the method comprising:
   contacting an interaction between Gal3 and insulin receptor with an antibody or binding fragment thereof that selectively binds to Gal3 and disrupts the interaction between Gal3 and insulin receptor.
2. The method of arrangement 1, wherein Gal3 is expressed by a cell.
3. The method of arrangement 1, wherein Gal3 is secreted by a cell.
4. The method of any one of arrangements 1-3, wherein insulin receptor is expressed by a cell.
5. The method of any one of arrangements 1-4, wherein the antibody or binding fragment thereof binds to an N-terminal domain of Gal3.
6. The method of any one of arrangements 1-5, wherein the antibody or binding fragment thereof binds to an epitope present within a region of Gal3 defined by Peptide 1 (ADNFSLHDALSGSGNPNPQG; **SEQ ID NO: 3**), Peptide 4 (GAGGYPGASYPGAYPGQAPP; **SEQ ID NO: 6**), Peptide 6 (GAYPGQAPPGAYPGAPGAYP; **SEQ ID NO: 8**), Peptide 7 (AYPGAPGAYPGAPAPGVYPG; **SEQ ID NO: 9**), or a combination thereof.
7. The method of any one of arrangements 1-6, wherein the antibody or binding fragment thereof binds to an epitope of Gal3 comprising an amino acid sequence of GxYPG, wherein X is alanine, glycine, or valine.
8. The method of any one of arrangements 1-7, wherein the interaction is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the antibody or binding fragment thereof.
9. The method of any one of arrangements 1-8, wherein the antibody or binding fragment thereof binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM.
10. The method of any one of arrangements 1-9, wherein the antibody comprises (1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein
   the V_{L}-CDR1 comprises an amino acid sequence having at least 60%, at least 70%, at least 80%, at least 90%, or 100% sequence identity to any amino acid sequence according to **SEQ ID NOs: 71-89,**
   the V_{L}-CDR2 comprises an amino acid sequence having at least 60%, at least 70%, at least 80%, at least 90%, or 100% sequence identity to any amino acid sequence according to **SEQ ID NOs: 90-100,**
   the V_{L}-CDR3 comprises an amino acid sequence having at least 60%, at least 70%, at least 80%, at least 90%, or 100% sequence identity to any amino acid sequence according to **SEQ ID NOs: 101-115,**
   the V_{H}-CDR1 comprises an amino acid sequence having at least 60%, at least 70%, at least 80%, at least 90%, or 100% sequence identity to any amino acid sequence according to **SEQ ID NOs: 27-38,**
   the V_{H}-CDR2 comprises an amino acid sequence having at least 60%, at least 70%, at least 80%, at least 90%, or 100% sequence identity to any amino acid sequence according to **SEQ ID NOs: 39-53,** and
   the V_{H}-CDR3 comprises an amino acid sequence having at least 60%, at least 70%, at least 80%, at least 90%, or 100% sequence identity to any amino acid sequence according to **SEQ ID NOs: 54-70.**
11. The method of any one of arrangements 1-10, wherein the antibody comprises a combination of a V_{L}-CDR1, a V_{L}-CDR2, a V_{L}-CDR3, a V_{H}-CDR1, a V_{H}-CDR2, and a V_{H}-CDR3 as illustrated in **Table 9.**
12. The method of any one of arrangements 1-11, wherein the antibody is selected from the group consisting of: 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, IMT-001 (TB001), and IMT-006 (TB006).
13. The method of any one of arrangements 1-12, wherein the antibody or binding fragment thereof comprises a humanized antibody.
14. The method of any one of arrangements 1-13, wherein the antibody or binding fragment thereof comprises a full-length antibody or a binding fragment thereof.
15. The method of any one of arrangements 1-14, wherein the antibody or binding fragment thereof comprises a bispecific antibody or a binding fragment thereof.
16. The method of any one of arrangements 1-15, wherein the antibody or binding fragment thereof comprises a monovalent Fab', a divalent Fab2, a single-chain variable fragment (scFv), a diabody, a minibody, a nanobody, a single-domain antibody (sdAb), or a camelid antibody, or binding fragment thereof.
17. The method of any one of arrangements 1-16, wherein the antibody or binding fragment thereof comprises an IgG framework.
18. The method of any one of arrangements 1-17, wherein the antibody or binding fragment thereof comprises an IgG1, IgG2, or IgG4 framework.
19. A method of treating diabetes mellitus in a subject in need thereof, the method comprising: administering to the subject an antibody or binding fragment thereof that selectively binds to Gal3 and disrupts an interaction between Gal3 and insulin receptor, thereby treating diabetes mellitus in the subject.
20. The method of arrangement 19, wherein the diabetes mellitus is insulin-dependent diabetes mellitus.
21. The method of arrangement 19, wherein the diabetes mellitus is insulin-independent diabetes mellitus.
22. The method of arrangement 19, wherein the diabetes mellitus is Type I diabetes mellitus.
23. The method of arrangement 19, wherein the diabetes mellitus is Type II diabetes mellitus.
24. The method of any one of arrangements 19-23, wherein the treating comprises decreasing glucose tolerance in the subject in need thereof.
25. The method of any one of arrangements 19-24, wherein the treating comprises decreasing insulin sensitivity in the subject in need thereof.
26. The method of any one of arrangements 19-25, wherein the treating comprises reducing weight gain in the subject in need thereof.
27. The method of any one of arrangements 19-26, wherein the treating comprises reducing liver steatosis in the subject in need thereof.
28. A method of treating non-alcoholic fatty liver disease (NAFLD) in a subject in need thereof, the method comprising administering to the subject an antibody or binding fragment thereof that selectively binds to Gal3 and disrupts an interaction between Gal3 and insulin receptor, thereby treating NAFLD in the subject.
29. A method of treating non-alcoholic steatohepatitis (NASH) in a subject in need thereof, the method comprising administering to the subject an antibody or binding fragment thereof that selectively binds to Gal3 and disrupts an interaction between Gal3 and insulin receptor, thereby treating NASH in the subject.
30. The method of any one of arrangements 19-29, wherein the antibody or binding fragment thereof binds to an N-terminal domain of Gal3.
31. The method of any one of arrangements 19-30, wherein the antibody or binding fragment thereof binds to an epitope present within a region of Gal3 defined by Peptide 1 (ADNFSLHDALSGSGNPNPQG; **SEQ ID NO: 3**), Peptide 4 (GAGGYPGASYPGAYPGQAPP; **SEQ ID NO: 6**), Peptide 6 (GAYPGQAPPGAYPGAPGAYP; **SEQ ID NO: 8**), Peptide 7 (AYPGAPGAYPGAPAPGVYPG; **SEQ ID NO: 9**), or a combination thereof.
32. The method of any one of arrangements 19-31, wherein the antibody or binding fragment thereof binds to an epitope of Gal3 comprising an amino acid sequence of GxYPG, wherein X is alanine, glycine, or valine.
33. The method of any one of arrangements 19-32, wherein the interaction is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the antibody or binding fragment thereof.
34. The method of any one of arrangements 19-33, wherein the antibody or binding fragment thereof binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM.
35. The method of any one of arrangements 19-34, wherein the antibody comprises (1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein
   the V_{L}-CDR1 comprises an amino acid sequence having at least 60%, at least 70%, at least 80%, at least 90%, or 100% sequence identity to any amino acid sequence according to **SEQ ID NOs: 71-89,**
   the V_{L}-CDR2 comprises an amino acid sequence having at least 60%, at least 70%, at least 80%, at least 90%, or 100% sequence identity to any amino acid sequence according to **SEQ ID NOs: 90-100,**
   the V_{L}-CDR3 comprises an amino acid sequence having at least 60%, at least 70%, at least 80%, at least 90%, or 100% sequence identity to any amino acid sequence according to **SEQ ID NOs: 101-115,**
   the V_{H}-CDR1 comprises an amino acid sequence having at least 60%, at least 70%, at least 80%, at least 90%, or 100% sequence identity to any amino acid sequence according to **SEQ ID NOs: 27-38,**
   the V_{H}-CDR2 comprises an amino acid sequence having at least 60%, at least 70%, at least 80%, at least 90%, or 100% sequence identity to any amino acid sequence according to **SEQ ID NOs: 39-53,** and
   the V_{H}-CDR3 comprises an amino acid sequence having at least 60%, at least 70%, at least 80%, at least 90%, or 100% sequence identity to any amino acid sequence according to **SEQ ID NOs: 54-70.**
36. The method of any one of arrangements 19-35, wherein the antibody comprises a combination of a V_{L}-CDR1, a V_{L}-CDR2, a V_{L}-CDR3, a V_{H}-CDR1, a V_{H}-CDR2, and a V_{H}-CDR3 as illustrated in **Table 9.**
37. The method of any one of arrangements 19-36, wherein the antibody is selected from the group consisting of: 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, IMT-001, and IMT-006.
38. The method of any one of arrangements 19-37, wherein the antibody or binding fragment thereof comprises a humanized antibody.
39. The method of any one of arrangements 19-38, wherein the antibody or binding fragment thereof comprises a full-length antibody or a binding fragment thereof.
40. The method of any one of arrangements 19-39, wherein the antibody or binding fragment thereof comprises a bispecific antibody or a binding fragment thereof.
41. The method of any one of arrangements 19-40, wherein the antibody or binding fragment thereof comprises a monovalent Fab', a divalent Fab2, a single-chain variable fragment (scFv), a diabody, a minibody, a nanobody, a single-domain antibody (sdAb), or a camelid antibody, or binding fragment thereof.
42. The method of any one of arrangements 19-41, wherein the antibody or binding fragment thereof comprises an IgG framework.
43. The method of any one of arrangements 19-42, wherein the antibody or binding fragment thereof comprises an IgG1, IgG2, or IgG4 framework.
44. The method of any one of arrangements 19-43, wherein the subject is diagnosed with Type I diabetes or Type II diabetes.
45. The method of any one of arrangements 19-44, wherein the antibody or binding fragment thereof is formulated for systemic administration.
46. The method of any one of arrangements 19-45, wherein the antibody or binding fragment thereof is formulated for parenteral administration.
47. The method of any one of arrangements 19-46, wherein the subject is a mammal.
48. The method of arrangement 47, wherein the mammal is a human.
49. An anti-Gal3 antibody comprising (1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein
   the V_{L}-CDR1 comprises an amino acid sequence having at least 60%, at least 70%, at least 80%, at least 90%, or 100% sequence identity to any amino acid sequence according to **SEQ ID NOs: 71-89,**
   the V_{L}-CDR2 comprises an amino acid sequence having at least 60%, at least 70%, at least 80%, at least 90%, or 100% sequence identity to any amino acid sequence according to **SEQ ID NOs: 90-100,**
   the V_{L}-CDR3 comprises an amino acid sequence having at least 60%, at least 70%, at least 80%, at least 90%, or 100% sequence identity to any amino acid sequence according to **SEQ ID NOs: 101-115,**
   the V_{H}-CDR1 comprises an amino acid sequence having at least 60%, at least 70%, at least 80%, at least 90%, or 100% sequence identity to any amino acid sequence according to **SEQ ID NOs: 27-38,**
   the V_{H}-CDR2 comprises an amino acid sequence having at least 60%, at least 70%, at least 80%, at least 90%, or 100% sequence identity to any amino acid sequence according to **SEQ ID NOs: 39-53,** and
   the V_{H}-CDR3 comprises an amino acid sequence having at least 60%, at least 70%, at least 80%, at least 90%, or 100% sequence identity to any amino acid sequence according to **SEQ ID NOs: 54-70.**
50. The anti-Gal3 antibody of arrangement 49, wherein the anti-Gal3 antibody comprises a combination of a V_{L}-CDR1, a V_{L}-CDR2, a V_{L}-CDR3, a V_{H}-CDR1, a V_{H}-CDR2, and a V_{H}-CDR3 as illustrated in **Table 9.**
51. The anti-Gal3 antibody of arrangement 49 or 50, wherein the anti-Gal3 antibody is selected from the group consisting of: 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, 7D8.2D8, 15F10.2D6, IMT-001 (TB001), and IMT-006 (TB006).
52. An antibody that binds to human Gal3 and competes with an anti-Gal3 antibody for binding to human Gal3, wherein the anti-Gal3 antibody is selected from the group consisting of: 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, 7D8.2D8, and 15F10.2D6, IMT001, and IMT006.
53. A method for identifying an antibody capable of disrupting an interaction between Gal3 and insulin receptor, the method comprising:
   (a) contacting Gal3 protein with an antibody that selectively binds to Gal3, thereby forming a Gal3-antibody complex;
   (b) contacting the Gal3-antibody complex with insulin receptor protein;
   (c) removing unbound insulin receptor protein; and
   (d) detecting insulin receptor protein bound to the Gal3-antibody complex;
      wherein the antibody is capable of disrupting an interaction of Gal3 and insulin receptor when insulin receptor protein is not detected in (d).
54. The method of arrangement 53, wherein the method comprises an immunoassay.
55. The method of arrangement 54, wherein the immunoassay is an enzyme-linked immunosorbent assay.
56. A method of disrupting an interaction between galectin-3 (Gal3) and insulin receptor or an integrin, or both, the method comprising:
   contacting an interaction between Gal3 and the insulin receptor or the integrin, or both, with an anti-Gal3 antibody or binding fragment thereof that selectively binds to Gal3 and disrupts the interaction between Gal3 and the insulin receptor or the integrin, or both.
57. The method of arrangement 56, wherein Gal3 is expressed by a cell.
58. The method of arrangement 56, wherein Gal3 is secreted by a cell.
59. The method of any one of arrangements 56-58, wherein the insulin receptor or the integrin, or both, is expressed by a cell.
60. The method of any one of arrangements 56-59, wherein the anti-Gal3 antibody or binding fragment thereof binds to an N-terminal domain of Gal3.
61. The method of any one of arrangements 56-60, wherein the anti-Gal3 antibody or binding fragment thereof binds to one or more peptides of **SEQ ID NOs: 3-26.**
62. The method of any one of arrangements 56-61, wherein the anti-Gal3 antibody or binding fragment thereof binds to an epitope present within a region of Gal3 defined by Peptide 1 (ADNFSLHDALSGSGNPNPQG; **SEQ ID NO: 3**), Peptide 4 (GAGGYPGASYPGAYPGQAPP; **SEQ ID NO: 6**), Peptide 6 (GAYPGQAPPGAYPGAPGAYP; **SEQ ID NO: 8**), Peptide 7 (AYPGAPGAYPGAPAPGVYPG; **SEQ ID NO: 9**), or a combination thereof.
63. The method of any one of arrangements 56-62, wherein the anti-Gal3 antibody or binding fragment thereof binds to an epitope of Gal3 comprising an amino acid sequence of GxYPG, wherein x is alanine, glycine, or valine.
64. The method of any one of arrangements 56-63, wherein the interaction is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the anti-Gal3 antibody or binding fragment thereof.
65. The method of any one of arrangements 56-64, wherein the anti-Gal3 antibody or binding fragment thereof binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM.
66. The method of any one of arrangements 56-65, wherein the anti-Gal3 antibody or binding fragment thereof comprises (1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein
   the V_{L}-CDR1 comprises an amino acid sequence having at least 60%, at least 70%, at least 80%, at least 90%, or 100% sequence identity to any amino acid sequence according to **SEQ ID NOs: 100-128,**
   the V_{L}-CDR2 comprises an amino acid sequence having at least 60%, at least 70%, at least 80%, at least 90%, or 100% sequence identity to any amino acid sequence according to **SEQ ID NOs: 129-144,**
   the V_{L}-CDR3 comprises an amino acid sequence having at least 60%, at least 70%, at least 80%, at least 90%, or 100% sequence identity to any amino acid sequence according to **SEQ ID NOs: 145-168,**
   the V_{H}-CDR1 comprises an amino acid sequence having at least 60%, at least 70%, at least 80%, at least 90%, or 100% sequence identity to any amino acid sequence according to **SEQ ID NOs: 27-47,**
   the V_{H}-CDR2 comprises an amino acid sequence having at least 60%, at least 70%, at least 80%, at least 90%, or 100% sequence identity to any amino acid sequence according to **SEQ ID NOs: 48-69,** and
   the V_{H}-CDR3 comprises an amino acid sequence having at least 60%, at least 70%, at least 80%, at least 90%, or 100% sequence identity to any amino acid sequence according to **SEQ ID NOs: 70-99.**
67. The method of arrangement 66, wherein the heavy chain variable region comprises an amino acid sequence having at least 75%, 80%, 85%, 90%, 95%, or 100% sequence identity to any sequence according to **SEQ ID NOs: 169-206.**
68. The method of arrangement 66 or 67, wherein the heavy chain variable region is selected from the group consisting of **SEQ ID NOs: 169-206.**
69. The method of any one of arrangements 66-68, wherein the light chain variable region comprises an amino acid sequence having at least 75%, 80%, 85%, 90%, 95%, or 100% sequence identity to any sequence according to **SEQ ID NOs: 207-245.**
70. The method of any one of arrangements 66-69, wherein the light chain variable region is selected from the group consisting of **SEQ ID NOs: 207-245.**
71. The method of any one of arrangements 56-70, wherein the anti-Gal3 antibody or binding fragment thereof comprises a combination of a V_{L}-CDR1, a V_{L}-CDR2, a V_{L}-CDR3, a V_{H}-CDR1, a V_{H}-CDR2, and a V_{H}-CDR3 as illustrated in **FIG. 36****.**
72. The method of any one of arrangements 56-71, wherein the anti-Gal3 antibody or binding fragment thereof comprises:
   a) the V_{H}-CDR1, V_{H}-CDR2, V_{H}-CDR3 of the V_{H}-CDR1, V_{H}-CDR2, V_{H}-CDR3 within **SEQ ID NO: 169** and the V_{L}-CDR1, V_{L}-CDR2, V_{L}-CDR3 of the V_{L}-CDR1, V_{L}-CDR2, V_{L}-CDR3 within **SEQ ID NO: 207;**
   b) the V_{H}-CDR1, V_{H}-CDR2, V_{H}-CDR3 of the V_{H}-CDR1, V_{H}-CDR2, V_{H}-CDR3 within **SEQ ID NO: 170** and the V_{L}-CDR1, V_{L}-CDR2, V_{L}-CDR3 of the V_{L}-CDR1, V_{L}-CDR2, V_{L}-CDR3 within **SEQ ID NO: 208;**
   c) the V_{H}-CDR1, V_{H}-CDR2, V_{H}-CDR3 of the V_{H}-CDR1, V_{H}-CDR2, V_{H}-CDR3 within **SEQ ID NO: 171** and the V_{L}-CDR1, V_{L}-CDR2, V_{L}-CDR3 of the V_{L}-CDR1, V_{L}-CDR2, V_{L}-CDR3 within **SEQ ID NO: 209;**
   d) the V_{H}-CDR1, V_{H}-CDR2, V_{H}-CDR3 of the V_{H}-CDR1, V_{H}-CDR2, V_{H}-CDR3 within **SEQ ID NO: 172** and the V_{L}-CDR1, V_{L}-CDR2, V_{L}-CDR3 of the V_{L}-CDR1, V_{L}-CDR2, V_{L}-CDR3 within **SEQ ID NO: 210;**
   e) the V_{H}-CDR1, V_{H}-CDR2, V_{H}-CDR3 of the V_{H}-CDR1, V_{H}-CDR2, V_{H}-CDR3 within **SEQ ID NO: 173** and the V_{L}-CDR1, V_{L}-CDR2, V_{L}-CDR3 of the V_{L}-CDR1, V_{L}-CDR2, V_{L}-CDR3 within **SEQ ID NO: 211;**
   f) the V_{H}-CDR1, V_{H}-CDR2, V_{H}-CDR3 of the V_{H}-CDR1, V_{H}-CDR2, V_{H}-CDR3 within **SEQ ID NO: 174** and the V_{L}-CDR1, V_{L}-CDR2, V_{L}-CDR3 of the V_{L}-CDR1, V_{L}-CDR2, V_{L}-CDR3 within **SEQ ID NO: 212;**
   g) the V_{H}-CDR1, V_{H}-CDR2, V_{H}-CDR3 of the V_{H}-CDR1, V_{H}-CDR2, V_{H}-CDR3 within **SEQ ID NO: 175** and the V_{L}-CDR1, V_{L}-CDR2, V_{L}-CDR3 of the V_{L}-CDR1, V_{L}-CDR2, V_{L}-CDR3 within **SEQ ID NO: 213;**
   h) the V_{H}-CDR1, V_{H}-CDR2, V_{H}-CDR3 of the V_{H}-CDR1, V_{H}-CDR2, V_{H}-CDR3 within **SEQ ID NO: 176** and the V_{L}-CDR1, V_{L}-CDR2, V_{L}-CDR3 of the V_{L}-CDR1, V_{L}-CDR2, V_{L}-CDR3 within **SEQ ID NO: 214;**
   i) the V_{H}-CDR1, V_{H}-CDR2, V_{H}-CDR3 of the V_{H}-CDR1, V_{H}-CDR2, V_{H}-CDR3 within **SEQ ID NO: 177** and the V_{L}-CDR1, V_{L}-CDR2, V_{L}-CDR3 of the V_{L}-CDR1, V_{L}-CDR2, V_{L}-CDR3 within **SEQ ID NO: 215;**
   j) the V_{H}-CDR1, V_{H}-CDR2, V_{H}-CDR3 of the V_{H}-CDR1, V_{H}-CDR2, V_{H}-CDR3 within **SEQ ID NO: 178** and the V_{L}-CDR1, V_{L}-CDR2, V_{L}-CDR3 of the V_{L}-CDR1, V_{L}-CDR2, V_{L}-CDR3 within **SEQ ID NO: 216;**
   k) the V_{H}-CDR1, V_{H}-CDR2, V_{H}-CDR3 of the V_{H}-CDR1, V_{H}-CDR2, V_{H}-CDR3 within **SEQ ID NO: 179** and the V_{L}-CDR1, V_{L}-CDR2, V_{L}-CDR3 of the V_{L}-CDR1, V_{L}-CDR2, V_{L}-CDR3 within **SEQ ID NO: 217;**
   1) the V_{H}-CDR1, V_{H}-CDR2, V_{H}-CDR3 of the V_{H}-CDR1, V_{H}-CDR2, V_{H}-CDR3 within **SEQ ID NO: 180** and the V_{L}-CDR1, V_{L}-CDR2, V_{L}-CDR3 of the V_{L}-CDR1, V_{L}-CDR2, V_{L}-CDR3 within **SEQ ID NO: 218;**
   m) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 219;**
   n) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 220;**
   o) the V_{H}-CDR1, V_{H}-CDR2, V_{H}-CDR3 of the V_{H}-CDR1, V_{H}-CDR2, V_{H}-CDR3 within **SEQ ID NO: 182** and the V_{L}-CDR1, V_{L}-CDR2, V_{L}-CDR3 of the V_{L}-CDR1, V_{L}-CDR2, V_{L}-CDR3 within **SEQ ID NO: 221;**
   p) the V_{H}-CDR1, V_{H}-CDR2, V_{H}-CDR3 of the V_{H}-CDR1, V_{H}-CDR2, V_{H}-CDR3 within **SEQ ID NO: 183** and the V_{L}-CDR1, V_{L}-CDR2, V_{L}-CDR3 of the V_{L}-CDR1, V_{L}-CDR2, V_{L}-CDR3 within **SEQ ID NO: 222;**
   q) the V_{H}-CDR1, V_{H}-CDR2, V_{H}-CDR3 of the V_{H}-CDR1, V_{H}-CDR2, V_{H}-CDR3 within **SEQ ID NO: 184** and the V_{L}-CDR1, V_{L}-CDR2, V_{L}-CDR3 of the V_{L}-CDR1, V_{L}-CDR2, V_{L}-CDR3 within **SEQ ID NO: 223;**
   r) the V_{H}-CDR1, V_{H}-CDR2, V_{H}-CDR3 of the V_{H}-CDR1, V_{H}-CDR2, V_{H}-CDR3 within **SEQ ID NO: 185** and the V_{L}-CDR1, V_{L}-CDR2, V_{L}-CDR3 of the V_{L}-CDR1, V_{L}-CDR2, V_{L}-CDR3 within **SEQ ID NO: 224;**
   s) the V_{H}-CDR1, V_{H}-CDR2, V_{H}-CDR3 of the V_{H}-CDR1, V_{H}-CDR2, V_{H}-CDR3 within **SEQ ID NO: 186** and the V_{L}-CDR1, V_{L}-CDR2, V_{L}-CDR3 of the V_{L}-CDR1, V_{L}-CDR2, V_{L}-CDR3 within **SEQ ID NO: 225;**
   t) the V_{H}-CDR1, V_{H}-CDR2, V_{H}-CDR3 of the V_{H}-CDR1, V_{H}-CDR2, V_{H}-CDR3 within **SEQ ID NO: 187** and the V_{L}-CDR1, V_{L}-CDR2, V_{L}-CDR3 of the V_{L}-CDR1, V_{L}-CDR2, V_{L}-CDR3 within **SEQ ID NO: 226;**
   u) the V_{H}-CDR1, V_{H}-CDR2, V_{H}-CDR3 of the V_{H}-CDR1, V_{H}-CDR2, V_{H}-CDR3 within **SEQ ID NO: 188** and the V_{L}-CDR1, V_{L}-CDR2, V_{L}-CDR3 of the V_{L}-CDR1, V_{L}-CDR2, V_{L}-CDR3 within **SEQ ID NO: 227;**
   v) the V_{H}-CDR1, V_{H}-CDR2, V_{H}-CDR3 of the V_{H}-CDR1, V_{H}-CDR2, V_{H}-CDR3 within **SEQ ID NO: 189** and the V_{L}-CDR1, V_{L}-CDR2, V_{L}-CDR3 of the V_{L}-CDR1, V_{L}-CDR2, V_{L}-CDR3 within **SEQ ID NO: 228;**
   w) the V_{H}-CDR1, V_{H}-CDR2, V_{H}-CDR3 of the V_{H}-CDR1, V_{H}-CDR2, V_{H}-CDR3 within **SEQ ID NO: 190** and the V_{L}-CDR1, V_{L}-CDR2, V_{L}-CDR3 of the V_{L}-CDR1, V_{L}-CDR2, V_{L}-CDR3 within **SEQ ID NO: 229;**
   x) the V_{H}-CDR1, V_{H}-CDR2, V_{H}-CDR3 of the V_{H}-CDR1, V_{H}-CDR2, V_{H}-CDR3 within **SEQ ID NO: 191** and the V_{L}-CDR1, V_{L}-CDR2, V_{L}-CDR3 of the V_{L}-CDR1, V_{L}-CDR2, V_{L}-CDR3 within **SEQ ID NO: 230;**
   y) the V_{H}-CDR1, V_{H}-CDR2, V_{H}-CDR3 of the V_{H}-CDR1, V_{H}-CDR2, V_{H}-CDR3 within **SEQ ID NO: 192** and the V_{L}-CDR1, V_{L}-CDR2, V_{L}-CDR3 of the V_{L}-CDR1, V_{L}-CDR2, V_{L}-CDR3 within **SEQ ID NO: 231;**
   z) the V_{H}-CDR1, V_{H}-CDR2, V_{H}-CDR3 of the V_{H}-CDR1, V_{H}-CDR2, V_{H}-CDR3 within **SEQ ID NO: 193** and the V_{L}-CDR1, V_{L}-CDR2, V_{L}-CDR3 of the V_{L}-CDR1, V_{L}-CDR2, V_{L}-CDR3 within **SEQ ID NO: 232;**
   aa) the V_{H}-CDR1, V_{H}-CDR2, V_{H}-CDR3 of the V_{H}-CDR1, V_{H}-CDR2, V_{H}-CDR3 within **SEQ ID NO: 194** and the V_{L}-CDR1, V_{L}-CDR2, V_{L}-CDR3 of the V_{L}-CDR1, V_{L}-CDR2, V_{L}-CDR3 within **SEQ ID NO: 233;**
   ab) the V_{H}-CDR1, V_{H}-CDR2, V_{H}-CDR3 of the V_{H}-CDR1, V_{H}-CDR2, V_{H}-CDR3 within **SEQ ID NO: 195** and the V_{L}-CDR1, V_{L}-CDR2, V_{L}-CDR3 of the V_{L}-CDR1, V_{L}-CDR2, V_{L}-CDR3 within **SEQ ID NO: 234;**
   ac) the V_{H}-CDR1, V_{H}-CDR2, V_{H}-CDR3 of the V_{H}-CDR1, V_{H}-CDR2, V_{H}-CDR3 within **SEQ ID NO: 196** and the V_{L}-CDR1, V_{L}-CDR2, V_{L}-CDR3 of the V_{L}-CDR1, V_{L}-CDR2, V_{L}-CDR3 within **SEQ ID NO: 235;**
   ad) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 236;**
   ae) the V_{H}-CDR1, V_{H}-CDR2, V_{H}-CDR3 of the V_{H}-CDR1, V_{H}-CDR2, V_{H}-CDR3 within **SEQ ID NO: 198** and the V_{L}-CDR1, V_{L}-CDR2, V_{L}-CDR3 of the V_{L}-CDR1, V_{L}-CDR2, V_{L}-CDR3 within **SEQ ID NO: 237;**
   af) the V_{H}-CDR1, V_{H}-CDR2, V_{H}-CDR3 of the V_{H}-CDR1, V_{H}-CDR2, V_{H}-CDR3 within **SEQ ID NO: 199** and the V_{L}-CDR1, V_{L}-CDR2, V_{L}-CDR3 of the V_{L}-CDR1, V_{L}-CDR2, V_{L}-CDR3 within **SEQ ID NO: 238;**
   ag) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 239;**
   ah) the V_{H}-CDR1, V_{H}-CDR2, V_{H}-CDR3 of the V_{H}-CDR1, V_{H}-CDR2, V_{H}-CDR3 within **SEQ ID NO: 200** and the V_{L}-CDR1, V_{L}-CDR2, V_{L}-CDR3 of the V_{L}-CDR1, V_{L}-CDR2, V_{L}-CDR3 within **SEQ ID NO: 240;**
   ai) the V_{H}-CDR1, V_{H}-CDR2, V_{H}-CDR3 of the V_{H}-CDR1, V_{H}-CDR2, V_{H}-CDR3 within **SEQ ID NO: 201** and the V_{L}-CDR1, V_{L}-CDR2, V_{L}-CDR3 of the V_{L}-CDR1, V_{L}-CDR2, V_{L}-CDR3 within **SEQ ID NO: 241;**
   aj) the V_{H}-CDR1, V_{H}-CDR2, V_{H}-CDR3 of the V_{H}-CDR1, V_{H}-CDR2, V_{H}-CDR3 within **SEQ ID NO: 202** and the V_{L}-CDR1, V_{L}-CDR2, V_{L}-CDR3 of the V_{L}-CDR1, V_{L}-CDR2, V_{L}-CDR3 within **SEQ ID NO: 242;**
   ak) the V_{H}-CDR1, V_{H}-CDR2, V_{H}-CDR3 of the V_{H}-CDR1, V_{H}-CDR2, V_{H}-CDR3 within **SEQ ID NO: 203** and the V_{L}-CDR1, V_{L}-CDR2, V_{L}-CDR3 of the V_{L}-CDR1, V_{L}-CDR2, V_{L}-CDR3 within **SEQ ID NO: 240;**
   al) the V_{H}-CDR1, V_{H}-CDR2, V_{H}-CDR3 of the V_{H}-CDR1, V_{H}-CDR2, V_{H}-CDR3 within **SEQ ID NO: 204** and the V_{L}-CDR1, V_{L}-CDR2, V_{L}-CDR3 of the V_{L}-CDR1, V_{L}-CDR2, V_{L}-CDR3 within **SEQ ID NO: 243;**
   am) the V_{H}-CDR1, V_{H}-CDR2, V_{H}-CDR3 of the V_{H}-CDR1, V_{H}-CDR2, V_{H}-CDR3 within **SEQ ID NO: 205** and the V_{L}-CDR1, V_{L}-CDR2, V_{L}-CDR3 of the V_{L}-CDR1, V_{L}-CDR2, V_{L}-CDR3 within **SEQ ID NO: 244;** or
   an) the V_{H}-CDR1, V_{H}-CDR2, V_{H}-CDR3 of the V_{H}-CDR1, V_{H}-CDR2, V_{H}-CDR3 within **SEQ ID NO: 206** and the V_{L}-CDR1, V_{L}-CDR2, V_{L}-CDR3 of the V_{L}-CDR1, V_{L}-CDR2, V_{L}-CDR3 within **SEQ ID NO: 245.**
73. The method of any one of arrangements 56-72, wherein the anti-Gal3 antibody or binding fragment thereof comprises:
   a) the heavy chain variable region of **SEQ ID NO: 169** and the light chain variable region of **SEQ ID NO: 207;**
   b) the heavy chain variable region of **SEQ ID NO: 170** and the light chain variable region of **SEQ ID NO: 208;**
   c) the heavy chain variable region of **SEQ ID NO: 171** and the light chain variable region of **SEQ ID NO: 209;**
   d) the heavy chain variable region of **SEQ ID NO: 172** and the light chain variable region of **SEQ ID NO: 210;**
   e) the heavy chain variable region of **SEQ ID NO: 173** and the light chain variable region of **SEQ ID NO: 211;**
   f) the heavy chain variable region of **SEQ ID NO: 174** and the light chain variable region of **SEQ ID NO: 212;**
   g) the heavy chain variable region of **SEQ ID NO: 175** and the light chain variable region of **SEQ ID NO: 213;**
   h) the heavy chain variable region of **SEQ ID NO: 176** and the light chain variable region of **SEQ ID NO: 214;**
   i) the heavy chain variable region of **SEQ ID NO: 177** and the light chain variable region of **SEQ ID NO: 215;**
   j) the heavy chain variable region of **SEQ ID NO: 178** and the light chain variable region of **SEQ ID NO: 216;**
   k) the heavy chain variable region of **SEQ ID NO: 179** and the light chain variable region of **SEQ ID NO: 217;**
   1) the heavy chain variable region of **SEQ ID NO: 180** and the light chain variable region of **SEQ ID NO: 218;**
   m) the heavy chain variable region of **SEQ ID NO: 181** and the light chain variable region of **SEQ ID NO: 219;**
   n) the heavy chain variable region of **SEQ ID NO: 181** and the light chain variable region of **SEQ ID NO: 220;**
   o) the heavy chain variable region of **SEQ ID NO: 182** and the light chain variable region of **SEQ ID NO: 221;**
   p) the heavy chain variable region of **SEQ ID NO: 183** and the light chain variable region of **SEQ ID NO: 222;**
   q) the heavy chain variable region of **SEQ ID NO: 184** and the light chain variable region of **SEQ ID NO: 223;**
   r) the heavy chain variable region of **SEQ ID NO: 185** and the light chain variable region of **SEQ ID NO: 224;**
   s) the heavy chain variable region of **SEQ ID NO: 186** and the light chain variable region of **SEQ ID NO: 225;**
   t) the heavy chain variable region of **SEQ ID NO: 187** and the light chain variable region of **SEQ ID NO: 226;**
   u) the heavy chain variable region of **SEQ ID NO: 188** and the light chain variable region of **SEQ ID NO: 227;**
   v) the heavy chain variable region of **SEQ ID NO: 189** and the light chain variable region of **SEQ ID NO: 228;**
   w) the heavy chain variable region of **SEQ ID NO: 190** and the light chain variable region of **SEQ ID NO: 229;**
   x) the heavy chain variable region of **SEQ ID NO: 191** and the light chain variable region of **SEQ ID NO: 230;**
   y) the heavy chain variable region of **SEQ ID NO: 192** and the light chain variable region of **SEQ ID NO: 231;**
   z) the heavy chain variable region of **SEQ ID NO: 193** and the light chain variable region of **SEQ ID NO: 232;**
   aa) the heavy chain variable region of **SEQ ID NO: 194** and the light chain variable region of **SEQ ID NO: 233;**
   ab) the heavy chain variable region of **SEQ ID NO: 195** and the light chain variable region of **SEQ ID NO: 234;**
   ac) the heavy chain variable region of **SEQ ID NO: 196** and the light chain variable region of **SEQ ID NO: 235;**
   ad) the heavy chain variable region of **SEQ ID NO: 197** and the light chain variable region of **SEQ ID NO: 236;**
   ae) the heavy chain variable region of **SEQ ID NO: 198** and the light chain variable region of **SEQ ID NO: 237;**
   af) the heavy chain variable region of **SEQ ID NO: 199** and the light chain variable region of **SEQ ID NO: 238;**
   ag) the heavy chain variable region of **SEQ ID NO: 197** and the light chain variable region of **SEQ ID NO: 239;**
   ah) the heavy chain variable region of **SEQ ID NO: 200** and the light chain variable region of **SEQ ID NO: 240;**
   ai) the heavy chain variable region of **SEQ ID NO: 201** and the light chain variable region of **SEQ ID NO: 241;**
   aj) the heavy chain variable region of **SEQ ID NO: 202** and the light chain variable region of **SEQ ID NO: 242;**
   ak) the heavy chain variable region of **SEQ ID NO: 203** and the light chain variable region of **SEQ ID NO: 240;**
   al) the heavy chain variable region of **SEQ ID NO: 204** and the light chain variable region of **SEQ ID NO: 243;**
   am) the heavy chain variable region of **SEQ ID NO: 205** and the light chain variable region of **SEQ ID NO: 244;** or
   an) the heavy chain variable region of **SEQ ID NO: 206** and the light chain variable region of **SEQ ID NO: 245.**
74. The method of any one of arrangements 56-73, wherein the anti-Gal3 antibody or binding fragment thereof is selected from the group consisting of: 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT-001(TB001), and IMT-006 (TB006).
75. The method of any one of arrangements 56-74, wherein the anti-Gal3 antibody or binding fragment thereof comprises a humanized antibody.
76. The method of any one of arrangements 56-75, wherein the anti-Gal3 antibody or binding fragment thereof comprises a full-length antibody or a binding fragment thereof.
77. The method of any one of arrangements 56-76, wherein the anti-Gal3 antibody or binding fragment thereof comprises a bispecific antibody or a binding fragment thereof.
78. The method of any one of arrangements 56-77, wherein the anti-Gal3 antibody or binding fragment thereof comprises a monovalent Fab', a divalent Fab2, a single-chain variable fragment (scFv), a diabody, a minibody, a nanobody, a single-domain antibody (sdAb), or a camelid antibody, or binding fragment thereof.
79. The method of any one of arrangements 56-78, wherein the anti-Gal3 antibody or binding fragment thereof comprises an IgG framework.
80. The method of any one of arrangements 56-79, wherein the anti-Gal3 antibody or binding fragment thereof comprises an IgG1, IgG2, or IgG4 framework.
81. The method of any one of arrangements 56-80, wherein disrupting the interaction between Gal3 and the integrin reduces lymphocyte adhesion.
82. The method of arrangement 81, wherein the lymphocyte adhesion is reduced by at least 50%, 55%, 60%, 65%, 70%, 75%, 80%, 85%, 90%, 95%, 96%, 97%, 98%, or 99%, or any percentage within a range defined by any two aforementioned percentages.
83. A method of treating diabetes mellitus in a subject in need thereof, the method comprising: administering to the subject an anti-Gal3 antibody or binding fragment thereof that selectively binds to Gal3 and disrupts an interaction between Gal3 and insulin receptor or an integrin, or both, thereby treating diabetes mellitus in the subject.
84. The method of arrangement 83, wherein the diabetes mellitus is insulin-dependent diabetes mellitus.
85. The method of arrangement 83, wherein the diabetes mellitus is insulin-independent diabetes mellitus.
86. The method of arrangement 83, wherein the diabetes mellitus is Type I diabetes mellitus.
87. The method of arrangement 83, wherein the diabetes mellitus is Type II diabetes mellitus.
88. The method of any one of arrangements 83-87, wherein the treating comprises decreasing glucose tolerance in the subject in need thereof.
89. The method of any one of arrangements 83-88, wherein the treating comprises decreasing insulin sensitivity in the subject in need thereof.
90. The method of any one of arrangements 83-89, wherein the treating comprises reducing weight gain in the subject in need thereof.
91. The method of any one of arrangements 83-90, wherein the treating comprises reducing liver steatosis in the subject in need thereof.
92. The method of any one of arrangements 83-91, further comprising selecting the subject as having diabetes mellitus or at risk of contracting diabetes mellitus prior to the administering step.
93. The method of any one of arrangements 83-92, further comprising detecting an amelioration of symptoms associated with diabetes mellitus in the subject after the administering step.
94. The method of any one of arrangements 83-93, wherein the insulin receptor comprises the sequence of **SEQ ID NO: 2.**
95. The method of any one of arrangements 83-94, wherein the integrin is selected from ITGa1, ITGa2, ITGa3, ITGa4, ITGa5, ITGa6, ITGa7, ITGa8, ITGa9, ITGa10, ITGa11, ITGaD, ITGaE, ITGaL, ITGaM, ITGaV, ITGa2B, ITGa2X, ITGb1, ITGb2, ITGb3, ITGb4, ITGb5, ITGb6, ITGb7, ITGb8, or any combination thereof.
96. The method of any one of arrangements 83-95, wherein the integrin comprises a sequence selected from **SEQ ID NO: 339-342.**
97. The method of any one of arrangements 83-96, wherein the anti-Gal3 antibody or binding fragment thereof is administered with one or more additional therapeutic compositions.
98. The method of arrangement 97, wherein the one or more additional therapeutic compositions comprises insulin, an insulin derivative or mimetic thereof, insulin aspart, insulin glulisine, insulin lispro, insulin isophane, insulin degludec, insulin detemir, insulin zinc, insulin glargine, vanadium, biguanides, metformin, phenformin, buformin, thiazolidinediones, rosiglitazone, pioglitazone, troglitazone, tolimidone, sulfonylureas, tolbutamide, acetohexamide, tolazamide, chlorpropamide, glipizide, glibenclamide, glimepiride, gliclazide, glyclopyramide, gliquidone, meglitinides, repaglinide, nateglinide, alpha-glucosidase inhibitors, miglitol, acarbose, voglibose, incretins, glucagon-like peptide 1, glucagon-like peptide agonists, exenatide, liraglutide, taspoglutide, lixisenatide, semaglutide, dulaglutide, gastric inhibitory peptide, dipeptidyl peptidase-4 inhibitors, vildagliptin, sitagliptin, saxagliptin, linagliptin, alogliptin, septagliptin, teneligpliptin, gemigliptin, pramlintide, dapagliflozin, canagliflozin, empagliflozin, or remogliflozin, or any combination thereof.
99. A method of treating inflammatory bowel syndrome in a subject in need thereof, the method comprising administering to the subject an anti-Gal3 antibody or binding fragment thereof that selectively binds to Gal3 and disrupts an interaction between Gal3 and an integrin, thereby treating inflammatory bowel syndrome in the subject.
100. The method of arrangement 99, wherein the inflammatory bowel syndrome is ulcerative colitis or Crohn's disease, or both.
101. The method of arrangement 99 or 100, further comprising selecting the subject as having inflammatory bowel syndrome or at risk of contracting inflammatory bowel syndrome prior to the administering step.
102. The method of any one of arrangements 99-101, further comprising detecting an amelioration of symptoms associated with inflammatory bowel syndrome in the subject after the administering step.
103. The method of any one of arrangements 99-102, wherein the disruption of the interaction between Gal3 and the integrin reduces lymphocyte adhesion in the subject.
104. The method of arrangement 103, wherein the lymphocyte adhesion is reduced by at least 50%, 55%, 60%, 65%, 70%, 75%, 80%, 85%, 90%, 95%, 96%, 97%, 98%, or 99%, or any percentage within a range defined by any two aforementioned percentages.
105. The method of any one of arrangements 99-104, wherein the integrin is selected from ITGa1, ITGa2, ITGa3, ITGa4, ITGa5, ITGa6, ITGa7, ITGa8, ITGa9, ITGa10, ITGa11, ITGaD, ITGaE, ITGaL, ITGaM, ITGaV, ITGa2B, ITGa2X, ITGb1, ITGb2, ITGb3, ITGb4, ITGb5, ITGb6, ITGb7, ITGb8, or any combination thereof.
106. The method of any one of arrangements 99-105, wherein the integrin comprises a sequence selected from **SEQ ID NO: 339-342.**
107. The method of any one of arrangements 99-106, wherein the anti-Gal3 antibody or binding fragment thereof is administered with one or more additional therapeutic compositions.
108. The method of arrangement 107, wherein the one or more additional therapeutic compositions comprises mesalazine, immunosuppressants, prednisone, TNF inhibitors, azathioprine, methotrexate, 6-mercaptopurine, or rifaximin, or any combination thereof.
109. A method of treating non-alcoholic fatty liver disease (NAFLD) in a subject in need thereof, the method comprising administering to the subject an anti-Gal3 antibody or binding fragment thereof that selectively binds to Gal3 and disrupts an interaction between Gal3 and insulin receptor, thereby treating NAFLD in the subject.
110. A method of treating non-alcoholic steatohepatitis (NASH) in a subject in need thereof, the method comprising administering to the subject an anti-Gal3 antibody or binding fragment thereof that selectively binds to Gal3 and disrupts an interaction between Gal3 and insulin receptor, thereby treating NASH in the subject.
111. The method of any one of arrangements 83-110, wherein the anti-Gal3 antibody or binding fragment thereof binds to an N-terminal domain of Gal3.
112. The method of any one of arrangements 83-111, wherein the anti-Gal3 antibody or binding fragment thereof binds to one or more peptides of **SEQ ID NOs: 3-26.**
113. The method of any one of arrangements 83-112, wherein the anti-Gal3 antibody or binding fragment thereof binds to an epitope present within a region of Gal3 defined by Peptide 1 (ADNFSLHDALSGSGNPNPQG; **SEQ ID NO: 3),** Peptide 4 (GAGGYPGASYPGAYPGQAPP; **SEQ ID NO: 6),** Peptide 6 (GAYPGQAPPGAYPGAPGAYP; **SEQ ID NO: 8),** Peptide 7 (AYPGAPGAYPGAPAPGVYPG; **SEQ ID NO: 9),** or a combination thereof.
114. The method of any one of arrangements 83-113, wherein the anti-Gal3 antibody or binding fragment thereof binds to an epitope of Gal3 comprising an amino acid sequence of GxYPG, wherein x is alanine, glycine, or valine.
115. The method of any one of arrangements 83-114, wherein the interaction is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the anti-Gal3 antibody or binding fragment thereof.
116. The method of any one of arrangements 83-115, wherein the anti-Gal3 antibody or binding fragment thereof binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM.
117. The method of any one of arrangements 83-116, wherein the anti-Gal3 antibody or binding fragment thereof comprises (1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein
   the V_{L}-CDR1 comprises an amino acid sequence having at least 60%, at least 70%, at least 80%, at least 90%, or 100% sequence identity to any amino acid sequence according to **SEQ ID NOs: 100-128,**
   the V_{L}-CDR2 comprises an amino acid sequence having at least 60%, at least 70%, at least 80%, at least 90%, or 100% sequence identity to any amino acid sequence according to **SEQ ID NOs: 129-144,**
   the V_{L}-CDR3 comprises an amino acid sequence having at least 60%, at least 70%, at least 80%, at least 90%, or 100% sequence identity to any amino acid sequence according to **SEQ ID NOs: 145-168,**
   the V_{H}-CDR1 comprises an amino acid sequence having at least 60%, at least 70%, at least 80%, at least 90%, or 100% sequence identity to any amino acid sequence according to **SEQ ID NOs: 27-47,**
   the V_{H}-CDR2 comprises an amino acid sequence having at least 60%, at least 70%, at least 80%, at least 90%, or 100% sequence identity to any amino acid sequence according to **SEQ ID NOs: 48-69,** and
   the V_{H}-CDR3 comprises an amino acid sequence having at least 60%, at least 70%, at least 80%, at least 90%, or 100% sequence identity to any amino acid sequence according to **SEQ ID NOs: 70-99.**
118. The method of arrangement 117, wherein the heavy chain variable region comprises an amino acid sequence having at least 75%, 80%, 85%, 90%, 95%, or 100% sequence identity to any sequence according to **SEQ ID NOs: 169-206.**
119. The method of arrangement 117 or 118, wherein the heavy chain variable region is selected from the group consisting of **SEQ ID NOs: 169-206.**
120. The method of any one of arrangements 117-119, wherein the light chain variable region comprises an amino acid sequence having at least 75%, 80%, 85%, 90%, 95%, or 100% sequence identity to any sequence according to **SEQ ID NOs: 207-245.**
121. The method of any one of arrangements 117-120, wherein the light chain variable region is selected from the group consisting of **SEQ ID NOs: 207-245.**
122. The method of any one of arrangements 83-121, wherein the anti-Gal3 antibody or binding fragment thereof comprises a combination of a V_{L}-CDR1, a V_{L}-CDR2, a V_{L}-CDR3, a V_{H}-CDR1, a V_{H}-CDR2, and a V_{H}-CDR3 as illustrated in **FIG. 36****.**
123. The method of any one of arrangements 83-122, wherein the anti-Gal3 antibody or binding fragment thereof comprises:
   a) the V_{H}-CDR1, V_{H}-CDR2, V_{H}-CDR3 of the V_{H}-CDR1, V_{H}-CDR2, V_{H}-CDR3 within **SEQ ID NO: 169** and the V_{L}-CDR1, V_{L}-CDR2, V_{L}-CDR3 of the V_{L}-CDR1, V_{L}-CDR2, V_{L}-CDR3 within **SEQ ID NO: 207;**
   b) the V_{H}-CDR1, V_{H}-CDR2, V_{H}-CDR3 of the V_{H}-CDR1, V_{H}-CDR2, V_{H}-CDR3 within **SEQ ID NO: 170** and the V_{L}-CDR1, V_{L}-CDR2, V_{L}-CDR3 of the V_{L}-CDR1, V_{L}-CDR2, V_{L}-CDR3 within **SEQ ID NO: 208;**
   c) the V_{H}-CDR1, V_{H}-CDR2, V_{H}-CDR3 of the V_{H}-CDR1, V_{H}-CDR2, V_{H}-CDR3 within **SEQ ID NO: 171** and the V_{L}-CDR1, V_{L}-CDR2, V_{L}-CDR3 of the V_{L}-CDR1, V_{L}-CDR2, V_{L}-CDR3 within **SEQ ID NO: 209;**
   d) the V_{H}-CDR1, V_{H}-CDR2, V_{H}-CDR3 of the V_{H}-CDR1, V_{H}-CDR2, V_{H}-CDR3 within **SEQ ID NO: 172** and the V_{L}-CDR1, V_{L}-CDR2, V_{L}-CDR3 of the V_{L}-CDR1, V_{L}-CDR2, V_{L}-CDR3 within **SEQ ID NO: 210;**
   e) the V_{H}-CDR1, V_{H}-CDR2, V_{H}-CDR3 of the V_{H}-CDR1, V_{H}-CDR2, V_{H}-CDR3 within **SEQ ID NO: 173** and the V_{L}-CDR1, V_{L}-CDR2, V_{L}-CDR3 of the V_{L}-CDR1, V_{L}-CDR2, V_{L}-CDR3 within **SEQ ID NO: 211;**
   f) the V_{H}-CDR1, V_{H}-CDR2, V_{H}-CDR3 of the V_{H}-CDR1, V_{H}-CDR2, V_{H}-CDR3 within **SEQ ID NO: 174** and the V_{L}-CDR1, V_{L}-CDR2, V_{L}-CDR3 of the V_{L}-CDR1, V_{L}-CDR2, V_{L}-CDR3 within **SEQ ID NO: 212;**
   g) the V_{H}-CDR1, V_{H}-CDR2, V_{H}-CDR3 of the V_{H}-CDR1, V_{H}-CDR2, V_{H}-CDR3 within **SEQ ID NO: 175** and the V_{L}-CDR1, V_{L}-CDR2, V_{L}-CDR3 of the V_{L}-CDR1, V_{L}-CDR2, V_{L}-CDR3 within **SEQ ID NO: 213;**
   h) the V_{H}-CDR1, V_{H}-CDR2, V_{H}-CDR3 of the V_{H}-CDR1, V_{H}-CDR2, V_{H}-CDR3 within **SEQ ID NO: 176** and the V_{L}-CDR1, V_{L}-CDR2, V_{L}-CDR3 of the V_{L}-CDR1, V_{L}-CDR2, V_{L}-CDR3 within **SEQ ID NO: 214;**
   i) the V_{H}-CDR1, V_{H}-CDR2, V_{H}-CDR3 of the V_{H}-CDR1, V_{H}-CDR2, V_{H}-CDR3 within **SEQ ID NO: 177** and the V_{L}-CDR1, V_{L}-CDR2, V_{L}-CDR3 of the V_{L}-CDR1, V_{L}-CDR2, V_{L}-CDR3 within **SEQ ID NO: 215;**
   j) the V_{H}-CDR1, V_{H}-CDR2, V_{H}-CDR3 of the V_{H}-CDR1, V_{H}-CDR2, V_{H}-CDR3 within **SEQ ID NO: 178** and the V_{L}-CDR1, V_{L}-CDR2, V_{L}-CDR3 of the V_{L}-CDR1, V_{L}-CDR2, V_{L}-CDR3 within **SEQ ID NO: 216;**
   k) the V_{H}-CDR1, V_{H}-CDR2, V_{H}-CDR3 of the V_{H}-CDR1, V_{H}-CDR2, V_{H}-CDR3 within **SEQ ID NO: 179** and the V_{L}-CDR1, V_{L}-CDR2, V_{L}-CDR3 of the V_{L}-CDR1, V_{L}-CDR2, V_{L}-CDR3 within **SEQ ID NO: 217;**
   1) the V_{H}-CDR1, V_{H}-CDR2, V_{H}-CDR3 of the V_{H}-CDR1, V_{H}-CDR2, V_{H}-CDR3 within **SEQ ID NO: 180** and the V_{L}-CDR1, V_{L}-CDR2, V_{L}-CDR3 of the V_{L}-CDR1, V_{L}-CDR2, V_{L}-CDR3 within **SEQ ID NO: 218;**
   m) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 219;**
   n) the V_{H}-CDR1, V_{H}-CDR2, V_{H}-CDR3 of the V_{H}-CDR1, V_{H}-CDR2, V_{H}-CDR3 within **SEQ ID NO: 181** and the V_{L}-CDR1, V_{L}-CDR2, V_{L}-CDR3 of the V_{L}-CDR1, V_{L}-CDR2, V_{L}-CDR3 within **SEQ ID NO: 220;**
   o) the V_{H}-CDR1, V_{H}-CDR2, V_{H}-CDR3 of the V_{H}-CDR1, V_{H}-CDR2, V_{H}-CDR3 within **SEQ ID NO: 182** and the V_{L}-CDR1, V_{L}-CDR2, V_{L}-CDR3 of the V_{L}-CDR1, V_{L}-CDR2, V_{L}-CDR3 within **SEQ ID NO: 221;**
   p) the V_{H}-CDR1, V_{H}-CDR2, V_{H}-CDR3 of the V_{H}-CDR1, V_{H}-CDR2, V_{H}-CDR3 within **SEQ ID NO: 183** and the V_{L}-CDR1, V_{L}-CDR2, V_{L}-CDR3 of the V_{L}-CDR1, V_{L}-CDR2, V_{L}-CDR3 within **SEQ ID NO: 222;**
   q) the V_{H}-CDR1, V_{H}-CDR2, V_{H}-CDR3 of the V_{H}-CDR1, V_{H}-CDR2, V_{H}-CDR3 within **SEQ ID NO: 184** and the V_{L}-CDR1, V_{L}-CDR2, V_{L}-CDR3 of the V_{L}-CDR1, V_{L}-CDR2, V_{L}-CDR3 within **SEQ ID NO: 223;**
   r) the V_{H}-CDR1, V_{H}-CDR2, V_{H}-CDR3 of the V_{H}-CDR1, V_{H}-CDR2, V_{H}-CDR3 within **SEQ ID NO: 185** and the V_{L}-CDR1, V_{L}-CDR2, V_{L}-CDR3 of the V_{L}-CDR1, V_{L}-CDR2, V_{L}-CDR3 within **SEQ ID NO: 224;**
   s) the V_{H}-CDR1, V_{H}-CDR2, V_{H}-CDR3 of the V_{H}-CDR1, V_{H}-CDR2, V_{H}-CDR3 within **SEQ ID NO: 186** and the V_{L}-CDR1, V_{L}-CDR2, V_{L}-CDR3 of the V_{L}-CDR1, V_{L}-CDR2, V_{L}-CDR3 within **SEQ ID NO: 225;**
   t) the V_{H}-CDR1, V_{H}-CDR2, V_{H}-CDR3 of the V_{H}-CDR1, V_{H}-CDR2, V_{H}-CDR3 within **SEQ ID NO: 187** and the V_{L}-CDR1, V_{L}-CDR2, V_{L}-CDR3 of the V_{L}-CDR1, V_{L}-CDR2, V_{L}-CDR3 within **SEQ ID NO: 226;**
   u) the V_{H}-CDR1, V_{H}-CDR2, V_{H}-CDR3 of the V_{H}-CDR1, V_{H}-CDR2, V_{H}-CDR3 within **SEQ ID NO: 188** and the V_{L}-CDR1, V_{L}-CDR2, V_{L}-CDR3 of the V_{L}-CDR1, V_{L}-CDR2, V_{L}-CDR3 within **SEQ ID NO: 227;**
   v) the V_{H}-CDR1, V_{H}-CDR2, V_{H}-CDR3 of the V_{H}-CDR1, V_{H}-CDR2, V_{H}-CDR3 within **SEQ ID NO: 189** and the V_{L}-CDR1, V_{L}-CDR2, V_{L}-CDR3 of the V_{L}-CDR1, V_{L}-CDR2, V_{L}-CDR3 within **SEQ ID NO: 228;**
   w) the V_{H}-CDR1, V_{H}-CDR2, V_{H}-CDR3 of the V_{H}-CDR1, V_{H}-CDR2, V_{H}-CDR3 within **SEQ ID NO: 190** and the V_{L}-CDR1, V_{L}-CDR2, V_{L}-CDR3 of the V_{L}-CDR1, V_{L}-CDR2, V_{L}-CDR3 within **SEQ ID NO: 229;**
   x) the V_{H}-CDR1, V_{H}-CDR2, V_{H}-CDR3 of the V_{H}-CDR1, V_{H}-CDR2, V_{H}-CDR3 within **SEQ ID NO: 191** and the V_{L}-CDR1, V_{L}-CDR2, V_{L}-CDR3 of the V_{L}-CDR1, V_{L}-CDR2, V_{L}-CDR3 within **SEQ ID NO: 230;**
   y) the V_{H}-CDR1, V_{H}-CDR2, V_{H}-CDR3 of the V_{H}-CDR1, V_{H}-CDR2, V_{H}-CDR3 within **SEQ ID NO: 192** and the V_{L}-CDR1, V_{L}-CDR2, V_{L}-CDR3 of the V_{L}-CDR1, V_{L}-CDR2, V_{L}-CDR3 within **SEQ ID NO: 231;**
   z) the V_{H}-CDR1, V_{H}-CDR2, V_{H}-CDR3 of the V_{H}-CDR1, V_{H}-CDR2, V_{H}-CDR3 within **SEQ ID NO: 193** and the V_{L}-CDR1, V_{L}-CDR2, V_{L}-CDR3 of the V_{L}-CDR1, V_{L}-CDR2, V_{L}-CDR3 within **SEQ ID NO: 232;**
   aa) the V_{H}-CDR1, V_{H}-CDR2, V_{H}-CDR3 of the V_{H}-CDR1, V_{H}-CDR2, V_{H}-CDR3 within **SEQ ID NO: 194** and the V_{L}-CDR1, V_{L}-CDR2, V_{L}-CDR3 of the V_{L}-CDR1, V_{L}-CDR2, V_{L}-CDR3 within **SEQ ID NO: 233;**
   ab) the V_{H}-CDR1, V_{H}-CDR2, V_{H}-CDR3 of the V_{H}-CDR1, V_{H}-CDR2, V_{H}-CDR3 within **SEQ ID NO: 195** and the V_{L}-CDR1, V_{L}-CDR2, V_{L}-CDR3 of the V_{L}-CDR1, V_{L}-CDR2, V_{L}-CDR3 within **SEQ ID NO: 234;**
   ac) the V_{H}-CDR1, V_{H}-CDR2, V_{H}-CDR3 of the V_{H}-CDR1, V_{H}-CDR2, V_{H}-CDR3 within **SEQ ID NO: 196** and the V_{L}-CDR1, V_{L}-CDR2, V_{L}-CDR3 of the V_{L}-CDR1, V_{L}-CDR2, V_{L}-CDR3 within **SEQ ID NO: 235;**
   ad) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 236;**
   ae) the V_{H}-CDR1, V_{H}-CDR2, V_{H}-CDR3 of the V_{H}-CDR1, V_{H}-CDR2, V_{H}-CDR3 within **SEQ ID NO: 198** and the V_{L}-CDR1, V_{L}-CDR2, V_{L}-CDR3 of the V_{L}-CDR1, V_{L}-CDR2, V_{L}-CDR3 within **SEQ ID NO: 237;**
   af) the V_{H}-CDR1, V_{H}-CDR2, V_{H}-CDR3 of the V_{H}-CDR1, V_{H}-CDR2, V_{H}-CDR3 within **SEQ ID NO: 199** and the V_{L}-CDR1, V_{L}-CDR2, V_{L}-CDR3 of the V_{L}-CDR1, V_{L}-CDR2, V_{L}-CDR3 within **SEQ ID NO: 238;**
   ag) the V_{H}-CDR1, V_{H}-CDR2, V_{H}-CDR3 of the V_{H}-CDR1, V_{H}-CDR2, V_{H}-CDR3 within **SEQ ID NO: 197** and the V_{L}-CDR1, V_{L}-CDR2, V_{L}-CDR3 of the V_{L}-CDR1, V_{L}-CDR2, V_{L}-CDR3 within **SEQ ID NO: 239;**
   ah) the V_{H}-CDR1, V_{H}-CDR2, V_{H}-CDR3 of the V_{H}-CDR1, V_{H}-CDR2, V_{H}-CDR3 within **SEQ ID NO: 200** and the V_{L}-CDR1, V_{L}-CDR2, V_{L}-CDR3 of the V_{L}-CDR1, V_{L}-CDR2, V_{L}-CDR3 within **SEQ ID NO: 240;**
   ai) the V_{H}-CDR1, V_{H}-CDR2, V_{H}-CDR3 of the V_{H}-CDR1, V_{H}-CDR2, V_{H}-CDR3 within **SEQ ID NO: 201** and the V_{L}-CDR1, V_{L}-CDR2, V_{L}-CDR3 of the V_{L}-CDR1, V_{L}-CDR2, V_{L}-CDR3 within **SEQ ID NO: 241;**
   aj) the V_{H}-CDR1, V_{H}-CDR2, V_{H}-CDR3 of the V_{H}-CDR1, V_{H}-CDR2, V_{H}-CDR3 within **SEQ ID NO: 202** and the V_{L}-CDR1, V_{L}-CDR2, V_{L}-CDR3 of the V_{L}-CDR1, V_{L}-CDR2, V_{L}-CDR3 within **SEQ ID NO: 242;**
   ak) the V_{H}-CDR1, V_{H}-CDR2, V_{H}-CDR3 of the V_{H}-CDR1, V_{H}-CDR2, V_{H}-CDR3 within **SEQ ID NO: 203** and the V_{L}-CDR1, V_{L}-CDR2, V_{L}-CDR3 of the V_{L}-CDR1, V_{L}-CDR2, V_{L}-CDR3 within **SEQ ID NO: 240;**
   al) the V_{H}-CDR1, V_{H}-CDR2, V_{H}-CDR3 of the V_{H}-CDR1, V_{H}-CDR2, V_{H}-CDR3 within **SEQ ID NO: 204** and the V_{L}-CDR1, V_{L}-CDR2, V_{L}-CDR3 of the V_{L}-CDR1, V_{L}-CDR2, V_{L}-CDR3 within **SEQ ID NO: 243;**
   am) the V_{H}-CDR1, V_{H}-CDR2, V_{H}-CDR3 of the V_{H}-CDR1, V_{H}-CDR2, V_{H}-CDR3 within **SEQ ID NO: 205** and the V_{L}-CDR1, V_{L}-CDR2, V_{L}-CDR3 of the V_{L}-CDR1, V_{L}-CDR2, V_{L}-CDR3 within **SEQ ID NO: 244;** or
   an) the V_{H}-CDR1, V_{H}-CDR2, V_{H}-CDR3 of the V_{H}-CDR1, V_{H}-CDR2, V_{H}-CDR3 within **SEQ ID NO: 206** and the V_{L}-CDR1, V_{L}-CDR2, V_{L}-CDR3 of the V_{L}-CDR1, V_{L}-CDR2, V_{L}-CDR3 within **SEQ ID NO: 245.**
124. The method of any one of arrangements 83-123, wherein the anti-Gal3 antibody or binding fragment thereof comprises:
   a) the heavy chain variable region of **SEQ ID NO: 169** and the light chain variable region of **SEQ ID NO: 207;**
   b) the heavy chain variable region of **SEQ ID NO: 170** and the light chain variable region of **SEQ ID NO: 208;**
   c) the heavy chain variable region of **SEQ ID NO: 171** and the light chain variable region of **SEQ ID NO: 209;**
   d) the heavy chain variable region of **SEQ ID NO: 172** and the light chain variable region of **SEQ ID NO: 210;**
   e) the heavy chain variable region of **SEQ ID NO: 173** and the light chain variable region of **SEQ ID NO: 211;**
   f) the heavy chain variable region of **SEQ ID NO: 174** and the light chain variable region of **SEQ ID NO: 212;**
   g) the heavy chain variable region of **SEQ ID NO: 175** and the light chain variable region of **SEQ ID NO: 213;**
   h) the heavy chain variable region of **SEQ ID NO: 176** and the light chain variable region of **SEQ ID NO: 214;**
   i) the heavy chain variable region of **SEQ ID NO: 177** and the light chain variable region of **SEQ ID NO: 215;**
   j) the heavy chain variable region of **SEQ ID NO: 178** and the light chain variable region of **SEQ ID NO: 216;**
   k) the heavy chain variable region of **SEQ ID NO: 179** and the light chain variable region of **SEQ ID NO: 217;**
   1) the heavy chain variable region of **SEQ ID NO: 180** and the light chain variable region of **SEQ ID NO: 218;**
   m) the heavy chain variable region of **SEQ ID NO: 181** and the light chain variable region of **SEQ ID NO: 219;**
   n) the heavy chain variable region of **SEQ ID NO: 181** and the light chain variable region of **SEQ ID NO: 220;**
   o) the heavy chain variable region of **SEQ ID NO: 182** and the light chain variable region of **SEQ ID NO: 221;**
   p) the heavy chain variable region of **SEQ ID NO: 183** and the light chain variable region of **SEQ ID NO: 222;**
   q) the heavy chain variable region of **SEQ ID NO: 184** and the light chain variable region of **SEQ ID NO: 223;**
   r) the heavy chain variable region of **SEQ ID NO: 185** and the light chain variable region of **SEQ ID NO: 224;**
   s) the heavy chain variable region of **SEQ ID NO: 186** and the light chain variable region of **SEQ ID NO: 225;**
   t) the heavy chain variable region of **SEQ ID NO: 187** and the light chain variable region of **SEQ ID NO: 226;**
   u) the heavy chain variable region of **SEQ ID NO: 188** and the light chain variable region of **SEQ ID NO: 227;**
   v) the heavy chain variable region of **SEQ ID NO: 189** and the light chain variable region of **SEQ ID NO: 228;**
   w) the heavy chain variable region of **SEQ ID NO: 190** and the light chain variable region of **SEQ ID NO: 229;**
   x) the heavy chain variable region of **SEQ ID NO: 191** and the light chain variable region of **SEQ ID NO: 230;**
   y) the heavy chain variable region of **SEQ ID NO: 192** and the light chain variable region of **SEQ ID NO: 231;**
   z) the heavy chain variable region of **SEQ ID NO: 193** and the light chain variable region of **SEQ ID NO: 232;**
   aa) the heavy chain variable region of **SEQ ID NO: 194** and the light chain variable region of **SEQ ID NO: 233;**
   ab) the heavy chain variable region of **SEQ ID NO: 195** and the light chain variable region of **SEQ ID NO: 234;**
   ac) the heavy chain variable region of **SEQ ID NO: 196** and the light chain variable region of **SEQ ID NO: 235;**
   ad) the heavy chain variable region of **SEQ ID NO: 197** and the light chain variable region of **SEQ ID NO: 236;**
   ae) the heavy chain variable region of **SEQ ID NO: 198** and the light chain variable region of **SEQ ID NO: 237;**
   af) the heavy chain variable region of **SEQ ID NO: 199** and the light chain variable region of **SEQ ID NO: 238;**
   ag) the heavy chain variable region of **SEQ ID NO: 197** and the light chain variable region of **SEQ ID NO: 239;**
   ah) the heavy chain variable region of **SEQ ID NO: 200** and the light chain variable region of **SEQ ID NO: 240;**
   ai) the heavy chain variable region of **SEQ ID NO: 201** and the light chain variable region of **SEQ ID NO: 241;**
   aj) the heavy chain variable region of **SEQ ID NO: 202** and the light chain variable region of **SEQ ID NO: 242;**
   ak) the heavy chain variable region of **SEQ ID NO: 203** and the light chain variable region of **SEQ ID NO: 240;**
   al) the heavy chain variable region of **SEQ ID NO: 204** and the light chain variable region of **SEQ ID NO: 243;**
   am) the heavy chain variable region of **SEQ ID NO: 205** and the light chain variable region of **SEQ ID NO: 244;** or
   an) the heavy chain variable region of **SEQ ID NO: 206** and the light chain variable region of **SEQ ID NO: 245.**
125. The method of any one of arrangements 83-124, wherein the anti-Gal3 antibody or binding fragment thereof is selected from the group consisting of: 13H12.2F8, 19D9.2E5, 14H10.2C9, 2D10.2B2, 4A11.2B5, 6H6.2D6, 20H5.A3, 19B5.2E6, 23H9.2E4, 20D11.2C6, 15G7.2A7, 4G2.2G6, 3B11.2G2, 13A12.2E5, 7D8.2D8, 15F10.2D6, 12G5.D7, 24D12.2H9, 13G4.2F8, 9H2.2H1, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT-001 (TB001), and IMT-006 (TB006).
126. The method of any one of arrangements 83-125, wherein the anti-Gal3 antibody or binding fragment thereof is administered enterally, orally, intranasally, parenterally, intracranially, subcutaneously, intramuscularly, intradermally, or intravenously, or any combination thereof.
127. The method of any one of arrangements 83-126, wherein the anti-Gal3 antibody or binding fragment thereof comprises a humanized antibody.
128. The method of any one of arrangements 83-127, wherein the anti-Gal3 antibody or binding fragment thereof comprises a full-length antibody or a binding fragment thereof.
129. The method of any one of arrangements 83-128, wherein the anti-Gal3 antibody or binding fragment thereof comprises a bispecific antibody or a binding fragment thereof.
130. The method of any one of arrangements 83-129, wherein the anti-Gal3 antibody or binding fragment thereof comprises a monovalent Fab', a divalent Fab2, a single-chain variable fragment (scFv), a diabody, a minibody, a nanobody, a single-domain antibody (sdAb), or a camelid antibody, or binding fragment thereof.
131. The method of any one of arrangements 83-130, wherein the anti-Gal3 antibody or binding fragment thereof comprises an IgG framework.
132. The method of any one of arrangements 83-131, wherein the anti-Gal3 antibody or binding fragment thereof comprises an IgG1, IgG2, or IgG4 framework.
133. The method of any one of arrangements 83-132, wherein the subject is diagnosed with Type I diabetes or Type II diabetes.
134. The method of any one of arrangements 83-133, wherein the anti-Gal3 antibody or binding fragment thereof is formulated for systemic administration.
135. The method of any one of arrangements 83-134, wherein the anti-Gal3 antibody or binding fragment thereof is formulated for parenteral administration.
136. The method of any one of arrangements 83-135, wherein more than one anti-Gal3 antibody or binding fragment is administered.
137. The method of any one of arrangements 83-136, wherein the subject is a mammal.
138. The method of arrangement 137, wherein the mammal is a human.
139. An anti-Gal3 antibody comprising (1) a light chain variable region comprising a V_{L}-CDR1, a V_{L}-CDR2, and a V_{L}-CDR3; and (2) a heavy chain variable region comprising a V_{H}-CDR1, a V_{H}-CDR2, and a V_{H}-CDR3, wherein
   the V_{L}-CDR1 comprises an amino acid sequence having at least 60%, at least 70%, at least 80%, at least 90%, or 100% sequence identity to any amino acid sequence according to **SEQ ID NOs: 100-128,**
   the V_{L}-CDR2 comprises an amino acid sequence having at least 60%, at least 70%, at least 80%, at least 90%, or 100% sequence identity to any amino acid sequence according to **SEQ ID NOs: 129-144,**
   the V_{L}-CDR3 comprises an amino acid sequence having at least 60%, at least 70%, at least 80%, at least 90%, or 100% sequence identity to any amino acid sequence according to **SEQ ID NOs: 145-168,**
   the V_{H}-CDR1 comprises an amino acid sequence having at least 60%, at least 70%, at least 80%, at least 90%, or 100% sequence identity to any amino acid sequence according to **SEQ ID NOs: 27-47,**
   the V_{H}-CDR2 comprises an amino acid sequence having at least 60%, at least 70%, at least 80%, at least 90%, or 100% sequence identity to any amino acid sequence according to **SEQ ID NOs: 48-69,** and
   the V_{H}-CDR3 comprises an amino acid sequence having at least 60%, at least 70%, at least 80%, at least 90%, or 100% sequence identity to any amino acid sequence according to **SEQ ID NOs: 70-99.**
140. The anti-Gal3 antibody of arrangement 139, wherein the anti-Gal3 antibody comprises a combination of a V_{L}-CDR1, a V_{L}-CDR2, a V_{L}-CDR3, a V_{H}-CDR1, a V_{H}-CDR2, and a V_{H}-CDR3 as illustrated in **FIG. 36****.**
141. The anti-Gal3 antibody of arrangement 139 or 140, wherein the anti-Gal3 antibody is selected from the group consisting of: 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, 7D8.2D8, 15F10.2D6, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT-001(TB001), and IMT-006 (TB006).
142. The anti-Gal3 antibody of any one of arrangements 139-141, wherein the anti-Gal3 antibody is selected from the group consisting of: F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, and 847.14H4.
143. A pharmaceutical formulation comprising the anti-Gal3 antibody of any one of arrangements 139-142.
144. The pharmaceutical formulation of arrangement 143 for use in treating diabetes mellitus.
145. The pharmaceutical formulation of arrangement 143 for use in treating inflammatory bowel disease.
146. The pharmaceutical formulation of arrangement 143 for use in treating NAFLD and/or NASH.
147. An antibody that binds to human Gal3 and competes with an anti-Gal3 antibody for binding to human Gal3, wherein the anti-Gal3 antibody is selected from the group consisting of: 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, 7D8.2D8, and 15F10.2D6, 23B10.2B12, 6B3.2D3, F846C.1B2, F846C.1F5, F846C.1H12, F846C.1H5, F846C.2H3, F846TC.14A2, F846TC.14E4, F846TC.16B5, F846TC.7F10, F847C.10B9, F847C.11B1, F847C.12F12, F847C.26F5, F847C.4B10, F849C.8D10, F849C.8H3, 847.14H4, mIMT001, IMT001(TB001), and IMT006 (TB006).
148. A method for identifying an antibody capable of disrupting an interaction between Gal3 and insulin receptor or an integrin, or both, the method comprising:
   (a) contacting Gal3 protein with an antibody or binding fragment thereof that selectively binds to Gal3, thereby forming a Gal3-antibody complex;
   (b) contacting the Gal3-antibody complex with the insulin receptor or the integrin, or both;
   (c) removing unbound insulin receptor or integrin, or both; and
   (d) detecting the insulin receptor or integrin, or both, bound to the Gal3-antibody complex;
      wherein the antibody or binding fragment thereof is capable of disrupting an interaction of Gal3 and insulin receptor or the integrin, or both, when the insulin receptor or the integrin, or both, is not detected in (d).
149. The method of arrangement 148, wherein the method comprises an immunoassay.
150. The method of arrangement 149, wherein the immunoassay is an enzyme-linked immunosorbent assay.
151. A method of producing an anti-Gal3 antibody or binding fragment thereof, comprising:
   expressing a nucleic acid that encodes for the anti-Gal3 antibody or binding fragment thereof in a cell; and
   isolating the expressed anti-Gal3 antibody or binding fragment thereof from the cell.
152. The method of arrangement 151, further comprising concentrating the anti-Gal3 antibody or binding fragment thereof to a desired concentration.
153. The method of arrangement 151 or 152, wherein the cell is a mammalian cell, insect cell, or bacterial cell.
154. The method of any one of arrangements 151-153, wherein the anti-Gal3 antibody or binding fragment is the anti-Gal3 antibody or binding fragment thereof of any one of the methods of arrangements 56-138.

### EXAMPLES

Some aspects of the embodiments discussed above are disclosed in further detail in the following examples, which are not in any way intended to limit the scope of the present disclosure. Those in the art will appreciate that many other embodiments also fall within the scope of the invention, as it is described herein above and in the claims.

### Example 1. Identification of Gal3-binding antibodies with and without Gal3-INSR blocking activity

To identify Gal3-binding antibodies with the capacity to block the assembly of Gal3 and INSR, an immunization campaign was run in mice. Balb/C, FVB, and CD-1F mice were inoculated at 7 day intervals with 50 µg of Gal3 protein fused to a linker-spaced 6-histidine tag, Gal3-ECD-His, (Acro GA3-H5129; Lot# 819-43PS1-5E) in combination with a TLR agonist adjuvant mix (50 µg MPL, 20 µg CpG, 10 µg Poly(I:C) and 10 µg R848) for 3 repetitions, followed by an inoculation with 50 µg of Gal3-His alone administered subcutaneously to the inguinal, back of the neck and base of the tail sites as well as hock and intraperitoneal sites. Animals were sacrificed in accordance with IACUC protocol and spleen, femurs, and lymph nodes (axillary, accessory axillary, mediastinal, superficial inguinal, iliac, sacral and popliteal) were harvested. A single cell suspension of immunized lymph node (LN), spleen and bone marrow cells were obtained using 2 sterile frosted glass slides in a tissue culture petri dish with 15 mL DMEM. Bone marrow was extracted from femurs via end-cap flushing with a 5 mL syringe fitted with an 18-gauge needle. Cells from 3 animals were pelleted with 5 minutes of centrifugation at 1200 RPM, resuspended in 10 mL of DMEM (GIBCO 10564-011) and nucleated cells were enumerated by hemocytometer count. Cells were pelleted at 1200 RPM and were resuspended in SC-Buffer (PBS, 2% FBS and 1 mM EDTA), and plasma cells were isolated with an EasySep^{™} Mouse CD138 Positive Selection Kit (StemCell Technologies) with the manufacturer recommended protocol. Enriched CD138-positive cells were pelleted with 5 minutes of centrifugation at 1200 RPM, resuspended in 50 mL electrofusion buffer (Eppendorf 940-00-220-6) and were enumerated. Separately, SP2/0-mIL6 myeloma cells (ATCC CRL2016) were pelleted with 5 minutes of centrifugation at 1200 RPM, resuspended in 50 mL electrofusion buffer and were enumerated. Myeloma cells and CD138-positive plasma cells were combined at a 1:1 ratio, volume was expanded to 50 mL with electrofusion buffer, cells were pelleted with 5 minutes of centrifugation at 1200 RPM and supernatant was discarded. After a repeated step of washing and pelleting in electrofusion buffer, cells were resuspended in electrofusion buffer to a concentration of 10x10^6 cells/mL, up to 9 mL of cell suspension was added to a BTX electrofusion chamber, and cells were fused with an 800V electrofusion protocol. Fused cells were rested for 5 minutes, transferred to a tissue culture dish containing 40 mL medium MM (DMEM, 15% FBS, 1% glutamax and 1% Pen/Strep), incubated for 1 hour at 37°C, 8% CO₂, resuspended with a pipette, pelleted with 5 minutes of centrifugation at 1200 RPM, resuspended in ClonaCell HY Liquid HAT Selection Medium (StemCell Technologies), and plated in 96-well tissue culture flat bottomed plates. After 10 days, supernatants were sampled and evaluated for binding to isolated Gal3 by ELISA. 50 µl of 0.1 µg/mL Gal3-ECD-His, (Acro GA3-H5129; Lot# 819-43PS1-5E) resuspended in diluent (PBS with 0.5% BSA) was added to each well for 45 minutes, supernatant was discarded and plates were washed with phosphate buffered saline (PBS) with 0.05% Tween20. 50 µl of 1:5 dilution of hybridoma supernatant in diluent was added to each well for 1 hour, followed by 5 successive 300 µl washes with PBS/0.05% Tween20, after which a 1:3000 dilution of goat anti-mouse Fc-specific antibody conjugated to horseradish peroxidase (Novex A16090) in 50 µl of diluent was added to each well for 1 hour followed by 5 successive 300 µl washes with PBS/0.05% Tween20. Following washing, 50 µl of ABTS (Novex #00-202-4) was added to each well for 20-30 minutes, prior to readout on a spectrophotometer (Molecular Devices) at absorbance of 405 nm.

Positively scoring wells were evaluated for the ability to block association of Gal3 and INSR. To identify Gal3-targeted antibodies with the ability to block the interaction of Gal3 and INSR, purified Gal3 and INSR proteins were incubated in the presence of Gal3-immunization hybridoma supernatants described above, or without antibody, and protein interaction was evaluated by ELISA. Human Galectin-3 protein (Acro Biosystems, GA3-H5129) was diluted in PBS (Corning, 21-030-CM) to a concentration of 3 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]). The plate was then blocked for an hour with 2% BSA (EMD Millipore, 126609) in PBST at room temperature with gentle rocking. Thereafter, the 2% BSA in PBST was discarded and antibody or inhibitor (3-fold dilutions beginning at 20 µg/ml, 60 µg/ml, or 180 µM) in 2% BSA in PBST was added to the wells. Afterwards, 6 µg/ml of human Insulin Receptor (Sino Biological,11086-H08H) in 2% BSA in PBST was added to the antibody or inhibitor in the wells in a 1:1 ratio. The plate was incubated for an hour at room temperature with gentle rocking. Thereafter, the plate was washed three times with PBST, and 0.3 µg/ml of human INSR Biotinylated Antibody (R&D Systems, BAF1544) in 2% BSA in PBST was added to the wells. The plate was incubated for an hour with gentle rocking and then washed three times with PBST. Avidin-HRP (1:2000) was then added to the wells. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

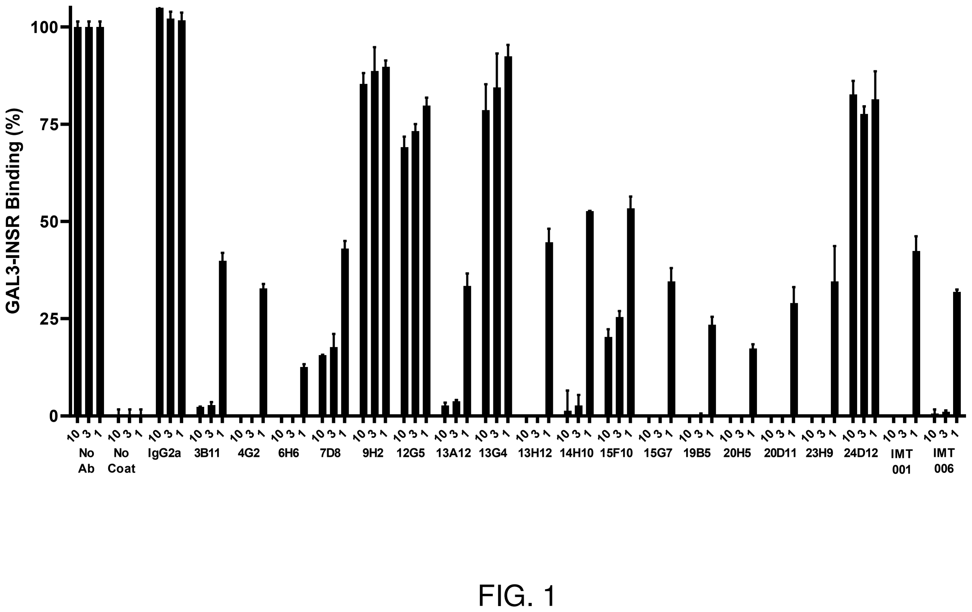

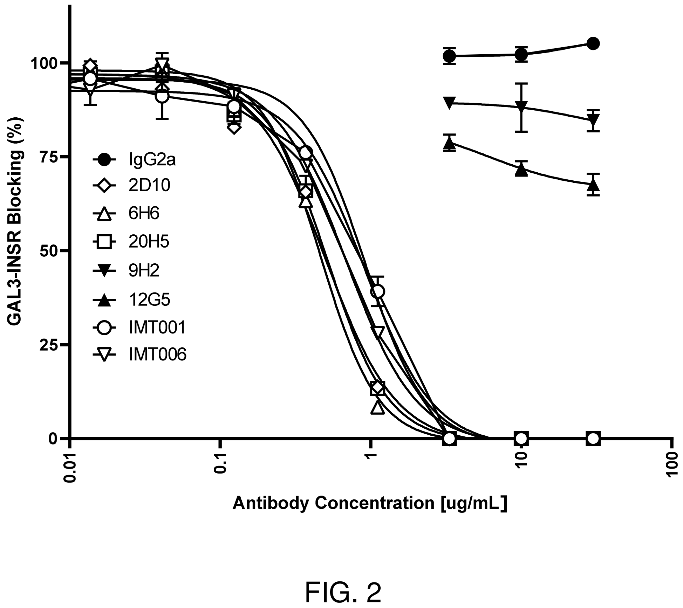

Several hGal3-binding antibodies with the ability to strongly block the binding of Gal3 to INSR were identified, depicted in **FIG. 1** and **FIG. 2****,** including 6H6.2D6, 20H5.A3, 20D11.2C6, 4G2.2G6, 13H12.2F8, 19B5.2E6, 15G7.2A7, 23H9.2E4, 19D9.2E5, 2D10.2B2, 4A11.2B5, 14H10.2C9, 3B11.2G2, 13A12.2E5, which reduced the binding of Gal3 and INSR at a concentration of 3 µg/mL to less than 5% of an unblocked sample. Additionally, monoclonal antibodies with reduced capacity to inhibit Gal3-INSR binding at 3 µg/mL to 15-25% of levels of an unblocked sample were identified, including 7D8.2D8 and 15F10.2D6. Finally, monoclonal antibodies with the capacity to minimally impact Gal3-INSR binding by 30% or less at 3 µg/ml were identified, including 9H2.2H1, 12G5.D7, 13G4.2F8, and 24D12.2H9.

To evaluate whether the ability to block Gal3 assembly with INSR was a function of antibody affinity to Gal3, the affinity of monoclonal Gal3 antibodies to Gal3 was evaluated by SPR. Kinetics experiments were performed on BiacoreT200 at 25°C in high performance mode. Ligand proteins, purified antibodies were captured onto a CM5 chip coupled with anti-human Fc or anti-mouse Fc antibody, three antibodies at a time onto flow cell #2, 3, and 4, respectively, while flow cell #1 was used as reference. The analyte Galectin-3 in HBS-EP buffer was injected over all four flow cells at concentrations of 100, 50, 25, 12.5, 6.25, 3.125 and 0 nM at a flow rate of 30 µL/min. The complex was allowed to associate and dissociate for 240 and 300 seconds, respectively. The surfaces were regenerated with a 30 second injection of 10 mM Glycine pH 1.7 (flow rate 30 µL/min). The data were fit to a simple 1:1 interaction model using the global data analysis option available within BiacoreT200 Evaluation software V2.0.

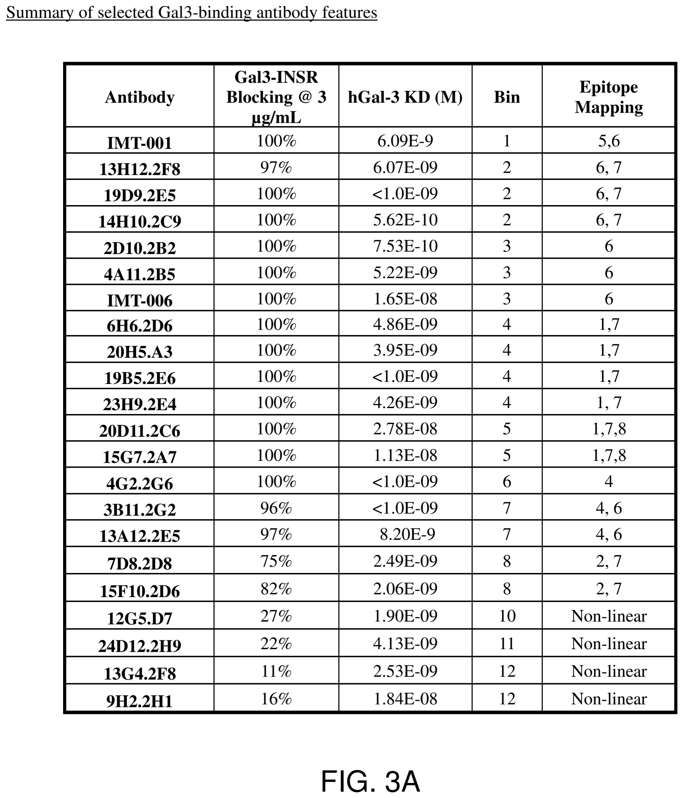

The affinity of Gal3 monoclonal antibodies was confirmed to be greater than 30 nM for all antibodies studied **(****FIG. 3A****).** Importantly, 15G7, an antibody which reduced assembly of Gal3 and INSR by greater than 99% at 3 µg/mL exhibited an affinity of 27.8 nM for Gal3, indicating that the ability to block the assembly of Gal3 and INSR is possible with antibodies with affinity at or below this level. In contrast, Gal3-targeted antibodies which poorly block Gal3-INSR assembly, 13G4.2F8, 9H2.2H1, 24D12.2H9, and 12G5.D7, exhibited affinities of 18.4, 2.53, 4.13, and 1.9 nM, respectively. Thus, antibody affinity to Gal3 of less than 10 nM is not sufficient to predict the ability to block assembly of Gal3 and INSR.

### Example 2. Gal3-targeted antibodies with and without Gal3-INSR blocking activity bind to distinct epitopes of Gal3

To identify the epitopes to which Gal3 antibodies with and without Gal3-INSR blocking activity bound, a library of 24 amino acid peptides representing portions of Gal3, depicted in **FIG. 27****,** was produced and the ability of each peptide to bind Gal3 antibodies was evaluated by ELISA. At least 2 µg/ml of hGal3 peptide in 50 µl of PBS or 0.1 µg/ml of full-length human Gal3 protein (GenScript) and human Galectin-3 protein (Acro Biosystems, GA3-H5129) were diluted in PBS (Corning, 21-030-CM) to concentrations of at least 2 µg/ml or 0.1 µg/ml, respectively, and added to the wells of a 96-well ELISA plate (Thermo Fisher, 44-2404-21). After incubating the plate at 4°C overnight, the plate was washed three times with PBST (PBS with 0.05% Tween 20 [VWR, 0777]). The plate was then blocked for an hour with 2% BSA (EMD Millipore, 126609) in PBST at room temperature with gentle rocking. Thereafter, the 2% BSA in PBST was discarded and human Galectin-3 hybridoma supernatants or antibodies were diluted in 2% BSA in PBST to concentrations of at least 0.1 µg/ml and added to the wells. The plate was incubated for an hour at room temperature with gentle rocking and then washed three times with PBST. Afterwards, Goat Anti-Mouse IgG-HRP (Jackson ImmunoResearch,115-036-1461) or Goat Anti-Rat IgG HRP (Abcam, ab205720) diluted in 2% BSA in PBST (1:4000) were added to the wells. The plate was incubated for 30 minutes to 1 hour at room temperature with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

Binding of Gal3-binding antibodies to the peptide array was observed at multiple locations, with the majority of binding observed in Peptides 1-8 **(SEQ ID NOs: 3-10),** summarized in **FIG. 3A****.** Significantly, all Gal3-binding antibodies with strong INSR-Gal3 blocking activity exhibited the ability to bind to peptides 4, 6, or 7, corresponding to peptide sequences in the N-terminal domain of Gal3. Specifically, six separate Gal3-binding antibodies with strong Gal3-INSR blocking activity (6H6.2D6, 20H5.A3, 20D11.2C6, 19B5.2E6, 15G7.2A7, and 23H9.2E4) all bound peptide 1 of Gal3, corresponding to amino acids 1-20 of Gal3, ADNFSLHDALSGSGNPNPQG **(SEQ ID NO: 3).** Conversely, no Gal3-targeted antibodies with poor Gal3-INSR blocking activity were observed to bind peptide 1. Taken together, these data indicate that binding to Gal3 peptide 1 is predictive of the ability to block the interaction of Gal3 with INSR. Similarly, two separate Gal3-binding antibodies with strong Gal3-INSR blocking activity (4G2.2G6 and 3B11.2G2) bound peptide 4 of Gal3, corresponding to amino acids 31-50 of Gal3, GAGGYPGASYPGAYPGQAPP **(SEQ ID NO: 6).** Conversely, no Gal3-targeted antibodies with poor Gal3-INSR blocking activity were observed to bind peptide 4. Taken together, these data indicate that binding to Gal3 peptide 4 is predictive of the ability to block the interaction of Gal3 with INSR. Further, seven Gal3-binding antibodies with strong Gal3-INSR blocking activity (13H12.2F8, 19D9.2E5, 14H10.2C9, 2D10.2B2, 4A11.2B5, 3B11.2D2, and 13A12.2E5) all bound peptide 6 of Gal3, corresponding to amino acids 51-70 of Gal3, GAYPGQAPPGAYPGAPGAYP **(SEQ ID NO: 8).** Conversely, no Gal3-targeted antibodies with poor Gal3-INSR blocking activity were observed to bind peptide 6. Taken together, these data indicate that binding to Gal3 peptide 6 is predictive of the ability to block the interaction of Gal3 with INSR. Additionally, eleven Gal3-binding antibodies with Gal3-INSR blocking activity (6H6.2D6, 20H5.A3, 20D11.2C6, 13H12.2F8, 19B5.2E6, 23H9.2E4, 15G7.2A7, 19D9.2E5, 14H10.2C9, 7D8.2D8, and 15F10.2D6) all bound peptide 7 of Gal3, corresponding to amino acids 61-80 of Gal3, AYPGAPGAYPGAPAPGVYPG **(SEQ ID NO: 9).** Conversely, no Gal3-targeted antibodies with poor Gal3-INSR blocking activity were observed to bind peptide 7. Taken together, these data indicate that binding to Gal3 peptide 7 is predictive of the ability to block the interaction of Gal3 with INSR. In total, these data indicate the utility of anti-Gal3 antibodies to Gal3 peptides 1, 4, 6, and 7 as predictive of the ability to block the interaction of Gal3 and INSR.

As illustrated in **FIG. 27****,** peptides 4, 6, and 7 share repeated amino acid sequences comprised of proline-glycine (PG) and tyrosine-proline-glycine (YPG), suggesting a common feature that may explain the ability of Gal3-targeted antibodies to bind to multiple Gal3 peptides. Further, the amino acid sequence glycine-x-tyrosine-proline-glycine (GxYPG), where x may be the amino acids alanine (A), glycine (G), or valine (V), is shared in peptides 4, 6, and 7, each of which possess two such sequences separated by 3 amino acids. Accordingly, the presence of two GxYPG sequences in close apposition is likely predictive of the ability to bind Gal3-targeted antibodies with the ability to block Gal3 and INSR. Additionally, the Grantham distance of alanine, glycine, and valine is Ala-Val: 64, Ala-Gly: 60, Val-Gly: 109, thereby predicting that amino acids with similarly low Grantham distances may similarly be able to substitute at the variable region, including proline and threonine.

### Example 3. Gal3-INSR antibodies with blocking activity compete for binding to Gal3

To determine whether Gal3-binding antibodies with Gal3-INSR blocking activity bind to the same or overlapping regions of the Gal3 molecule, antibody binning assays were performed to assess the ability of antibodies to simultaneously bind Gal3. Amine-reactive probes were loaded onto a Gator biosensor (Probe Life, Palo Alto, CA), equilibrated in dH20 for 60 seconds, dipped into 100 µl EDC 0.2M /NHS 0.05M activation buffer for 30 seconds, then dipped into a solution of 20 µg/µl human Gal3-His in 10 mM NaOAc buffer, pH 5 until binding was saturated, and quenched in 1 M ethanolamine pH 8.5 for 300 seconds. Following Gal3-His loading, tips were dipped in 20 µg/mL saturating antibody, then successively dipped into 5 µg/mL competing antibody. As shown in **FIG. 3A-B****,** antibodies with competitive binding profiles were assigned bins and associations to blocking activity were made.

Separate bins of competitive antibody binding patterns to Gal3 were established. Significantly, strong associations between bin and blocking Gal3-INSR blocking activity were observed. All antibodies from bins 1, 2, 3, 4, 5, 6, and 7 strongly inhibited Gal3 binding to INSR, summarized in **FIG. 3A****.** In contrast, antibodies in bin 8 were somewhat weaker blockers of Gal3 blocking to INSR, despite strong affinity to Gal3. Antibodies in bin 10, 11, and 12 uniformly did not have the ability to significantly inhibit the association of Gal3 and INSR. Thus, the competitive binding bins of 1, 2, 3, 4, 5, 6, and 7, are useful to predict the ability of Gal3-binding antibodies to block the assembly of Gal3 and INSR.

### Example 4. Gal3-INSR blocking antibody IMT001-4 (TB001) reduces weight gain, insulin resistance, glucose insensitivity, liver steatosis, and liver dysfunction in high-fat diet fed diabetic mice.

To evaluate whether Gal3-INSR blockade by Gal3-targeted antibodies functionally impacted diabetes biology, a mouse model of type II diabetes was employed. Male C57BL6J mice were purchased from Jackson Lab and fed with high fat diet (HFD, 60% kcal from fat, Research Diet, Catalog #12492i) starting from 7-week old for 8 weeks, 10 or 16 weeks. Age-matched male C57BL6J mice fed with standard chow diet were used as control mice. The mice were housed in standard facilities and disposable standard cages with filter tops at room temperature with a 06:00-18:00 day-night cycle. Mice were fed with standard chow diet or HFD ad libitum except when fasted as indicated in the experiments. Glucose tolerance test (GTT) and insulin tolerance test (ITT) were done at 15 weeks and 16 weeks old (8 weeks and 9 weeks after HFD), respectively, to confirm the insulin resistant and glucose intolerant phenotype. For GTT, mice were fasted for 6 hours by transferring mice to clean cage with no food or faeces but with drinking water and then injected with 2 g/kg glucose (Sigma) and blood was drawn to measure glucose levels at 0, 15, 30, 60, 90, and 120 minutes after glucose injection. For ITT, mice were fasted for 6 hours, and then injected with 0.75 IU/ kg body weight of Humalin-R (Eli Lilly) intraperitoneally. Blood from the tail was measured for glucose content using HemoCue Glucose 201 Analyzer at 0, 15, 30, 45, 60 minutes. Based on area under the curve (AUC) from ITT, HFD fed mice were randomized into two groups: human IgG4 isotype group and IMT001-4 treated group. At 17 weeks of age, mice were given antibody treatments by intraperitoneal injection twice a week for 5 doses before ITT and GTT were done at 19 weeks old and 20 weeks old. Human IgG4 isotype and IMT001-4 were dosed at 10 mg/kg with 100 µl/mouse and BM was dosed at 10 mg/ml with 100 µl/mouse. Treatments continued until the mice were sacked at 21 weeks old. The mice body weight was monitored one week after the mice arrived at the facility. Body weight was measured once a week using a balance. Mouse blood was collected using cardiac puncture. Liver, gastrocnemius muscle, and posterior subcutaneous white adipose fat were collected and weighed.

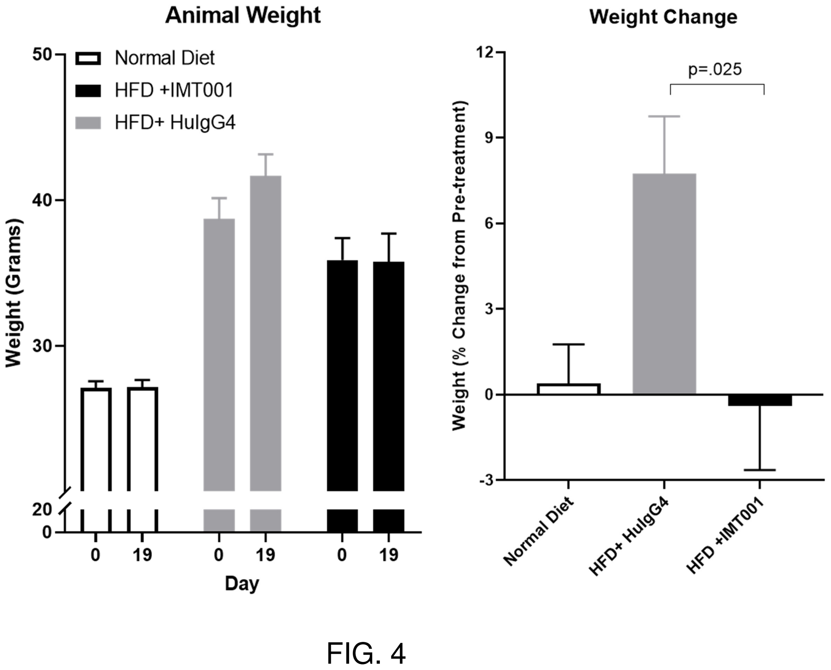

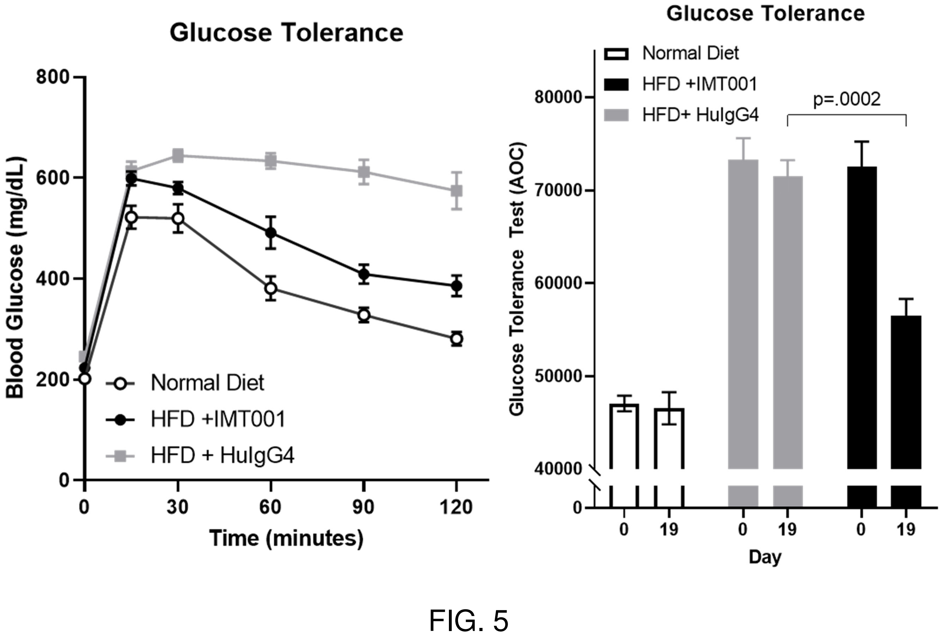

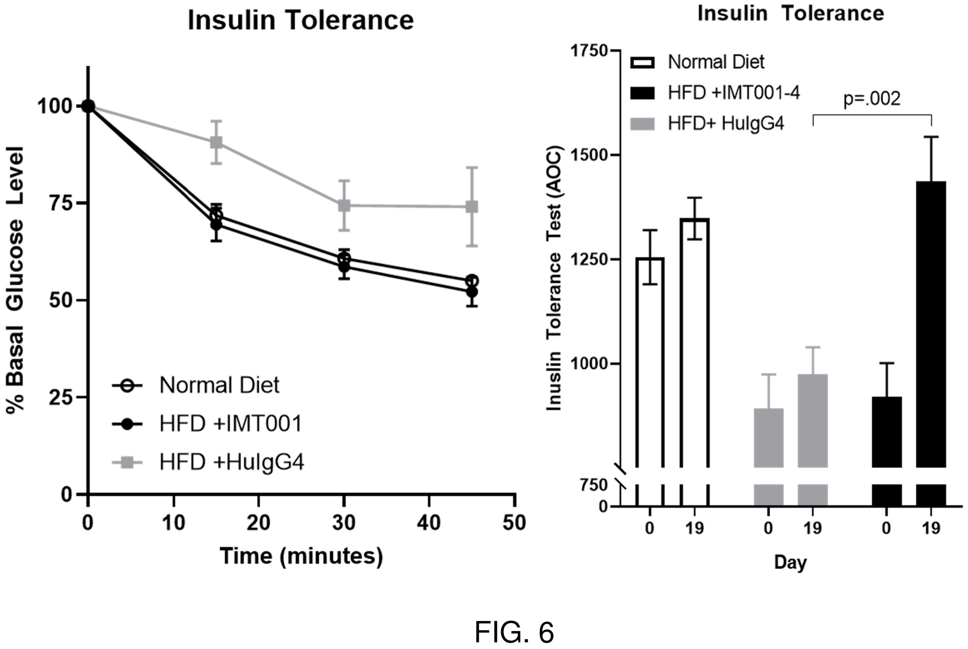

High-fat diet (HFD) fed mice were confirmed to have elevated basal glucose levels, and gained weight more rapidly than control-diet fed animals. Significantly, whereas HFD-fed mice treated with isotype control exhibited significant weight gain of over 7% body mass over the 19 day study, HFD-fed animals treated with IMT001-4 did not gain any appreciable weight **(****FIG. 4****).** Further, whereas isotype-control treated HFD-fed mice exhibited delayed response to glucose challenge, HFD-fed animals treated with IMT001-4 cleared glucose significantly more rapidly, in a manner more similar to mice fed with a normal diet **(****FIG. 5****).** Further, whereas HFD-fed animals treated with isotype control exhibited delayed glucose reduction in response to insulin challenge, IMT001-4 treated HFD-fed mice exhibited glucose clearance indistinguishable from non-diabetic mice fed a normal diet **(****FIG. 6****).** Collectively, these data support the finding that the Gal3-INSR blocking antibody IMT001-4 resolves multiple aspects of diabetes, including insulin resistance, glucose insensitivity, and weight gain.

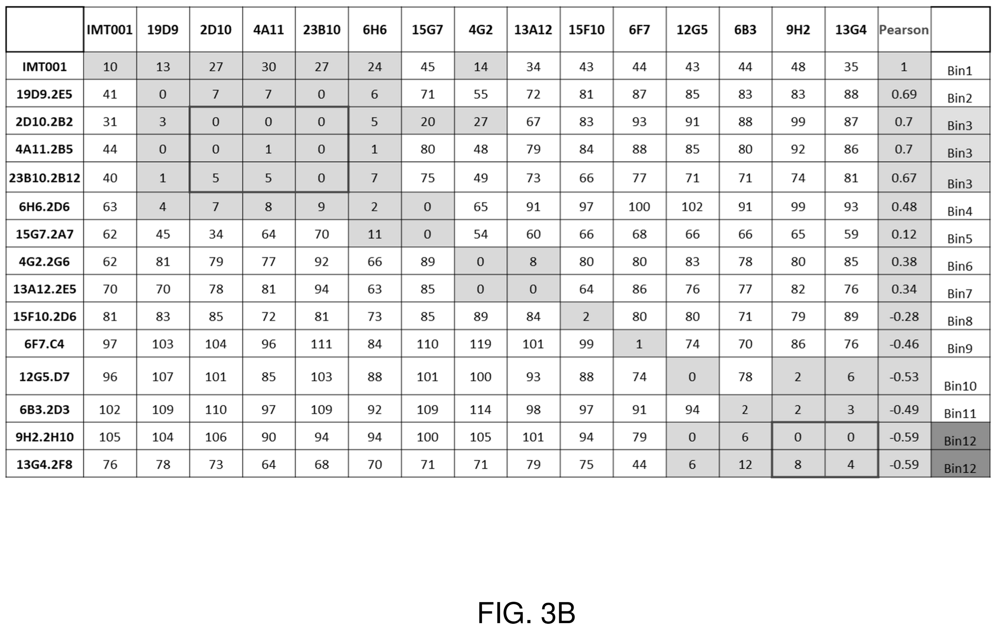

To further evaluate the ability of IMT001-4 to impact the pathological sequalae of diabetes, liver sections from animals treated as in **FIGs. 3-5** were examined for signs of fat accumulation, e.g., steatosis. Briefly, liver specimens were fixed in 4% paraformaldehyde for 24 hours, placed in 70% EtOH for 3 days, embedded into paraffin and 10 µm sections were cut and mounted to glass slides and stained with hematoxylin and eosin and visualized on a Revolve microscope at 40X magnification. Images were evaluated by ImageJ to quantitate the extent of steatosis.

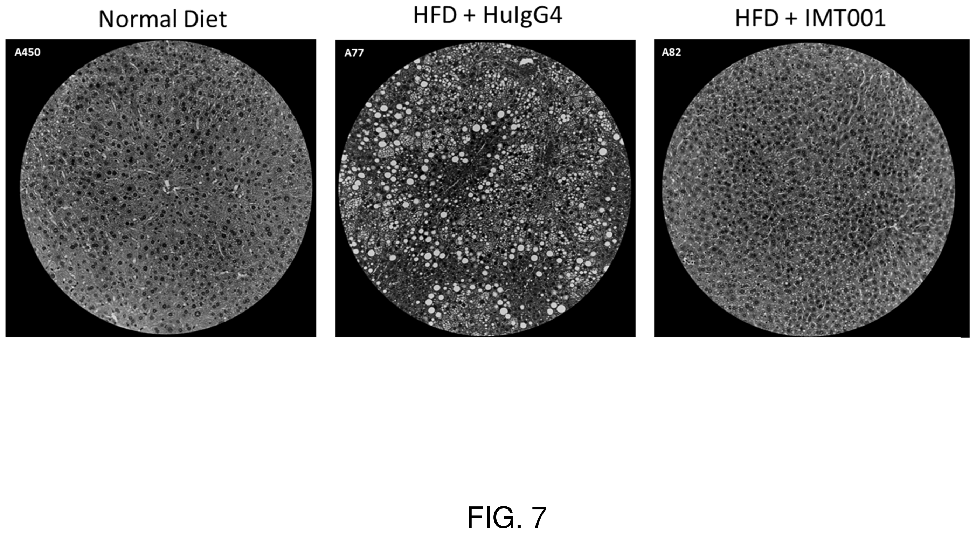

Moderate levels of steatosis were observed in HFD-fed animals treated with control antibody, exemplified by the presence of circular-non stained regions in H&E stained sections **(****FIG. 7****).** In contrast, HFD-fed animals treated with IMT001-4 exhibited negligible levels of steatosis and were difficult to distinguish from livers of non-diabetic animals fed with a normal diet. Visual observations were confirmed by image quantification, which demonstrated a complete reversion of disease state in IMT001-4 treated animals relative to isotype-control treated animals. Accordingly, the Gal3-targeted Gal3-INSR blocking antibody, IMT001-4, has utility in reducing liver steatosis.

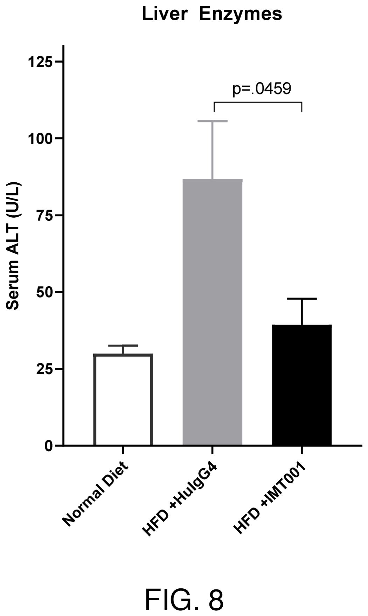

To further characterize the liver disease observed in histological samples, serum markers of liver dysfunction were evaluated in animals treated as above by ELISA. Briefly, mouse plasma samples were obtained from whole blood sample in lithium heparin collection tubes. 100 µl of serum was dispensed into the rotor of a VetScan VS2 Blood Chemistry Analyzer (Abaxis) through the sample port. ALT levels were determined as per the manufacturer's specification. Consistent with the observed elevated levels of steatosis observed in isotype-control treated HFD-fed diabetic mice relative to normal-diet fed non-diabetic mice, isotype-control HFD-fed diabetic mice exhibited nearly a 3-fold increase in ALT relative to normal-diet fed non-diabetic mice **(****FIG. 8****).** Strikingly, ALT levels were significantly decreased in IMT001-4 treated HFD-fed mice relative to HFD-fed isotype-control treated mice, to levels on-par with normal-diet fed non-diabetic mice. Accordingly, the Gal3-targeted Gal3-INSR blocking antibody, IMT001-4, has utility in reducing serum liver enzymes.

### Example 5. GAL3 specifically binds to ITGb1

To evaluate the possibility that human galectin-3 (GAL3) could physically interact with human integrin beta-1 (ITGb1), ELISA assessments with purified GAL3 and ITGb1, were conducted. Human GAL3 protein (TrueBinding, QCB200349; R&D Systems, 8259-GA) was diluted in PBS (Corning, 21-030-CM) to concentrations of 10, 5, and 2.5 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGb1 (Sino Biological, 10587-H08H1) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. After the plate was blocked, the 2% BSA in PBST was discarded and 10, 5, and 2.5 µg/ml of biotinylated recombinant ITGb1 in 2% BSA in PBST was added to the wells. The plate was incubated for an hour at room temperature with gentle rocking. Thereafter, the plate was washed three times with PBST. Avidin HRP (Biolegend, 405103; 1:2000 dilution) was diluted in 2% BSA in PBST and then added to the wells. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

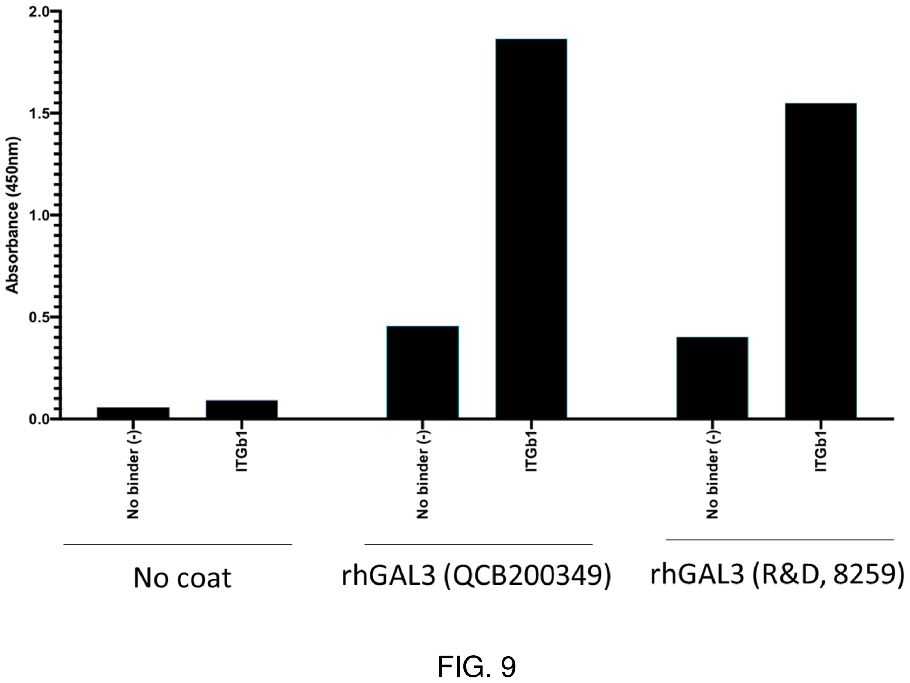

As depicted in **FIG. 9****,** ITGb1 strongly binds GAL3-coated wells. ITGb1 does not significantly bind to uncoated ELISA wells. Likewise, no significant binding signal is observed in wells with only GAL3 coating.

### Example 6: Identification of GAL3-binding antibodies with GAL3-ITGb1 blocking activity

To identify GAL3-targeted antibodies with the ability to block the interaction of GAL3 and ITGbl, purified GAL3 and ITGb1 were incubated in the presence (or absence) of various GAL3-targeted or control antibodies, or without antibody, and protein interaction was evaluated by ELISA.

Human GAL3 (TrueBinding, QCB200352) was diluted in PBS (Corning, 21-030-CM) to a concentration of 4 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGb1 (Sino Biological, 10587-H08H1) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. Thereafter, the 2% BSA in PBST was discarded and 30 µl of control or anti-GAL3 antibodies at 20, 6.6, or 2.2 µg/ml was added to each well, followed by the addition of 30 µl of 2 µg/ml of biotinylated ITGb1 in 2% BSA in PBST. The plate was incubated for an hour at room temperature with gentle rocking. Thereafter, the plate was washed three times with PBST. Avidin HRP (Biolegend, 405103) was then added to the wells at 1:2000 dilution in 2% BSA in PBST. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm. Percent blockade of the GAL3-ITGb1 interaction was calculated as the fraction of signal obtained in the absence of antibody with the background signal subtracted.

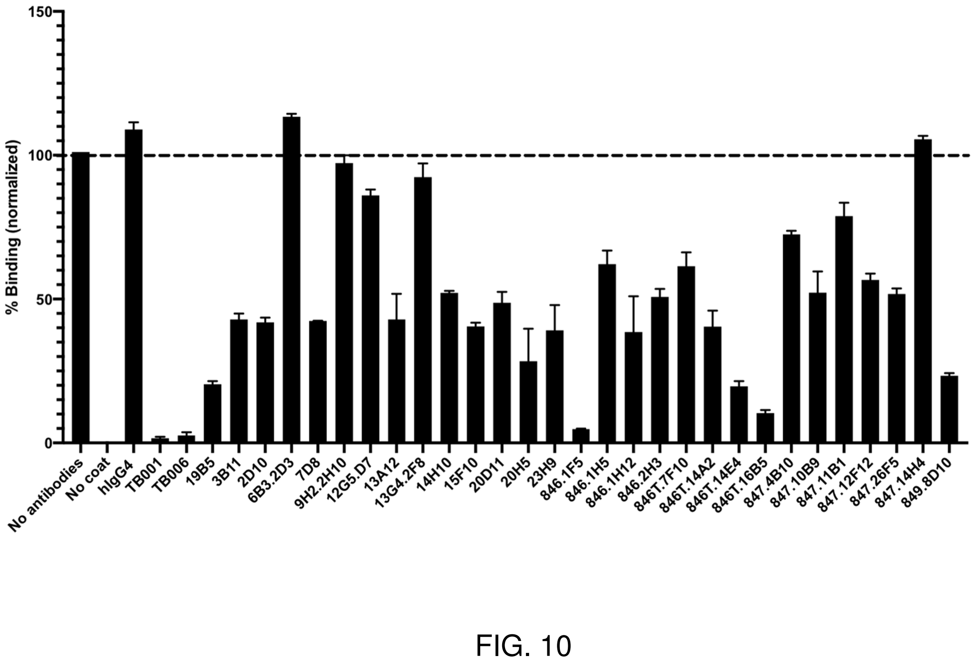

As shown in **FIG. 10****,** anti-GAL3 antibodies display differential blocking of the interaction between GAL3 and ITGb1. Three antibodies display blocking > 90% (TB001, TB006, and 846.1F5), twelve antibodies display intermediate blocking between 40 and 60% (3B11, 2D10, 7D8, 13A12, 14H10, 15F10, 20D11, 846.2H3, 846T.14A2, 847.10B9, 847.12F12, and 847.26F5), and three antibodies display no blocking ability (6B3.2D3, 847.14H4, and data not shown). Since anti-GAL3 antibodies display differential ability to block the GAL3 interaction with ITGb1, this shows that GAL3 binding alone is not sufficient to disrupt the interaction of GAL3 and ITGb1, and specific properties are required for this disrupting activity.

### Example 7: GAL3 specifically binds to ITGa3

To evaluate the possibility that human galectin-3 (GAL3) could physically interact with human integrin alpha-3 (ITGa3), ELISA assessments with purified GAL3 and ITGa3, were conducted. Human GAL3 protein (TrueBinding, QCB200349; TrueBinding, QCB200352) was diluted in PBS (Corning, 21-030-CM) to concentrations of 4, 2, and 1 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGa3 protein antigen (Novus Biological, NBP2-48514PEP) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. After the plate was blocked, the 2% BSA in PBST was discarded and 4, 2, and 1 µg/ml of biotinylated recombinant ITGa3 in 2% BSA in PBST was added to the wells. The plate was incubated for an hour at room temperature with gentle rocking. Thereafter, the plate was washed three times with PBST. Avidin HRP (Biolegend, 405103; 1:2000 dilution) was diluted in 2% BSA in PBST and then added to the wells. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

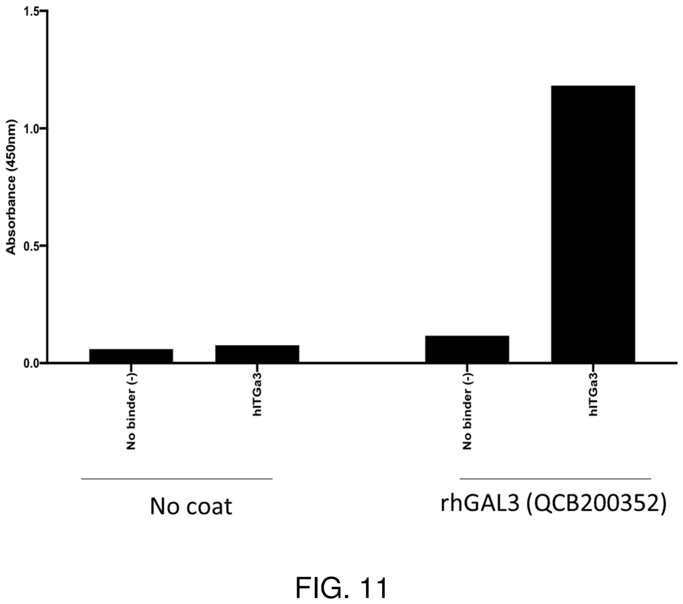

As depicted in **FIG. 11****,** ITGa3 strongly binds GAL3-coated wells. ITGa3 does not significantly bind to uncoated ELISA wells. Likewise, no significant binding signal is observed in wells with only GAL3 coating.

### Example 8: Identification of GAL3-binding antibodies with GAL3-ITGa3 blocking activity

To identify GAL3-targeted antibodies with the ability to block the interaction of GAL3 and ITGa3, purified GAL3 and ITGa3 were incubated in the presence (or absence) of various GAL3-targeted or control antibodies, or without antibody, and protein interaction was evaluated by ELISA.

Human GAL3 (TrueBinding, QCB200352) was diluted in PBS (Corning, 21-030-CM) to a concentration of 4 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGa3 protein antigen (Novus Biological, NBP2-48514PEP) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. Thereafter, the 2% BSA in PBST was discarded and 30 µl of control or anti-GAL3 antibodies at 20, 6.6, or 2.2 µg/ml was added to each well, followed by the addition of 30 µl of 4 µg/ml of biotinylated ITGa3 in 2% BSA in PBST. The plate was incubated for an hour at room temperature with gentle rocking. Thereafter, the plate was washed three times with PBST. Avidin HRP (Biolegend, 405103) was then added to the wells at 1:2000 dilution in 2% BSA in PBST. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm. Percent blockade of the GAL3-ITGa3 interaction was calculated as the fraction of signal obtained in the absence of antibody with the background signal subtracted.

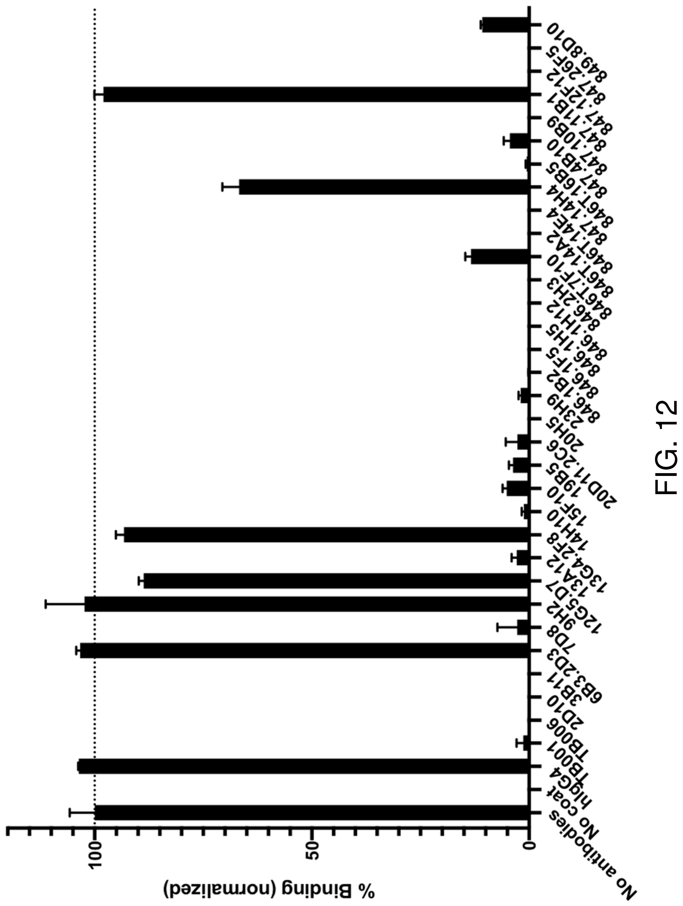

As shown in **FIG. 12****,** anti-GAL3 antibodies display differential blocking of the interaction between GAL3 and ITGa3. Twenty-four antibodies display > 90% blocking ability (TB001, TB006, 2D10, 3B11, 7D8, 13A12, 14H10, 15F10, 19B5, 20D11.2C6, 20H5, 23H9, 846.1B2, 846.1F5, 846.1H5, 846.1H12, 846.2H3, 846T.14A2, 846T.14E4, 846T.16B5, 847.4B10, 847.10B9, 84712F12, and 847.26F5), while thirteen antibodies display < 35% blocking ability (6B3.2D3, 9H2.2H10, 12G5.D7, 13G4.2F8, 847.14H4, 847.11B1, and data not shown). Since anti-GAL3 antibodies display differential ability to block the GAL3 interaction with ITGa3, this shows that GAL3 binding alone is not sufficient to disrupt the interaction of GAL3 and ITGa3, and specific properties are required for this disrupting activity.

### Example 9: GAL3 specifically binds to ITGb3

To evaluate the possibility that human galectin-3 (GAL3) could physically interact with human integrin beta-3 (ITGb3), ELISA assessments with purified GAL3 and ITGb3, were conducted. Human GAL3 protein (TrueBinding, QCB200349; R&D Systems, 8259-GA) was diluted in PBS (Corning, 21-030-CM) to concentrations of 10, 5, and 2.5 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGb3 protein (Abbexa, abx067294) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. After the plate was blocked, the 2% BSA in PBST was discarded and 10, 5, and 2.5 µg/ml of biotinylated recombinant ITGb3 in 2% BSA in PBST was added to the wells. The plate was incubated for an hour at room temperature with gentle rocking. Thereafter, the plate was washed three times with PBST. Avidin HRP (Biolegend, 405103; 1:2000 dilution) was diluted in 2% BSA in PBST and then added to the wells. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

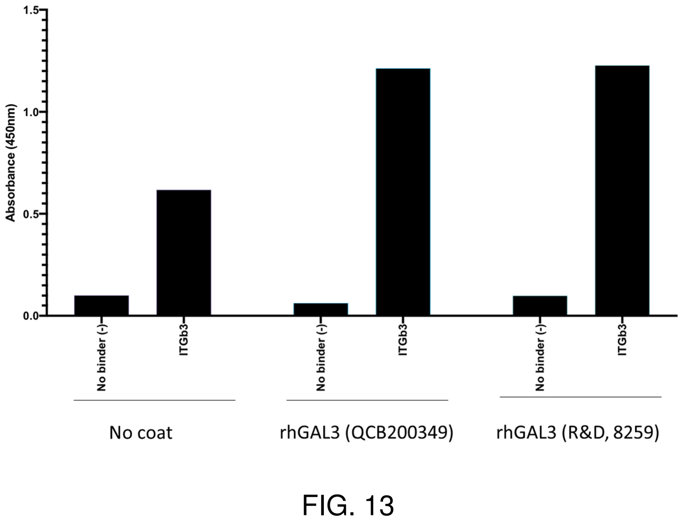

As depicted in FIG. 13, ITGb3 strongly binds GAL3-coated wells. ITGb3 does not significantly bind to uncoated ELISA wells. Likewise, no significant binding signal is observed in wells with only GAL3 coating.

### Example 10: Identification of GAL3-binding antibodies with GAL3-ITGb3 blocking activity

To identify GAL3-targeted antibodies with the ability to block the interaction of GAL3 and ITGb3, purified GAL3 and ITGb3 were incubated in the presence (or absence) of various GAL3-targeted or control antibodies, or without antibody, and protein interaction was evaluated by ELISA.

Human GAL3 (TrueBinding, QCB200352) was diluted in PBS (Corning, 21-030-CM) to a concentration of 4 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGb3 protein (Abbexa, abx067294) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. Thereafter, the 2% BSA in PBST was discarded and 30 µl of control or anti-GAL3 antibodies at 20, 6.6, or 2.2 µg/ml was added to each well, followed by the addition of 30 µl of 2 µg/ml of biotinylated ITGb3 in 2% BSA in PBST. The plate was incubated for an hour at room temperature with gentle rocking. Thereafter, the plate was washed three times with PBST. Avidin HRP (Biolegend, 405103) was then added to the wells at 1:2000 dilution in 2% BSA in PBST. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm. Percent blockade of the GAL3-ITGb3 interaction was calculated as the fraction of signal obtained in the absence of antibody with the background signal subtracted.

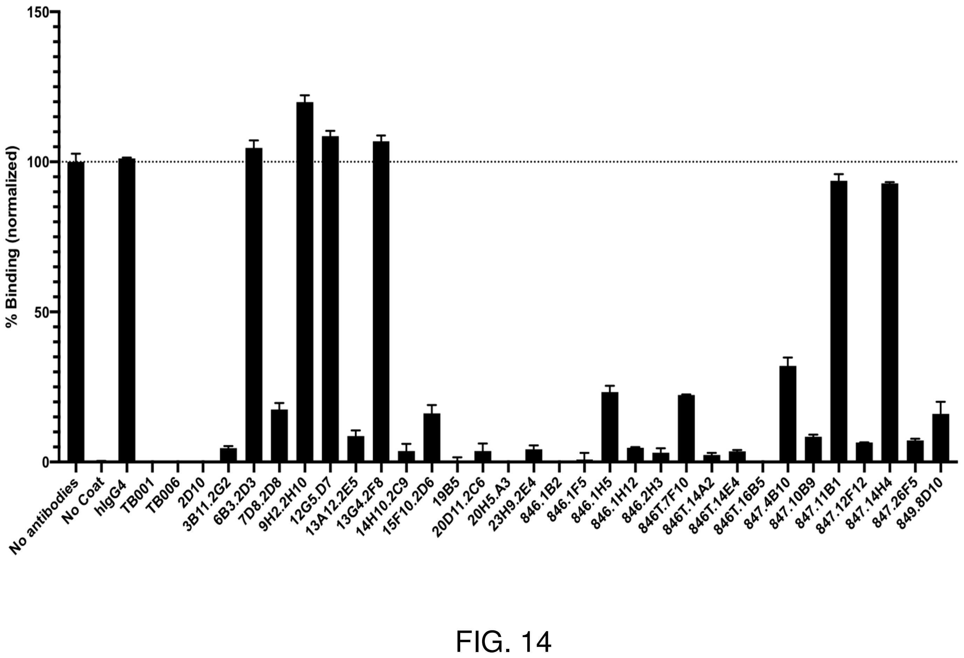

As shown in **FIG. 14****,** anti-GAL3 antibodies display differential blocking of the interaction between GAL3 and ITGb3. Twenty antibodies display > 90% blocking ability (TB001, TB006, 2D10, 3B11.2G2, 13A12.2E5, 14H10.2C9, 19B5, 20D11.2C6, 20H5.A3, 23H9.2E4, 846.1B2, 846.1F5, 846.1H12, 846.2H3, 846T.14A2, 846T.14E4, 846T.16B5, 847.10B9, 847.12F12, and 847.26F5), while thirteen antibodies display < 10% blocking ability (6B3.2D3, 9H2.2H10, 12G5.D7, 13G4.2F8, 847.11B1, 847.14H4, and data not shown). Since anti-GAL3 antibodies display differential ability to block the GAL3 interaction with ITGb3, this shows that GAL3 binding alone is not sufficient to disrupt the interaction of GAL3 and ITGb3, and specific properties are required for this disrupting activity.

### Example 11: GAL3 specifically binds to ITGaV

To evaluate the possibility that human galectin-3 (GAL3) could physically interact with human integrin alpha-V (ITGaV), ELISA assessments with purified GAL3 and ITGaV, were conducted. Human GAL3 protein (TrueBinding, QCB200349; TrueBinding, QCB200352) was diluted in PBS (Corning, 21-030-CM) to concentrations of 4, 2, and 1 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGaV protein (abbexa, abx166523) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. After the plate was blocked, the 2% BSA in PBST was discarded and 4, 2, and 1 µg/ml of biotinylated recombinant ITGaV in 2% BSA in PBST was added to the wells. The plate was incubated for an hour at room temperature with gentle rocking. Thereafter, the plate was washed three times with PBST. Avidin HRP (Biolegend, 405103; 1:2000 dilution) was diluted in 2% BSA in PBST and then added to the wells. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

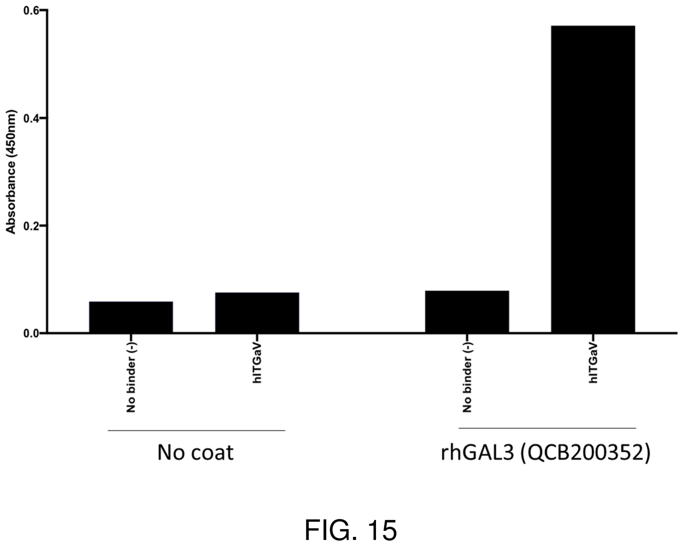

As depicted in **FIG. 15****,** ITGaV strongly binds GAL3-coated wells. ITGaV does not significantly bind to uncoated ELISA wells. Likewise, no significant binding signal is observed in wells with only GAL3 coating.

### Example 12: Identification of GAL3-binding antibodies with GAL3-ITGaV blocking activity

To identify GAL3-targeted antibodies with the ability to block the interaction of GAL3 and ITGaV, purified GAL3 and ITGaV were incubated in the presence (or absence) of various GAL3-targeted or control antibodies, or without antibody, and protein interaction was evaluated by ELISA.

Human GAL3 (TrueBinding, QCB200352) was diluted in PBS (Corning, 21-030-CM) to a concentration of 4 µg/ml and added to the wells of a 96-well ELISA plate (Thermo Fisher, 44-2404-21). After incubating the plate at 4°C overnight, the plate was washed three times with PBST (PBS with 0.05% Tween 20 [VWR, 0777]) and then blocked for an hour with 2% BSA (EMD Millipore, 126609) in PBST at room temperature with gentle rocking. Recombinant human ITGaV protein (abbexa, abx166523) was biotinylated with EZ Link Sulfo-NHS-LC-Biotin (ThermoFisher Scientific, A39257) and desalted using a Zeba Spin Desalting Column (ThermoFisher Scientific, 89882), following the manufacturer's instructions. Thereafter, the 2% BSA in PBST was discarded and 30 µl of control or anti-GAL3 antibodies at 20, 6.6, or 2.2 µg/ml was added to each well, followed by the addition of 30 µl of 8 µg/ml of biotinylated ITGaV in 2% BSA in PBST. The plate was incubated for an hour at room temperature with gentle rocking. Thereafter, the plate was washed three times with PBST. Avidin HRP (Biolegend, 405103) was then added to the wells at 1:2000 dilution in 2% BSA in PBST. The plate was incubated at room temperature for an hour with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm. Percent blockade of the GAL3-ITGaV interaction was calculated as the fraction of signal obtained in the absence of antibody with the background signal subtracted.

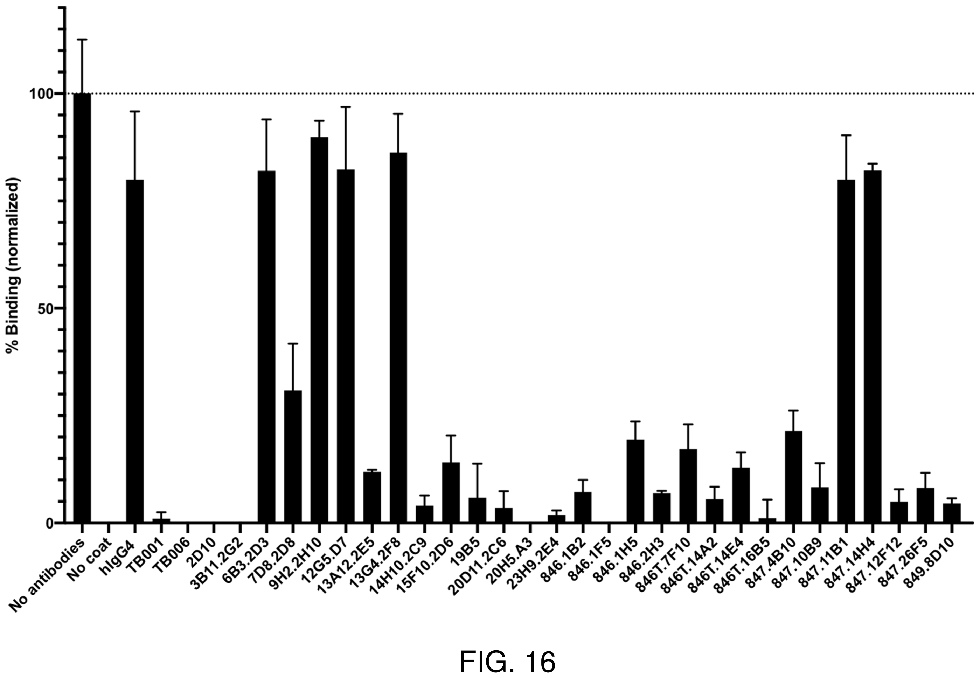

As shown in **FIG. 16****,** anti-GAL3 antibodies display differential blocking of the interaction between GAL3 and ITGaV. Eighteen antibodies display > 90% blocking ability (TB001, TB006, 2D10, 3B11.2G2, 14H10.2C9, 19B5, 20D11.2C6, 20H5.A3, 23H9.2E4, 846.1B2, 846.1F5, 846.2H3, 846T.14A2, 846T.16B5, 847.10B9, 847.12F12, 847.26F5, and 849.8D10), two antibodies display intermediate (between 40 and 60%) blocking ability (data not shown), and ten antibodies display < 30% blocking ability (6B3.2D3, 9H2.2H10, 12G5.D7, 13G4.2F8, 847.11B1, 847.14H4, and data not shown). Since anti-GAL3 antibodies display differential ability to block the GAL3 interaction with ITGaV, this shows that GAL3 binding alone is not sufficient to disrupt the interaction of GAL3 and ITGaV, and specific properties are required for this disrupting activity.

### Example 13: Anti-GAL3 antibodies that block GAL3 interaction with integrins inhibit Jurkat T cell adhesion.

To evaluate whether anti-GAL3 antibodies that block GAL3 interaction with integrin negatively influence lymphocyte homing (an important aspect in development of inflammatory diseases), a cell-based assay utilizing lymphocyte Jurkat T cell line was employed. Briefly, recombinant Gal-3 (10mg/ml in PBS) and antibody (5 mg/ml in PBS) were incubated for 30 minutes on ice prior to the coating mixture transfer to the 96-well plate and additional overnight incubation. The coating mixture was then removed and the plate was blocked with 0.5% BSA for 45 minutes. Jurkat T cells (10⁵ cells per well) were plated in serum free RPMI. Following thirty-minute incubation, the cells were gently washed once with serum free medium and fixed with 4% PFA for 10 minutes. The adhered cells were further stained with SYTOX^{™} Orange Nucleic Acid staining (ThermoFisher, S11368) and analyzed by Cytation 1 Cell Imaging Multi-Mode Reader (Biotek).

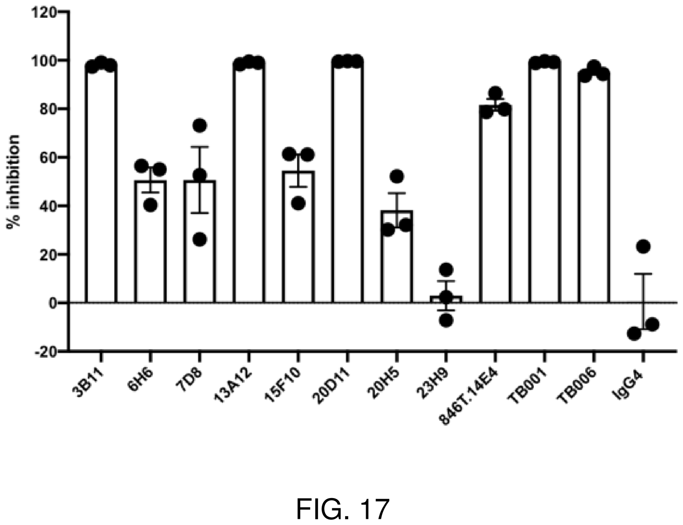

As depicted in **FIG. 17****,** four anti-GAL3 antibodies inhibited Jurkat T cell adhesion with > 90% efficacy (3B11, 20D11, TB001, TB006). Five anti-GAL3 antibodies inhibited Jurkat T cell adhesion between 40% and 80% (6H6, 7D8, 15F10, 20H5, and 846T.14E4). Clone 23H9 did not inhibit lymphocyte homing. Taken together, specific properties of anti-GAL3 antibodies are required to inhibit Jurkat T cell adhesion.

### Example 14: Anti-GAL3 antibodies have therapeutic potential in a model of advanced Type II diabetes

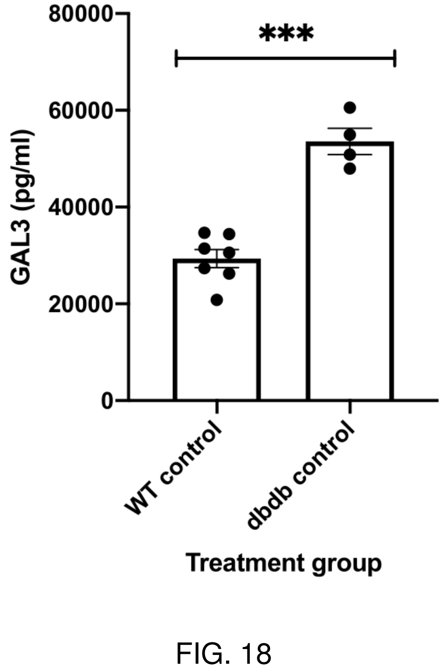

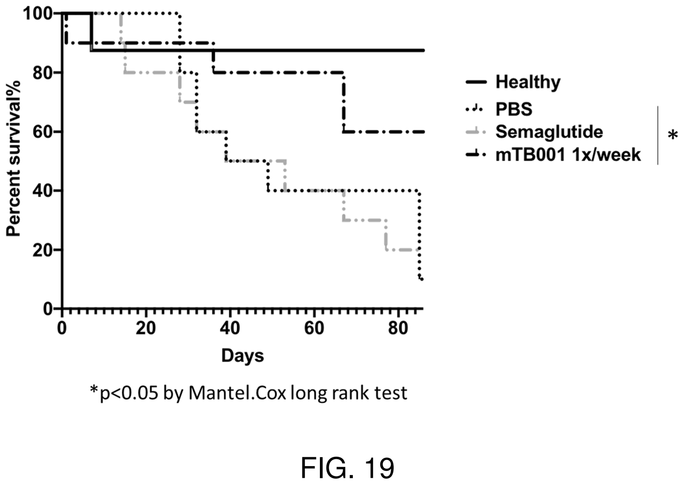

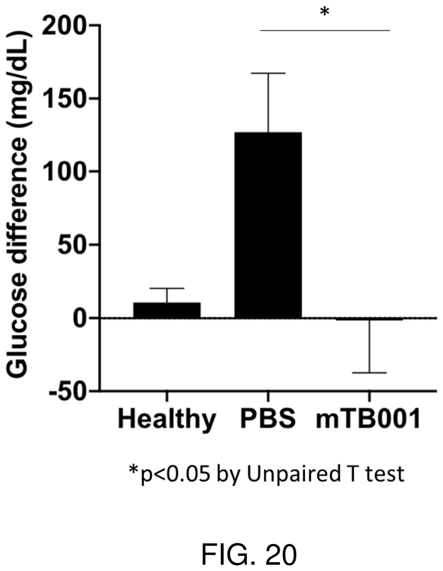

In order to investigate the effect of therapeutic anti-GAL-3 antibodies on a severe case of type II diabetes, a mouse model using High Fat Diet (HFD)-fed Db/Db mice was implemented. Four-weeks-old male Bks-Db mice (000642) obtained from Jackson lab were fed with 60% kcal HFD (Research Diet, 12492i) for eight weeks. Control animals (male C57BL6/J (000662), Jackson lab) were fed normal chow diet. Db/Db animals had increased levels circulating Gal-3 **(****FIG. 18****).** Animals were randomized based on the body composition measured by employing EchoMRI-500^{™} (EchoMRI). The HFD-fed Db/Db animals were treated with negative control (PBS), positive control (Semaglutide), or anti-GAL3 TB001 antibodies Q1W (once weekly). Wild type animals were used as a control (Healthy) group. The animals were dosed for twelve weeks. TB001 (anti-GAL3) treatment significantly prolonged survival of Db/Db animals **(****FIG. 19****).** The prolonged survival of the TB001-treated animals was also associated with a lack of change in the levels of fasting blood glucose compared to PBS-treated groups as depicted in **FIG. 20****.** The fasting blood glucose was measured as described in Example 15. Briefly, the mice were fasted for 4-6 hours in clean cages with water and no food. Blood from the tail was diluted 2.5 times before using HemoCue Glucose 201 Analyzer and cuvettes (Mercedes Scientific 110706) to measure the blood glucose level.

### Example 15: Anti-GAL3 antibodies have therapeutic potential in a mouse model of Type I diabetes

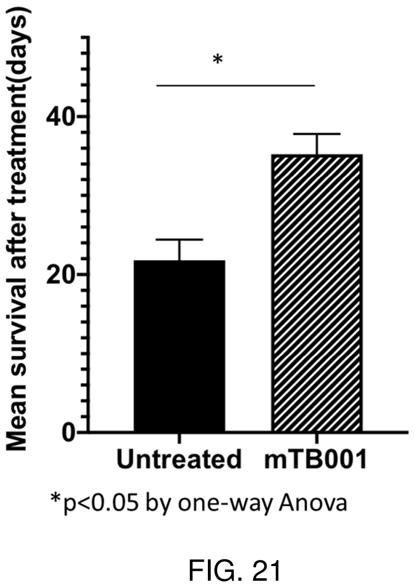

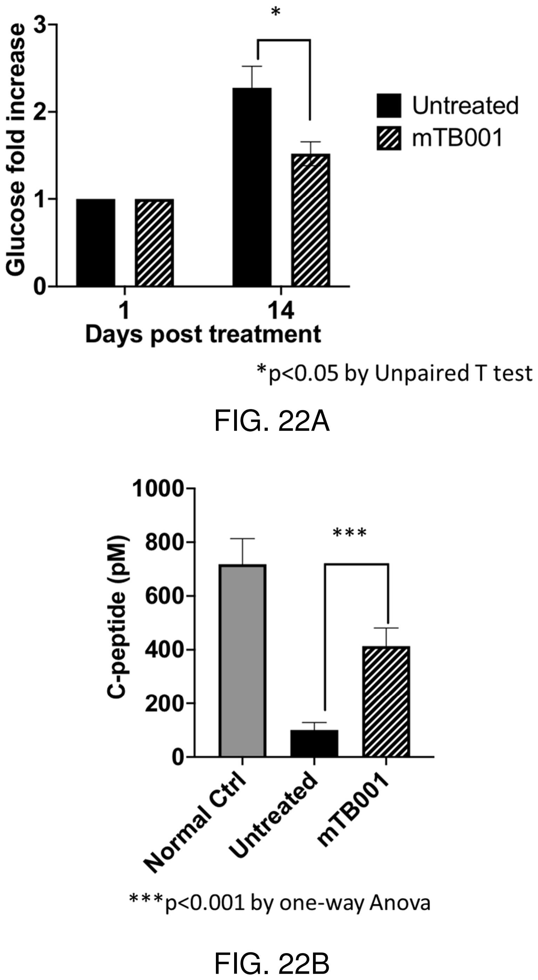

To assess the therapeutic potential of anti-GAL3 antibody in Type I diabetes, a mouse model of diabetes type I was used. Briefly, seven-weeks-old female NOD/ShiLtJ mice (Jackson lab (001976)) and control group animals NOR/LtJ (Jackson Lab (002050)) were housed in standard disposable cages with filter tops at room temperature with a 6:00-18:00 day-night cycle. Mice were allowed to rest for a week before initiating glucose monitoring. The antibody treatment was initiated when glucose levels reach 250-400 (mg/dL) for two consecutive days. To establish glucose levels, the blood samples were collected from tail vein of the experimental animals following housing in clean cages with water and no food for 4-6 hours. The collected samples were analyzed by HemoCue Glucose 201 Analyzer and cuvettes (Mercedes Scientific 110706). The animals were dosed twice a week (10mg/kg) with anti-GAL3 antibodies (mTB001) or PBS control. The animals were sacrificed when the glucose levels reached >1000 (mg/dL) or until they exhibited morbidity symptoms (i.e. hunched posture, hypothermia, and hypoactivity). At the end stage, the plasma samples were collected from symptomatic animals. C-peptide levels were determined by Alpco Mouse C-peptide ELISA kit (Alpco, 80-CPTMS-E01). As depicted in **FIG. 21****,** anti-GAL3 treatment prolonged survival of NOD/ShiLtJ animals and restored beta-cell function by stabilizing the levels of blood glucose **(****FIG. 22A****)** and restoring C-peptide levels **(****FIG. 22B****).** Therefore, anti-Gal3 treatment decreases the symptoms of both Type II and Type I diabetes.

### Example 16: Anti-GAL3 antibodies have therapeutic potential in a mouse model of chronic inflammatory bowel disease (IBD)

A mouse model of chronic inflammatory bowel disease was implemented to study the effect of Gal-3 antibodies. Briefly, seven-weeks-old male C57BL/6J mice were housed in standard disposable cages with filter tops at room temperature with a 6:00-8:00 day-night cycle. Mice were allowed to rest for one week before initiating the treatment.

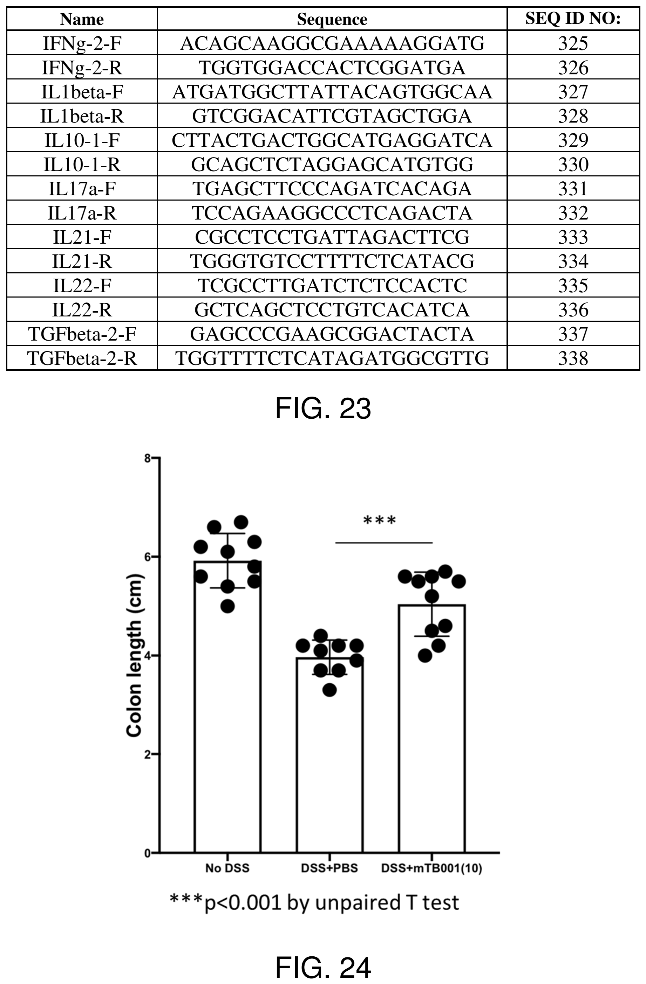

Mice were randomized into the four treatment groups: no dextran sulfate sodium salt (DSS), 3% DSS in water + PBS, 3% DSS + mTB001 (10mg/kg), 3% DSS + mTB001 (1mg/kg). DSS water mixture was given for five consecutive days followed by seven days of normal water for a total of three DSS cycles and two normal water cycles. Body weight, stool consistency, gross bleeding score, and Hemoccult score (Fisher, SK-61130) were monitored daily. At necropsy, blood, colon, jejunum, ileum, duodenum, and spleen were preserved and flash frozen. Plasma Inflammatory cytokines were measured using Multi-spot Assay Systems (MSD) Proinflammatory Panel 1 (mouse) kit (MSD, K15048D). RT-qPCR was performed on frozen colon samples. Briefly, the colon tissue was disrupted using Qiazol lysis buffer (Qiagen, 79306) and the Qiagen Tissue Lyser II (Qiagen, 85300). RNA was extracted using RNeasy mini kit (Qiagen, 74104). cDNA was synthesized with iScript Reverse Transcription Supermix (BioRad, 1708841) at BioRad C1000 touch thermal cycler (BioRad, 1851138). RT-qPCR was performed using SsoAdvanced Universal SYBR green supermix (BioRad, 1725272) at CFX384 Touch Real-Time PCR detection system (BioRad, 1955485) using the primers in **FIG. 23** (SEQ ID NO: 325-338).

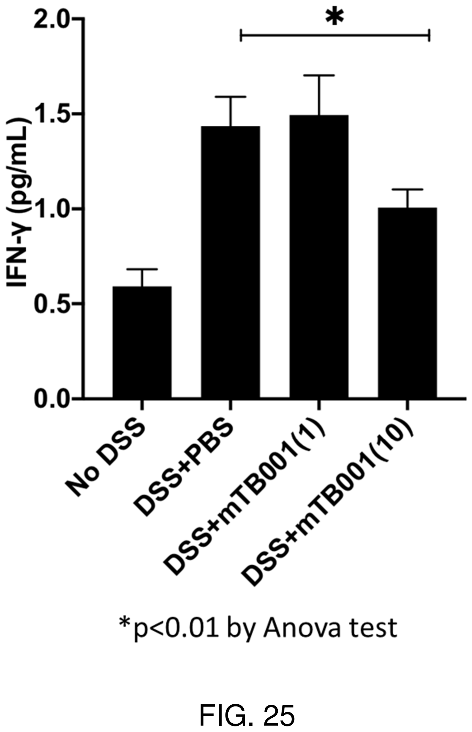

Anti-galectin-3 treatment restored DSS-induced reduction in the colon length **(****FIG. 24****)** and reduced DSS-induced inflammation determined by the circulating levels of IFN-gamma **(****FIG. 25****).** In addition, mRNA levels of several pro-inflammatory genes (e.g. IFN-gamma, IL17a, IL-1beta, IL-21, IL-22) were reduced in response to TB001 treatment.

### Example 17. Sequences of anti-Gal3 antibodies

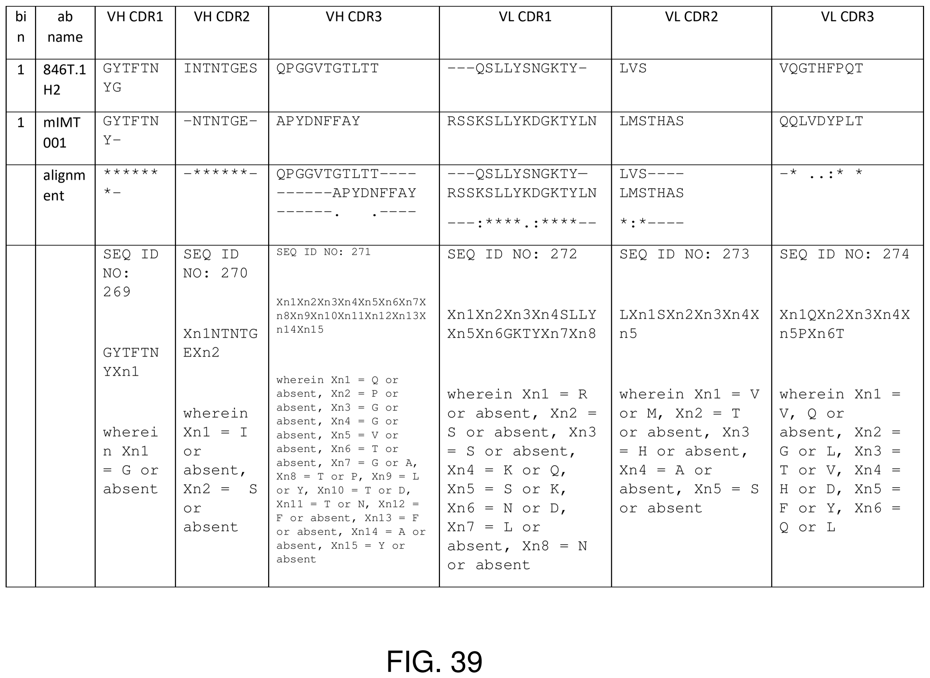

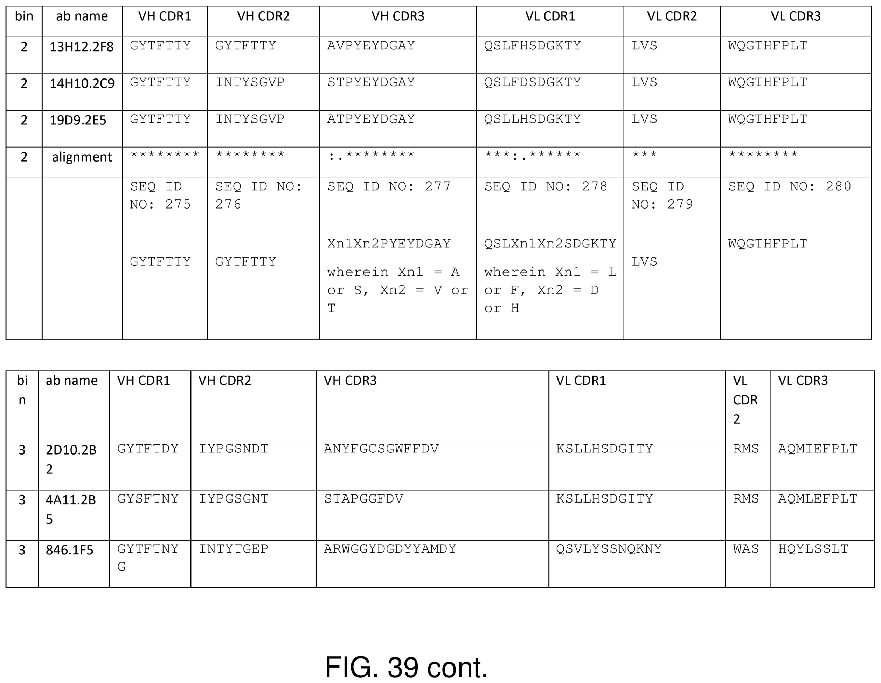

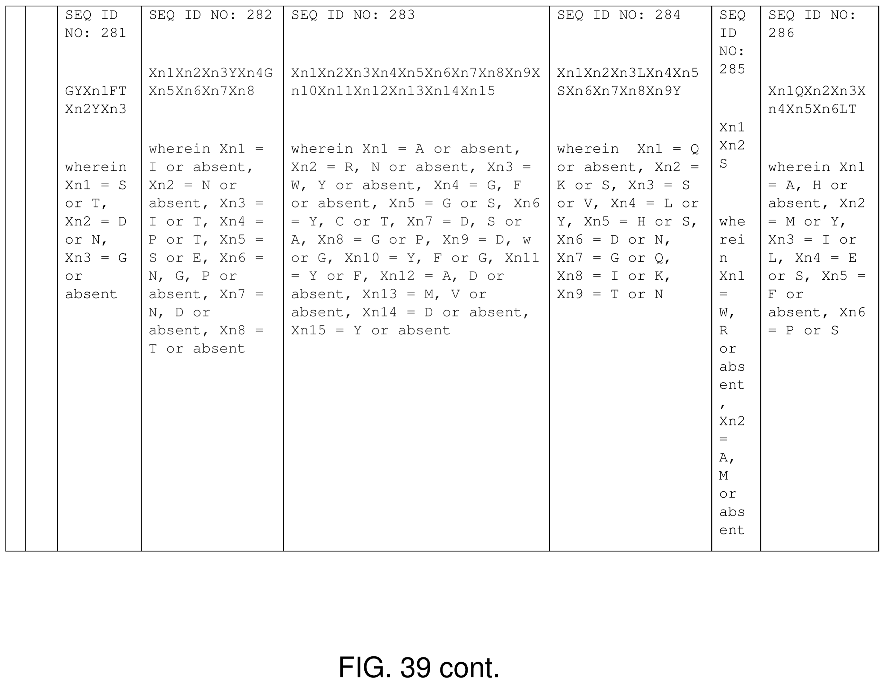

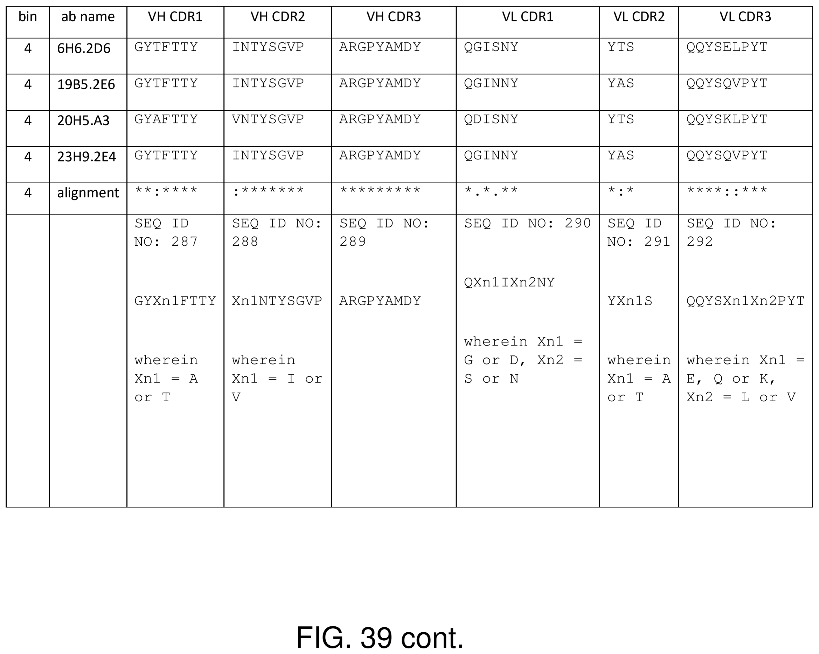

Complementarity determining regions of GAL3 binding antibodies from various bins were aligned using Clustal Omega **(****FIG. 39****).** Bin 1 antibodies shared significant homology in VH CDR1 and CDR2, as well as in regions of VL CDR1 and CDR3. Bin 2 antibodies shared significant homology in all CDRs examined, with relatively conservative A/S, V/T, H/D, and L/F substitutions observed. Bin 3 antibodies were somewhat more diverse, with significant sequence homology in CDR1, but relatively divergent in other CDR regions. Bin 4 antibodies shared significant homology in all CDRs examined, with relatively conservative A/T, I/V, D/G, S/N, QK, and V/L substitutions observed. Bin 5 antibodies also shared significant homology in all CDRs, with relatively conservative Y/F, N/K, substitutions observed in addition to less conservative T/I, N/Y substitutions. Finally, bin 7 antibody CDRs were observed to be nearly identical, with a single V/L substitution in VL CDR2 distinguishing 3B11.2G2 from 13A12.2E5. Alignments with any of the other sequences provided in **FIGs. 28A-C****,** **29A-C****,** **30-33** can be done with techniques known in the art.

### Example 18: Binning and peptide binding assay of additional exemplary anti-Gal3 antibodies

A large-scale antibody binning assay was performed on additional exemplary anti-Gal3 antibodies.

The epitope binning assay was done in a sandwich format on the high-throughput SPR-based Carterra LSA unit (CarterraBio, Salt Lake City, UT). First, the purified antibodies were diluted to 10 µg/ml concentration in 10 mM NaOAc (pH 5.0) and then were covalently coupled via amine group to HC200M chip activated by EDC and S-NHS to immobilize antibodies to different positions of a 384-spot array. One hundred thirty-eight binning cycles were run on the array of immobilized antibodies. In each cycle, first, human Gal3 (AcroBio GA3-H5129) was injected over the entire array to bind to different antibodies (primary antibody), followed by one antibody (secondary antibody) selected among the panel of 150 anti-GAL3 antibodies tested. At the end of each cycle, the array was regenerated by 10 mM Glycine (pH 2.0) to remove bound antigen and secondary antibody from the array. The data analysis was done using the Epitope software by CarterraBio.

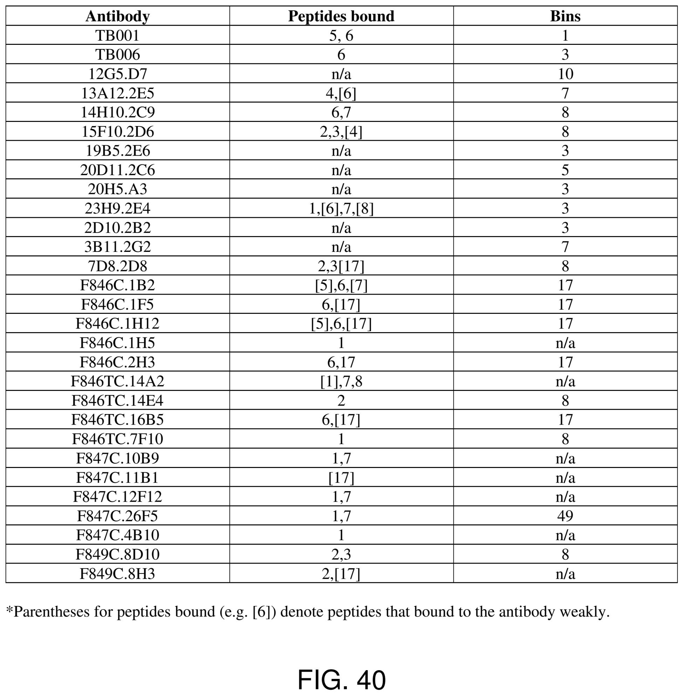

Binning results are shown in **FIG. 40****.** In total, 49 distinct bins were identified for 120 anti-GAL3 antibodies that demonstrate binding to hGAL3 (30 antibodies out of 150 tested did not bind hGAL3 when immobilized on HC200M chip and were thus excluded from further analysis). Antibodies that strongly blocked the association of GAL3 and APP695 belonged to a number of distinct bins defined below. This highlights the utility of these bins as predictors of GAL3 binding activity.

Antibody IMT001 (TB001) defined bin 1. Clones IMT006 (TB006), 19B5.2E6, 20H5.A3, 23H9.2E4, 2D10.2B2 exhibited mutual competitive binding for Gal3, but did not prevent binding of the rest of the clones, thus defining bin 3. Clones 3B11.2G2, 13A12.2E5 exhibited mutual competitive binding for Gal3, but did not prevent binding of the rest of the clones, thus defining bin 7. Clones 14H10.2C9, 15F10.2D6, 7D8.2D8, F846TC.14E4, F846TC.7F10, F849C.8D10 exhibited mutual competitive binding for Gal3, but did not prevent binding of the rest of the clones, thus defining bin 8. Clones F846C.1B2, F846C.1F5, F846C.1H12, F846C.2H3, F846TC.16B5 exhibited mutual competitive binding for Gal3, but did not prevent binding of the rest of the clones, thus defining bin 17. Clones F847C.10B9, F847C.12F12, and F847C.26F5 exhibited mutual competitive binding for Gal3, but did not prevent binding of the rest of the clones, thus defining bin 49. In addition, a number of clones did not compete for binding to Gal3 with other antibodies tested, therefore defining bins 5 (clone 20D11.2C6) and bin 10 (clone 12G5.D7), respectively.

Gal3 peptide binding activity of additional exemplary anti-Gal3 antibodies was assessed with the same peptides as described in FIG. 27 **(SEQ ID NOs: 3-26).** Human Gal3 peptides (LifeTein, custom order) and human Gal3 proteins (R&D Systems, 8259-GA; TrueBinding, QCB200349) were diluted in PBS (Corning, 21-030-CM) to concentrations of at least 100 µg/mL (peptides) or 1 µg/mL (proteins), and added to the wells of several 96-well ELISA plates (Thermo Fisher, 44-2404-21). After incubating the plates at 4°C overnight, the plates were washed three times with PBST (PBS with 0.05% Tween 20 [VWR, 0777]). The plates were then blocked for an hour with 2% BSA (EMD Millipore, 126609) in PBST at room temperature with gentle rocking. Thereafter, the 2% BSA in PBST was discarded and Gal3-binding antibodies (reformatted hIgG4 [S228P]) were diluted in 2% BSA in PBST to concentrations of 5 µg/mL and added to the wells. The plates were incubated for an hour at room temperature with gentle rocking and then washed three times with PBST. Afterwards, Peroxidase AffiniPure F(ab')₂ Fragment Goat Anti-Human IgG (H+L) polyclonal antibody (Jackson ImmunoResearch, 109-036-003), was diluted in 2% BSA in PBST (1:4000) and added to the wells. The plates were incubated for 1 hour at room temperature with gentle rocking and then washed three times with PBST. TMB substrate (Thermo Scientific, 34029) was then added to each well. The reaction was stopped with 1M HCl (JT Baker, 5620-02) and read using a plate reader (Molecular Devices) at absorbance of 450 nm.

Peptide binding results are depicted in FIG. 40. Binding of Gal3-binding antibodies to the peptide array was observed at multiple locations, with the majority of binding observed in peptides 1-8 with some weak binding to peptide 17. 13 separate Gal3-binding antibodies (19B5.2E6, 20D11.2C6, 20H5.A3, 23H9.2E4, 2D10.2B2, F846C.1H5, F846TC.14A2, F846TC.7F10, F847C.10B9, F847C.26F5, F847C.4B10, F847C.12F12, 15FG7.2A7) all bound peptide 1 of Gal3, corresponding to amino acids ADNFSLHDALSGSGNPNPQG **(SEQ ID NO: 3)** of Gal3. Similarly, 15 separate Gal3-binding antibodies (IMT006 (TB006), 13A12.2E5, 14H10.2C9, 20D11.2C6, 20H5.A3, 23H9.2E4, 2D10.2B2, 3B11.2G2, F846C.1B2, F846C.1F5, F846C.1H12, F846C.2H3, F846TC.16B5, 15FG7.2A7, and IMT001 (TB001)) bound peptide 6 of Gal3, corresponding to amino acids GAYPGQAPPGAYPGAPGAYP **(SEQ ID NO: 8)** of Gal3. Further, 13 Gal3-binding antibodies (14H10.2C9, 19B5.2E6, 20D11.2C6, 20H5.A3, 23H9.2E4, 2D10.2B2, 3B11.2G2, F846C.1B2, F846TC.14A2, F847C.10B9, F847C.26F5, F847C.12F12, 15FG7.2A7) all bound peptide 7 of Gal3, corresponding to amino acids AYPGAPGAYPGAPAPGVYPG **(SEQ ID NO: 9)** of Gal3.

### Example 19: Anti-Gal3 antibodies for use in the treatment of diabetes

Patients present with diabetes mellitus type I or type II. One or more anti-Gal3 antibodies or binding fragments thereof are administered to the patients enterally, orally, intranasally, parenterally, intracranially, subcutaneously, intramuscularly, intradermally, or intravenously.

The anti-Gal3 antibodies or binding fragments thereof are administered as doses in at an amount of 1 ng (or in the alternative: 10, 100, 1000 ng, or 1, 10, *50,* 100, 200, 300, 400, 500, 600, 700, 800, 900, 1000 µg, or 1, 10, 100, 200, 300, 400, 500, 600, 700, 800, 900, 1000 mg, or any amount within a range defined by any two of the aforementioned amounts, or any other amount appropriate for optimal efficacy in humans). The doses are administered every 1 day (or in the alternative: every 2, 3, 4, 5, 6, 7, 8, 9, 10, 11, 12, 24, 36, or 48 days or any time within a range defined by any two of the aforementioned times).

A treatment of the diabetes or symptoms associated with diabetes is observed in the patients following administration of the anti-Gal3 antibodies or binding fragments thereof. Administration of the anti-Gal3 antibodies or binding fragments may be performed in conjunction with another therapy for diabetes, for example, insulin, an insulin derivative or mimetic thereof, insulin aspart, insulin glulisine, insulin lispro, insulin isophane, insulin degludec, insulin detemir, insulin zinc, insulin glargine, vanadium, biguanides, metformin, phenformin, buformin, thiazolidinediones, rosiglitazone, pioglitazone, troglitazone, tolimidone, sulfonylureas, tolbutamide, acetohexamide, tolazamide, chlorpropamide, glipizide, glibenclamide, glimepiride, gliclazide, glyclopyramide, gliquidone, meglitinides, repaglinide, nateglinide, alpha-glucosidase inhibitors, miglitol, acarbose, voglibose, incretins, glucagon-like peptide 1, glucagon-like peptide agonists, exenatide, liraglutide, taspoglutide, lixisenatide, semaglutide, dulaglutide, gastric inhibitory peptide, dipeptidyl peptidase-4 inhibitors, vildagliptin, sitagliptin, saxagliptin, linagliptin, alogliptin, septagliptin, teneligpliptin, gemigliptin, pramlintide, dapagliflozin, canagliflozin, empagliflozin, or remogliflozin.

### Example 20: Anti-Gal3 antibodies for use in the treatment of inflammatory bowel disease

Patients present with inflammatory bowel disease, such as but not limited to Crohn's disease or ulcerative colitis. One or more anti-Gal3 antibodies or binding fragments thereof are administered to the patients enterally, orally, intranasally, parenterally, intracranially, subcutaneously, intramuscularly, intradermally, or intravenously.

The anti-Gal3 antibodies or binding fragments thereof are administered as doses in at an amount of 1 ng (or in the alternative: 10, 100, 1000 ng, or 1, 10, *50,* 100, 200, 300, 400, 500, 600, 700, 800, 900, 1000 µg, or 1, 10, 100, 200, 300, 400, 500, 600, 700, 800, 900, 1000 mg, or any amount within a range defined by any two of the aforementioned amounts, or any other amount appropriate for optimal efficacy in humans). The doses are administered every 1 day (or in the alternative: every 2, 3, 4, 5, 6, 7, 8, 9, 10, 11, 12, 24, 36, or 48 days or any time within a range defined by any two of the aforementioned times).

A treatment of the inflammatory bowel disease or symptoms associated with inflammatory bowel disease is observed in the patients following administration of the anti-Gal3 antibodies or binding fragments thereof. Administration of the anti-Gal3 antibodies or binding fragments may be performed in conjunction with another therapy for inflammatory bowel disease, for example, surgery, mesalazine, immunosuppressants, prednisone, TNF inhibitors, azathioprine, methotrexate, 6-mercaptopurine, or rifaximin.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

All references cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification, the specification is intended to supersede and/or take precedence over any such contradictory material.

## Claims

1. An anti-Gal3 antibody for use in treating diabetes mellitus, inflammatory bowel syndrome, non-alcoholic fatty liver disease (NAFLD) or non-alcoholic steatohepatitis (NASH) in a subject in need thereof, wherein the anti-Gal3 antibody or binding fragment thereof selectively binds to Gal3 and disrupts an interaction between Gal3 and insulin receptor or an integrin, or both, thereby treating diabetes mellitus, inflammatory bowel syndrome, non-alcoholic fatty liver disease (NAFLD) or non-alcoholic steatohepatitis (NASH) in the subject, wherein the anti-Gal3 antibody or binding fragment thereof binds to an epitope present within a region of Gal3 defined by Peptide 6 (GAYPGQAPPGAYPGAPGAYP; **SEQ ID NO: 8).**

2. An anti-Gal3 antibody for its use according to claim 1, wherein
a) the diabetes mellitus is insulin-dependent diabetes mellitus; or
b) the diabetes mellitus is insulin-independent diabetes mellitus; or
c) the diabetes mellitus is Type I diabetes mellitus; or
d) the diabetes mellitus is Type II diabetes mellitus; or
e) the treating comprises decreasing glucose tolerance in the subject in need thereof; or
f) the treating comprises decreasing insulin sensitivity in the subject in need thereof; or
g) the treating comprises reducing weight gain in the subject in need thereof; or
h) the treating comprises reducing liver steatosis in the subject in need thereof; or
i) the use further comprises selecting the subject as having diabetes mellitus or at risk of contracting diabetes mellitus prior to administration; or
j) the use further comprises detecting an amelioration of symptoms associated with diabetes mellitus in the subject after administration, or
k) the insulin receptor comprises the sequence of **SEQ ID NO: 2;** or
1) the integrin is selected from ITGa1, ITGa2, ITGa3, ITGa4, ITGa5, ITGa6, ITGa7, ITGa8, ITGa9, ITGa10, ITGa11, ITGaD, ITGaE, ITGaL, ITGaM, ITGaV, ITGa2B, ITGa2X, ITGb1, ITGb2, ITGb3, ITGb4, ITGb5, ITGb6, ITGb7, ITGb8, or any combination thereof; or
m) the integrin comprises a sequence selected from **SEQ ID NO: 339-342;** or
n) the anti-Gal3 antibody or binding fragment thereof is administered with one or more additional therapeutic compositions; or
o) the anti-Gal3 antibody or binding fragment thereof is administered with one or more additional therapeutic compositions, wherein the one or more additional therapeutic compositions comprises insulin, an insulin derivative or mimetic thereof, insulin aspart, insulin glulisine, insulin lispro, insulin isophane, insulin degludec, insulin detemir, insulin zinc, insulin glargine, vanadium, biguanides, metformin, phenformin, buformin, thiazolidinediones, rosiglitazone, pioglitazone, troglitazone, tolimidone, sulfonylureas, tolbutamide, acetohexamide, tolazamide, chlorpropamide, glipizide, glibenclamide, glimepiride, gliclazide, glyclopyramide, gliquidone, meglitinides, repaglinide, nateglinide, alpha-glucosidase inhibitors, miglitol, acarbose, voglibose, incretins, glucagon-like peptide 1, glucagon-like peptide agonists, exenatide, liraglutide, taspoglutide, lixisenatide, semaglutide, dulaglutide, gastric inhibitory peptide, dipeptidyl peptidase-4 inhibitors, vildagliptin, sitagliptin, saxagliptin, linagliptin, alogliptin, septagliptin, teneligpliptin, gemigliptin, pramlintide, dapagliflozin, canagliflozin, empagliflozin, or remogliflozin, or any combination thereof; or
p) the inflammatory bowel syndrome is ulcerative colitis or Crohn's disease, or both; or
q) the use further comprises selecting the subject as having inflammatory bowel syndrome or at risk of contracting inflammatory bowel syndrome prior to administration; or
r) the use further comprises detecting an amelioration of symptoms associated with inflammatory bowel syndrome in the subject after administration; or
s) the disruption of the interaction between Gal3 and the integrin reduces lymphocyte adhesion in the subject by at least 50%, 55%, 60%, 65%, 70%, 75%, 80%, 85%, 90%, 95%, 96%, 97%, 98%, or 99%, or any percentage within a range defined by any two aforementioned percentages; or
t) the anti-Gal3 antibody or binding fragment thereof is administered with one or more additional therapeutic compositions, wherein the one or more additional therapeutic compositions comprises mesalazine, immunosuppressants, prednisone, TNF inhibitors, azathioprine, methotrexate, 6-mercaptopurine, or rifaximin, or any combination thereof; or
u) the interaction is reduced to less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1%, of an interaction in absence of the anti-Gal3 antibody or binding fragment thereof; or
v) the anti-Gal3 antibody or binding fragment thereof binds to Gal3 with a dissociation constant (KD) of less than 1 nM, less than 1.2 nM, less than 2 nM, less than 5 nM, less than 10 nM, less than 13.5 nM, less than 15 nM, less than 20 nM, less than 25 nM, or less than 30 nM; or
w) the anti-Gal3 antibody comprises a heavy chain variable region comprising an amino acid sequence having at least 75%, 80%, 85%, 90%, 95%, or 100% sequence identity to any sequence according to **SEQ ID NOs: 205, 169-204, or 206;** or
x) the anti-Gal3 antibody comprises a heavy chain variable region selected from the group consisting of **SEQ ID NOs: 205, 169-204, or 206;** or
y) the anti-Gal3 antibody comprises a light chain variable region comprises an amino acid sequence having at least 75%, 80%, 85%, 90%, 95%, or 100% sequence identity to any sequence according to **SEQ ID NOs: 244, 207-243, or 245;** or
z) the anti-Gal3 antibody comprises a light chain variable region selected from the group consisting of **SEQ ID NOs: 244, 207-243, or 245;** or
aa) the anti-Gal3 antibody or binding fragment thereof is administered enterally, orally, intranasally, parenterally, intracranially, subcutaneously, intramuscularly, intradermally, or intravenously, or any combination thereof; or
bb) the anti-Gal3 antibody or binding fragment thereof comprises a humanized antibody; or
cc) the anti-Gal3 antibody or binding fragment thereof comprises a full-length antibody or a binding fragment thereof; or
dd) the anti-Gal3 antibody or binding fragment thereof comprises a bispecific antibody or a binding fragment thereof; or
ee) the anti-Gal3 antibody or binding fragment thereof comprises a monovalent Fab', a divalent Fab2, a single-chain variable fragment (scFv), a diabody, a minibody, a nanobody, a singledomain antibody (sdAb), or a camelid antibody, or binding fragment thereof; or
ff) the anti-Gal3 antibody or binding fragment thereof comprises an IgG framework; or
mm) the anti-Gal3 antibody or binding fragment thereof comprises an IgG1, IgG2, or IgG4 framework; or
gg) the subject is diagnosed with Type I diabetes or Type II diabetes; or
hh) the anti-Gal3 antibody or binding fragment thereof is formulated for systemic administration; or
ii) the anti-Gal3 antibody or binding fragment thereof is formulated for parenteral administration; or
jj) more than one anti-Gal3 antibody or binding fragment is administered; or
kk) the subject is a mammal; or
11) the subject is a human.

3. An anti-Gal3 antibody for its use according to claim 1, wherein the anti-Gal3 antibody or binding fragment thereof comprises (1) a light chain variable region comprising a VL-CDR1, a VL-CDR2, and a VL-CDR3; and (2) a heavy chain variable region comprising a VH-CDR1, a VH-CDR2, and a VH-CDR3, wherein
the VL-CDR1 comprises an amino acid sequence having at least 60%, at least 70%, at least 80%, at least 90%, or 100% sequence identity to any amino acid sequence according to **SEQ ID NOs: 103, 100-102, or 104-128,**
the VL-CDR2 comprises an amino acid sequence having at least 60%, at least 70%, at least 80%, at least 90%, or 100% sequence identity to any amino acid sequence according to **SEQ ID NOs: 130, 129, or 131-144,**
the VL-CDR3 comprises an amino acid sequence having at least 60%, at least 70%, at least 80%, at least 90%, or 100% sequence identity to any amino acid sequence according to **SEQ ID NOs: 146, 145, or 147-168,**
the VH-CDR1 comprises an amino acid sequence having at least 60%, at least 70%, at least 80%, at least 90%, or 100% sequence identity to any amino acid sequence according to **SEQ ID NOs: 30, 27-29, or 31-47,**
the VH-CDR2 comprises an amino acid sequence having at least 60%, at least 70%, at least 80%, at least 90%, or 100% sequence identity to any amino acid sequence according to **SEQ ID NOs: 51, 48-50, or 52-69,** and
the VH-CDR3 comprises an amino acid sequence having at least 60%, at least 70%, at least 80%, at least 90%, or 100% sequence identity to any amino acid sequence according to **SEQ ID NOs: 75, 70-74, or 76-99.**

4. An anti-Gal3 antibody for its use according to claim 1, wherein the anti-Gal3 antibody or binding fragment thereof comprises:
a) the VH-CDR1, VH-CDR2, VH-CDR3 of the VH-CDR1, VH-CDR2, VH-CDR3 within **SEQ ID NO: 205** and the VL-CDR1, VL-CDR2, VL-CDR3 of the VL-CDR1, VL-CDR2, VL-CDR3 within **SEQ ID NO: 244;** or
b) the VH-CDR1, VH-CDR2, VH-CDR3 of the VH-CDR1, VH-CDR2, VH-CDR3 within **SEQ ID NO: 169** and the VL-CDR1, VL-CDR2, VL-CDR3 of the VL-CDR1, VL-CDR2, VLCDR3 within **SEQ ID NO: 207;** or
c) the VH-CDR1, VH-CDR2, VH-CDR3 of the VH-CDR1, VH-CDR2, VH-CDR3 within **SEQ ID NO: 170** and the VL-CDR1, VL-CDR2, VL-CDR3 of the VL-CDR1, VL-CDR2, VLCDR3 within **SEQ ID NO: 208;** or
d) the VH-CDR1, VH-CDR2, VH-CDR3 of the VH-CDR1, VH-CDR2, VH-CDR3 within **SEQ ID NO: 171** and the VL-CDR1, VL-CDR2, VL-CDR3 of the VL-CDR1, VL-CDR2, VLCDR3 within **SEQ ID NO: 209;** or
e) the V_{H}-CDR1, VH-CDR2, VH-CDR3 of the VH-CDR1, VH-CDR2, VH-CDR3 within **SEQ ID NO: 172** and the VL-CDR1, VL-CDR2, VL-CDR3 of the VL-CDR1, VL-CDR2, VLCDR3 within **SEQ ID NO: 210;** or
f) the VH-CDR1, VH-CDR2, VH-CDR3 of the VH-CDR1, VH-CDR2, VH-CDR3 within **SEQ ID NO: 173** and the VL-CDR1, VL-CDR2, VL-CDR3 of the VL-CDR1, VL-CDR2, VLCDR3 within **SEQ ID NO: 211;** or
g) the V_{H}-CDR1, VH-CDR2, VH-CDR3 of the VH-CDR1, VH-CDR2, VH-CDR3 within **SEQ ID NO: 174** and the VL-CDR1, VL-CDR2, VL-CDR3 of the VL-CDR1, VL-CDR2, VLCDR3 within **SEQ ID NO: 212;** or
h) the VH-CDR1, VH-CDR2, VH-CDR3 of the VH-CDR1, VH-CDR2, VH-CDR3 within **SEQ ID NO: 175** and the VL-CDR1, VL-CDR2, VL-CDR3 of the VL-CDR1, VL-CDR2, VLCDR3 within **SEQ ID NO: 213;** or
i) the VH-CDR1, VH-CDR2, VH-CDR3 of the VH-CDR1, VH-CDR2, VH-CDR3 within **SEQ ID NO: 176** and the VL-CDR1, VL-CDR2, VL-CDR3 of the VL-CDR1, VL-CDR2, VLCDR3 within **SEQ ID NO: 214;** or
j) the VH-CDRI, VH-CDR2, VH-CDR3 of the VH-CDR1, VH-CDR2, VH-CDR3 within **SEQ ID NO: 177** and the VL-CDR1, VL-CDR2, VL-CDR3 of the VL-CDR1, VL-CDR2, VLCDR3 within **SEQ ID NO: 215;** or
k) the VH-CDR1, VH-CDR2, VH-CDR3 of the VH-CDR1, VH-CDR2, VH-CDR3 within **SEQ ID NO: 178** and the VL-CDR1, VL-CDR2, VL-CDR3 of the VL-CDR1, VL-CDR2, VLCDR3 within **SEQ ID NO: 216;** or
1) the VH-CDR1, VH-CDR2, VH-CDR3 of the VH-CDR1, VH-CDR2, VH-CDR3 within **SEQ ID NO: 179** and the VL-CDR1, VL-CDR2, VL-CDR3 of the VL-CDR1, VL-CDR2, VLCDR3 within **SEQ ID NO: 217;** or
m) the VH-CDR1, VH-CDR2, VH-CDR3 of the VH-CDR1, VH-CDR2, VH-CDR3 within **SEQ ID NO: 180** and the VL-CDR1, VL-CDR2, VL-CDR3 of the VL-CDR1, VL-CDR2, VLCDR3 within **SEQ ID NO: 218;** or
n) the VH-CDR1, VH-CDR2, VH-CDR3 of the VH-CDR1, VH-CDR2, VH-CDR3 within **SEQ ID NO: 181** and the VL-CDR1, VL-CDR2, VL-CDR3 of the VL-CDR1, VL-CDR2, VLCDR3 within **SEQ ID NO: 219;** or
o) the VH-CDR1, VH-CDR2, VH-CDR3 of the VH-CDR1, VH-CDR2, VH-CDR3 within **SEQ ID NO: 181** and the VL-CDR1, VL-CDR2, VL-CDR3 of the VL-CDR1, VL-CDR2, VLCDR3 within **SEQ ID NO: 220;** or
p) the VH-CDR1, VH-CDR2, VH-CDR3 of the VH-CDR1, VH-CDR2, VH-CDR3 within **SEQ ID NO: 182** and the VL-CDR1, VL-CDR2, VL-CDR3 of the VL-CDR1, VL-CDR2, VLCDR3 within **SEQ ID NO: 221;** or
q) the VH-CDR1, VH-CDR2, VH-CDR3 of the VH-CDR1, VH-CDR2, VH-CDR3 within **SEQ ID NO: 183** and the VL-CDR1, VL-CDR2, VL-CDR3 of the VL-CDR1, VL-CDR2, VLCDR3 within **SEQ ID NO: 222;** or
r) the VH-CDR1, VH-CDR2, VH-CDR3 of the VH-CDR1, VH-CDR2, VH-CDR3 within **SEQ ID NO: 184** and the VL-CDR1, VL-CDR2, VL-CDR3 of the VL-CDR1, VL-CDR2, VLCDR3 within **SEQ ID NO: 223;** or
s) the VH-CDR1, VH-CDR2, VH-CDR3 of the VH-CDR1, VH-CDR2, VH-CDR3 within **SEQ ID NO: 185** and the VL-CDR1, VL-CDR2, VL-CDR3 of the VL-CDR1, VL-CDR2, VLCDR3 within **SEQ ID NO: 224;** or
t) the VH-CDR1, VH-CDR2, VH-CDR3 of the VH-CDR1, VH-CDR2, VH-CDR3 within **SEQ ID NO: 186** and the VL-CDR1, VL-CDR2, VL-CDR3 of the VL-CDR1, VL-CDR2, VLCDR3 within **SEQ ID NO: 225;** or
u) the VH-CDR1, VH-CDR2, VH-CDR3 of the VH-CDR1, VH-CDR2, VH-CDR3 within **SEQ ID NO: 187** and the VL-CDR1, VL-CDR2, VL-CDR3 of the VL-CDR1, VL-CDR2, VLCDR3 within **SEQ ID NO: 226;** or
v) the VH-CDR1, VH-CDR2, VH-CDR3 of the VH-CDR1, VH-CDR2, VH-CDR3 within **SEQ ID NO: 188** and the VL-CDR1, VL-CDR2, VL-CDR3 of the VL-CDR1, VL-CDR2, VLCDR3 within **SEQ ID NO: 227;** or
w) the VH-CDR1, VH-CDR2, VH-CDR3 of the VH-CDR1, VH-CDR2, VH-CDR3 within **SEQ ID NO: 189** and the VL-CDR1, VL-CDR2, VL-CDR3 of the VL-CDR1, VL-CDR2, VLCDR3 within **SEQ ID NO: 228;** or
x) the VH-CDR1, VH-CDR2, VH-CDR3 of the VH-CDR1, VH-CDR2, VH-CDR3 within **SEQ ID NO: 190** and the VL-CDR1, VL-CDR2, VL-CDR3 of the VL-CDR1, VL-CDR2, VLCDR3 within **SEQ ID NO: 229;** or
y) the VH-CDR1, VH-CDR2, VH-CDR3 of the VH-CDR1, VH-CDR2, VH-CDR3 within **SEQ ID NO: 191** and the VL-CDR1, VL-CDR2, VL-CDR3 of the VL-CDR1, VL-CDR2, VLCDR3 within **SEQ ID NO: 230;** or
z) the VH-CDR1, VH-CDR2, VH-CDR3 of the VH-CDR1, VH-CDR2, VH-CDR3 within **SEQ ID NO: 192** and the VL-CDR1, VL-CDR2, VL-CDR3 of the VL-CDR1, VL-CDR2, VLCDR3 within **SEQ ID NO: 231;** or
aa) the VH-CDR1, VH-CDR2, VH-CDR3 of the VH-CDR1, VH-CDR2, VH-CDR3 within **SEQ ID NO: 193** and the VL-CDR1, VL-CDR2, VL-CDR3 of the VL-CDR1, VL-CDR2, VLCDR3 within **SEQ ID NO: 232;** or
ab) the VH-CDR1, VH-CDR2, VH-CDR3 of the VH-CDR1, VH-CDR2, VH-CDR3 within **SEQ ID NO: 194** and the VL-CDR1, VL-CDR2, VL-CDR3 of the VL-CDR1, VL-CDR2, VLCDR3 within **SEQ ID NO: 233;** or
ac) the VH-CDR1, VH-CDR2, VH-CDR3 of the VH-CDR1, VH-CDR2, VH-CDR3 within **SEQ ID NO: 195** and the VL-CDR1, VL-CDR2, VL-CDR3 of the VL-CDR1, VL-CDR2, VLCDR3 within **SEQ ID NO: 234;** or
ad) the VH-CDR1, VH-CDR2, VH-CDR3 of the VH-CDR1, VH-CDR2, VH-CDR3 within **SEQ ID NO: 196** and the VL-CDR1, VL-CDR2, VL-CDR3 of the VL-CDR1, VL-CDR2, VLCDR3 within **SEQ ID NO: 235;** or
ae) the VH-CDR1, VH-CDR2, VH-CDR3 of the VH-CDR1, VH-CDR2, VH-CDR3 within **SEQ ID NO: 197** and the VL-CDR1, VL-CDR2, VL-CDR3 of the VL-CDR1, VL-CDR2, VLCDR3 within **SEQ ID NO: 236;** or
af) the VH-CDR1, VH-CDR2, VH-CDR3 of the VH-CDR1, VH-CDR2, VH-CDR3 within **SEQ ID NO: 198** and the VL-CDR1, VL-CDR2, VL-CDR3 of the VL-CDR1, VL-CDR2, VLCDR3 within **SEQ ID NO: 237;** or
ag) the VH-CDR1, VH-CDR2, VH-CDR3 of the VH-CDR1, VH-CDR2, VH-CDR3 within **SEQ ID NO: 199** and the VL-CDR1, VL-CDR2, VL-CDR3 of the VL-CDR1, VL-CDR2, VLCDR3 within **SEQ ID NO: 238;** or
ah) the VH-CDR1, VH-CDR2, VH-CDR3 of the VH-CDR1, VH-CDR2, VH-CDR3 within **SEQ ID NO: 197** and the VL-CDR1, VL-CDR2, VL-CDR3 of the VL-CDR1, VL-CDR2, VLCDR3 within **SEQ ID NO: 239;** or
ai) the VH-CDR1, VH-CDR2, VH-CDR3 of the VH-CDR1, VH-CDR2, VH-CDR3 within **SEQ ID NO: 200** and the VL-CDR1, VL-CDR2, VL-CDR3 of the VL-CDR1, VL-CDR2, VLCDR3 within **SEQ ID NO: 240;** or
aj) the VH-CDR1, VH-CDR2, VH-CDR3 of the VH-CDR1, VH-CDR2, VH-CDR3 within
**SEQ ID NO: 201** and the VL-CDR1, VL-CDR2, VL-CDR3 of the VL-CDR1, VL-CDR2, VLCDR3 within **SEQ ID NO: 241;** or
ak) the VH-CDR1, VH-CDR2, VH-CDR3 of the VH-CDR1, VH-CDR2, VH-CDR3 within
**SEQ ID NO: 202** and the VL-CDR1, VL-CDR2, VL-CDR3 of the VL-CDR1, VL-CDR2, VLCDR3 within **SEQ ID NO: 242;** or
al) the VH-CDR1, VH-CDR2, VH-CDR3 of the VH-CDR1, VH-CDR2, VH-CDR3 within **SEQ ID NO: 203** and the VL-CDR1, VL-CDR2, VL-CDR3 of the VL-CDR1, VL-CDR2, VLCDR3 within **SEQ ID NO: 240;** or
am) the VH-CDR1, VH-CDR2, VH-CDR3 of the VH-CDR1, VH-CDR2, VH-CDR3 within **SEQ ID NO: 204** and the VL-CDR1, VL-CDR2, VL-CDR3 of the VL-CDR1, VL-CDR2, VLCDR3 within **SEQ ID NO: 243;** or
an) the VH-CDR1, VH-CDR2, VH-CDR3 of the VH-CDR1, VH-CDR2, VH-CDR3 within **SEQ ID NO: 206** and the VL-CDR1, VL-CDR2, VL-CDR3 of the VL-CDR1, VL-CDR2, VLCDR3 within **SEQ ID NO: 245.**

5. An anti-Gal3 antibody for its use according to claim 1, wherein the anti-Gal3 antibody or binding fragment thereof comprises:
a) the heavy chain variable region of **SEQ ID NO: 205** and the light chain variable region of **SEQ ID NO: 244;** or
b) the heavy chain variable region of **SEQ ID NO: 169** and the light chain variable region of **SEQ ID NO: 207;** or
c) the heavy chain variable region of **SEQ ID NO: 170** and the light chain variable region of **SEQ ID NO: 208;** or
d) the heavy chain variable region of **SEQ ID NO: 171** and the light chain variable region of **SEQ ID NO: 209;** or
e) the heavy chain variable region of **SEQ ID NO: 172** and the light chain variable region of **SEQ ID NO: 210;** or
f) the heavy chain variable region of **SEQ ID NO: 173** and the light chain variable region of **SEQ ID NO: 211;** or
g) the heavy chain variable region of **SEQ ID NO: 174** and the light chain variable region of **SEQ ID NO: 212;** or
h) the heavy chain variable region of **SEQ ID NO: 175** and the light chain variable region of **SEQ ID NO: 213;** or
i) the heavy chain variable region of **SEQ ID NO: 176** and the light chain variable region of **SEQ ID NO: 214;** or
j) the heavy chain variable region of **SEQ ID NO: 177** and the light chain variable region of **SEQ ID NO: 215;** or
k) the heavy chain variable region of **SEQ ID NO: 178** and the light chain variable region of **SEQ ID NO: 216;** or
1) the heavy chain variable region of **SEQ ID NO: 179** and the light chain variable region of **SEQ ID NO: 217;** or
m) the heavy chain variable region of **SEQ ID NO: 180** and the light chain variable region of **SEQ ID NO: 218;** or
n) the heavy chain variable region of **SEQ ID NO: 181** and the light chain variable region of **SEQ ID NO: 219;** or
o) the heavy chain variable region of **SEQ ID NO: 181** and the light chain variable region of **SEQ ID NO: 220;** or
p) the heavy chain variable region of **SEQ ID NO: 182** and the light chain variable region of **SEQ ID NO: 221;** or
q) the heavy chain variable region of **SEQ ID NO: 183** and the light chain variable region of **SEQ ID NO: 222;** or
r) the heavy chain variable region of **SEQ ID NO: 184** and the light chain variable region of **SEQ ID NO: 223;** or
s) the heavy chain variable region of **SEQ ID NO: 185** and the light chain variable region of **SEQ ID NO: 224;** or
t) the heavy chain variable region of **SEQ ID NO: 186** and the light chain variable region of **SEQ ID NO: 225;** or
u) the heavy chain variable region of **SEQ ID NO: 187** and the light chain variable region of **SEQ ID NO: 226;** or
v) the heavy chain variable region of **SEQ ID NO: 188** and the light chain variable region of **SEQ ID NO: 227;** or
w) the heavy chain variable region of **SEQ ID NO: 189** and the light chain variable region of **SEQ ID NO: 228;** or
x) the heavy chain variable region of **SEQ ID NO: 190** and the light chain variable region of **SEQ ID NO: 229;** or
y) the heavy chain variable region of **SEQ ID NO: 191** and the light chain variable region of **SEQ ID NO: 230;** or
z) the heavy chain variable region of **SEQ ID NO: 192** and the light chain variable region of **SEQ ID NO: 231;** or
aa) the heavy chain variable region of **SEQ ID NO: 193** and the light chain variable region of **SEQ ID NO: 232;** or
ab) the heavy chain variable region of **SEQ ID NO: 194** and the light chain variable region of **SEQ ID NO: 233;** or
ac) the heavy chain variable region of **SEQ ID NO: 195** and the light chain variable region of **SEQ ID NO: 234;** or
ad) the heavy chain variable region of **SEQ ID NO: 196** and the light chain variable region of **SEQ ID NO: 235;** or
ae) the heavy chain variable region of **SEQ ID NO: 197** and the light chain variable region of **SEQ ID NO: 236;** or
af) the heavy chain variable region of **SEQ ID NO: 198** and the light chain variable region of **SEQ ID NO: 237;** or
ag) the heavy chain variable region of **SEQ ID NO: 199** and the light chain variable region of **SEQ ID NO: 238;** or
ah) the heavy chain variable region of **SEQ ID NO: 197** and the light chain variable region of **SEQ ID NO: 239;** or
ai) the heavy chain variable region of **SEQ ID NO: 200** and the light chain variable region of **SEQ ID NO: 240;** or
aj) the heavy chain variable region of **SEQ ID NO: 201** and the light chain variable region of **SEQ ID NO: 241;** or
ak) the heavy chain variable region of **SEQ ID NO: 202** and the light chain variable region of **SEQ ID NO: 242;** or
al) the heavy chain variable region of **SEQ ID NO: 203** and the light chain variable region of **SEQ ID NO: 240;** or
am) the heavy chain variable region of **SEQ ID NO: 204** and the light chain variable region of **SEQ ID NO: 243;** or
an) the heavy chain variable region of **SEQ ID NO: 206** and the light chain variable region of **SEQ ID NO: 245.**

6. An anti-Gal3 antibody comprising (1) a light chain variable region comprising a VL-CDR1, a VL-CDR2, and a VL-CDR3; and (2) a heavy chain variable region comprising a VH-CDR1, a VH-CDR2, and a VH-CDR3, wherein
the VL-CDR1 comprises an amino acid sequence having at least 60%, at least 70%, at least 80%, at least 90%, or 100% sequence identity to any amino acid sequence according to **SEQ ID NOs: 103, 100-102, or 104-128,**
the VL-CDR2 comprises an amino acid sequence having at least 60%, at least 70%, at least 80%, at least 90%, or 100% sequence identity to any amino acid sequence according to **SEQ ID NOs: 130, 129, or 131-144,**
the VL-CDR3 comprises an amino acid sequence having at least 60%, at least 70%, at least 80%, at least 90%, or 100% sequence identity to any amino acid sequence according to **SEQ ID NOs: 146, 145, or 147-168,**
the VH-CDR1 comprises an amino acid sequence having at least 60%, at least 70%, at least 80%, at least 90%, or 100% sequence identity to any amino acid sequence according to **SEQ ID NOs: 30, 27-29, or 31-47,**
the VH-CDR2 comprises an amino acid sequence having at least 60%, at least 70%, at least 80%, at least 90%, or 100% sequence identity to any amino acid sequence according to **SEQ ID NOs: 51, 48-50, or 52-69,** and
the VH-CDR3 comprises an amino acid sequence having at least 60%, at least 70%, at least 80%, at least 90%, or 100% sequence identity to any amino acid sequence according to **SEQ ID NOs: 75, 70-74, or 76-99.**

7. A pharmaceutical formulation comprising the anti-Gal3 antibody of claim 6.

8. The pharmaceutical formulation of claim 7 for use in treating diabetes mellitus; inflammatory bowel disease; NAFLD and/or NASH.

9. A method for identifying an antibody capable of disrupting an interaction between Gal3 and insulin receptor or an integrin, or both, the method comprising:
(a) contacting Gal3 protein with an antibody or binding fragment thereof that selectively binds to Gal3, thereby forming a Gal3-antibody complex;
(b) contacting the Gal3-antibody complex with the insulin receptor or the integrin, or both;
(c) removing unbound insulin receptor or integrin, or both; and
(d) detecting the insulin receptor or integrin, or both, bound to the Gal3-antibody complex; wherein the antibody or binding fragment thereof is capable of disrupting an interaction of Gal3 and insulin receptor or the integrin, or both, when the insulin receptor or the integrin, or both, is not detected in (d).
